(19) Europäisches Patentamt
European Patent Office
Office européen des brevets

(11) Veröffentlichungsnummer: **0 587 134 A2**

(12) **EUROPÄISCHE PATENTANMELDUNG**

(21) Anmeldenummer: **93114401.8**

(22) Anmeldetag: **08.09.93**

(51) Int. Cl.5: **C07D 233/70**, C07D 233/72, C07D 249/12, C07D 401/10, C07D 453/02, A61K 31/415, A61K 31/445

(30) Priorität: **11.09.92 DE 4230470**
**26.01.93 DE 4302052**
**22.03.93 DE 4309213**

(43) Veröffentlichungstag der Anmeldung:
**16.03.94 Patentblatt 94/11**

(84) Benannte Vertragsstaaten:
**AT BE CH DE DK ES FR GB GR IE IT LI LU NL PT SE**

(71) Anmelder: **Dr. Karl Thomae GmbH**

**D-88397 Biberach(DE)**

(72) Erfinder: **Himmelsbach, Frank, Dr. Dipl.-Chem.**
**Ahornweg 16**
**D-88441 Mittelbiberach(DE)**
Erfinder: **Austel, Volkhard, Dr. Dipl.-Chem.**
**Kapellenweg 7**
**D-88400 Biberach(DE)**
Erfinder: **Linz, Günter, Dr. Dipl.-Chem.**
**Erlenweg 8**
**D-88441 Mittelbiberach(DE)**
Erfinder: **Pieper, Helmut, Dr. Dipl.-Chem.**
**Kapellenweg 5**
**D-88400 Biberach(DE)**
Erfinder: **Guth, Brian, Dr.Dr. Dipl.-Chem.**
**Am Schlegelberg 24**
**D-88447 Warthausen(DE)**
Erfinder: **Müller, Thomas, Dr. Dipl.-Chem.**
**Alter Postplatz 17**
**D-88400 Biberach(DE)**
Erfinder: **Weisenberger, Johannes, Dr. Dipl.-Chem.**
**Haydnweg 5**
**D-88400 Biberach(DE)**

(54) **Cyclische Harnstoffderivate, diese Verbindungen enthaltende Arzneimittel und Verfahren zu ihrer Herstellung.**

(57) Die Erfindung betrifft cyclische Harnstoffderivate der allgemeinen Formel

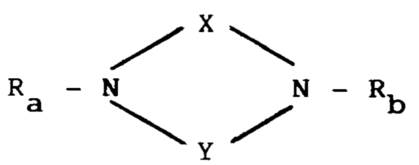

,(I)

in der
$R_a$, $R_b$, X und Y wie im Anspruch 1 definiert sind, deren Tautomere, deren Stereoisomere und deren Salze, insbesondere deren physiologisch verträgliche Salze mit anorganischen oder organischen Säuren oder Basen, welche wertvolle pharmakologische Eigenschaften aufweisen, vorzugsweise aggregationshemmende Wirkungen, die Verbindungen enthaltende Arzneimittel und Verfahren zu ihrer Herstellung.

EP 0 587 134 A2

Die Erfindung betrifft cyclische Harnstoffderivate der allgemeinen Formel

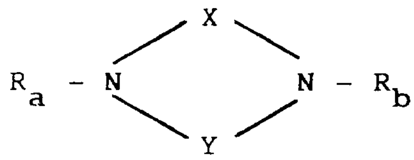

,(I)

deren Tautomere, deren Stereoisomere und deren Salze, insbesondere deren physiologisch verträgliche Salze mit anorganischen oder organischen Säuren oder Basen, welche u. a. wertvolle pharmakologische Eigenschaften aufweisen, vorzugsweise aggregationshemmende Wirkungen,
mit Ausnahme von
1-(4-Amidino-phenyl)-3[4-(2-carboxy-ethyl)-cyclohexyl]-imidazolidin-2-on,
1-(4-Amidino-phenyl)-3-[4-(2-methoxycarbonyl-ethyl)-cyclohexyl]-imidazolidin-2-on,
1-(4-Cyano-phenyl)-3-[4-(2-methoxycarbonyl-ethyl)-cyclohexyl]-imidazolidin-2-on,
1-(4-Amidino-phenyl)-3-[4-[2-(5-tetrazolyl)-ethyl]-phenyl]-imidazolidin-2-on,
1-(4-Aminomethyl-cyclohexyl)-3-[4-(2-carboxy-ethyl)-phenyl]-imidazolidin-2-on,
1-(4-Aminomethyl-cyclohexyl)-3-[4-(2-methoxycarbonyl-ethyl)-phenyl]-imidazolidin-2-on,
4-(4'-Amidino-4-biphenylyl)-1-(2-carboxy-ethyl)-3-phenyl-3H-imidazol-2-on,
4-(4'-Amidino-4-biphenylyl)-1-(2-methoxycarbonyl-ethyl)-3-phenyl-3H-imidazol-2-on,
4-(4'-Cyano-4-biphenylyl)-1-(2-methoxycarbonyl-ethyl)-3-phenyl-3H-imidazol-2-on,
4-[4'-Amidino-4-biphenylyl)-1-(2-carboxy-ethyl)-3-phenyl-imidazolidin-2-on,
4-(4'-Amidino-4-biphenylyl)-1-(2-methoxycarbonyl-ethyl)-3-phenyl-imidazolidin-2-on,
1-(4'-Amidino-4-biphenylyl)-3-(2-ethoxycarbonyl-ethyl)-3H-imidazol-2-on,
1-(4'-Cyano-4-biphenylyl)-3-(2-ethoxycarbonyl-ethyl)-3H-imidazol-2-on,
1-(4'-Amidino-4-biphenylyl)-3-(2-ethoxycarbonyl-ethyl)-imidazolidin-2,4-dion,
1-(4'-Cyano-4-biphenylyl)-3-(2-ethoxycarbonyl-ethyl)-imidazolidin-2,4-dion,
1-(4'-Amidino-4-biphenylyl)-3-(2-ethoxycarbonyl-ethyl)-3,4,5,6-tetrahydro-1H-pyrimidin-2-on,
1-(4'-Cyano-4-biphenylyl)-3-(2-ethoxycarbonyl-ethyl)-3,4,5,6-tetrahydro-1H-pyrimidin-2-on,
2-(4-Amidino-phenyl)-4-[4-(2-isopropyloxycarbonyl-ethyl)-phenyl]-5-methyl-4H-1,2,4-triazol-3-on,
4-[4-(2-Isopropyloxycarbonyl-ethyl)-phenyl]-2-(4-methoxycarbonylamidino-phenyl)-5-methyl-4H-1,2,4-triazol-3-on,
1-(4-Amidino-phenyl)-3-[4-(2-ethoxycarbonyl-ethyl)-phenyl]-3H-imidazol-2-on,
1-(4-Ethoxycarbonylamidino-phenyl)-3-[4-(2-ethoxycarbonyl-ethyl)-phenyl]-3H-imidazol-2-on,
1-(4-Ethoxycarbonylamidino-phenyl)-3-[4-(2-ethoxycarbonyl-ethyl)-phenyl]-imidazolidin-2-on,
1-(4-Methoxycarbonylamidino-phenyl)-3-[4-(2-methoxycarbonyl-ethyl)-cyclohexyl]-imidazolidin-2-on,
4-[4-(2-Isobutyloxycarbonyl-ethyl)-phenyl]-2-(4-methoxycarbonylamidino-phenyl)-5-methyl-4H-1,2,4-triazol-3-on,
2-(4-Amidino-phenyl)-4-[4-(2-isobutyloxycarbonyl-ethyl)-phenyl]-5-methyl-4H-1,2,4-triazol-3-on,
1-(1-Amino-5-indanyl)-3-[4-(2-carboxy-ethyl)-phenyl]-imidazolidin-2-on,
1-(1-Amino-5-indanyl)-3-[4-(2-methoxycarbonyl-ethyl)-phenyl]-imidazolidin-2-on,
2-(4-Amidino-phenyl)-5-ethyl-4-[4-(2-isopropyloxycarbonyl-ethyl)-phenyl]-4H-1,2,4-triazol-3-on,
1-(4-Amidino-phenyl)-3-[4-(2-isopropyloxycarbonyl-ethyl)-phenyl]-4-methyl-3H-imidazol-2-on,
1-(4-Amidino-phenyl)-3-[4-(2-isopropyloxycarbonyl-ethyl)-phenyl]-3H-imidazol-2-on,
1-(4-Amidino-phenyl)-3-[4-(2-isopropyloxycarbonyl-ethyl)-phenyl]-imidazolidin-2,4-dion,
2-(4-Amidino-phenyl)-4-[4-(2-carboxy-ethyl)-phenyl]-5-trifluormethyl-4H-1,2,4-triazol-3-on und
3-(4-Cyano-phenyl)-1-(4-ethoxycarbonyl-butyl)-3H-imidazo-[4,5-b]pyridin-2-on,
diese Verbindungen enthaltende Arzneimittel, deren Verwendung und Verfahren zu ihrer Herstellung.

In der obigen allgemeinen Formel I bedeutet mit den Maßgaben, daß

(i) X eine am Stickstoffatom durch eine Cyanogruppe substituierte Carbiminogruppe, eine 1-Nitro-ethen-2,2-diyl- oder 1,1-Dicyano-ethen-2,2-diyl-Gruppe,

(ii) Y eine gegebenenfalls durch $R_c$ oder $R_d$ oder $R_c$ und $R_d$ substituierte 1,2-Cycloalkylengruppe mit 5 bis 7 Kohlenstoffatomen,

(iii) A eine Aminoalkylgruppe mit 1 bis 5 Kohlenstoffatomen, eine Amino-, Amidino- oder Guanidinogruppe, wobei in jeder der vorstehend erwähnten Gruppen jeweils an einem der Stickstoffatome ein Wasserstoffatom durch eine $R_1$-CO-O-($R_2CR_3$)-O-CO-Gruppe ersetzt ist,

(iv) B eine gegebenenfalls durch eine oder zwei Alkylgruppen substituierte Cycloalkylengruppe mit 3 bis 7 Kohlenstoffatomen,
eine gegebenenfalls durch einen Rest $R_e$ substituierte Cycloalkylengruppe mit 4 oder 5 Kohlenstoffatomen, welche zusätzlich durch 1 bis 4 Alkylgruppen substituiert sein kann und in der im Cycloalkylenteil jeweils eine >CH-Einheit durch ein Stickstoffatom ersetzt ist,
eine gegebenenfalls durch einen Rest $R_e$ substituierte Cycloalkylengruppe mit 6 bis 8 Kohlenstoffatomen, welche zusätzlich durch 1 bis 4 Alkylgruppen substituiert sein kann und in der im Cycloalkylenteil jeweils eine >CH-Einheit durch ein Stickstoffatom ersetzt ist und zusätzlich eine weitere in 4-Stellung befindliche >CH-Einheit durch ein Stickstoffatom ersetzt sein kann, wobei außerdem in den vorstehend erwähnten 6- bis 8-gliedrigen Ringen jeweils eine oder zwei zu einem Stickstoffatom benachbarte Methylengruppen jeweils durch eine Carbonylgruppe ersetzt sein können,
eine gegebenenfalls durch eine oder zwei Alkylgruppen substituierte Cycloalkenylengruppe mit 5 bis 7 Kohlenstoffatomen, welche zusätzlich durch eine oder zwei Methylgruppen substituiert sein kann und in der im Cycloalkenylenteil jeweils eine >CH-Einheit durch ein Stickstoffatom ersetzt ist,
eine Cycloalkylengruppe mit 5 bis 7 Kohlenstoffatomen, in der eine >CH-Einheit durch ein Stickstoffatom, welches mit dem Rest A verknüpft ist, ersetzt ist und zusätzlich zwei Wasserstoffatome am Kohlenstoffgerüst durch eine geradkettige Alkylenbrücke ersetzt sind, wobei diese Brücke 2 bis 6 Kohlenstoffatome enthält, wenn die zwei Wasserstoffatome sich am selben Kohlenstoffatom befinden, oder 1 bis 5 Kohlenstoffatome enthält, wenn sie zwei Wasserstoffatome an benachbarten Kohlenstoffatomen ersetzt, oder 1 bis 4 Kohlenstoffatome enthält, wenn sie zwei Wasserstoffatome an Kohlenstoffatomen ersetzt, die durch ein weiteres Atom getrennt sind, oder 1 bis 3 Kohlenstoffatome enthält, wenn sie zwei Wasserstoffatome an Kohlenstoffatomen ersetzt, die durch zwei Atome getrennt sind, wobei die vorstehenden Gruppen jeweils durch eine oder zwei Alkylgruppen substituiert sein können,
eine über den Rest W mit dem Rest C verknüpfte Alkylengruppe mit 1 bis 6 Kohlenstoffatomen, oder
B zusammen mit A eine gegebenenfalls durch 1 oder 2 Alkylgruppen substituierte Pyridylgruppe oder eine Piperidinylgruppe, in der das Wasserstoffatom in 1-Stellung zusammen mit einem Wasserstoffatom in 2-Stellung durch eine geradkettige Alkylengruppe mit 3 oder 4 Kohlenstoffatomen ersetzt ist, oder eine Piperidinylgruppe, in der das Wasserstoffatom in 1-Stellung zusammen mit einem Wasserstoffatom in 3-Stellung durch eine geradkettige Alkylengruppe mit 2 bis 4 Kohlenstoffatomen ersetzt ist, oder eine Piperidinylgruppe, in der das Wasserstoffatom in 1-Stellung zusammen mit einem Wasserstoffatom in 4-Stellung durch eine geradkettige Alkylengruppe mit 1 bis 3 Kohlenstoffatomen ersetzt ist, wobei die an die 4-Stellung gebundene Methylengruppe einer Ethylenkette durch eine Hydroxymethylen- oder Carbonylgruppe ersetzt sein kann und zusätzlich das Stickstoffatom dieser vorstehend erwähnten bicyclischen Gruppen, welche im Kohlenstoffgerüst durch eine oder zwei Alkylgruppen substituiert sein können, durch Boran komplexiert oder durch eine im Phenylkern gegebenenfalls durch 1 bis 2 Methoxygruppen substituierte Benzylgruppe quarternisiert sein kann,
(v) C eine Alkylengruppe mit 1 bis 6 Kohlenstoffatomen, in der eine Methylengruppe durch ein Sauerstoff- oder Schwefelatom oder durch eine Sulfinyl-, Sulfonyl- oder $-NR_4$-Gruppe ersetzt ist oder in der eine Ethylengruppe durch eine $-CONR_5$- oder $-NR_5CO$-Gruppe ersetzt ist,
eine gegebenenfalls durch eine oder zwei Alkylgruppen substituierte 3- bis 7-gliedrige Cycloalkylengruppe,
eine Cyclohexylengruppe, in der zwei durch drei Bindungen getrennte Kohlenstoffatome zusätzlich durch eine geradkettige Alkylengruppe mit 1 bis 3 Kohlenstoffatomen verknüpft sind, wobei diese bicyclischen Gruppen jeweils außerdem durch ein bis zwei Alkylgruppen substituiert sein können,
eine Indanylen-, Naphthylen-, 1,2,3,4-Tetrahydronaphthylen- oder Benzosuberanylengruppe, wobei die gesättigten Ringe jeweils durch 1 oder 2 Alkylgruppen substituiert sein können und die aromatischen Ringe jeweils durch ein Fluor-, Chlor-, Brom- oder Jodatom, durch eine Alkyl-, Trifluormethyl-, Hydroxy-, Alkoxy-, Alkylsulfenyl-, Alkylsulfinyl-, Alkylsulfonyl- oder Cyanogruppe substituiert sein können und in denen zusätzlich eine oder zwei Methingruppen jeweils durch ein Stickstoffatom ersetzt sein können,
eine gegebenenfalls durch den Rest $R_e$ substituierte Cycloalkylengruppe mit 4 oder 5 Kohlenstoffatomen, welche zusätzlich durch 1 bis 4 Alkylgruppen substituiert sein kann und in denen im Cycloalkylenteil jeweils eine >CH-Einheit durch ein Stickstoffatom ersetzt ist,
eine gegebenenfalls durch den Rest $R_e$ substituierte Cycloalkylengruppe mit 6 bis 8 Kohlenstoffatomen, welche zusätzlich durch 1 bis 4 Alkylgruppen substituiert sein kann und in denen im Cycloalkylenteil jeweils eine >CH-Einheit durch ein Stickstoffatom ersetzt ist und zusätzlich eine weitere in 4-Stellung befindliche >CH-Einheit durch ein Stickstoffatom ersetzt sein kann, wobei außerdem in den vorstehend erwähnten 6- bis 8-gliedrigen Ringen jeweils eine oder zwei zu einem Stickstoffatom benachbarte Methylengruppen jeweils durch eine Carbonylgruppe ersetzt sein können,

eine gegebenenfalls durch eine oder zwei Alkylgruppen substituierte Cycloalkenylengruppe mit 5 bis 7 Kohlenstoffatomen, welche zusätzlich durch eine oder zwei Methylgruppen substituiert sein kann und in denen im Cycloalkenylenteil jeweils eine >CH-Einheit durch ein Stickstoffatom ersetzt ist,
eine Cycloalkylengruppe mit 5 bis 7 Kohlenstoffatomen, in der eine >CH-Einheit durch ein Stickstoffatom, welches mit dem Rest A verknüpft ist, ersetzt ist und zusätzlich zwei Wasserstoffatome am Kohlenstoffgerüst durch eine geradkettige Alkylenbrücke ersetzt sind, wobei diese Brücke 2 bis 6 Kohlenstoffatome enthält, wenn die zwei Wasserstoffatome sich am selben Kohlenstoffatom befinden, oder 1 bis 5 Kohlenstoffatome enthält, wenn sie zwei Wasserstoffatome an benachbarten Kohlenstoffatomen ersetzt, oder 1 bis 4 Kohlenstoffatome enthält, wenn sie zwei Wasserstoffatome an Kohlenstoffatomen ersetzt, die durch ein weiteres Atom getrennt sind, oder 1 bis 3 Kohlenstoffatome enthält, wenn sie zwei Wasserstoffatome an Kohlenstoffatomen ersetzt, die durch zwei Atome getrennt sind, wobei die vorstehenden Gruppen jeweils durch eine oder zwei Alkylgruppen substituiert sein können, oder
C zusammen mit A und B eine gegebenenfalls jeweils durch eine oder zwei Alkylgruppen substituierte Pyridyl- oder 1-(4-Pyridyl)-piperidinylgruppe oder eine Piperidinylgruppe, in der das Wasserstoffatom in 1-Stellung zusammen mit einem Wasserstoffatom in 2-Stellung durch eine geradkettige Alkylengruppe mit 3 oder 4 Kohlenstoffatomen ersetzt ist, oder eine Piperidinylgruppe, in der das Wasserstoffatom in 1-Stellung zusammen mit einem Wasserstoffatom in 3-Stellung durch eine geradkettige Alkylengruppe mit 2 bis 4 Kohlenstoffatomen ersetzt ist, oder eine Piperidinylgruppe, in der das Wasserstoffatom in 1-Stellung zusammen mit einem Wasserstoffatom in 4-Stellung durch eine geradkettige Alkylengruppe mit 1 bis 3 Kohlenstoffatomen ersetzt ist, wobei die an die 4-Stellung gebundene Methylengruppe einer Ethylenkette durch eine Hydroxymethylen- oder Carbonylgruppe ersetzt sein kann und zusätzlich das Stickstoffatom dieser vorstehend erwähnten bicyclischen Gruppen, welche im Kohlenstoffgerüst durch eine oder zwei Alkylgruppen substituiert sein können, durch Boran komplexiert oder durch eine im Phenylkern gegebenenfalls durch 1 bis 2 Methoxygruppen substituierte Benzylgruppe quartärnisiert sein kann, oder
C eine Alkylengruppe mit 1 bis 6 Kohlenstoffatomen und B eine Bindung,
(vi) D eine 1,3-Arylengruppe,
eine Indanylen-, Naphthylen-, 1,2,3,4-Tetrahydronaphthylen- oder Benzosuberanylengruppe, wobei die gesättigten Ringe jeweils durch 1 oder 2 Alkylgruppen substituiert sein können und die aromatischen Ringe jeweils durch ein Fluor-, Chlor-, Brom- oder Jodatom, durch eine Alkyl-, Trifluormethyl-, Hydroxy-, Alkoxy-, Alkylsulfenyl-, Alkylsulfinyl-, Alkylsulfonyl- oder Cyanogruppe substituiert sein können und in denen zusätzlich eine oder zwei Methingruppen jeweils durch ein Stickstoffatom ersetzt sein können,
eine gegebenenfalls durch eine oder zwei Alkylgruppen substituierte Cycloalkylengruppe mit 3 bis 7 Kohlenstoffatomen oder
eine Cyclohexylengruppe, in der zwei durch drei Bindungen getrennte Kohlenstoffatome zusätzlich durch eine geradkettige Alkylengruppe mit 1 bis 3 Kohlenstoffatomen verknüpft sind, wobei diese bicyclischen Gruppen jeweils außerdem durch ein bis zwei Alkylgruppen substituiert sein können,
(vii) E eine geradkettige Alkylengruppe mit 1 bis 6 Kohlenstoffatomen oder eine geradkettige Alkenylengruppe mit 2 bis 6 Kohlenstoffatomen, die jeweils durch eine Alkylgruppe mit 1 bis 8 Kohlenstoffatomen, durch eine Aryl- oder Heteroarylgruppe, durch eine $HNR_6$- oder N-Phenylalkyl-$NR_6$-Gruppe oder durch eine N-Alkyl-$NR_6$-Gruppe mit 1 bis 8 Kohlenstoffatomen im Alkylteil substituiert ist,
eine gegebenenfalls durch eine oder zwei Alkylgruppen substituierte Cycloalkylengruppe mit 5 bis 7 Kohlenstoffatomen, in der eine >CH-Einheit durch ein Stickstoffatom ersetzt ist,
eine durch eine Hydroxy-, Amino-, Aryl- oder Heteroarylgruppe, durch eine Alkoxy- oder Alkylaminogruppe mit jeweils 1 bis 8 Kohlenstoffatomen, durch eine Dialkylaminogruppe mit insgesamt 2 bis 10 Kohlenstoffatomen, durch eine $HNR_6$- oder N-Phenylalkyl-$NR_6$-Gruppe oder durch eine N-Alkyl-$NR_6$-Gruppe mit 1 bis 8 Kohlenstoffatomen im Alkylteil substituierte Cycloalkylengruppe mit 4 bis 7 Kohlenstoffatomen im Cycloalkylenteil, oder
eine über den Rest W mit dem Rest D verknüpfte Alkylengruppe, wobei die Alkylengruppe zusätzlich durch eine oder zwei Alkylgruppen mit jeweils 1 bis 8 Kohlenstoffatomen, durch eine Hydroxy-, Amino-, Aryl- oder Heteroarylgruppe, durch eine Alkoxy- oder Alkylaminogruppe mit jeweils 1 bis 8 Kohlenstoffatomen, durch eine Dialkylaminogruppe mit insgesamt 2 bis 10 Kohlenstoffatomen, durch eine $HNR_6$- oder N-Phenylalkyl-$NR_6$-Gruppe oder durch eine N-Alkyl-$NR_6$-Gruppe mit 1 bis 8 Kohlenstoffatomen im Alkylteil substituiert ist,
(viii) F eine Alkoxycarbonylgruppe mit insgesamt 3 bis 9 Kohlenstoffatomen, eine $R_7O$-CO-, Phosphono-, O-Alkyl-phosphono-, O,O'-Dialkyl-phosphono-, Tetrazol-5-yl- oder $R_8CO$-O-$CHR_9$-O-CO-Gruppe und
(ix) der dritte der Reste $R_a$ bis $R_d$ eine Perfluoralkyl- oder Arylgruppe

darstellt, wobei mindestens eine der obigen Bedingungen (i) bis (ix) erfüllt sein müssen,

X eine gegebenenfalls am Stickstoffatom durch eine Alkyl- oder Cyanogruppe substituierte Carbiminogruppe, eine Carbonyl-, Thiocarbonyl-, Sulfonyl-, 1-Nitro-ethen-2,2-diyl- oder 1,1-Dicyano-ethen-2,2-diyl-Gruppe,
Y eine gegebenenfalls durch $R_c$ oder $R_d$ oder $R_c$ und $R_d$ substituierte geradkettige Alkylengruppe mit 2 bis 4 Kohlenstoffatomen, die zusätzlich durch eine oder zwei Alkylgruppen substituiert sein kann, und in der zusätzlich eine oder zwei Methylengruppen jeweils durch eine Carbonylgruppe ersetzt sein können,
eine gegebenenfalls durch $R_c$ oder $R_d$ oder $R_c$ und $R_d$ substituierte geradkettige Alkenylengruppe mit 2 bis 4 Kohlenstoffatomen, in der zusätzlich eine gegebenenfalls vorhandene Methylengruppe durch eine Carbonylgruppe ersetzt sein kann,
eine gegebenenfalls durch $R_c$ oder $R_d$ oder $R_c$ und $R_d$ substituierte 1,2-Cycloalkylengruppe mit 5 bis 7 Kohlenstoffatomen,
eine gegebenenfalls durch $R_c$ oder $R_d$ oder $R_c$ und $R_d$ substituierte 1,2-Cycloalkenylengruppe mit 5 bis 7 Kohlenstoffatomen,
eine 1,2-Arylengruppe,
eine 1,2-Phenylengruppe, in der eine oder zwei Methingruppen jeweils durch ein Stickstoffatom ersetzt sind oder in der eine oder zwei -CH = CH-Gruppen jeweils durch eine -CO-NH-Gruppe ersetzt sind oder in der eine Methingruppe durch ein Stickstoffatom und eine -CH = CH-Gruppe durch eine -CO-NH-Gruppe ersetzt ist, wobei die vorstehend erwähnten heterocyclischen Gruppen zusätzlich durch eine oder zwei Alkylgruppen substituiert sein können, oder
eine gegebenenfalls durch $R_c$ oder $R_d$ substituierte -CO-NH-, -NH-CO-, -CH = N- oder -N = CH-Gruppe,
der erste der Reste $R_a$ bis $R_d$ eine Gruppe der Formel

A - B - C -,

in der
A eine Aminoalkylgruppe mit 1 bis 5 Kohlenstoffatomen, eine Amino-, Amidino- oder Guanidinogruppe, wobei in jeder der vorstehend erwähnten Gruppen jeweils an einem der Stickstoffatome ein oder zwei Wasserstoffatome jeweils durch eine Alkylgruppe oder ein Wasserstoffatom durch eine Alkoxycarbonylgruppe mit insgesamt 2 bis 5 Kohlenstoffatomen, durch eine Benzyloxycarbonylgruppe oder durch eine $R_1$-CO-O-($R_2CR_3$)-O-CO-Gruppe ersetzt sein kann, wobei
$R_1$ eine Alkylgruppe mit 1 bis 8 Kohlenstoffatomen, eine Cycloalkylgruppe mit 5 bis 7 Kohlenstoffatomen, eine Phenyl- oder Phenylalkylgruppe,
$R_2$ ein Wasserstoffatom, eine Alkylgruppe, eine Cycloalkylgruppe mit 5 bis 7 Kohlenstoffatomen oder eine Phenylgruppe und
$R_3$ ein Wasserstoffatom oder eine Alkylgruppe darstellen,
eine Cyano- oder Cyanoalkylgruppe oder
auch, falls A an ein Stickstoffatom der Reste B oder C gebunden ist, das nicht Teil einer Lactamgruppe ist, ein Wasserstoffatom, eine Alkylgruppe, eine Benzylgruppe, in der der Phenylteil durch 1 bis 2 Methoxygruppen substituiert sein kann, eine Formyl-, Acetyl- oder Trifluoracetylgruppe, eine Alkoxycarbonylgruppe mit insgesamt 2 bis 5 Kohlenstoffatomen, eine Benzyloxycarbonylgruppe oder eine $R_1$-CO-O-($R_2CR_3$)-O-CO-Gruppe, wobei $R_1$ bis $R_3$ wie vorstehend erwähnt definiert sind,
B eine Bindung,
eine Alkylengruppe mit 1 bis 6 Kohlenstoffatomen,
eine Arylengruppe,
eine Pyridinylen-, Pyrimidinylen-, Pyrazinylen- oder Pyridazinylengruppe, in denen eine oder zwei -CH = N-Gruppen jeweils durch eine -CO-NH-Gruppe ersetzt sein können und eines der Stickstoffatome statt an ein Wasserstoffatom auch an den Rest C gebunden sein kann, sofern dieser nicht mit einem Heteroatom oder einer Carbonylgruppe an den Rest B anschließt, wobei die vorstehend erwähnten heterocyclischen Gruppen zusätzlich durch eine oder zwei Alkylgruppen substituiert sein können,
eine gegebenenfalls durch eine oder zwei Alkylgruppen substituierte Cycloalkylengruppe mit 3 bis 7 Kohlenstoffatomen,
eine gegebenenfalls durch einen Rest $R_e$ substituierte Cycloalkylengruppe mit 4 oder 5 Kohlenstoffatomen, welche zusätzlich durch 1 bis 4 Alkylgruppen substituiert sein kann und in der im Cycloalkylenteil jeweils eine $>$CH-Einheit durch ein Stickstoffatom, welches mit dem Rest A verknüpft ist, ersetzt ist, wobei
$R_e$ mit der Maßgabe, daß ein Heteroatom des Restes $R_e$ durch mindestens zwei Kohlenstoffatome von dem Ringstickstoffatom der cyclischen Iminogruppe getrennt ist, eine Alkyl-, Hydroxy-, Alkoxy-, Phenylalkoxy-, Alkylsulfenyl-, Alkylsulfinyl-, Alkylsulfonyl-, Cyano-, Carboxy-, Alkoxycarbonyl-, Phenylalkoxycarbonyl-, Alkylcarbonyl-, Aminocarbonyl-, Alkylaminocarbonyl-, Dialkylaminocarbonyl-, Amino-, Alkylamino-, Dialkylamino- oder Phenylgruppe darstellt,

eine gegebenenfalls durch einen Rest $R_e$ substituierte Cycloalkylengruppe mit 6 bis 8 Kohlenstoffatomen, welche zusätzlich durch 1 bis 4 Alkylgruppen substituiert sein kann und in denen im Cycloalkylenteil jeweils eine >CH-Einheit durch ein Stickstoffatom, welches mit dem Rest A verknüpft ist, ersetzt ist und zusätzlich eine weitere in 4-Stellung befindliche >CH-Einheit durch ein Stickstoffatom ersetzt sein kann, wobei außerdem in den vorstehend erwähnten 6- bis 8-gliedrigen Ringen jeweils eine oder zwei zu einem Stickstoffatom benachbarte Methylengruppen jeweils durch eine Carbonylgruppe ersetzt sein können, wobei $R_e$ wie vorstehend erwähnt definiert ist,

eine gegebenenfalls durch eine oder zwei Alkylgruppen substituierte Cycloalkenylengruppe mit 5 bis 7 Kohlenstoffatomen, welche zusätzlich durch eine oder zwei Methylgruppen substituiert sein kann und in denen im Cycloalkenylenteil jeweils eine >CH-Einheit durch ein Stickstoffatom, welches mit dem Rest A verknüpft ist, ersetzt ist, wobei das Stickstoffatom durch mindestens eine gegebenenfalls mono- oder disubstituierte Methylengruppe von der Doppelbindung getrennt ist,

eine Cycloalkylengruppe mit 5 bis 7 Kohlenstoffatomen, in der eine >CH-Einheit durch ein Stickstoffatom, welches mit dem Rest A verknüpft ist, ersetzt ist und zusätzlich zwei Wasserstoffatome am Kohlenstoffgerüst durch eine geradkettige Alkylenbrücke ersetzt sind, wobei diese Brücke 2 bis 6 Kohlenstoffatome enthält, wenn die zwei Wasserstoffatome sich am selben Kohlenstoffatom befinden, oder 1 bis 5 Kohlenstoffatome enthält, wenn sie zwei Wasserstoffatome an benachbarten Kohlenstoffatomen ersetzt, oder 1 bis 4 Kohlenstoffatome enthält, wenn sie zwei Wasserstoffatome an Kohlenstoffatomen ersetzt, die durch ein weiteres Atom getrennt sind, oder 1 bis 3 Kohlenstoffatome enthält, wenn sie zwei Wasserstoffatome an Kohlenstoffatomen ersetzt, die durch zwei Atome getrennt sind, wobei die vorstehenden Gruppen jeweils durch eine oder zwei Alkylgruppen substituiert sein können, oder

eine über den Rest W mit dem Rest C verknüpfte Alkylengruppe mit 1 bis 6 Kohlenstoffatomen, wobei W ein Sauerstoff- oder Schwefelatom, eine Sulfinyl-, Sulfonyl-, $-NR_4-$, $-NR_5CO-$ oder $-CONR_5-$Gruppe darstellt, wobei

$R_4$ ein Wasserstoffatom, eine Alkyl-, Alkylcarbonyl-, Alkylsulfonyl-, Arylcarbonyl-, Heteroarylcarbonyl-, Arylsulfonyl- oder Heteroarylsulfonylgruppe und

$R_5$ ein Wasserstoffatom oder eine Alkylgruppe darstellen,

oder B zusammen mit A eine gegebenenfalls durch 1 oder 2 Alkylgruppen substituierte Pyridylgruppe oder eine Piperidinylgruppe, in der das Wasserstoffatom in 1-Stellung zusammen mit einem Wasserstoffatom in 2-Stellung durch eine geradkettige Alkylengruppe mit 3 oder 4 Kohlenstoffatomen ersetzt ist, oder eine Piperidinylgruppe, in der das Wasserstoffatom in 1-Stellung zusammen mit einem Wasserstoffatom in 3-Stellung durch eine geradkettige Alkylengruppe mit 2 bis 4 Kohlenstoffatomen ersetzt ist, oder eine Piperidinylgruppe, in der das Wasserstoffatom in 1-Stellung zusammen mit einem Wasserstoffatom in 4-Stellung durch eine geradkettige Alkylengruppe mit 1 bis 3 Kohlenstoffatomen ersetzt ist, wobei die an die 4-Stellung gebundene Methylengruppe einer Ethylenkette durch eine Hydroxymethylen- oder Carbonylgruppe ersetzt sein kann und zusätzlich das Stickstoffatom dieser vorstehend erwähnten bicyclischen Gruppen, welche im Kohlenstoffgerüst durch eine oder zwei Alkylgruppen substituiert sein können, durch Boran komplexiert oder durch eine im Phenylkern gegebenenfalls durch 1 bis 2 Methoxygruppen substituierte Benzylgruppe quarternisiert sein kann, und

C eine Alkylengruppe mit 1 bis 6 Kohlenstoffatomen, in der eine Methylengruppe durch ein Sauerstoff- oder Schwefelatom oder durch eine Sulfinyl-, Sulfonyl- oder $-NR_4$-Gruppe ersetzt sein kann, oder in der eine Ethylengruppe durch eine $-CONR_5-$ oder $-NR_5CO$-Gruppe ersetzt sein kann, wobei $R_4$ und $R_5$ wie vorstehend erwähnt definiert sind,

eine Alkenylengruppe mit 2 bis 6 Kohlenstoffatomen,

eine über die Carbonylgruppe mit dem Rest B verbundene Alkylencarbonylgruppe mit insgesamt 2 bis 7 Kohlenstoffatomen,

eine Arylengruppe,

eine Pyridinylen-, Pyrimidinylen-, Pyrazinylen- oder Pyridazinylengruppe, in denen eine oder zwei -CH=N-Gruppen jeweils durch eine -CO-NH-Gruppe ersetzt sein können und eines der Stickstoffatome statt an ein Wasserstoffatom auch an den Rest B gebunden sein kann, sofern dieser nicht eine Bindung bedeutet oder nicht mit einem Heteroatom oder einer Carbonylgruppe an den Rest C anschließt, wobei die vorstehend erwähnten heterocyclischen Gruppen zusätzlich durch eine oder zwei Alkylgruppen substituiert sein können,

eine gegebenenfalls durch eine oder zwei Alkylgruppen substituierte 3- bis 7-gliedrige Cycloalkylengruppe oder

eine Cyclohexylengruppe, in der zwei durch drei Bindungen getrennte Kohlenstoffatome zusätzlich durch eine geradkettige Alkylengruppe mit 1 bis 3 Kohlenstoffatomen verknüpft sind, wobei diese bicyclischen Gruppen jeweils außerdem durch ein bis zwei Alkylgruppen substituiert sein können, oder

C zusammen mit A und B eine gegebenenfalls jeweils durch eine oder zwei Alkylgruppen substituierte Pyridyl- oder 1-(4-Pyridyl)-piperidinylgruppe oder eine Piperidinylgruppe, in der das Wasserstoffatom in 1-Stellung zusammen mit einem Wasserstoffatom in 2-Stellung durch eine geradkettige Alkylengruppe mit 3 oder 4 Kohlenstoffatomen ersetzt ist, oder eine Piperidinylgruppe, in der das Wasserstoffatom in 1-Stellung zusammen mit einem Wasserstoffatom in 3-Stellung durch eine geradkettige Alkylengruppe mit 2 bis 4 Kohlenstoffatomen ersetzt ist, oder eine Piperidinylgruppe, in der das Wasserstoffatom in 1-Stellung zusammen mit einem Wasserstoffatom in 4-Stellung durch eine geradkettige Alkylengruppe mit 1 bis 3 Kohlenstoffatomen ersetzt ist, wobei die an die 4-Stellung gebundene Methylengruppe einer Ethylenkette durch eine Hydroxymethylen- oder Carbonylgruppe ersetzt sein kann und zusätzlich das Stickstoffatom dieser vorstehend erwähnten bicyclischen Gruppen, welche im Kohlenstoffgerüst durch eine oder zwei Alkylgruppen substituiert sein können, durch Boran komplexiert oder durch eine im Phenylkern gegebenenfalls durch 1 bis 2 Methoxygruppen substituierte Benzylgruppe quarternisiert sein kann, oder

C, wenn B eine Bindung darstellt, auch

(a) eine Indanylen-, Naphthylen-, 1,2,3,4-Tetrahydronaphthylen- oder Benzosuberanylengruppe, in denen jeweils einer der Ringe an den Rest A und der andere der Ringe an den cyclischen Rest der allgemeinen Formel I gebunden ist, wobei die gesättigten Ringe jeweils durch 1 oder 2 Alkylgruppen substituiert sein können und die aromatischen Ringe jeweils durch ein Fluor-, Chlor-, Brom- oder Jodatom, durch eine Alkyl-, Trifluormethyl-, Hydroxy-, Alkoxy-, Alkylsulfenyl-, Alkylsulfinyl-, Alkylsulfonyl- oder Cyanogruppe substituiert sein können und in denen zusätzlich eine oder zwei Methingruppen jeweils durch ein Stickstoffatom ersetzt sein können,

(b) eine gegebenenfalls durch den Rest $R_e$ substituierte Cycloalkylengruppe mit 4 oder 5 Kohlenstoffatomen, welche zusätzlich durch 1 bis 4 Alkylgruppen substituiert sein kann und in denen im Cycloalkylenteil jeweils eine $>$CH-Einheit durch ein Stickstoffatom, welches mit dem Rest A verknüpft ist, ersetzt ist, wobei $R_e$ wie vorstehend erwähnt definiert ist,

(c) eine gegebenenfalls durch den Rest $R_e$ substituierte Cycloalkylengruppe mit 6 bis 8 Kohlenstoffatomen, welche zusätzlich durch 1 bis 4 Alkylgruppen substituiert sein kann und in denen im Cycloalkylenteil jeweils eine $>$CH-Einheit durch ein Stickstoffatom, welches mit dem Rest A verknüpft ist, ersetzt ist und zusätzlich eine weitere in 4-Stellung befindliche $>$CH-Einheit durch ein Stickstoffatom ersetzt sein kann, wobei außerdem in den vorstehend erwähnten 6- bis 8-gliedrigen Ringen jeweils eine oder zwei zu einem Stickstoffatom benachbarte Methylengruppen jeweils durch eine Carbonylgruppe ersetzt sein können, wobei $R_e$ wie vorstehend erwähnt definiert ist,

(d) eine gegebenenfalls durch eine oder zwei Alkylgruppen substituierte Cycloalkenylengruppe mit 5 bis 7 Kohlenstoffatomen, welche zusätzlich durch eine oder zwei Methylgruppen substituiert sein kann und in denen im Cycloalkenylenteil jeweils eine $>$CH-Einheit durch ein Stickstoffatom, welches mit dem Rest A verknüpft ist, ersetzt ist, wobei das Stickstoffatom durch mindestens eine gegebenenfalls mono- oder disubstituierte Methylengruppe von der Doppelbindung getrennt ist, oder

e) eine Cycloalkylengruppe mit 5 bis 7 Kohlenstoffatomen, in der eine $>$CH-Einheit durch ein Stickstoffatom, welches mit dem Rest A verknüpft ist, ersetzt ist und zusätzlich zwei Wasserstoffatome am Kohlenstoffgerüst durch eine geradkettige Alkylenbrücke ersetzt sind, wobei diese Brücke 2 bis 6 Kohlenstoffatome enthält, wenn die zwei Wasserstoffatome sich am selben Kohlenstoffatom befinden, oder 1 bis 5 Kohlenstoffatome enthält, wenn sie zwei Wasserstoffatome an benachbarten Kohlenstoffatomen ersetzt, oder 1 bis 4 Kohlenstoffatome enthält, wenn sie zwei Wasserstoffatome an Kohlenstoffatomen ersetzt, die durch ein weiteres Atom getrennt sind, oder 1 bis 3 Kohlenstoffatome enthält, wenn sie zwei Wasserstoffatome an Kohlenstoffatomen ersetzt, die durch zwei Atome getrennt sind, wobei die vorstehenden Gruppen jeweils durch eine oder zwei Alkylgruppen substituiert sein können, darstellen,

der zweite der Reste $R_a$ bis $R_d$ eine Gruppe der Formel

F - E - D -,

in der

D eine Alkylengruppe mit 1 bis 6 Kohlenstoffatomen, in der eine Methylengruppe durch ein Sauerstoff- oder Schwefelatom, durch eine Sulfinyl-, Sulfonyl- oder $-NR_4$-Gruppe ersetzt sein kann, oder in der eine Ethylengruppe durch eine $-CO-NR_5-$ oder $-NR_5-CO$-Gruppe ersetzt sein kann, wobei $R_4$ und $R_5$ wie vorstehend erwähnt definiert sind,

eine Alkenylengruppe mit 2 bis 6 Kohlenstoffatomen,

eine Arylengruppe,

eine Pyridinylen-, Pyrimidinylen-, Pyrazinylen- oder Pyridazinylengruppe, in denen eine oder zwei -CH=N-Gruppen jeweils durch eine -CO-NH-Gruppe ersetzt sein können und eines der Stickstoffatome statt an ein

Wasserstoffatom auch an den Rest E gebunden sein kann, sofern dieser nicht eine Bindung bedeutet oder nicht mit einem Heteroatom oder einer Carbonylgruppe an den Rest D anschließt, wobei die vorstehend erwähnten heterocyclischen Gruppen zusätzlich durch eine oder zwei Alkylgruppen substituiert sein können,
eine Indanylen-, Naphthylen-, 1,2,3,4-Tetrahydronaphthylen- oder Benzosuberanylengruppe, in denen jeweils einer der Ringe an den Rest E und der andere der Ringe an den cyclischen Rest der allgemeinen Formel I gebunden ist, wobei die gesättigten Ringe jeweils durch 1 oder 2 Alkylgruppen substituiert sein können und die aromatischen Ringe jeweils durch ein Fluor-, Chlor-, Brom- oder Jodatom, durch eine Alkyl-, Trifluormethyl-, Hydroxy-, Alkoxy-, Alkylsulfenyl-, Alkylsulfinyl-, Alkylsulfonyl- oder Cyanogruppe substituiert sein können und in denen zusätzlich eine oder zwei Methingruppen jeweils durch ein Stickstoffatom ersetzt sein können,
eine gegebenenfalls durch eine oder zwei Alkylgruppen substituierte Cycloalkylengruppe mit 3 bis 7 Kohlenstoffatomen,
eine gegebenenfalls durch eine oder zwei Alkylgruppen substituierte Cycloalkylengruppe mit 4 bis 7 Kohlenstoffatomen, in der eine $>$CH-Einheit durch ein Stickstoffatom ersetzt ist, wobei außerdem in den vorstehend erwähnten 4- bis 7-gliedrigen Ringen eine und, ab einer Ringgröße von 5, jeweils eine oder zwei zu einem Stickstoffatom benachbarte Methylengruppen jeweils durch eine Carbonylgruppe ersetzt sein können,
eine gegebenenfalls durch eine oder zwei Alkylgruppen substituierte Cycloalkylengruppe mit 6 oder 7 Kohlenstoffatomen, in der zwei zueinander in 1,4-Stellung befindliche $>$CH-Einheiten jeweils durch ein Stickstoffatom ersetzt sind, wobei außerdem in den vorstehend erwähnten 6- oder 7-gliedrigen Ringen jeweils eine oder zwei zu einem Stickstoffatom benachbarte Methylengruppen jeweils durch eine Carbonylgruppe ersetzt sein können,
eine Cyclohexylengruppe, in der zwei durch drei Bindungen getrennte Kohlenstoffatome zusätzlich durch eine geradkettige Alkylengruppe mit 1 bis 3 Kohlenstoffatomen verknüpft sind, wobei diese bicyclischen Gruppen jeweils außerdem durch ein bis zwei Alkylgruppen substituiert sein können,
oder, wenn E eine cyclische Iminogruppe darstellt, auch eine Alkylencarbonylgruppe mit insgesamt 2 bis 6 Kohlenstoffatomen, wobei die Carbonylgruppe jeweils an das Stickstoffatom der cyclischen Iminogruppe der Gruppe E gebunden ist,
oder auch, falls E keine Bindung darstellt, eine Bindung,
E eine Bindung,
eine Alkylengruppe mit 1 bis 6 Kohlenstoffatomen oder eine Alkenylengruppe mit 2 bis 6 Kohlenstoffatomen, die jeweils durch eine oder zwei Alkylgruppen mit jeweils 1 bis 8 Kohlenstoffatomen, durch eine Hydroxy-, Amino-, Aryl- oder Heteroarylgruppe, durch eine Alkoxy- oder Alkylaminogruppe mit jeweils 1 bis 8 Kohlenstoffatomen, durch eine Dialkylaminogruppe mit insgesamt 2 bis 10 Kohlenstoffatomen, durch eine $HNR_6$- oder N-Phenylalkyl-$NR_6$-Gruppe oder durch eine N-Alkyl-$NR_6$-Gruppe mit 1 bis 8 Kohlenstoffatomen im Alkylteil substituiert sein können, wobei

$R_6$ eine Alkylcarbonyl- oder Alkylsulfonylgruppe mit jeweils 1 bis 8 Kohlenstoffatomen im Alkylteil, eine Alkyloxycarbonylgruppe mit insgesamt 2 bis 5 Kohlenstoffatomen, eine Cycloalkylcarbonyl- oder Cycloalkylsulfonylgruppe mit jeweils 5 bis 7 Kohlenstoffatomen im Cycloalkylteil, eine Arylalkylcarbonyl-, Arylalkylsulfonyl-, Arylalkoxycarbonyl-, Arylcarbonyl-, Arylsulfonyl-, Heteroarylalkylcarbonyl-, Heteroarylalkylsulfonyl-, Heteroarylalkoxycarbonyl-, Heteroarylcarbonyl- oder Heteroarylsulfonylgruppe darstellt,

eine Arylengruppe,
eine Pyridinylen-, Pyrimidinylen-, Pyrazinylen- oder Pyridazinylengruppe, in denen eine oder zwei -CH=N-Gruppen jeweils durch eine -CO-NH-Gruppe ersetzt sein können und eines der Stickstoffatome statt an ein Wasserstoffatom auch an den Rest D gebunden sein kann, sofern dieser nicht eine Bindung bedeutet oder nicht mit einem Heteroatom oder einer Carbonylgruppe an den Rest E anschließt, wobei die vorstehend erwähnten heterocyclischen Gruppen zusätzlich durch eine oder zwei Alkylgruppen substituiert sein können,
eine gegebenenfalls durch eine oder zwei Alkylgruppen substituierte Cycloalkylengruppe mit 5 bis 7 Kohlenstoffatomen, in der eine $>$CH-Einheit durch ein Stickstoffatom, welches mit einem Kohlenstoffatom des Restes D verknüpft ist, ersetzt ist,
eine gegebenenfalls durch eine oder zwei Alkylgruppen mit jeweils 1 bis 8 Kohlenstoffatomen, durch eine Hydroxy-, Amino-, Aryl- oder Heteroarylgruppe, durch eine Alkoxy- oder Alkylaminogruppe mit jeweils 1 bis 8 Kohlenstoffatomen, durch eine Dialkylaminogruppe mit insgesamt 2 bis 10 Kohlenstoffatomen, durch eine $HNR_6$- oder N-Phenylalkyl-$NR_6$-Gruppe oder durch eine N-Alkyl-$NR_6$-Gruppe mit 1 bis 8 Kohlenstoffatomen im Alkylteil substituierte Cycloalkylengruppe mit 4 bis 7 Kohlenstoffatomen im Cycloalkylenteil, wobei $R_6$ wie vorstehend definiert ist,

oder auch, falls D keine Bindung darstellt, eine über den Rest W mit dem Rest D verknüpfte Alkylengruppe, in der W wie eingangs erwähnt definiert ist und die Alkylengruppe zusätzlich durch eine oder zwei Alkylgruppen mit jeweils 1 bis 8 Kohlenstoffatomen, durch eine Hydroxy-, Amino-, Aryl- oder Heteroarylgruppe, durch eine Alkoxy- oder Alkylaminogruppe mit jeweils 1 bis 8 Kohlenstoffatomen, durch eine Dialkylaminogruppe mit insgesamt 2 bis 10 Kohlenstoffatomen, durch eine $HNR_6$- oder N-phenylalkyl-$NR_6$-Gruppe oder durch eine N-Alkyl-$NR_6$-Gruppe mit 1 bis 8 Kohlenstoffatomen im Alkylteil substituiert sein kann, wobei das Heteroatom des zusätzlichen Substituenten durch mindestens 2 Kohlenstoffatome von einem Heteroatom des Restes W getrennt ist und $R_6$ wie vorstehend definiert ist, sowie mit der Maßgabe, daß D zusammen mit E keine $-(CH_2)_n-CONH-CH_2CH_2-$Gruppe darstellt, wobei

n die Zahl 1,2,3 oder 4 bedeutet und der mit dem Stickstoffatom verbundene Ethylenteil gegebenenfalls wie vorstehend erwähnt substituiert sein kann, und

F eine Carbonylgruppe, die durch eine Hydroxygruppe, durch eine Alkoxygruppe mit 1 bis 8 Kohlenstoffatomen, durch eine Arylalkoxygruppe oder durch eine $R_7O$-Gruppe substituiert ist, wobei

$R_7$ eine Cycloalkylgruppe mit 4 bis 8 Kohlenstoffatomen oder eine Cycloalkylalkylgruppe mit 3 bis 8 Kohlenstoffatomen im Cycloalkylteil, in denen jeweils die Cycloalkylgruppe durch eine Alkylgruppe, durch eine Alkylgruppe und durch 1 bis 3 Methylgruppen, durch eine Alkoxy- oder Dialkylaminogruppe substituiert und zusätzlich eine Methylengruppe in einem 4 bis 8 gliedrigen Cycloalkylteil durch ein Sauerstoff- oder Schwefelatom, durch eine Sulfinyl-, Sulfonyl- oder Alkyliminogruppe ersetzt sein kann, eine Bicycloalkyl- oder Bicycloalkylalkylgruppe, in denen jeweils der Bicycloalkylteil jeweils 6 bis 10 Kohlenstoffatome enthält und zusätzlich durch 1 bis 3 Methylgruppen substituiert sein kann, eine Benzocycloalkylgruppe mit 9 bis 12 Kohlenstoffatomen oder eine Arylgruppe darstellt,

eine Phosphono-, O-Alkyl-phosphono-, O,O'-Dialkyl-phosphono-, Tetrazol-5-yl- oder $R_8CO-O-CHR_9-O-CO$-Gruppe darstellen, wobei

$R_8$ eine Alkyl- oder Alkoxygruppe mit jeweils 1 bis 8 Kohlenstoffatomen, eine Cycloalkyl- oder Cycloalkoxygruppe mit jeweils 5 bis 7 Kohlenstoffatomen im Cycloalkylteil, eine Aryl-, Aryloxy-, Arylalkyl- oder Arylalkoxygruppe und

$R_9$ ein Wasserstoffatom, eine Cycloalkylgruppe mit 5 bis 7 Kohlenstoffatomen, eine Alkyl- oder Arylgruppe darstellen,

und der kürzeste Abstand zwischen dem Rest F und dem von dem Rest F am weitesten entfernten Stickstoffatom der Gruppe A-B-C- mindestens 11 Bindungen beträgt,

der dritte der Reste $R_a$ bis $R_d$ ein Wasserstoffatom, eine Alkyl-, Perfluoralkyl-, Alkoxy-, Alkylsulfenyl-, Alkylsulfinyl-, Alkylsulfonyl-, Amino-, Alkylamino-, Dialkylamino-, Aryl-, Arylalkyl-, Heteroaryl- oder Heteroarylalkylgruppe und

der vierte der Reste $R_a$ bis $R_d$ ein Wasserstoffatom oder eine Alkyl-, Aryl- oder Arylalkylgruppe,

wobei, soweit nichts anderes erwähnt wurde,

unter den bei der Definition der vorstehenden Reste erwähnten Arylteilen eine Phenylgruppe, die jeweils durch $R_{10}$ monosubstituiert, durch $R_{11}$ mono-, di- oder trisubstituiert oder durch $R_{10}$ monosubstituiert und zusätzlich durch $R_{11}$ mono- oder disubstituiert sein kann, wobei die Substituenten gleich oder verschieden sein können und

$R_{10}$ eine Cyano-, Carboxy-, Aminocarbonyl-, Alkylaminocarbonyl-, Dialkylaminocarbonyl-, Alkoxycarbonyl-, Alkylcarbonyl-, Alkylsulfenyl-, Alkylsulfinyl-, Alkylsulfonyl-, Alkylsulfonyloxy-, Perfluoralkyl-, Trifluormethoxy-, Nitro-, Amino-, Alkylamino-, Dialkylamino-, Alkylcarbonylamino-, Phenylalkylcarbonylamino-, Phenylcarbonylamino-, Alkylsulfonylamino-, Phenylalkylsulfonylamino-, Phenylsulfonylamino-, N-Alkyl-alkylcarbonylamino-, N-Alkyl-phenylalkylcarbonylamino-, N-Alkyl-phenylcarbonylamino-, N-Alkyl-alkylsulfonylamino-, N-Alkyl-phenylalkylsulfonylamino-, N-Alkyl-phenylsulfonylamino-, Aminosulfonyl-, Alkylaminosulfonyl- oder Dialkylaminosulfonylgruppe und

$R_{11}$ eine Alkyl-, Hydroxy- oder Alkoxygruppe, ein Fluor-, Chlor-, Brom- oder Jodatom darstellen, wobei zwei Reste $R_{11}$ sofern diese an benachbarte Kohlenstoffatome gebunden sind, auch eine Alkylengruppe mit 3 bis 6 Kohlenstoffatomen, eine 1,3-Butadien-1,4-diylengruppe oder eine Methylendioxygruppe darstellen können,

unter den bei der Definition der vorstehenden Resten erwähnten Arylenteilen eine Phenylengruppe die jeweils durch $R_{10}$ monosubstituiert, durch $R_{11}$ mono- oder disubstituiert oder durch $R_{10}$ monosubstituiert und zusätzlich durch $R_{11}$ monosubstituiert sein kann, wobei die Substituenten gleich oder verschieden sein können und wie vorstehend erwähnt definiert sind,

unter den bei der Definition der vorstehenden Reste erwähnten Heteroarylteilen ein 5-gliedriger heteroaromatischer Ring, welcher ein Sauerstoff-, Schwefel- oder Stickstoffatom, ein Stickstoffatom und ein Sauerstoff-, Schwefel- oder Stickstoffatom oder zwei Stickstoffatome und ein Sauerstoff-, Schwefel- oder Stickstoffatom enthält, oder ein 6-gliedriger heteroaromatischer Ring, welcher 1, 2 oder 3 Stickstoffatome enthält

und in dem zusätzlich eine oder zwei -CH=N-Gruppen jeweils durch eine -CO-$NR_5$-Gruppe ersetzt sein können, wobei $R_5$ wie vorstehend erwähnt definiert ist, und zusätzlich die vorstehend erwähnten heteroaromatischen Ringe durch eine oder zwei Alkylgruppen oder am Kohlenstoffgerüst auch durch ein Fluor-, Chlor-, Brom- oder Jodatom, durch eine Hydroxy- oder Alkoxygruppe substituiert sein können,
zu verstehen ist, sowie, soweit nichts anderes erwähnt wurde, die vorstehend erwähnten Alkyl-, Alkylen- oder Alkoxyteile jeweils 1 bis 4 Kohlenstoffatome enthalten können und jedes Kohlenstoffatom in den vorstehend erwähnten Alkylen- und Cycloalkylenteilen höchstens mit einem Heteroatom verknüpft ist.

Bevorzugte Verbindungen der obigen allgemeinen Formel I sind mit Ausnahme von
1-[4-(4-Amidino-phenyl)-cyclohexyl]-3-(2-carboxy-ethyl)-imidazolidin-2-on,
1-[4-(4-Amidino-phenyl)-cyclohexyl]-3-(2-methoxycarbonyl-ethyl)-imidazolidin-2-on,
1-[4-(4-Amidino-phenyl)-cyclohexyl]-3-(2-methoxycarbonyl-ethyl)-3H-imidazol-2-on,
1-[4-(4-Amidino-phenyl)-cyclohexyl]-3-(2-carboxy-ethyl)-3H-imidazol-2-on,
1-[4-(4-Cyano-phenyl)-cyclohexyl]-3-(2-ethoxycarbonyl-ethyl)-3H-imidazol-2-on,
1-(4-Amidino-phenyl)-3-[4-(2-carboxy-ethyl)-cyclohexyl]-imidazolidin-2-on,
1-(4-Amidino-phenyl)-3-[4-(2-methoxycarbonyl-ethyl)-cyclohexyl]-imidazolidin-2-on,
1-(4-Cyano-phenyl)-3-[4-(2-methoxycarbonyl-ethyl)-cyclohexyl]-imidazolidin-2-on,
1-(4-Amidino-phenyl)-3-[4-(2-phosphono-ethyl)-phenyl]-imidazolidin-2-on,
1-(4-Amidino-phenyl)-3-[4-[2-(O-methyl-phosphono)-ethyl]-phenyl]-imidazolidin-2-on,
1-(4-Cyano-phenyl)-3-[4-(2-phosphono-ethyl)-phenyl]-imidazolidin-2-on,
1-(4-Cyano-phenyl)-3-[4-[2-(0-methyl-phosphono)-ethyl]-phenyl]-imidazolidin-2-on,
1-(1-Amidino-4-piperidinyl)-3-[4-(2-carboxy-ethyl)-phenyl]-imidazolidin-2-on,
1-(1-Amidino-4-piperidinyl)-3-[4-(2-methoxycarbonyl-ethyl)-phenyl]-imidazolidin-2-on,
1-(4-Aminomethyl-cyclohexyl)-3-[4-(2-carboxy-ethyl)-phenyl]-imidazolidin-2-on,
1-(4-Aminomethyl-cyclohexyl)-3-[4-(2-methoxycarbonyl-ethyl)-phenyl]-imidazolidin-2-on,
1-(4-Amidino-phenyl)-3-[4-(2-carboxy-ethyl)-phenyl]-4-phenyl-3H-imidazol-2-on,
1-(4-Amidino-phenyl)-3-[4-(2-methoxycarbonyl-ethyl)-phenyl]-4-phenyl-3H-imidazol-2-on,
1-(4-Amidino-phenyl)-3-[4-(2-carboxy-ethyl)-phenyl]-4-phenyl-imidazolidin-2-on,
1-(4-Amidino-phenyl)-3-[4-(2-methoxycarbonyl-ethyl)-phenyl]-4-phenyl-imidazolidin-2-on,
4-(4-Amidino-phenyl)-1-[4-(2-carboxy-ethyl)-phenyl]-3-phenyl-3H-imidazol-2-on,
4-(4-Amidino-phenyl)-1-[4-(2-methoxycarbonyl-ethyl)-phenyl]-3-phenyl-3H-imidazol-2-on,
4-(4-Cyano-phenyl)-1-[4-(2-methoxycarbonyl-ethyl)-phenyl]-3-phenyl-3H-imidazol-2-on,
4-(4-Amidino-phenyl)-1-[4-(2-carboxy-ethyl)-phenyl]-3-phenyl-3H-imidazol-2-thion,
4-(4-Amidino-phenyl)-1-[4-(2-methoxycarbonyl-ethyl)-phenyl]-3-phenyl-3H-imidazol-2-thion,
4-(4-Cyano-phenyl)-1-[4-(2-methoxycarbonyl-ethyl)-phenyl]-3-phenyl-3H-imidazol-2-thion,
4-(4-Amidino-phenyl)-1-[4-(2-carboxy-ethyl)-phenyl]-5-methyl-3-phenyl-3H-imidazol-2-on,
4-(4-Amidino-phenyl)-1-[4-(2-methoxycarbonyl-ethyl)-phenyl]-5-methyl-3-phenyl-3H-imidazol-2-on,
4-(4-Cyano-phenyl)-1-[4-(2-methoxycarbonyl-ethyl)-phenyl]-5-methyl-3-phenyl-3H-imidazol-2-on,
4-(4-Amidino-phenyl)-1-[4-(2-carboxy-ethyl)-phenyl]-3-phenyl-imidazolidin-2-on,
4-(4-Amidino-phenyl)-1-[4-(2-methoxycarbonyl-ethyl)-phenyl]-3-phenyl-imidazolidin-2-on,
1-(4-Amidino-phenyl)-4-[4-(2-carboxy-ethyl)-phenyl]-3-phenyl-3H-imidazol-2-on,
1-(4-Amidino-phenyl)-4-[4-(2-methoxycarbonyl-ethyl)-phenyl]-3-phenyl-3H-imidazol-2-on,
1-(4-Cyano-phenyl)-4-[4-(2-methoxycarbonyl-ethyl)-phenyl]-3-phenyl-3H-imidazol-2-on,
1-(4-Amidino-phenyl)-4-[4-(2-carboxy-ethyl)-phenyl]-3-phenyl-imidazolidin-2-on,
1-(4-Amidino-phenyl)-4-[4-(2-methoxycarbonyl-ethyl)-phenyl]-3-phenyl-imidazolidin-2-on,
1-(4-Amidino-phenyl)-4-[4-(2-carboxy-ethyl)-phenyl]-3-phenyl-3H-imidazol-2-thion,
1-(4-Amidino-phenyl)-4-[4-(2-methoxycarbonyl-ethyl)-phenyl]-3-phenyl-3H-imidazol-2-thion,
1-(4-Cyano-phenyl)-4-[4-(2-methoxycarbonyl-ethyl)-phenyl]-3-phenyl-3H-imidazol-2-thion,
1-(4-Amidino-phenyl)-4-[4-(2-carboxy-ethyl)-phenyl]-5-methyl-3-phenyl-3H-imidazol-2-on,
1-(4-Amidino-phenyl)-4-[4-(2-methoxycarbonyl-ethyl)-phenyl]-5-methyl-3-phenyl-3H-imidazol-2-on,
1-(4-Cyano-phenyl)-4-[4-(2-methoxycarbonyl-ethyl)-phenyl]-5-methyl-3-phenyl-3H-imidazol-2-on,
4-(4'-Amidino-4-biphenylyl)-1-(2-carboxy-ethyl)-3-phenyl-3H-imidazol-2-on,
4-(4'-Amidino-4-biphenylyl)-1-(2-methoxycarbonyl-ethyl)-3-phenyl-3H-imidazol-2-on,
4-(4'-Cyano-4-biphenylyl)-1-(2-methoxycarbonyl-ethyl)-3-phenyl-3H-imidazol-2-on,
4-[4'-Amidino-4-biphenylyl)-1-(2-carboxy-ethyl)-3-phenyl-imidazolidin-2-on,
4-(4'-Amidino-4-biphenylyl)-1-(2-methoxycarbonyl-ethyl)-3-phenyl-imidazolidin-2-on,
4-(4'-Amidino-4-biphenylyl)-1-(2-carboxy-ethyl)-3-phenyl-3H-imidazol-2-thion,
4-(4'-Amidino-4-biphenylyl)-1-(2-methoxycarbonyl-ethyl)-3-phenyl-3H-imidazol-2-thion,
4-(4'-Cyano-4-biphenylyl)-1-(2-methoxycarbonyl-ethyl)-3-phenyl-3H-imidazol-2-thion,

4-(4'-Amidino-4-biphenylyl)-1-(2-carboxy-ethyl)-5-methyl-3-phenyl-3H-imidazol-2-on,
4-(4'-Amidino-4-biphenylyl)-1-(2-methoxycarbonyl-ethyl)-5-methyl-3-phenyl-3H-imidazol-2-on,
4-(4'-Cyano-4-biphenylyl)-1-(2-methoxycarbonyl-ethyl)-5-methyl-3-phenyl-3H-imidazol-2-on,
1-(4-Amidino-phenyl)-3-[4-(2-butyloxycarbonyl-ethyl)-phenyl]-imidazolidin-2-on,
1-(4'-Amidino-4-biphenylyl)-3-(2-ethoxycarbonyl-ethyl)-3H-imidazol-2-on,
1-(4'-Cyano-4-biphenylyl)-3-(2-ethoxycarbonyl-ethyl)-3H-imidazol-2-on,
1-(4'-Amidino-4-biphenylyl)-3-(2-ethoxycarbonyl-ethyl)-imidazolidin-2,4-dion,
1-(4'-Cyano-4-biphenylyl)-3-(2-ethoxycarbonyl-ethyl)-imidazolidin-2,4-dion,
1-(4'-Amidino-4-biphenylyl)-3-(2-ethoxycarbonyl-ethyl)-3,4,5,6-tetrahydro-1H-pyrimidin-2-on,
1-(4'-Cyano-4-biphenylyl)-3-(2-ethoxycarbonyl-ethyl)-3,4,5,6-tetrahydro-1H-pyrimidin-2-on,
2-(4-Amidino-phenyl)-4-[4-(2-isopropyloxycarbonyl-ethyl)-phenyl]-5-methyl-4H-1,2,4-triazol-3-on,
4-[4-(2-Isopropyloxycarbcnyl-ethyl)-phenyl]-2-(4-methoxycarbonylamidino-phenyl)-5-methyl-4H-1,2,4-triazol-3-on,
1-(4-Amidino-phenyl)-3-[4-(2-ethoxycarbonyl-ethyl)-phenyl]-3H-imidazol-2-on,
1-(4-Ethoxycarbonylamidino-phenyl)-3-[4-(2-ethoxycarbonyl-ethyl)-phenyl]-3H-imidazol-2-on,
1-(4-Ethoxycarbonylamidino-phenyl)-3-[4-(2-ethoxycarbonyl-ethyl)-phenyl]-imidazolidin-2-on,
1-(4-Methoxycarbonylamidino-phenyl)-3-[4-(2-methoxycarbonyl-ethyl)-cyclohexyl]-imidazolidin-2-on,
4-[4-(2-Isobutyloxycarbonyl-ethyl)-phenyl]-2-(4-methoxycarbonylamidino-phenyl)-5-methyl-4H-1,2,4-triazol-3-on,
2-(4-Amidino-phenyl)-4-[4-(2-isobutyloxycarbonyl-ethyl)-phenyl]-5-methyl-4H-1,2,4-triazol-3-on,
1-(1-Amino-5-indanyl)-3-[4-(2-carboxy-ethyl)-phenyl]-imidazolidin-2-on,
1-(1-Amino-5-indanyl)-3-[4-(2-methoxycarbonyl-ethyl)-phenyl]-imidazolidin-2-on,
1-(1-Amino-1,2,3,4-tetrahydro-6-naphthyl)-3-[4-(2-carboxy-ethyl)-phenyl]-imidazolidin-2-on,
1-(1-Amino-1,2,3,4-tetrahydro-6-naphthyl)-3-[4-(2-methoxycarbonyl-ethyl)-phenyl]-imidazolidin-2-on,
1-(4-Amino-cyclohexyl)-3-[4-(3-carboxy-propyl)-phenyl]-imidazolidin-2-on,
1-(4-Amino-cyclohexyl)-3-[4-(3-methoxycarbonyl-propyl)-phenyl]-imidazolidin-2-on,
1-(4-Amino-cyclohexyl)-3-[4-(2-carboxy-ethyl)-phenyl]-imidazolidin-2-on,
1-(4-Amino-cyclohexyl)-3-[4-(2-methoxycarbonyl-ethyl)-phenyl]-imidazolidin-2-on,
1-(4-Aminomethyl-phenyl)-3-[3-(2-carboxy-ethyl)-phenyl]-imidazolidin-2-on,
1-(4-Aminomethyl-phenyl)-3-[3-(2-methoxycarbonyl-ethyl)-phenyl]-imidazolidin-2-on,
1-(4-Amino-cyclohexyl)-3-[4-[(2-carboxy-ethyl)-aminocarbonyl]-phenyl]-imidazolidin-2-on,
1-(4-Amino-cyclohexyl)-3-[4-[(2-methoxycarbonyl-ethyl)-aminocarbonyl]-phenyl]-imidazolidin-2-on,
1-[4-[(2-Carboxy-ethyl)-aminocarbonyl]-phenyl]-3-(4-piperidinyl)-imidazolidin-2-on,
1-[4-[(2-Methoxycarbonyl-ethyl)-aminocarbonyl]-phenyl]-3-(4-piperidinyl)-imidazolidin-2-on,
1-[4-(1-Amino-cyclopropyl)-phenyl]-3-[4-(2-carboxy-ethyl)-phenyl]-imidazolidin-2-on,
1-[4-(1-Amino-cyclopropyl)-phenyl]-3-[4-(2-methoxycarbonyl-ethyl)-phenyl]-imidazolidin-2-on,
1-[4-(1-Amino-cyclopentyl)-phenyl]-3-[4-(2-carboxy-ethyl)-phenyl]-imidazolidin-2-on,
1-[4-(1-Amino-cyclopentyl)-phenyl]-3-[4-[(2-methoxycarbonyl-ethyl)-phenyl]-imidazolidin-2-on,
2-(4-Amidino-phenyl)-5-ethyl-4-[4-(2-isopropyloxycarbonyl-ethyl)-phenyl]-4H-1,2,4-triazol-3-on,
1-(4-Amidino-phenyl)-3-[4-(2-isopropyloxycarbonyl-ethyl)-phenyl]-4-methyl-3H-imidazol-2-on,
4-(4-Amidino-phenyl)-2-[4-(2-isopropyloxycarbonyl-ethyl)-phenyl]-4H-1,2,4-triazol-3-on,
1-(4-Amidino-phenyl)-3-[4-(2-isopropyloxycarbonyl-ethyl)-phenyl]-imidazolidin-2-on,
1-(4-Amidino-phenyl)-3-[4-(2-isopropyloxycarbonyl-ethyl)-phenyl]-3H-imidazol-2-on,
1-(4-Amidino-phenyl)-3-[4-(2-isopropyloxycarbonyl-ethyl)-phenyl]-imidazolidin-2,4-dion,
1-[4-[(2-Carboxy-ethyl)-aminocarbonyl]-phenyl]-3-(1-methyl-4-piperidinyl)-imidazolidin-2-on,
2-(4-Amidino-phenyl)-4-[4-(2-phosphono-ethyl)-phenyl]-5-methyl-4H-1,2,4-triazol-3-on,
2-(4-Amidino-phenyl)-4-[4-[2-(0-methyl-phosphono)-ethyl]-phenyl)-5-methyl-4H-1,2,4-triazol-3-on,
2-(4-Amidino-phenyl)-4-[4-(2-carboxy-ethyl)-phenyl]-5-phenyl-4H-1,2,4-triazol-3-on,
2-(4-Amidino-phenyl)-4-[4-(2-methoxycarbonyl-ethyl)-phenyl]-5-phenyl-4H-1,2,4-triazol-3-on,
3-(4-Amidino-phenyl)-1-[4-(2-carboxy-ethyl)-phenyl]-4-trifluormethyl-3H-imidazol-2-on,
3-(4-Amidino-phenyl(-1-[4-(2-methoxycarbonyl-ethyl(-phenyl]-4-trifluormethyl-3H-imidazol-2-on,
2-(4-Amidino-phenyl)-4-[4-(2-carboxy-ethyl)-phenyl]-5-trifluormethyl-4H-1,2,4-triazol-3-on,
2-(4-Amidino-phenyl)-4-[4-(2-methoxycarbonyl-ethyl)-phenyl]-5-trifluormethyl-4H-1,2,4-triazol-3-on und
3-(4-Cyano-phenyl)-1-(4-ethoxycarbonyl-butyl)-3H-imidazo-[4,5-b]pyridin-2-on,
sowie mit den Maßgaben, daß

(i) X eine am Stickstoffatom durch eine Cyanogruppe substituierte Carbiminogruppe,

(ii) Y eine gegebenenfalls durch $R_c$ oder $R_d$ oder $R_c$ und $R_d$ substituierte 1,2-Cycloalkylengruppe mit 5 bis 7 Kohlenstoffatomen,

(iii) A eine Aminoalkylgruppe mit 1 bis 5 Kohlenstoffatomen, eine Amino-, Amidino- oder Guanidinogruppe, wobei in jeder der vorstehend erwähnten Gruppen jeweils an einem der Stickstoffatome ein Wasserstoffatom durch eine $R_1$-CO-O-($R_2CR_3$)-O-CO-Gruppe ersetzt ist,
(iv) B eine gegebenenfalls durch eine oder zwei Alkylgruppen substituierte Cycloalkylengruppe mit 3 bis 7 Kohlenstoffatomen,
eine gegebenenfalls durch einen Rest $R_e$ substituierte Cycloalkylengruppe mit 4 oder 5 Kohlenstoffatomen, welche zusätzlich durch 1 bis 4 Alkylgruppen substituiert sein kann und in der im Cycloalkylenteil jeweils eine >CH-Einheit durch ein Stickstoffatom ersetzt ist,
eine gegebenenfalls durch einen Rest $R_e$ substituierte Cycloalkylengruppe mit 6 bis 8 Kohlenstoffatomen, welche zusätzlich durch 1 bis 4 Alkylgruppen substituiert sein kann und in der im Cycloalkylenteil jeweils eine >CH-Einheit durch ein Stickstoffatom ersetzt ist und zusätzlich eine weitere in 4-Stellung befindliche >CH-Einheit durch ein Stickstoffatom ersetzt sein kann, wobei außerdem in den vorstehend erwähnten 6- bis 8-gliedrigen Ringen jeweils eine oder zwei zu einem Stickstoffatom benachbarte Methylengruppen jeweils durch eine Carbonylgruppe ersetzt sein können,
eine gegebenenfalls durch eine oder zwei Alkylgruppen substituierte Cycloalkenylengruppe mit 5 bis 7 Kohlenstoffatomen, welche zusätzlich durch eine oder zwei Methylgruppen substituiert sein kann und in der im Cycloalkenylenteil jeweils eine >CH-Einheit durch ein Stickstoffatom ersetzt ist,
eine Piperidinylengruppe, in der zwei Wasserstoffatome am Kohlenstoffgerüst durch eine geradkettige Alkylenbrücke ersetzt sind, wobei diese Brücke 2 bis 6 Kohlenstoffatome enthält, wenn die zwei Wasserstoffatome sich am selben Kohlenstoffatom befinden, oder 1 bis 5 Kohlenstoffatome enthält, wenn sie zwei Wasserstoffatome an benachbarten Kohlenstoffatomen ersetzt, oder 1 bis 4 Kohlenstoffatome enthält, wenn sie zwei Wasserstoffatome an Kohlenstoffatomen ersetzt, die durch ein weiteres Atom getrennt sind, oder 1 bis 3 Kohlenstoffatome enthält, wenn sie zwei Wasserstoffatome an Kohlenstoffatomen ersetzt, die durch zwei Atome getrennt sind, wobei die vorstehenden Gruppen jeweils durch eine oder zwei Alkylgruppen substituiert sein können,
eine über den Rest W mit dem Rest C verknüpfte Alkylengruppe oder
B zusammen mit A eine gegebenenfalls durch 1 oder 2 Alkylgruppen substituierte Pyridylgruppe oder eine Piperidinylgruppe, in der das Wasserstoffatom in 1-Stellung zusammen mit einem Wasserstoffatom in 2-Stellung durch eine geradkettige Alkylengruppe mit 3 oder 4 Kohlenstoffatomen ersetzt ist, oder eine Piperidinylgruppe, in der das Wasserstoffatom in 1-Stellung zusammen mit einem Wasserstoffatom in 3-Stellung durch eine geradkettige Alkylengruppe mit 2 bis 4 Kohlenstoffatomen ersetzt ist, oder eine Piperidinylgruppe, in der das Wasserstoffatom in 1-Stellung zusammen mit einem Wasserstoffatom in 4-Stellung durch eine geradkettige Alkylengruppe mit 1 bis 3 Kohlenstoffatomen ersetzt ist, wobei die an die 4-Stellung gebundene Methylengruppe einer Ethylenkette durch eine Hydroxymethylen- oder Carbonylgruppe ersetzt sein kann und zusätzlich das Stickstoffatom dieser vorstehend erwähnten bicyclischen Gruppen, welche im Kohlenstoffgerüst durch eine oder zwei Alkylgruppen substituiert sein können, durch Boran komplexiert oder durch eine im Phenylkern gegebenenfalls durch 1 bis 2 Methoxygruppen substituierte Benzylgruppe quarternisiert sein kann,
(v) C eine Alkylengruppe mit 1 bis 6 Kohlenstoffatomen, in der eine Methylengruppe durch ein Sauerstoff- oder Schwefelatom oder durch eine Sulfinyl-, Sulfonyl- oder -$NR_4$-Gruppe ersetzt ist oder in der eine Ethylengruppe durch eine -$CONR_5$- oder -$NR_5CO$-Gruppe ersetzt ist,
eine gegebenenfalls durch eine oder zwei Alkylgruppen substituierte 5- bis 7-gliedrige Cycloalkylengruppe,
eine Cyclohexylengruppe, in der zwei durch drei Bindungen getrennte Kohlenstoffatome zusätzlich durch eine geradkettige Alkylengruppe mit 1 bis 3 Kohlenstoffatomen verknüpft sind, wobei diese bicyclischen Gruppen jeweils außerdem durch ein bis zwei Alkylgruppen substituiert sein können,
eine Indanylen-, Naphthylen-, 1,2,3,4-Tetrahydronaphthylen- oder Benzosuberanylengruppe, wobei die gesättigten Ringe jeweils durch 1 oder 2 Alkylgruppen substituiert sein können und die aromatischen Ringe jeweils durch ein Fluor-, Chlor-, Brom- oder Jodatom, durch eine Alkyl-, Trifluormethyl-, Hydroxy-, Alkoxy-, Alkylsulfenyl-, Alkylsulfinyl-, Alkylsulfonyl- oder Cyanogruppe substituiert sein können,
eine gegebenenfalls durch den Rest $R_e$ substituierte Cycloalkylengruppe mit 5 bis 7 Kohlenstoffatomen, welche zusätzlich durch 1 bis 4 Alkylgruppen substituiert sein kann und in denen im Cycloalkylenteil jeweils eine >CH-Einheit durch ein Stickstoffatom ersetzt ist,
eine Piperidinylengruppe, in der zwei Wasserstoffatome am Kohlenstoffgerüst durch eine geradkettige Alkylenbrücke ersetzt sind, wobei diese Brücke 2 bis 6 Kohlenstoffatome enthält, wenn die zwei Wasserstoffatome sich am selben Kohlenstoffatom befinden, oder 1 bis 5 Kohlenstoffatome enthält, wenn sie zwei Wasserstoffatome an benachbarten Kohlenstoffatomen ersetzt, oder 1 bis 4 Kohlenstoffatome enthält, wenn sie zwei Wasserstoffatome an Kohlenstoffatomen ersetzt, die durch ein weiteres Atom

getrennt sind, oder 1 bis 3 Kohlenstoffatome enthält, wenn sie zwei Wasserstoffatome an Kohlenstoffatomen ersetzt, die durch zwei Atome getrennt sind, wobei die vorstehenden Gruppen jeweils durch eine oder zwei Alkylgruppen substituiert sein können, oder

C zusammen mit A und B eine gegebenenfalls jeweils durch eine oder zwei Alkylgruppen substituierte Pyridyl- oder 1-(4-Pyridyl)-piperidinylgruppe oder eine Piperidinylgruppe, in der das Wasserstoffatom in 1-Stellung zusammen mit einem Wasserstoffatom in 4-Stellung durch eine geradkettige Alkylengruppe mit 1 bis 3 Kohlenstoffatomen ersetzt ist, wobei die an die 4-Stellung gebundene Methylengruppe einer Ethylenkette durch eine Hydroxymethylen- oder Carbonylgruppe ersetzt sein kann und zusätzlich das Stickstoffatom dieser vorstehend erwähnten bicyclischen Gruppen, welche im Kohlenstoffgerüst durch eine oder zwei Alkylgruppen substituiert sein können, durch Boran komplexiert oder durch eine im Phenylkern gegebenenfalls durch 1 bis 2 Methoxygruppen substituierte Benzylgruppe quarternisiert sein kann, oder

C eine Alkylengruppe mit 1 bis 6 Kohlenstoffatomen und B eine Bindung,

(vi) D eine 1,3-Arylengruppe,

eine Indanylen-, Naphthylen-, 1,2,3,4-Tetrahydronaphthylen- oder Benzosuberanylengruppe, wobei die gesättigten Ringe jeweils durch 1 oder 2 Alkylgruppen substituiert sein können und die aromatischen Ringe jeweils durch ein Fluor-, Chlor-, Brom- oder Jodatom, durch eine Alkyl-, Trifluormethyl-, Hydroxy-, Alkoxy-, Alkylsulfenyl-, Alkylsulfinyl-, Alkylsulfonyl- oder Cyanogruppe substituiert sein können,

eine gegebenenfalls durch eine oder zwei Alkylgruppen substituierte Cycloalkylengruppe mit 4 bis 7 Kohlenstoffatomen oder

eine Cyclohexylengruppe, in der zwei durch drei Bindungen getrennte Kohlenstoffatome zusätzlich durch eine geradkettige Alkylengruppe mit 1 bis 3 Kohlenstoffatomen verknüpft sind, wobei diese bicyclischen Gruppen jeweils außerdem durch ein bis zwei Alkylgruppen substituiert sein können,

(vii) E eine geradkettige Alkylengruppe mit 1 bis 6 Kohlenstoffatomen, die durch eine Alkylgruppe mit 1 bis 6 Kohlenstoffatomen, durch eine Arylgruppe oder durch eine $HNR_6$- oder N-Alkyl-$NR_6$-Gruppe substituiert ist,

eine gegebenenfalls durch eine oder zwei Alkylgruppen substituierte Cycloalkylengruppe mit 5 bis 7 Kohlenstoffatomen, in der eine $>$CH-Einheit durch ein Stickstoffatom ersetzt ist,

eine durch eine Hydroxy- oder Aminogruppe, durch eine Alkoxygruppe mit 1 bis 6 Kohlenstoffatomen oder durch eine $HNR_6$- oder N-Alkyl-$NR_6$-Gruppe substituierte Cycloalkylengruppe mit 4 bis 7 Kohlenstoffatomen im Cycloalkylenteil oder

eine über den Rest W mit dem Rest D verknüpfte Alkylengruppe, wobei die Alkylengruppe zusätzlich durch eine oder zwei Alkylgruppen mit jeweils 1 bis 6 Kohlenstoffatomen, durch eine Hydroxy-, Amino- oder Arylgruppe, durch eine Alkoxygruppe mit 1 bis 6 Kohlenstoffatomen oder durch eine $HNR_6$- oder N-Alkyl-$NR_6$-Gruppe substituiert ist,

(viii) F eine Alkoxycarbonylgruppe mit insgesamt 3 bis 9 Kohlenstoffatomen, eine $R_7$O-CO-, Phosphono-, O-Alkyl-phosphono-, O,O'-Dialkyl-phosphono- oder $R_8$CO-O-$CHR_9$-O-CO-Gruppe und

(ix) der dritte der Reste $R_a$ bis $R_d$ eine Trifluormethyl- oder Arylgruppe

darstellt, wobei mindestens eine der obigen Bedingungen (i) bis (ix) erfüllt sein müssen, diejenigen in denen X eine gegebenenfalls am Stickstoffatom durch eine Alkyl- oder Cyanogruppe substituierte Carbiminogruppe, eine Carbonyl-, Thiocarbonyl- oder Sulfonylgruppe,

Y eine gegebenenfalls durch $R_c$ oder $R_d$ oder $R_c$ und $R_d$ substituierte geradkettige Alkylengruppe mit 2 oder 3 Kohlenstoffatomen, die zusätzlich durch eine oder zwei Alkylgruppen substituiert sein kann, und in der zusätzlich eine Methylengruppe durch eine Carbonylgruppe ersetzt sein kann,

eine gegebenenfalls durch $R_c$ oder $R_d$ oder $R_c$ und $R_d$ substituierte geradkettige Alkenylengruppe mit 2 oder 3 Kohlenstoffatomen, in der zusätzlich eine gegebenenfalls vorhandene Methylengruppe durch eine Carbonylgruppe ersetzt sein kann,

eine gegebenenfalls durch $R_c$ oder $R_d$ oder $R_c$ und $R_d$ substituierte 1,2-Cycloalkylengruppe mit 5 bis 7 Kohlenstoffatomen,

eine 1,2-Cycloalkenylengruppe mit 5 bis 7 Kohlenstoffatomen,

eine 1,2-Arylengruppe,

eine 1,2-Phenylengruppe, in der eine oder zwei Methingruppen jeweils durch ein Stickstoffatom ersetzt sind, wobei die vorstehend erwähnten heterocyclischen Gruppen zusätzlich durch eine oder zwei Alkylgruppen substituiert sein können, oder

eine gegebenenfalls durch $R_c$ oder $R_d$ substituierte -CO-NH-, -NH-CO-, -CH=N- oder -N=CH-Gruppe,

der erste der Reste $R_a$ bis $R_d$ eine Gruppe der Formel

A - B - C -,

in der

A eine Aminoalkylgruppe mit 1 bis 5 Kohlenstoffatomen, eine Amino-, Amidino- oder Guanidinogruppe, wobei in jeder der vorstehend erwähnten Gruppen jeweils an einem der Stickstoffatome ein oder zwei Wasserstoffatome jeweils durch eine Alkylgruppe oder ein Wasserstoffatom durch eine Alkoxycarbonylgruppe mit insgesamt 2 bis 5 Kohlenstoffatomen, durch eine Benzyloxycarbonylgruppe oder durch eine $R_1$-CO-O-($R_2CR_3$)-O-CO-Gruppe ersetzt sein kann, wobei

$R_1$ eine Alkylgruppe mit 1 bis 8 Kohlenstoffatomen, eine Cycloalkylgruppe mit 5 bis 7 Kohlenstoffatomen, eine Phenyl- oder Phenylalkylgruppe,

$R_2$ ein Wasserstoffatom oder eine Alkylgruppe und

$R_3$ ein Wasserstoffatom darstellen,

eine Cyano- oder Cyanoalkylgruppe oder

auch, falls A an ein Stickstoffatom der Reste B oder C gebunden ist, das nicht Teil einer Lactamgruppe ist, ein Wasserstoffatom, eine Alkylgruppe, eine Benzylgruppe, in der der Phenylteil durch 1 bis 2 Methoxygruppen substituiert sein kann, eine Formyl-, Acetyl- oder Trifluoracetylgruppe, eine Alkoxycarbonylgruppe mit insgesamt 2 bis 5 Kohlenstoffatomen, eine Benzyloxycarbonylgruppe oder eine $R_1$-CO-O-($R_2CR_3$)-O-CO-Gruppe, wobei $R_1$ bis $R_3$ wie vorstehend erwähnt definiert sind,

B eine Bindung,

eine Alkylengruppe,

eine Arylengruppe,

eine gegebenenfalls durch eine oder zwei Alkylgruppen substituierte Pyridinylen-, Pyrimidinylen-, Pyrazinylen- oder Pyridazinylengruppe,

eine gegebenenfalls durch eine oder zwei Alkylgruppen substituierte Cycloalkylengruppe mit 3 bis 7 Kohlenstoffatomen,

eine gegebenenfalls durch einen Rest $R_e$ substituierte Cycloalkylengruppe mit 4 oder 5 Kohlenstoffatomen, welche zusätzlich durch 1 bis 4 Alkylgruppen substituiert sein kann und in denen im Cycloalkylenteil jeweils eine >CH-Einheit durch ein Stickstoffatom, welches mit dem Rest A verknüpft ist, ersetzt ist, wobei

$R_e$ mit der Maßgabe, daß ein Heteroatom des Restes $R_e$ durch mindestens zwei Kohlenstoffatome von dem Ringstickstoffatom der cyclischen Iminogruppe getrennt ist, eine Alkyl-, Hydroxy-, Alkoxy-, Cyano-, Carboxy-, Alkoxycarbonyl-, Alkylcarbonyl-, Aminocarbonyl-, Alkylaminocarbonyl- oder Dialkylaminocarbonylgruppe darstellt,

eine gegebenenfalls durch einen Rest $R_e$ substituierte Cycloalkylengruppe mit 6 bis 8 Kohlenstoffatomen, welche zusätzlich durch 1 bis 4 Alkylgruppen substituiert sein kann und in denen im Cycloalkylenteil jeweils eine >CH-Einheit durch ein Stickstoffatom, welches mit dem Rest A verknüpft ist, ersetzt ist und zusätzlich eine weitere in 4-Stellung befindliche >CH-Einheit durch ein Stickstoffatom ersetzt sein kann, wobei außerdem in den vorstehend erwähnten 6- bis 8-gliedrigen Ringen jeweils eine oder zwei zu einem Stickstoffatom benachbarte Methylengruppen jeweils durch eine Carbonylgruppe ersetzt sein können, wobei $R_e$ wie vorstehend erwähnt definiert ist,

eine gegebenenfalls durch eine oder zwei Alkylgruppen substituierte Cycloalkenylengruppe mit 5 bis 7 Kohlenstoffatomen, welche zusätzlich durch eine oder zwei Methylgruppen substituiert sein kann und in denen im Cycloalkenylenteil jeweils eine >CH-Einheit durch ein Stickstoffatom, welches mit dem Rest A verknüpft ist, ersetzt ist, wobei das Stickstoffatom durch mindestens eine gegebenenfalls mono- oder disubstituierte Methylengruppe von der Doppelbindung getrennt ist,

eine Piperidinylengruppe, in der zwei Wasserstoffatome am Kohlenstoffgerüst durch eine geradkettige Alkylenbrücke ersetzt sind, wobei diese Brücke 2 bis 6 Kohlenstoffatome enthält, wenn die zwei Wasserstoffatome sich am selben Kohlenstoffatom befinden, oder 1 bis 5 Kohlenstoffatome enthält, wenn sie zwei Wasserstoffatome an benachbarten Kohlenstoffatomen ersetzt, oder 1 bis 4 Kohlenstoffatome enthält, wenn sie zwei Wasserstoffatome an Kohlenstoffatomen ersetzt, die durch ein weiteres Atom getrennt sind, oder 1 bis 3 Kohlenstoffatome enthält, wenn sie zwei Wasserstoffatome an Kohlenstoffatomen ersetzt, die durch zwei Atome getrennt sind, wobei die vorstehenden Gruppen jeweils durch eine oder zwei Alkylgruppen substituiert sein können,

eine über den Rest W mit dem Rest C verknüpfte Alkylengruppe, wobei W ein Sauerstoff- oder Schwefelatom, eine Sulfinyl-, Sulfonyl-, -$NR_4$-, -$NR_5$CO- oder -CO$NR_5$-Gruppe darstellt, wobei

$R_4$ ein Wasserstoffatom, eine Alkyl-, Alkylcarbonyl-, Alkylsulfonyl-, Arylcarbonyl- oder Arylsulfonylgruppe und

$R_5$ ein Wasserstoffatom oder eine Alkylgruppe darstellen,

oder B zusammen mit A eine gegebenenfalls durch 1 oder 2 Alkylgruppen substituierte Pyridylgruppe oder eine Piperidinylgruppe, in der das Wasserstoffatom in 1-Stellung zusammen mit einem Wasserstoffatom in

2-Stellung durch eine geradkettige Alkylengruppe mit 3 oder 4 Kohlenstoffatomen ersetzt ist, oder eine Piperidinylgruppe, in der das Wasserstoffatom in 1-Stellung zusammen mit einem Wasserstoffatom in 3-Stellung durch eine geradkettige Alkylengruppe mit 2 bis 4 Kohlenstoffatomen ersetzt ist, oder eine Piperidinylgruppe, in der das Wasserstoffatom in 1-Stellung zusammen mit einem Wasserstoffatom in 4-Stellung durch eine geradkettige Alkylengruppe mit 1 bis 3 Kohlenstoffatomen ersetzt ist, wobei die an die 4-Stellung gebundene Methylengruppe einer Ethylenkette durch eine Hydroxymethylen- oder Carbonylgruppe ersetzt sein kann und zusätzlich das Stickstoffatom dieser vorstehend erwähnten bicyclischen Gruppen, welche im Kohlenstoffgerüst durch eine oder zwei Alkylgruppen substituiert sein können, durch Boran komplexiert oder durch eine im Phenylkern gegebenenfalls durch 1 bis 2 Methoxygruppen substituierte Benzylgruppe quarternisiert sein kann, und

C eine Alkylengruppe mit 1 bis 6 Kohlenstoffatomen, in der eine Methylengruppe durch ein Sauerstoff- oder Schwefelatom oder durch eine Sulfinyl-, Sulfonyl- oder $-NR_4$-Gruppe ersetzt sein kann, oder in der eine Ethylengruppe durch eine $-CONR_5-$ oder $-NR_5CO$-Gruppe ersetzt sein kann, wobei $R_4$ und $R_5$ wie vorstehend erwähnt definiert sind,

eine Alkenylengruppe mit 2 bis 6 Kohlenstoffatomen,

eine über die Carbonylgruppe mit dem Rest B verbundene Alkylencarbonylgruppe mit insgesamt 2 bis 6 Kohlenstoffatomen,

eine Arylengruppe,

eine Pyridinylen-, Pyrimidinylen-, Pyrazinylen- oder Pyridazinylengruppe, in denen eine oder zwei -CH=N-Gruppen jeweils durch eine -CO-NH-Gruppe ersetzt sein können und eines der Stickstoffatome statt an ein Wasserstoffatom auch an den Rest B gebunden sein kann, sofern dieser nicht eine Bindung bedeutet oder nicht mit einem Heteroatom oder einer Carbonylgruppe an den Rest C anschließt, wobei die vorstehend erwähnten heterocyclischen Gruppen zusätzlich durch eine oder zwei Alkylgruppen substituiert sein können,

eine gegebenenfalls durch eine oder zwei Alkylgruppen substituierte 5- bis 7-gliedrige Cycloalkylengruppe,

eine Cyclohexylengruppe, in der zwei durch drei Bindungen getrennte Kohlenstoffatome zusätzlich durch eine geradkettige Alkylengruppe mit 1 bis 3 Kohlenstoffatomen verknüpft sind, wobei diese bicyclischen Gruppen jeweils außerdem durch ein bis zwei Alkylgruppen substituiert sein können, oder

C zusammen mit A und B eine gegebenenfalls jeweils durch eine oder zwei Alkylgruppen substituierte Pyridyl- oder 1-(4-Pyridyl)-piperidinylgruppe oder eine Piperidinylgruppe, in der das Wasserstoffatom in 1-Stellung zusammen mit einem Wasserstoffatom in 4-Stellung durch eine geradkettige Alkylengruppe mit 1 bis 3 Kohlenstoffatomen ersetzt ist, wobei die an die 4-Stellung gebundene Methylengruppe einer Ethylenkette durch eine Hydroxymethylen- oder Carbonylgruppe ersetzt sein kann und zusätzlich das Stickstoffatom dieser vorstehend erwähnten bicyclischen Gruppen, welche im Kohlenstoffgerüst durch eine oder zwei Alkylgruppen substituiert sein können, durch Boran komplexiert oder durch eine im Phenylkern gegebenenfalls durch 1 bis 2 Methoxygruppen substituierte Benzylgruppe quarternisiert sein kann, oder

C, wenn B eine Bindung darstellt, auch

(a) eine Indanylen-, Naphthylen-, 1,2,3,4-Tetrahydronaphthylen- oder Benzosuberanylengruppe, in denen jeweils einer der Ringe an den Rest A und der andere der Ringe an den cyclischen Rest der allgemeinen Formel I gebunden ist, wobei die gesättigten Ringe jeweils durch 1 oder 2 Alkylgruppen substituiert sein können und die aromatischen Ringe jeweils durch ein Fluor-, Chlor-, Brom- oder Jodatom, durch eine Alkyl-, Trifluormethyl-, Hydroxy-, Alkoxy-, Alkylsulfenyl-, Alkylsulfinyl-, Alkylsulfonyl- oder Cyanogruppe substituiert sein können,

(b) eine gegebenenfalls durch den Rest $R_e$ substituierte Cycloalkylengruppe mit 5 bis 7 Kohlenstoffatomen, welche zusätzlich durch 1 bis 4 Alkylgruppen substituiert sein kann und in denen im Cycloalkylenteil jeweils eine $>$CH-Einheit durch ein Stickstoffatom, welches mit dem Rest A verknüpft ist, ersetzt ist, wobei $R_e$ wie vorstehend erwähnt definiert ist, oder

(c) eine Piperidinylengruppe, in der zwei Wasserstoffatome am Kohlenstoffgerüst durch eine geradkettige Alkylenbrücke ersetzt sind, wobei diese Brücke 2 bis 6 Kohlenstoffatome enthält, wenn die zwei Wasserstoffatome sich am selben Kohlenstoffatom befinden, oder 1 bis 5 Kohlenstoffatome enthält, wenn sie zwei Wasserstoffatome an benachbarten Kohlenstoffatomen ersetzt, oder 1 bis 4 Kohlenstoffatome enthält, wenn sie zwei Wasserstoffatome an Kohlenstoffatomen ersetzt, die durch ein weiteres Atom getrennt sind, oder 1 bis 3 Kohlenstoffatome enthält, wenn sie zwei Wasserstoffatome an Kohlenstoffatomen ersetzt, die durch zwei Atome getrennt sind, wobei die vorstehenden Gruppen jeweils durch eine oder zwei Alkylgruppen substituiert sein können, darstellen,

der zweite der Reste $R_a$ bis $R_d$ eine Gruppe der Formel

F - E - D -,

in der

D eine Alkylengruppe mit 1 bis 6 Kohlenstoffatomen, in der eine Methylengruppe- durch ein Sauerstoff- oder Schwefelatom, durch eine Sulfinyl-, Sulfonyl- oder -$NR_4$-Gruppe ersetzt sein kann, oder in der eine Ethylengruppe durch eine -CO-$NR_5$- oder -$NR_5$-CO-Gruppe ersetzt sein kann, wobei $R_4$ und $R_5$ wie vorstehend erwähnt definiert sind,

eine Arylengruppe,

eine Pyridinylen-, Pyrimidinylen-, Pyrazinylen- oder Pyridazinylengruppe, in denen eine oder zwei -CH = N-Gruppen jeweils durch eine -CO-NH-Gruppe ersetzt sein können und eines der Stickstoffatome statt an ein Wasserstoffatom auch an den Rest E gebunden sein kann, sofern dieser nicht eine Bindung bedeutet oder nicht mit einem Heteroatom oder einer Carbonylgruppe an den Rest D anschließt, wobei die vorstehend erwähnten heterocyclischen Gruppen zusätzlich durch eine oder zwei Alkylgruppen substituiert sein können,

eine Indanylen-, Naphthylen-, 1,2,3,4-Tetrahydronaphthylen- oder Benzosuberanylengruppe, in denen jeweils einer der Ringe an den Rest E und der andere der Ringe an den cyclischen Rest der allgemeinen Formel I gebunden ist, wobei die gesättigten Ringe jeweils durch 1 oder 2 Alkylgruppen substituiert sein können und die aromatischen Ringe jeweils durch ein Fluor-, Chlor-, Brom- oder Jodatom, durch eine Alkyl-, Trifluormethyl-, Hydroxy-, Alkoxy-, Alkylsulfenyl-, Alkylsulfinyl-, Alkylsulfonyl- oder Cyanogruppe substituiert sein können,

eine gegebenenfalls durch eine oder zwei Alkylgruppen substituierte Cycloalkylengruppe mit 4 bis 7 Kohlenstoffatomen,

eine gegebenenfalls durch eine oder zwei Alkylgruppen substituierte Cycloalkylengruppe mit 5 bis 7 Kohlenstoffatomen, in der eine >CH-Einheit durch ein Stickstoffatom ersetzt ist, wobei außerdem in den vorstehend erwähnten 5- bis 7-gliedrigen Ringen jeweils eine zu einem Stickstoffatom benachbarte Methylengruppe durch eine Carbonylgruppe ersetzt sein kann,

eine gegebenenfalls durch eine oder zwei Alkylgruppen substituierte Piperazinylengruppe, in der jeweils eine zu einem Stickstoffatom benachbarte Methylengruppe durch eine Carbonylgruppe ersetzt sein kann,

eine Cyclohexylengruppe, in der zwei durch drei Bindungen getrennte Kohlenstoffatome zusätzlich durch eine geradkettige Alkylengruppe mit 1 bis 3 Kohlenstoffatomen verknüpft sind, wobei diese bicyclischen Gruppen jeweils außerdem durch ein bis zwei Alkylgruppen substituiert sein können,

oder, wenn E eine cyclische Iminogruppe darstellt, auch eine Alkylencarbonylgruppe mit insgesamt 2 bis 6 Kohlenstoffatomen, wobei die Carbonylgruppe jeweils an das Stickstoffatom der cyclischen Iminogruppe der Gruppe E gebunden ist,

oder auch, falls E keine Bindung darstellt, eine Bindung,

E eine Bindung,

eine Alkylengruppe mit 1 bis 6 Kohlenstoffatomen, die durch eine oder zwei Alkylgruppen mit jeweils 1 bis 6 Kohlenstoffatomen, durch eine Hydroxy-, Amino- oder Arylgruppe, durch eine Alkoxygruppe mit 1 bis 6 Kohlenstoffatomen, durch eine $HNR_6$- oder N-Alkyl-$NR_6$-Gruppe substituiert sein kann, wobei

$R_6$ eine Alkylcarbonyl- oder Alkylsulfonylgruppe mit jeweils 1 bis 6 Kohlenstoffatomen im Alkylteil, eine Alkyloxycarbonylgruppe mit insgesamt 2 bis 5 Kohlenstoffatomen, eine Cycloalkylcarbonyl- oder Cycloalkylsulfonylgruppe mit jeweils 5 bis 7 Kohlenstoffatomen im Cycloalkylteil, eine Arylalkylcarbonyl-, Arylalkylsulfonyl-, Arylalkoxycarbonyl-, Arylcarbonyl- oder Arylsulfonylgruppe darstellt,

eine Alkenylengruppe mit 2 bis 6 Kohlenstoffatomen,

eine Arylengruppe,

eine gegebenenfalls durch eine oder zwei Alkylgruppen substituierte Pyridinylen-, Pyrimidinylen-, Pyrazinylen- oder Pyridazinylengruppe,

eine gegebenenfalls durch eine oder zwei Alkylgruppen substituierte Cycloalkylengruppe mit 5 bis 7 Kohlenstoffatomen, in der eine >CH-Einheit durch ein Stickstoffatom, welches mit einem Kohlenstoffatom des Restes D verknüpft ist, ersetzt ist,

eine gegebenenfalls durch eine oder zwei Alkylgruppen mit jeweils 1 bis 6 Kohlenstoffatomen, durch eine Hydroxy-, Amino- oder Arylgruppe, durch eine Alkoxygruppe mit 1 bis 6 Kohlenstoffatomen, durch eine $HNR_6$- oder N-Alkyl-$NR_6$-Gruppe substituierte Cycloalkylengruppe mit 4 bis 7 Kohlenstoffatomen im Cycloalkylenteil, wobei $R_6$ wie vorstehend definiert ist,

oder auch, falls D keine Bindung darstellt, eine über den Rest W mit dem Rest D verknüpfte Alkylengruppe, in der W wie eingangs erwähnt definiert ist und die Alkylengruppe zusätzlich durch eine oder zwei Alkylgruppen mit jeweils 1 bis 6 Kohlenstoffatomen, durch eine Hydroxy-, Amino- oder Arylgruppe, durch eine Alkoxygruppe mit 1 bis 6 Kohlenstoffatomen, durch eine $HNR_6$- oder N-Alkyl-$NR_6$-Gruppe substituiert sein kann, wobei das Heteroatom des zusätzlichen Substituenten durch mindestens 2 Kohlenstoffatome von

einem Heteroatom des Restes W getrennt ist und $R_6$ wie vorstehend definiert ist, sowie mit der Maßgabe, daß D zusammen mit E keine $-(CH_2)_n-CONH-CH_2CH_2-$Gruppe darstellt, wobei

n die Zahl 1,2,3 oder 4 bedeutet und der mit dem Stickstoffatom verbundene Ethylenteil gegebenenfalls wie vorstehend erwähnt substituiert sein kann, und

F eine Carbonylgruppe, die durch eine Hydroxygruppe, durch eine Alkoxygruppe mit 1 bis 8 Kohlenstoffatomen, durch eine Arylalkoxygruppe oder durch eine $R_7O$-Gruppe substituiert ist, wobei

$R_7$ eine Cycloalkylgruppe mit 5 bis 7 Kohlenstoffatomen oder eine Cycloalkylalkylgruppe mit 5 bis 7 Kohlenstoffatomen im Cycloalkylteil, in denen jeweils die Cycloalkylgruppe durch eine Alkylgruppe, durch eine Alkylgruppe und durch 1 bis 3 Methylgruppen oder durch eine Alkoxygruppe substituiert und zusätzlich eine Methylengruppe in einem 5 bis 7-gliedrigen Cycloalkylteil durch ein Sauerstoffatom oder durch eine Alkyliminogruppe ersetzt sein kann, eine Benzocycloalkylgruppe mit 9 bis 12 Kohlenstoffatomen oder eine Arylgruppe darstellt,

eine Phosphono-, O-Alkyl-phosphono-, O,O'-Dialkyl-phosphono-, Tetrazol-5-yl- oder $R_8CO-O-CHR_9-O-CO-$Gruppe darstellen, wobei

$R_8$ eine Alkyl- oder Alkoxygruppe mit jeweils 1 bis 8 Kohlenstoffatomen, eine Cycloalkyl- oder Cycloalkoxygruppe mit jeweils 5 bis 7 Kohlenstoffatomen im Cycloalkylteil, eine Arylalkyl- oder Arylalkoxygruppe und

$R_9$ ein Wasserstoffatom oder eine Alkylgruppe darstellen,

und der kürzeste Abstand zwischen dem Rest F und dem von dem Rest F am weitesten entfernten Stickstoffatom der Gruppe A-B-C- mindestens 11 Bindungen beträgt,

der dritte der Reste $R_a$ bis $R_d$ ein Wasserstoffatom, eine Alkyl-, Trifluormethyl-, Aryl- oder Arylalkylgruppe und

der vierte der Reste $R_a$ bis $R_d$ ein Wasserstoffatom, eine Alkyl- oder Arylgruppe bedeuten,

wobei, soweit nichts anderes erwähnt wurde,

unter den bei der Definition der vorstehenden Reste erwähnten Arylteilen eine Phenylgruppe, die jeweils durch $R_{10}$ monosubstituiert, durch $R_{11}$ mono-, di- oder trisubstituiert oder durch $R_{10}$ monosubstituiert und zusätzlich durch $R_{11}$ mono- oder disubstituiert sein kann, wobei die Substituenten gleich oder verschieden sein können und

$R_{10}$ eine Cyano-, Carboxy-, Aminocarbonyl-, Alkylaminocarbonyl-, Dialkylaminocarbonyl-, Alkoxycarbonyl-, Alkylcarbonyl-, Alkylsulfenyl-, Alkylsulfinyl-, Alkylsulfonyl-, Alkylsulfonyloxy-, Trifluormethyl-, Trifluormethoxy-, Nitro-, Amino-, Alkylamino-, Dialkylamino-, Alkylcarbonylamino-, Phenylalkylcarbonylamino-, Phenylcarbonylamino-, Alkylsulfonylamino-, Phenylalkylsulfonylamino-, Phenylsulfonylamino-, N-Alkyl-alkylcarbonylamino-, N-Alkyl-phenylalkylcarbonylamino-, N-Alkyl-phenylcarbonylamino-, N-Alkyl-alkylsulfonylamino-, N-Alkyl-phenylalkylsulfonylamino-, N-Alkyl-phenylsulfonylamino-, Aminosulfonyl-, Alkylaminosulfonyl- oder Dialkylaminosulfonylgruppe und

$R_{11}$ eine Alkyl-, Hydroxy- oder Alkoxygruppe, ein Fluor-, Chlor-, Brom- oder Jodatom darstellen, wobei zwei Reste $R_{11}$ sofern diese an benachbarte Kohlenstoffatome gebunden sind, auch eine Alkylengruppe mit 3 bis 6 Kohlenstoffatomen, eine 1,3-Butadien-1,4-diylengruppe oder eine Methylendioxygruppe darstellen können,

unter den bei der Definition der vorstehenden Resten erwähnten Arylenteilen eine Phenylengruppe die jeweils durch $R_{10}$ monosubstituiert, durch $R_{11}$ mono- oder disubstituiert oder durch $R_{10}$ monosubstituiert und zusätzlich durch $R_{11}$ monosubstituiert sein kann, wobei die Substituenten gleich oder verschieden sein können und wie vorstehend erwähnt definiert sind,

zu verstehen ist, sowie, soweit nichts anderes erwähnt wurde, die vorstehend erwähnten Alkyl-, Alkylen- oder Alkoxyteile jeweils 1 bis 4 Kohlenstoffatome enthalten können, und jedes Kohlenstoffatom in den vorstehend erwähnten Alkylen- und Cycloalkylenteilen höchstens mit einem Heteroatom verknüpft ist, deren Tautomere, deren Stereoisomere und Salze.

Besonders bevorzugte Verbindungen der obigen allgemeinen Formel I sind mit Ausnahme von

1-(4-Amidino-phenyl)-3-[4-(2-carboxy-ethyl)-cyclohexyl]-imidazolidin-2-on,
1-(4-Amidino-phenyl)-3-[4-(2-methoxycarbonyl-ethyl)-cyclohexyl]-imidazolidin-2-on,
1-(4-Cyano-phenyl)-3-[4-(2-methoxycarbonyl-ethyl)-cyclohexyl]-imidazolidin-2-on,
1-(4-Amidino-phenyl)-3-[4-(2-phosphono-ethyl)-phenyl]-imidazolidin-2-on,
1-(4-Amidino-phenyl)-3-[4-[2-(O-methyl-phosphono)-ethyl]-phenyl]-imidazolidin-2-on,
1-(4-Cyano-phenyl)-3-[4-(2-phosphono-ethyl)-phenyl]-imidazolidin-2-on,
1-(4-Cyano-phenyl)-3-[4-[2-(0-methyl-phosphono)-ethyl]-phenyl]-imidazolidin-2-on,
1-(4-Aminomethyl-cyclohexyl)-3-[4-(2-carboxy-ethyl)-phenyl]-imidazolidin-2-on,
1-(4-Aminomethyl-cyclohexyl)-3-[4-(2-methoxycarbonyl-ethyl)-phenyl]-imidazolidin-2-on,
1-(4-Amidino-phenyl)-3-[4-(2-carboxy-ethyl)-phenyl]-4-phenyl-3H-imidazol-2-on,

1-(4-Amidino-phenyl)-3-[4-(2-methoxycarbonyl-ethyl)-phenyl]-4-phenyl-3H-imidazol-2-on,
1-(4-Amidino-phenyl)-3-[4-(2-carboxy-ethyl)-phenyl]-4-phenyl-imidazolidin-2-on,
1-(4-Amidino-phenyl)-3-[4-(2-methoxycarbonyl-ethyl)-phenyl]-4-phenyl-imidazolidin-2-on,
4-(4-Amidino-phenyl)-1-[4-(2-carboxy-ethyl)-phenyl]-3-phenyl-3H-imidazol-2-on,
4-(4-Amidino-phenyl)-1-[4-(2-methoxycarbonyl-ethyl)-phenyl]-3-phenyl-3H-imidazol-2-on,
4-(4-Cyano-phenyl)-1-[4-(2-methoxycarbonyl-ethyl)-phenyl]-3-phenyl-3H-imidazol-2-on,
4-(4-Amidino-phenyl)-1-[4-(2-carboxy-ethyl)-phenyl]-5-methyl-3-phenyl-3H-imidazol-2-on,
4-(4-Amidino-phenyl)-1-[4-(2-methoxycarbonyl-ethyl)-phenyl]-5-methyl-3-phenyl-3H-imidazol-2⁻on,
4-(4-Cyano-phenyl)-1-[4-(2-methoxycarbonyl-ethyl)-phenyl]-5-methyl-3-phenyl-3H-imidazol-2-on,
4-(4-Amidino-phenyl)-1-[4-(2-carboxy-ethyl)-phenyl]-3-phenyl-imidazolidin-2-on,
4-(4-Amidino-phenyl)-1-[4-(2-methoxycarbonyl-ethyl)-phenyl]-3-phenyl-imidazolidin-2-on,
1-(4-Amidino-phenyl)-4-[4-(2-carboxy-ethyl)-phenyl]-3-phenyl-3H-imidazol-2-on,
1-(4-Amidino-phenyl)-4-[4-(2-methoxycarbonyl-ethyl)-phenyl]-3-phenyl-3H-imidazol-2-on,
1-(4-Cyano-phenyl)-4-[4-(2-methoxycarbonyl-ethyl)-phenyl]-3-phenyl-3H-imidazol-2-on,
1-(4-Amidino-phenyl)-4-[4-(2-carboxy-ethyl)-phenyl]-3-phenyl-imidazolidin-2-on,
1-(4-Amidino-phenyl)-4-[4-(2-methoxycarbonyl-ethyl)-phenyl]-3-phenyl-imidazolidin-2-on,
1-(4-Amidino-phenyl)-4-[4-(2-carboxy-ethyl)-phenyl]-5-methyl-3-phenyl-3H-imidazol-2-on,
1-(4-Amidino-phenyl)-4-[4-(2-methoxycarbonyl-ethyl)-phenyl]-5-methyl-3-phenyl-3H-imidazol-2-on,
1-(4-Cyano-phenyl)-4-[4-(2-methoxycarbonyl-ethyl)-phenyl]-5-methyl-3-phenyl-3H-imidazol-2-on,
1-(4-Amidino-phenyl)-3-[4-(2-butyloxycarbonyl-ethyl)-phenyl]-imidazolidin-2-on,
2-(4-Amidino-phenyl)-4-[4-(2-isopropyloxycarbonyl-ethyl)-phenyl]-5-methyl-4H-1,2,4-triazol-3-on,
4-[4-(2-Isopropyloxycarbonyl-ethyl)-phenyl]-2-(4-methoxycarbonylamidino-phenyl)-5-methyl-4H-1,2,4-triazol-3-on,
1-(4-Amidino-phenyl)-3-[4-(2-ethoxycarbonyl-ethyl)-phenyl]-3H-imidazol-2-on,
1-(4-Ethoxycarbonylamidino-phenyl)-3-[4-(2-ethoxycarbonyl-ethyl)-phenyl]-3H-imidazol-2-on,
1-(4-Ethoxycarbonylamidino-phenyl)-3-[4-(2-ethoxycarbonyl-ethyl)-phenyl]-imidazolidin-2-on,
1-(4-Methoxycarbonylamidino-phenyl)-3-[4-(2-methoxycarbonyl-ethyl)-cyclohexyl]-imidazolidin-2-on,
4-[4-(2-Isobutyloxycarbonyl-ethyl)-phenyl]-2-(4-methoxycarbonylamidino-phenyl)-5-methyl-4H-1,2,4-triazol-3-on,
2-(4-Amidino-phenyl)-4-[4[(2-isobutyloxycarbonyl-ethyl)-phenyl]-5-methyl-4H-1,2,4-triazol-3-on,
1-(1-Amino-5-indanyl)-3-[4-(2-carboxy-ethyl)-phenyl]-imidazolidin-2-on,
1-(1-Amino-5-indanyl)-3-[4-(2-methoxycarbonyl-ethyl)-phenyl}-imidazolidin-2-on,
1-(1-Amino-1,2,3,4-tetrahydro-6-naphthyl)-3-[4-(2-carboxy-ethyl)-phenyl]-imidazolidin-2-on,
1-(1-Amino-1,2,3,4-tetrahydro-6-naphthyl)-3-[4-(2-methoxycarbonyl-ethyl)-phenyl]-imidazolidin-2-on,
1-(4-Amino-cyclohexyl)-3-[4-(3-carboxy-propyl)-phenyl)-imidazolidin-2-on,
1-(4-Amino-cyclohexyl)-3-[4-(3-methoxycarbonyl-propyl)-phenyl]-imidazolidin-2-on,
1-(4-Amino-cyclohexyl)-3-[4-(2-carboxy-ethyl)-phenyl]-imidazolidin-2-on,
1-(4-Amino-cyclohexyl)-3-[4-(2-methoxycarbonyl-ethyl)-phenyl]-imidazolidin-2-on,
1-(4-Aminomethyl-phenyl)-3-[3-(2-carboxy-ethyl)-phenyl]-imidazolidin-2-on,
1-(4-Aminomethyl-phenyl)-3-[3-(2-methoxycarbonyl-ethyl)-phenyl]-imidazolidin-2-on,
1-[4-(1-Amino-cyclopropyl)-phenyl]-3-[4-(2-carboxy-ethyl)-phenyl]-imidazolidin-2-on,
1-[4-(1-Amino-cyclopropyl)-phenyl]-3-[4-(2-methoxycarbonyl-ethyl)-phenyl]-imidazolidin-2-on,
1-[4-(1-Amino-cyclopentyl)-phenyl]-3-[4-(2-carboxy-ethyl)-phenyl]-imidazolidin-2-on,
1-[4-(1-Amino-cyclopentyl)-phenyl]-3-[4-[(2-methoxycarbonyl-ethyl)-phenyl]-imidazolidin-2-on,
2-(4-Amidino-phenyl)-5-ethyl-4-[4-(2-isopropyloxycarbonyl-ethyl)-phenyl]-4H-1,2,4-triazol-3-on,
1-(4-Amidino-phenyl)-3-[4-(2-isopropyloxycarbonyl-ethyl)-phenyl]-4-methyl-3H-imidazol-2-on,
4-(4-Amidino-phenyl)-2-[4-(2-isopropyloxycarbonyl-ethyl)-phenyl]-4H-1,2,4-triazol-3-on,
1-(4-Amidino-phenyl)-3-[4-(2-isopropyloxycarbonyl-ethyl)-phenyl]-imidazolidin-2-on,
1-(4-Amidino-phenyl)-3-[4-(2-isopropyloxycarbonyl-ethyl)-phenyl]-3H-imidazol-2-on,
1-(4-Amidino-phenyl)-3-[4-(2-isopropyloxycarbonyl-ethyl)-phenyl]-imidazolidin-2,4-dion,
2-(4-Amidino-phenyl)-4-[4-(2-phosphono-ethyl)-phenyl]-5-methyl-4H-1,2,4-triazol-3-on,
2-(4-Amidino-phenyl)-4-[4-[2-(0-methyl-phosphono)-ethyl]-phenyl]-5-methyl-4H-1,2,4-triazol-3-on,
2-(4-Amidino-phenyl)-4-[4-(2-carboxy-ethyl)-phenyl]-5-phenyl-4H-1,2,4-triazol-3-on,
2-(4-Amidino-phenyl)-4-[4-(2-methoxycarbonyl-ethyl)-phenyl]-5-phenyl-4H-1,2,4-triazol-3-on,
3-(4-Amidino-phenyl)-1-[4-(2-carboxy-ethyl)-phenyl]-4-trifluormethyl-3H-imidazol-2-on,
3-(4-Amidino-phenyl)-1-[4-(2-methoxycarbonyl-ethyl)-phenyl]-4-trifluormethyl-3H-imidazol-2-on,
2-(4-Amidino-phenyl)-4-[4-(2-carboxy-ethyl)-phenyl]-5-trifluormethyl-4H-1,2,4-triazol-3-on,
2-(4-Amidino-phenyl)-4-[4-(2-methoxycarbonyl-ethyl)-phenyl]-5-trifluormethyl-4H-1,2,4-triazol-3-on und

3-(4-Cyano-phenyl)-1-(4-ethoxycarbonyl-butyl)-3H-imidazo-[4,5-b]pyridin-2-on,
sowie mit den Maßgaben, daß

(i) X eine am Stickstoffatom durch eine Cyanogruppe substituierte Carbiminogruppe,

(ii) Y eine gegebenenfalls durch eine oder zwei Alkylgruppen substituierte 1,2-Cyclopentylen- oder 1,2-Cyclohexylengruppe,

(iii) A eine durch eine $R_1$-CO-O-($R_2$C$R_3$)-O-CO-Gruppe substituierte Amidinogruppe,

(iv) B eine gegebenenfalls durch eine oder zwei Alkylgruppen substituierte Pyrrolidinylengruppe,
eine gegebenenfalls durch eine bis vier Alkylgruppen substituierte Piperidinylengruppe, welche zusätzlich in 4-Stellung durch eine Cyano-, Aminocarbonyl-, Alkylaminocarbonyl-, Dialkylaminocarbonyl-, Alkylcarbonyl- oder Alkoxygruppe substituiert sein kann,
eine Piperidinylengruppe, in der zwei Wasserstoffatome am Kohlenstoffgerüst durch eine geradkettige Alkylenbrücke ersetzt sind, wobei diese Brücke 2 bis 5 Kohlenstoffatome enthält, wenn die zwei Wasserstoffatome sich am selben Kohlenstoffatom befinden, oder 1 bis 4 Kohlenstoffatome enthält, wenn sie zwei Wasserstoffatome an benachbarten Kohlenstoffatomen ersetzt, oder 2 oder 3 Kohlenstoffatome enthält, wenn sie zwei Wasserstoffatome an Kohlenstoffatomen ersetzt, die durch ein weiteres Atom getrennt sind, oder 1 oder 2 Kohlenstoffatome enthält, wenn sie zwei Wasserstoffatome an Kohlenstoffatomen ersetzt, die durch zwei Atome getrennt sind,
eine gegebenenfalls durch eine oder zwei Alkylgruppen substituierte 1,2,3,6-Tetrahydro-pyridinylengruppe,
eine gegebenenfalls durch eine oder zwei Alkylgruppen substituierte Azacycloheptylengruppe,
eine gegebenenfalls durch eine oder zwei Alkylgruppen substituierte Piperazinylengruppe,
eine gegebenenfalls durch eine oder zwei Alkylgruppen substituierte 2-Oxo-piperazinylengruppe,
eine Alkylenoxygruppe,
eine gegebenenfalls durch eine oder zwei Alkylgruppen substituierte Cycloalkylengruppe mit 3 bis 6 Kohlenstoffatomen oder
B zusammen mit A eine gegebenenfalls durch eine oder zwei Alkylgruppen substituierte Pyridylgruppe oder eine Piperidinylgruppe, in der das Wasserstoffatom in 1-Stellung zusammen mit einem Wasserstoffatom in 4-Stellung durch eine Methylen-, Ethylen- oder 1,3-Propylengruppe ersetzt ist, wobei die an die 4-Stellung gebundene Methylengruppe einer Ethylenkette durch eine Carbonyl- oder Hydroxymethylengruppe ersetzt sein kann und zusätzlich das Stickstoffatom der vorstehend erwähnten bicyclischen Gruppen, welche im Kohlenstoffgerüst zusätzlich durch eine oder zwei Alkylgruppen substituiert sein können, durch Boran komplexiert oder durch eine Benzylgruppe quarternisiert sein kann,

(v) C eine Alkylenoxygruppe,
eine Cyclohexylengruppe, in der zwei durch drei Bindungen getrennte Kohlenstoffatome zusätzlich durch eine Methylen- oder Ethylengruppe verknüpft sein können,
eine gegebenenfalls durch eine oder zwei Alkylgruppen substituierte Piperidinylengruppe oder
eine Indanylen-, Naphthylen- oder 1,2,3,4-Tetrahydro-naphthylengruppe oder
C zusammen mit A und B eine gegebenenfalls jeweils durch eine oder zwei Alkylgruppen substituierte Pyridyl- oder 1-(4-Pyridyl)-piperidinylgruppe oder eine Piperidinylgruppe, in der das Wasserstoffatom in 1-Stellung zusammen mit einem Wasserstoffatom in 4-Stellung durch eine Methylen- oder Ethylengruppe ersetzt ist und zusätzlich das Stickstoffatom dieser vorstehend erwähnten bicyclischen Gruppen, welche im Kohlenstoffgerüst zusätzlich durch eine oder zwei Alkylgruppen substituiert sein können, durch Boran komplexiert oder durch eine Benzylgruppe quarternisiert sein kann, oder
C eine Alkylengruppe mit 1 bis 6 Kohlenstoffatomen und B eine Bindung,

(vi) D eine 1,3-Arylengruppe,
eine gegebenenfalls durch eine oder zwei Alkylgruppen substituierte Cycloalkylengruppe mit 5 bis 7 Kohlenstoffatomen,
eine Cyclohexylengruppe, in der zwei durch drei Bindungen getrennte Kohlenstoffatome zusätzlich durch eine Methylen- oder Ethylengruppe verknüpft sind,
eine Indanylen-, Naphthylen- oder 1,2,3,4-Tetrahydro-naphthylengruppe,

(vii) E eine geradkettige Alkylengruppe, die durch eine Alkylgruppe mit 1 bis 6 Kohlenstoffatomen, durch eine Arylgruppe oder durch eine $HNR_6$-Gruppe substituiert ist,
eine über das Sauerstoffatom mit dem Rest D verknüpfte Alkylenoxygruppe, die durch eine Alkylgruppe mit 1 bis 6 Kohlenstoffatomen, durch eine Aryl-, Hydroxy- oder Aminogruppe, durch eine Alkoxygruppe mit 1 bis 6 Kohlenstoffatomen oder durch eine $HNR_6$-Gruppe substituiert ist, oder
eine gegebenenfalls durch eine oder zwei Alkylgruppen substituierte Piperidinylengruppe,

(viii) F eine Alkoxycarbonylgruppe mit insgesamt 3 bis 7 Kohlenstoffatomen, eine $R_7$O-CO-, Phosphono-, O-Alkyl-phosphono-, O,O'-Dialkyl-phosphono- oder $R_8$CO-O-CH$R_9$-O-CO-Gruppe und

(ix) der dritte der Reste $R_a$ bis $R_d$ eine Trifluormethyl- oder Arylgruppe

darstellt, wobei mindestens eine der obigen Bedingungen (i) bis (ix) erfüllt sein müssen, diejenigen in denen X eine am Stickstoffatom durch eine Cyanogruppe substituierte Carbiminogruppe, eine Carbonyl- oder Sulfonylgruppe,

Y eine gegebenenfalls durch $R_c$ oder $R_c$ und $R_d$ substituierte $-CH_2CH_2-$, $-CH_2CH_2CH_2-$, -CH = CH-, $-CH_2CO-$ oder $-COCH_2$-Gruppe,

eine gegebenenfalls durch $R_c$ substituierte -N = CH- oder -CH = N-Gruppe,

eine 1,2-Arylengruppe,

eine 1,2-Phenylengruppe, in der eine oder zwei Methingruppen jeweils durch ein Stickstoffatom ersetzt sind, wobei die vorstehend erwähnten heterocyclischen Gruppen zusätzlich durch eine oder zwei Alkylgruppen substituiert sein können, oder

eine gegebenenfalls durch eine oder zwei Alkylgruppen substituierte 1,2-Cyclopentylen- oder 1,2-Cyclohexylengruppe,

der erste der Reste $R_a$ bis $R_d$ eine Gruppe der Formel

A - B - C -,

in der

A eine gegebenenfalls durch eine Alkoxycarbonylgruppe mit insgesamt 2 oder 3 Kohlenstoffatomen, durch eine Benzyloxycarbonyl- oder $R_1$-CO-O-($R_2CR_3$)-O-CO-Gruppe substituierte Amidinogruppe, wobei

$R_1$ eine Alkylgruppe,

$R_2$ ein Wasserstoffatom oder eine Methylgruppe und

$R_3$ ein Wasserstoffatom darstellen,

eine Cyano-, Cyanomethyl-, Cyanoethyl-, Amino-, Aminomethyl-, Aminoethyl- oder Aminopropylgruppe oder, falls A an ein Stickstoffatom der Reste B oder C gebunden ist, ein Wasserstoffatom, eine Methyl-, Ethyl-, Benzyl-, tert.Butyloxycarbonyl-, Benzyloxycarbonyl-, Trifluoracetyl- oder $R_1$CO-O-($R_2CR_3$)-O-CO-Gruppe, wobei $R_1$ bis $R_3$ wie vorstehend erwähnt definiert sind,

B eine Bindung,

eine gegebenenfalls durch ein oder zwei Alkylgruppen substituierte Pyrrolidinylengruppe,

eine gegebenenfalls durch eine bis vier Alkylgruppen substituierte Piperidinylengruppe, welche zusätzlich in 4-Stellung durch eine Cyano-, Aminocarbonyl-, Alkylaminocarbonyl-, Dialkylaminocarbonyl-, Alkylcarbonyl- oder Alkoxygruppe substituiert sein kann,

eine Piperidinylengruppe, in der zwei Wasserstoffatome am Kohlenstoffgerüst durch eine geradkettige Alkylenbrücke ersetzt sind, wobei diese Brücke 2 bis 5 Kohlenstoffatome enthält, wenn die zwei Wasserstoffatome sich am selben Kohlenstoffatom befinden, oder 1 bis 4 Kohlenstoffatome enthält, wenn sie zwei Wasserstoffatome an benachbarten Kohlenstoffatomen ersetzt, oder 2 oder 3 Kohlenstoffatome enthält, wenn sie zwei Wasserstoffatome an Kohlenstoffatomen ersetzt, die durch ein weiteres Atom getrennt sind, oder 1 oder 2 Kohlenstoffatome enthält, wenn sie zwei Wasserstoffatome an Kohlenstoffatomen ersetzt, die durch zwei Atome getrennt sind,

eine gegebenenfalls durch eine oder zwei Alkylgruppen substituierte 1,2,3,6-Tetrahydro-pyridinylengruppe,

eine gegebenenfalls durch eine oder zwei Alkylgruppen substituierte Azacycloheptylengruppe,

eine gegebenenfalls durch eine oder zwei Alkylgruppen substituierte Piperazinylengruppe, wobei jeweils ein Stickstoffatom der bei der Definition des Restes B vorstehend erwähnten cyclischen Iminogruppen mit dem Rest A verknüpft ist,

eine gegebenenfalls durch eine oder zwei Alkylgruppen substituierte 2-Oxo-piperazinylengruppe, in der das Stickstoffatom in 4-Stellung mit dem Rest A verknüpft ist,

eine Alkylengruppe,

eine Alkylenoxygruppe, wobei das Sauerstoffatom mit dem Rest C verknüpft ist und die Stickstoffatome des Restes A jeweils durch mindestens zwei Kohlenstoffatome von dem Sauerstoffatom getrennt sind,

eine gegebenenfalls durch eine oder zwei Alkylgruppen substituierte Cycloalkylengruppe mit 3 bis 6 Kohlenstoffatomen oder

B zusammen mit A eine gegebenenfalls durch eine oder zwei Alkylgruppen substituierte Pyridylgruppe oder eine Piperidinylgruppe, in der das Wasserstoffatom in 1-Stellung zusammen mit einem Wasserstoffatom in 4-Stellung durch eine Methylen-, Ethylen- oder 1,3-Propylengruppe ersetzt ist, wobei die an die 4-Stellung gebundene Methylengruppe einer Ethylenkette durch eine Carbonyl- oder Hydroxymethylengruppe ersetzt sein kann und zusätzlich das Stickstoffatom der vorstehend erwähnten bicyclischen Gruppen, welche im Kohlenstoffgerüst zusätzlich durch eine oder zwei Alkylgruppen substituiert sein können, durch Boran komplexiert oder durch eine Benzylgruppe quarternisiert sein kann, und

C eine Alkylengruppe mit 1 bis 6 Kohlenstoffatomen,
eine Alkylenoxygruppe, wobei das Sauerstoffatom jeweils an ein Kohlenstoffatom des Restes B oder an eine der von B zusammen mit A gebildeten bicyclischen Gruppen gebunden ist und zwischen dem Sauerstoffatom der Alkylenoxygruppe und dem Stickstoffatom des cyclischen Restes der allgemeinen Formel I jeweils mindestens zwei Kohlenstoffatome stehen,
eine Alkenylengruppe mit 2 bis 4 Kohlenstoffatomen, wobei diese jeweils nicht über den Vinylenteil mit einem Heteroatom des Restes A, des Restes B oder des cyclischen Restes der allgemeinen Formel I verknüpft sein kann,
eine Cyclohexylengruppe, in der zwei durch drei Bindungen getrennte Kohlenstoffatome zusätzlich durch eine Methylen- oder Ethylengruppe verknüpft sein können,
eine Arylengruppe oder eine gegebenenfalls durch eine oder zwei Alkylgruppen substituierte Pyridinylen-, Pyrimidinylen-, Pyrazinylen- oder Pyridazinylengruppe oder,
falls B eine gegebenenfalls durch eine oder zwei Alkylgruppen substituierte Piperazinylengruppe darstellt, auch eine Alkylencarbonylgruppe, wobei die Carbonylgruppe mit einem Stickstoffatom der gegebenenfalls durch eine oder zwei Alkylgruppen substituierten Piperazinylengruppe des Restes B verknüpft ist, oder
C zusammen mit A und B eine gegebenenfalls jeweils durch eine oder zwei Alkylgruppen substituierte Pyridyl- oder 1-(4-Pyridyl)-piperidinylgruppe oder eine Piperidinylgruppe, in der das Wasserstoffatom in 1-Stellung zusammen mit einem Wasserstoffatom in 4-Stellung durch eine Methylen- oder Ethylengruppe ersetzt ist und zusätzlich das Stickstoffatom dieser vorstehend erwähnten bicyclischen Gruppen, welche im Kohlenstoffgerüst zusätzlich durch eine oder zwei Alkylgruppen substituiert sein können, durch Boran komplexiert oder durch eine Benzylgruppe quarternisiert sein kann, oder
C, wenn B eine Bindung darstellt, auch eine gegebenenfalls durch eine oder zwei Alkylgruppen substituierte Piperidinylengruppe, in der das Stickstoffatom mit dem Rest A verknüpft ist, oder eine Indanylen-, Naphthylen- oder 1,2,3,4-Tetrahydro-naphthylengruppe, wobei jeweils einer der Ringe an den Rest A und der andere der Ringe an den cyclischen Rest der allgemeinen Formel I gebunden ist, darstellen,
der zweite der Reste $R_a$ bis $R_d$ eine Gruppe der Formel

F - E - D -,

in der
D eine Alkylengruppe,
eine Arylengruppe,
eine gegebenenfalls durch eine oder zwei Alkylgruppen substituierte Pyridinylen-, Pyrimidinylen-, Pyrazinylen- oder Pyridazinylengruppe,
eine gegebenenfalls durch eine oder zwei Alkylgruppen substituierte Cycloalkylengruppe mit 5 bis 7 Kohlenstoffatomen,
eine Cyclohexylengruppe, in der zwei durch drei Bindungen getrennte Kohlenstoffatome zusätzlich durch eine Methylen- oder Ethylengruppe verknüpft sind,
eine Indanylen-, Naphthylen- oder 1,2,3,4-Tetrahydro-naphthylengruppe, wobei jeweils einer der Ringe an den Rest E und der andere der Ringe an den cyclischen Rest der allgemeinen Formel I gebunden ist,
oder, falls E eine gegebenenfalls durch eine oder zwei Alkylgruppen substituierte Piperidinylengruppe darstellt, eine Alkylencarbonylgruppe, wobei die Carbonylgruppe mit dem Stickstoffatom der gegebenenfalls durch eine oder zwei Alkylgruppen substituierten Piperidinylengruppe des Restes E verknüpft ist,
E eine Bindung,
eine Alkenylengruppe mit 2 bis 4 Kohlenstoffatomen,
eine Alkylengruppe, die durch eine Alkylgruppe mit 1 bis 6 Kohlenstoffatomen, durch eine Aryl-, Hydroxy- oder Aminogruppe, durch eine Alkoxygruppe mit 1 bis 6 Kohlenstoffatomen oder durch eine $HNR_6$-Gruppe substituiert sein kann, wobei
$R_6$ eine Alkylcarbonyl- oder Alkylsulfonylgruppe mit jeweils 1 bis 6 Kohlenstoffatomen im Alkylteil, eine Cycloalkylcarbonyl- oder Cycloalkylsulfonylgruppe mit jeweils 5 bis 7 Kohlenstoffatomen im Cycloalkylteil, eine Arylcarbonyl-, Arylsulfonyl-, Arylalkylcarbonyl- oder Arylalkylsulfonylgruppe darstellt,
eine über das Sauerstoffatom mit dem Rest D verknüpfte Alkylenoxygruppe, die durch eine Alkylgruppe mit 1 bis 6 Kohlenstoffatomen, durch eine Aryl-, Hydroxy- oder Aminogruppe, durch eine Alkoxygruppe mit 1 bis 6 Kohlenstoffatomen oder durch eine $HNR_6$-Gruppe substituiert sein kann, wobei das Heteroatom des zusätzlichen Substituenten durch mindestens 2 Kohlenstoffatome von dem Sauerstoffatom der Alkylenoxygruppe getrennt ist und $R_6$ wie vorstehend erwähnt definiert ist,
eine Arylengruppe,
eine gegebenenfalls durch eine oder zwei Alkylgruppen substituierte Cyclohexylengruppe

oder, falls D eine Alkylencarbonylgruppe darstellt, eine gegebenenfalls durch eine oder zwei Alkylgruppen substituierte Piperidinylengruppe, wobei das Stickstoffatom mit der Carbonylgruppe der Alkylencarbonylgruppe des Restes D verknüpft ist, und

F eine Carbonylgruppe, die durch eine Hydroxygruppe, durch eine Alkoxygruppe mit 1 bis 6 Kohlenstoffatomen, durch eine Arylalkoxygruppe oder durch eine $R_7$O-Gruppe substituiert ist, wobei

$R_7$ eine Cycloalkylgruppe mit 5 bis 7 Kohlenstoffatomen, eine Cycloylkylalkylgruppe mit 5 bis 7 Kohlenstoffatomen im Cycloalkylteil, eine Benzocycloalkylgruppe mit 9 bis 11 Kohlenstoffatomen oder eine Arylgruppe darstellt,

eine Phosphono-, O-Alkyl-phosphono-, O,O'-Dialkyl-phosphono-, Tetrazol-5-yl- oder $R_8$CO-O-CH$R_9$-O-CO-Gruppe darstellen, wobei

$R_8$ eine Cycloalkyl- oder Cycloalkoxygruppe mit jeweils 5 bis 7 Kohlenstoffatomen im Cycloalkylteil, eine Alkyl- oder Alkoxygruppe mit jeweils 1 bis 6 Kohlenstoffatomen und

$R_9$ ein Wasserstoffatom oder eine Alkylgruppe darstellen,

und der kürzeste Abstand zwischen dem Rest F und dem von dem Rest F am weitesten entfernten Stickstoffatom der Gruppe A-B-C- mindestens 11 Bindungen beträgt,

der dritte der Reste $R_a$ bis $R_d$ ein Wasserstoffatom, eine Alkyl-, Trifluormethyl-, Aryl- oder Arylalkylgruppe und

der vierte der Reste $R_a$ bis $R_d$ ein Wasserstoffatom oder eine Alkylgruppe bedeuten,

wobei, soweit nichts anderes erwähnt wurde,

unter den bei der Definition der vorstehenden Reste erwähnten Arylteilen eine Phenylgruppe, die jeweils durch $R_{10}$ monosubstituiert, durch $R_{11}$ mono- oder disubstituiert oder durch $R_{10}$ monosubstituiert und zusätzlich durch $R_{11}$ monosubstituiert sein kann, wobei die Substituenten gleich oder verschieden sein können und

$R_{10}$ eine Cyano-, Aminocarbonyl-, Alkylaminocarbonyl-, Dialkylaminocarbonyl-, Alkylcarbonyl-, Alkylsulfenyl-, Alkylsulfinyl-, Alkylsulfonyl-, Alkylsulfonyloxy-, Trifluormethyl-, Nitro-, Amino-, Alkylcarbonylamino-, N-Alkyl-alkylcarbonylamino-, Alkylsulfonylamino- oder N-Alkyl-alkylsulfonylaminogruppe und

$R_{11}$ eine Alkyl-, Hydroxy- oder Alkoxygruppe, ein Fluor-, Chlor- oder Bromatom darstellen, wobei zwei Reste $R_{11}$ sofern diese an benachbarte Kohlenstoffatome gebunden sind, auch eine Alkylengruppe mit 3 oder 4 Kohlenstoffatomen, eine 1,3-Butadien-1,4-diylengruppe oder eine Methylendioxygruppe darstellen können,

unter den bei der Definition der vorstehenden Resten erwähnten Arylenteilen eine Phenylengruppe die jeweils durch $R_{10}$ monosubstituiert, durch $R_{11}$ mono- oder disubstituiert oder durch $R_{10}$ monosubstituiert und zusätzlich durch $R_{11}$ monosubstituiert sein kann, wobei die Substituenten gleich oder verschieden sein können und wie vorstehend erwähnt definiert sind,

zu verstehen ist, sowie, soweit nichts anderes erwähnt wurde, die vorstehend erwähnten Alkyl-, Alkylen- oder Alkoxyteile jeweils 1 bis 4 Kohlenstoffatome enthalten können und jedes Kohlenstoffatom in den vorstehend erwähnten Alkylen- und Cycloalkylenteilen höchstens mit einem Heteroatom verknüpft ist,

insbesondere diejenigen Verbindungen mit Ausnahme von 1-(4-Amidino-phenyl)-3-[4-(2-carboxy-ethyl)-cyclohexyl]-imidazolidin-2-on,
1-(4-Amidino-phenyl)-3-[4-(2-methoxycarbonyl-ethyl)-cyclohexyl]-imidazolidin-2-on,
1-(4-Amidino-phenyl)-3-[4-(2-phosphono-ethyl)-phenyl]-imidazolidin-2-on,
1-(4-Amidino-phenyl)-3-[4-[2-(O-methyl-phosphono)-ethyl]-phenyl]-imidazolidin-2-on,
1-(4-Aminomethyl-cyclohexyl)-3-[4-(2-carboxy-ethyl)-phenyl]-imidazolidin-2-on,
1-(4-Aminomethyl-cyclohexyl)-3-[4-(2-methoxycarbonyl-ethyl)-phenyl]-imidazolidin-2-on,
1-(4-Amidino-phenyl)-3-[4-(2-butyloxycarbonyl-ethyl)-phenyl]-imidazolidin-2-on,
2-(4-Amidino-phenyl)-4-[4-(2-isopropyloxycarbonyl-ethyl)-phenyl]-5-methyl-4H-1,2,4-triazol-3-on,
1-(4-Amidino-phenyl)-3-[4-(2-ethoxycarbonyl-ethyl)-phenyl]-3H-imidazol-2-on,
2-(4-Amidino-phenyl)-4-[4-(2-isobutyloxycarbonyl-ethyl)-phenyl]-5-methyl-4H-1,2,4-triazol-3-on,
1-(4-Amino-cyclohexyl)-3-[4-(3-carboxy-propyl)-phenyl]-imidazolidin-2-on,
1-(4-Amino-cyclohexyl)-3-[4-(3-methoxycarbonyl-propyl)-phenyl]-imidazolidin-2-on,
1-(4-Amino-cyclohexyl)-3-[4-(2-carboxy-ethyl)-phenyl]-imidazolidin-2-on,
1-(4-Amino-cyclohesyl)-3-[4-(2-methoxycarbonyl-ethyl)-phenyl]-imidazolidin-2-on,
1-(4-Aminomethyl-phenyl)-3-[3-(2-carboxy-ethyl)-phenyl]-imidazolidin-2-on,
1-(4-Aminomethyl-phenyl)-3-[3-(2-methoxycarbonyl-ethyl)-phenyl]-imidazolidin-2-on,
1-(4-Amidino-phenyl)-3-[4-(2-isopropyloxycarbonyl-ethyl)-phenyl]-4-methyl-3H-imidazol-2-on,
4-(4-Amidino-phenyl)-2-[4-(2-isopropyloxycarbonyl-ethyl)-phenyl]-4H-1,2,4-triazol-3-on,
1-(4-Amidino-phenyl)-3-[4-(2-isopropyloxycarbonyl-ethyl)-phenyl]-imidazolidin-2-on,
1-(4-Amidino-phenyl)-3-[4-(2-isopropyloxycarbonyl-ethyl)-phenyl]-3H-imidazol-2-on,

1-(4-Amidino-phenyl)-3-[4-(2-isopropyloxycarbonyl-ethyl)-phenyl]-imidazolidin-2,4-dion,
2-(4-Amidino-phenyl)-4-[4-(2-phosphono-ethyl)-phenyl]-5-methyl-4H-1,2,4-triazol-3-on und
2-(4-Amidino-phenyl)-4-[4-[2-(0-methyl-phosphono)-ethyl]-phenyl]-5-methyl-4H-1,2,4-triazol-3-on,
sowie mit den Maßgaben, daß

(i) X eine am Stickstoffatom durch eine Cyanogruppe substituierte Carbiminogruppe,
(ii) Y eine 1,2-Cyclohexylengruppe,
(iii) A eine durch eine $R_1$-CO-O-($R_2CR_3$)-O-CO-Gruppe substituierte Amidinogruppe,
(iv) B eine 1,3-Pyrrolidinylen- oder 1,3-Piperidinylengruppe,
eine gegebenenfalls durch eine bis vier Methylgruppen substituierte 1,4-Piperidinylengruppe, die zusätzlich in 4-Stellung durch eine Cyano- oder Aminocarbonylgruppe substituiert sein kann,
eine 1,4-Azacycloheptylengruppe,
eine 1,4-Piperazinylengruppe,
eine 2-Oxo-1,4-piperazinylengruppe,
eine -$CH_2CH_2O$-Gruppe,
eine 1,2-Cyclopentylengruppe oder
B zusammen mit A eine 4-Piperidinylgruppe, in der das Wasserstoffatom in 1-Stellung zusammen mit einem Wasserstoffatom in 4-Stellung durch eine Methylen- oder Ethylengruppe ersetzt ist, wobei die an die 4-Stellung gebundene Methylengruppe einer Ethylenkette durch eine Carbonylgruppe ersetzt sein kann,
(v) C eine -O-$CH_2$- oder -O-$CH_2CH_2$-Gruppe,
eine 1,4-Cyclohexylengruppe, in der ein Wasserstoffatom in 1-Stellung zusammen mit einem Wasserstoffatom in 4-Stellung durch eine Ethylengruppe ersetzt sein kann,
eine 1,4-Piperidinylengruppe,
eine 1,2,3,4-Tetrahydro-2,6-naphthylengruppe oder
C zusammen mit A und B eine 4-Piperidinylgruppe, in der das Wasserstoffatom in 1-Stellung zusammen mit einem Wasserstoffatom in 4-Stellung durch eine Methylen- oder Ethylengruppe ersetzt ist, oder
C eine Alkylengruppe mit 1 bis 4 Kohlenstoffatomen und B eine Bindung,
(vi) D eine gegebenenfalls durch ein Fluor- oder Chloratom, durch eine Methyl-, Trifluormethyl- oder Cyanogruppe substituierte 1,3-Phenylengruppe,
eine 1,4-Cyclohexylengruppe, in der ein Wasserstoffatom in 1-Stellung, zusammen mit einem Wasserstoffatom in 4-Stellung durch eine Ethylengruppe ersetzt sein kann,
eine 1,5-Indanylen-, 2,6-Naphthylen- oder 1,2,3,4-Tetrahydro-2,6-naphthylengruppe,
(vii) E eine geradkettige Alkylengruppe mit 1 bis 4 Kohlenstoffatomen, die durch eine Alkylgruppe mit 1 bis 4 Kohlenstoffatomen, durch eine Phenylgruppe oder durch eine Alkylsulfonylaminogruppe mit 1 bis 4 Kohlenstoffatomen substituiert ist, oder
eine 1,4-Piperidinylengruppe,
(viii) F eine Alkoxycarbonylgruppe mit insgesamt 3 bis 6 Kohlenstoffatomen, eine $R_7$O-CO-, $R_8$CO-O-$CHR_9$-O-CO-, Phosphono-, O-Methyl-phosphono- oder O-Ethyl-phosphono-Gruppe

darstellt, wobei mindestens eine der obigen Bedingungen (i) bis (viii) erfüllt sein müssen, in denen
X eine am Stickstoffatom durch eine Cyanogruppe substituierte Carbiminogruppe, eine Carbonyl- oder Sulfonylgruppe,
Y eine gegebenenfalls durch $R_c$ oder $R_c$ und $R_d$ substituierte -$CH_2CH_2$-, -$CH_2CH_2CH_2$-, -CH=CH-, -$CH_2$CO- oder -CO$CH_2$-Gruppe,
eine gegebenenfalls durch $R_c$ substituierte -N=CH- oder -CH=N-Gruppe,
eine 1,2-Phenylen- oder 1,2-Cyclohexylengruppe,
der erste der Reste $R_a$ bis $R_d$ eine Gruppe der Formel

A - B - C -,

in der
A eine gegebenenfalls durch eine $R_1$-CO-O-($R_2CR_3$)-O-CO-Gruppe substituierte Amidinogruppe, wobei
$R_1$ eine Methylgruppe,
$R_2$ ein Wasserstoffatom oder eine Methylgruppe und
$R_3$ ein Wasserstoffatom darstellen,
eine Amino- oder Aminomethylgruppe oder, falls A an ein Stickstoffatom der Reste B oder C gebunden ist, ein Wasserstoffatom oder eine Methylgruppe,
B eine Bindung,
eine 1,3-Pyrrolidinylen- oder 1,3-Piperidinylengruppe,

eine gegebenenfalls durch eine bis vier Methylgruppen substituierte 1,4-Piperidinylengruppe, die zusätzlich in 4-Stellung durch eine Cyano- oder Aminocarbonylgruppe substituiert sein kann,
eine 1,4-Azacycloheptylengruppe,
eine 1,4-Piperazinylengruppe, wobei jeweils ein Stickstoffatom der bei der Definition des Restes B vorstehend erwähnten Gruppen mit dem Rest A verknüpft ist,
eine 2-Oxo-1,4-piperazinylengruppe, in der das Stickstoffatom in 4-Stellung mit dem Rest A verknüpft ist,
eine $-CH_2CH_2O$-Gruppe, wobei das Sauerstoffatom mit dem Rest C verknüpft ist,
eine 1,2-Cyclopentylengruppe oder
B zusammen mit A eine 4-Piperidinylgruppe, in der das Wasserstoffatom in 1-Stellung zusammen mit einem Wasserstoffatom in 4-Stellung durch eine Methylen- oder Ethylengruppe ersetzt ist, wobei die an die 4-Stellung gebundene Methylengruppe einer Ethylenkette durch eine Carbonylgruppe ersetzt sein kann, und
C eine Alkylengruppe mit 1 bis 4 Kohlenstoffatomen,
eine $-O-CH_2-$ oder $-O-CH_2CH_2$-Gruppe, wobei das Sauerstoffatom jeweils an ein Kohlenstoffatom des Restes B oder an eine der von B zusammen mit A gebildeten bicyclischen Gruppen gebunden ist und die Methylengruppe der $-O-CH_2$-Gruppe an ein Kohlenstoffatom des cyclischen Restes der allgemeinen Formel I gebunden ist,
eine 1,4-Cyclohexylengruppe, in der ein Wasserstoffatom in 1-Stellung zusammen mit einem Wasserstoffatom in 4-Stellung durch eine Ethylengruppe ersetzt sein kann,
eine 1,4-Phenylengruppe oder
C zusammen mit A und B eine 4-Piperidinylgruppe, in der das Wasserstoffatom in 1-Stellung zusammen mit einem Wasserstoffatom in 4-Stellung durch eine Methylen- oder Ethylengruppe ersetzt ist,
oder C, falls B eine 1,4-Piperazinylengruppe darstellt, auch eine $-CO-CH_2$-Gruppe, wobei die Carbonylgruppe an ein Stickstoffatom der 1,4-Piperazinylengruppe des Restes B gebunden ist, oder
C, wenn B eine Bindung darstellt, auch eine 1,4-Piperidinylengruppe, in der das Stickstoffatom mit dem Rest A verknüpft ist, oder eine 1,2,3,4-Tetrahydro-2,6-napthylengruppe, die in 2-Stellung mit dem Rest A verknüpft ist, darstellen,
der zweite der Reste $R_a$ bis $R_d$ eine Gruppe der Formel

F - E - D -,

in der
D eine Alkylengruppe mit 1 bis 4 Kohlenstoffatomen,
eine gegebenenfalls durch ein Fluor- oder Chloratom, durch eine Methyl-, Trifluormethyl- oder Cyanogruppe substituierte 1,3- oder 1,4-Phenylengruppe,
eine 1,4-Cyclohexylengruppe, in der ein Wasserstoffatom in 1-Stellung, zusammen mit einem Wasserstoffatom in 4-Stellung durch eine Ethylengruppe ersetzt sein kann,
eine 1,5-Indanylen-, 2,6-Naphthylen- oder 1,2,3,4-Tetrahydro-2,6-naphthylengruppe,
oder, falls E eine 1,4-Piperidinylengruppe darstellt, eine $-COCH_2$-Gruppe, wobei die Carbonylgruppe mit dem Stickstoffatom der 1,4-Piperidinylengruppe des Restes E verknüpft ist,
E eine Bindung,
eine $-CH=CH$-Gruppe,
eine Alkylengruppe mit 1 bis 4 Kohlenstoffatomen, die durch eine Alkylgruppe mit 1 bis 4 Kohlenstoffatomen, durch eine Phenylgruppe oder durch eine Alkylsulfonylaminogruppe mit 1 bis 4 Kohlenstoffatomen substituiert sein kann,
eine $-O-CH_2$-Gruppe, wobei das Sauerstoffatom mit dem Rest D verknüpft ist,
eine 1,4-Cyclohexylengruppe
oder, falls D eine $-COCH_2$-Gruppe darstellt, eine 1,4-Piperidinylengruppe, wobei das Stickstoffatom mit der Carbonylgruppe der $-COCH_2$-Gruppe des Restes D verknüpft ist, und
F eine Carbonylgruppe, die durch eine Hydroxygruppe, durch eine Alkoxygruppe mit 1 bis 5 Kohlenstoffatomen, durch eine Phenylalkoxygruppe mit 1 bis 3 Kohlenstoffatomen im Alkoxyteil oder durch eine $R_7O$-Gruppe substituiert ist, wobei
$R_7$ eine Cycloalkylgruppe mit 5 bis 7 Kohlenstoffatomen, eine Cyclohexylmethyl- oder Indanylgruppe darstellt,
eine $R_8CO-O-CHR_9-O-CO-$, Phosphono-, O-Methyl-phosphono- oder O-Ethyl-phosphono-Gruppe, wobei
$R_8$ eine Cycloalkoxygruppe mit 5 bis 7 Kohlenstoffatomen im Cycloalkylteil, eine Alkyl- oder Alkoxygruppe mit jeweils 1 bis 4 Kohlenstoffatomen und
$R_9$ ein Wasserstoffatom oder eine Methylgruppe darstellen,

und der kürzeste Abstand zwischen dem Rest F und dem von dem Rest F am weitesten entfernten Stickstoffatom der Gruppe A-B-C- mindestens 11 Bindungen beträgt,
der dritte der Reste $R_a$ bis $R_d$ ein Wasserstoffatom, eine Methyl-, Trifluormethyl- oder Phenylgruppe und der vierte der Reste $R_a$ bis $R_d$ ein Wasserstoffatom oder eine Methylgruppe bedeuten,
und jedes Kohlenstoffatom in den vorstehend erwähnten Alkylen- und Cycloalkylenteilen höchstens mit einem Heteroatom verknüpft ist,
deren Tautomere, deren Stereoisomere und deren Salze.

Ganz besonders bevorzugte Verbindung der obigen allgemeinen Formel I sind mit Ausnahme von
1-(4-Amidino-phenyl)-3-[4-(2-phosphono-ethyl)-phenyl]-imidazolidin-2-on,
1-(4-Amidino-phenyl)-3-[4-(2-butyloxycarbonyl-ethyl)-phenyl]-imidazolidin-2-on,
2-(4-Amidino-phenyl)-4-[4-(2-isopropyloxycarbonyl-ethyl)-phenyl]-5-methyl-4H-1,2,4-triazol-3-on,
1-(4-Amidino-phenyl)-3-[4-(2-ethoxycarbonyl-ethyl)-phenyl]-3H-imidazol-2-on,
2-(4-Amidino-phenyl)-4-[4-(2-isobutyloxycarbonyl-ethyl)-phenyl]-5-methyl-4H-1,2,4-triazol-3-on,
1-(4-Aminomethyl-phenyl)-3-[3-(2-carboxy-ethyl)-phenyl]-imidazolidin-2-on,
1-(4-Aminomethyl-phenyl)-3-[3-(2-methoxycarbonyl-ethyl)-phenyl]-imidazolidin-2-on,
1-(4-Amidino-phenyl)-3-[4-(2-isopropyloxycarbonyl-ethyl)-phenyl]-4-methyl-3H-imidazol-2-on,
1-(4-Amidino-phenyl)-3-[4-(2-isopropyloxycarbonyl-ethyl)-phenyl]-imidazolidin-2-on,
1-(4-Amidino-phenyl)-3-[4-(2-isopropyloxycarbonyl-ethyl)-phenyl]-3H-imidazol-2-on,
1-(4-Amidino-phenyl)-3-[4-(2-isopropyloxycarbonyl-ethyl)-phenyl]-imidazolidin-2,4-dion und
2-(4-Amidino-phenyl)-4-[4-(2-phosphono-ethyl)-phenyl]-5-methyl-4H-1,2,4-triazol-3-on,
sowie mit den Maßgaben, daß

(i) X eine am Stickstoffatom durch eine Cyanogruppe substituierte Carbiminogruppe,
(ii) Y eine 1,2-Cyclohexylengruppe,
(iii) A eine durch eine $R_1$-CO-O-($R_2CR_3$)-O-CO-Gruppe substituierte Amidinogruppe,
(iv) B eine 1,4-Azacycloheptylengruppe, eine gegebenenfalls durch 1 bis 4 Methylgruppen substituierte 1,4-Piperidinylengruppe, die zusätzlich in 4-Stellung durch eine Cyano- oder Aminocarbonylgruppe substituiert sein kann, eine 1,4-Piperazinylengruppe oder
A und B zusammen eine 2-Amino-ethoxy- oder 4-Chinuclidinylgruppe,
(v) C eine 1,4-Piperidinylen-, 1,2,3,4-Tetrahydro-2,6-naphthylen- oder 1,4-Bicyclo[2.2.2]octanylen-Gruppe,
(vi) D eine 1,3-Phenylen-, cis-1,4-Cyclohexylen- oder trans-1,4-Cyclohexylengruppe,
(vii) E eine geradkettige Alkylenkette mit 2 bis 4 Kohlenstoffatomen, die durch eine Alkylgruppe mit 1 bis 4 Kohlenstoffatomen, durch eine Phenylgruppe oder durch eine Alkylsulfonylaminogruppe mit 1 bis 4 Kohlenstoffatomen substituiert ist, oder
eine 1,4-Piperidinylengruppe,
(viii) F eine Alkoxycarbonylgruppe mit insgesamt 3 bis 6 Kohlenstoffatomen eine $R_7$O-CO-, $R_8$CO-O-$CHR_9$-O-CO-, Phosphono- oder O-Ethyl-phosphono-Gruppe darstellt, wobei mindestens eine der obigen Bedingungen (i) bis (viii) erfüllt sein müssen,

diejenigen, in denen
X eine am Stickstoffatom durch eine Cyanogruppe substituierte Carbiminogruppe, eine Carbonyl- oder Sulfonylgruppe,
Y eine gegebenenfalls durch $R_c$ oder $R_c$ und $R_d$ substituierte $-CH_2CH_2-$, $-CH_2CH_2CH_2-$ oder -CH=CH-Gruppe,
eine gegebenenfalls durch $R_c$ substituierte -N=CH-Gruppe,
eine $-CH_2CO-$ oder $-COCH_2-$Gruppe,
eine 1,2-Phenylen- oder 1,2-Cyclohexylengruppe, wobei

$R_c$ ein Wasserstoffatom, eine Methyl-, Trifluormethyl- oder Phenylgruppe und
$R_d$ ein Wasserstoffatom oder eine Methylgruppe darstellen,

$R_a$ eine Gruppe der Formel

A - B - C -,

in der
A eine gegebenenfalls durch eine $R_1$-CO-O-($R_2CR_3$)-O-CO-Gruppe substituierte Amidinogruppe, wobei

$R_1$ eine Methylgruppe,
$R_2$ ein Wasserstoffatom oder eine Methylgruppe und
$R_3$ ein Wasserstoffatom darstellen,

eine Aminomethylgruppe oder, falls A an ein Stickstoffatom der Reste B oder C gebunden ist, ein Wasserstoffatom oder eine Methylgruppe,

B eine Bindung, eine 1,4-Azacycloheptylengruppe, eine gegebenenfalls durch 1 bis 4 Methylgruppen substituierte 1,4-Piperidinylengruppe, die zusätzlich in 4-Stellung durch eine Cyano- oder Aminocarbonylgruppe substituiert sein kann, oder eine 1,4-Piperazinylengruppe, wobei jeweils ein Stickstoffatom der vorstehend erwähnten Gruppen mit dem Rest A verknüpft ist, oder

A und B zusammen eine 2-Amino-ethoxy- oder 4-Chinuclidinylgruppe und

C eine $-CH_2CH_2$-Gruppe, eine 1,4-Phenylen-, 1,4-Cyclohexylen- oder 1,4-Bicyclo[2.2.2]octanylen-Gruppe oder, wenn B eine Bindung darstellt, auch eine 1,4-Piperidinylengruppe, die über ein Stickstoffatom mit dem Rest A verknüpft ist, oder eine 1,2,3,4-Tetrahydro-2,6-naphthylengruppe, die in 2-Stellung mit dem Rest A verknüpft ist,

oder, falls B eine 1,4-Piperazinylengruppe darstellt, auch eine über die Carbonylgruppe mit der 1,4-Piperazinylengruppe des Restes B verbundene $-COCH_2$-Gruppe,

$R_b$ eine Gruppe der Formel

F - E - D -,

in der

D eine geradkettige Alkylengruppe mit 1 bis 4 Kohlenstoffatomen, eine 1,3-Phenylen- oder 1,4-Cyclohexylengruppe oder eine gegebenenfalls durch ein Fluoratom, durch eine Methyl-, Trifluormethyl- oder Cyanogruppe substituierte 1,4-Phenylengruppe, eine 2,6-Naphthylen- oder 1,2,3,4-Tetrahydro-2,6-naphthylengruppe oder, falls E eine 1,4-Piperidinylengruppe darstellt, eine über die Carbonylgruppe mit der 1,4-Piperidinylengruppe des Restes E verknüpfte $-COCH_2$-Gruppe,

E eine Bindung, eine $-CH=CH$-Gruppe, eine gegebenenfalls durch eine Alkylgruppe mit 1 bis 4 Kohlenstoffatomen, durch eine Phenylgruppe oder durch eine Alkylsulfonylaminogruppe mit 1 bis 4 Kohlenstoffatomen substituierte geradkettige Alkylengruppe mit 2 bis 4 Kohlenstoffatomen, eine $-O-CH_2$-Gruppe, wobei das Sauerstoffatom mit dem Rest D verknüpft ist, oder, falls D eine $-COCH_2$-Gruppe darstellt, eine 1,4-Piperidinylengruppe, wobei das Stickstoffatom mit der Carbonylgruppe der $-COCH_2$-Gruppe des Restes D verknüpft ist, und

F eine Carbonylgruppe, die durch eine Hydroxygruppe, durch eine Alkoxygruppe mit 1 bis 5 Kohlenstoffatomen oder durch eine $R_7O$-Gruppe substituiert ist, wobei

$R_7$ eine Cyclopentyl-, Cyclohexyl-, Cyclohexylmethyl- oder 5-Indanylgruppe darstellt,

eine $R_8CO-O-CHR_9-O-CO-$, Phosphono- oder O-Ethyl-phosphono-Gruppe darstellen, wobei

$R_8$ eine tert.Butyl-, Ethoxy- oder Cyclohexyloxygruppe und

$R_9$ ein Wasserstoffatom oder eine Methylgruppe darstellen,

bedeuten und der kürzeste Abstand zwischen dem Rest F und dem von dem Rest F am weitesten entfernten Stickstoffatom der Gruppe A-B-C- mindestens 11 Bindungen beträgt,

deren Tautomere, deren Stereoisomere und deren Salze.

Als besonders bevorzugte cyclische Harnstoffderivate der allgemeinen Formel I seien beispielsweise folgende erwähnt:

(a) 2-(4-Amidinophenyl)-4-[4-(2-carboxy-1-propyl)phenyl]-5-methyl-4H-1,2,4-triazol-3-on,
(b) 4-[4-(2-Carboxyethyl)phenyl]-5-methyl-2-[2-(4-piperidinyl)ethyl]-4H-1,2,4-triazol-3-on,
(c) 1-[4-(2-Carboxyethyl)phenyl]-3-[2-(4-piperidinyl)ethyl]-imidazolidin-2-on,
(d) 4-[4-(2-Carboxy-1-pentyl)phenyl]-5-methyl-2-[2-(4-piperidinyl)ethyl]-4H-1,2,4-triazol-3-on,
(e) 1-(4-Amidinophenyl)-3-[4-[2-(n-butylsulfonylamino)-2-carboxy-ethyl]phenyl]-imidazolidin-2-on,
(f) 2-(4-Amidinophenyl)-4-[trans-4-(2-carboxyethyl)cyclohexyl]-4H-1,2,4-triazol-3-on,
(g) 2-(4-Amidinophenyl)-4-[4-[2-(cyclohexyloxycarbonyl)-ethyl]phenyl]-5-methyl-4H-1,2,4-triazol-3-on,
(h) 2-(4-Amidinophenyl)-4-[trans-4-(2-carboxyethyl)cyclohexyl]-5-methyl-4H-1,2,4-triazol-3-on,
(i) 1-(4-Amidinophenyl)-3-[trans-4-(2-carboxyethyl)cyclohexyl]-imidazolidin-2-on,
(j) 1-(4-Amidinophenyl)-3-[trans-4-(2-carboxyethyl)cyclohexyl]-imidazolidin-2,4-dion,
(k) 1-(4-Amidinophenyl)-3-[trans-4-[2-(methoxycarbonyl)-ethyl]cyclohexyl]-imidazolidin-2-on,
(l) 1-(trans-4-Carboxycyclohexyl)-3-[4-(4-cyano-4-piperidinyl)phenyl]-imidazolidin-2-on,
(m) 1-[4-(4-Aminocarbonyl-4-piperidinyl)phenyl]-3-(trans-4-carboxycyclohexyl)-imidazolidin-2-on,
(n) 1-[4-[2-(Ethoxycarbonyl)ethyl]phenyl]-3-[2-(4-piperidinyl)ethyl]-imidazolidin-2-on,
(o) 1-(trans-4-Carboxycyclohexyl)-3-[4-(4-piperidinyl)-phenyl]-imidazolidin-2-on,
(p) 1-[trans-4-(2-Carboxyethyl)cyclohexyl]-3-[2-(4-piperidinyl)ethyl]-imidazolidin-2-on,
(q) 1-[4-(2-Carboxyethyl)phenyl]-3-[2-(4-chinuclidinyl)-ethyl]-imidazolidin-2-on,
(r) 1-[2-(4-Chinuclidinyl)ethyl]-3-[4-[2-(ethoxycarbonyl)-ethyl]phenyl]-imidazolidin-2-on,
(s) 1-[trans-4-(5-Indanyloxycarbonyl)cyclohexyl]-3-[4-(4-piperidinyl)phenyl]-imidazolidin-2-on,

(t) 1-[trans-4-[[1-(Cyclohexyloxycarbonyloxy)ethyl]oxycarbonyl]cyclohexyl]-3-[4-(4-piperidinyl)phenyl]-imidazolidin-2-on,
(u) 1-[trans-4-(Ethoxycarbonyl)cyclohexyl]-3-[4-(4-piperidinyl)phenyl]-imidazolidin-2-on,
(v) 1-[trans-4-[2-(Ethoxycarbonyl)ethyl]cyclohexyl]-3-[2-(4-piperidinyl)ethyl]-imidazolidin-2-on,
(w) 1-(2-Aminomethyl-1,2,3,4-tetrahydro-6-naphthyl)-3-(trans-4-carboxycyclohexyl)-imidazolidin-2-on,
(x) 1-[trans-4-[(Carboxymethyl)oxy]cyclohexyl]-3-[2-(4-piperidinyl)ethyl]-imidazolidin-2-on,
(y) 1-[4-(2-Carboxyethenyl)-2-fluor-phenyl]-3-[2-(4-piperidinyl)ethyl]-imidazolidin-2-on,
(z) 1-(trans-4-Carboxycyclohexyl)-3-[4-(4-piperidinyl)cyclohexyl]-imidazolidin-2-on,
(aa) 1-(trans-4-Carboxycyclohexyl)-3-[4-(4-chinuclidinyl)-phenyl]-imidazolidin-2-on,
(bb) 1-(trans-4-Carboxycyclohexyl)-3-[4-(4-methyl-4-piperidinyl)phenyl]-imidazolidin-2-on,
(cc) 1-[4-(4-Chinuclidinyl)phenyl]-3-[trans-4-(ethoxycarbonyl)cyclohexyl]-imidazolidin-2-on,
(dd) 1-[trans-4-(Ethoxycarbonyl)cyclohexyl]-3-[4-(4-methyl-4-piperidinyl)phenyl]-imidazolidin-2-on,
(ee) 1-[4-(2-Carboxyethenyl)phenyl]-3-[2-(4-piperidinyl)-ethyl]-imidazolidin-2-on,
(ff) 1-[trans-4-(Ethoxycarbonyl)cyclohexyl]-3-[4-(4-piperidinyl)phenyl]-3H-imidazol-2-on,
(gg) 1-[4-(Aminomethyl)-bicyclo[2.2.2]octan-1-yl]-3-[4-(2-carboxyethyl)phenyl]-imidazolidin-2-on,
(hh) 1-[4-(2-Carboxy-1-phenyl-ethyl)phenyl]-3-[2-(4-piperidinyl)ethyl]-imidazolidin-2-on,
(ii) 1-(2-Carboxy-1,2,3,4-tetrahydro-6-naphthyl)-3-[2-(4-piperidinyl)ethyl]-imidazolidin-2-on und
(jj) 1-(trans-4-Carboxycyclohexyl)-3-[4-[(2-aminoethyl)oxy]-phenyl]-imidazolidin-2-on,

deren Stereoisomere und deren Salze.

Die neuen Verbindungen lassen sich beispielsweise nach folgenden Verfahren herstellen:

a) Zur Herstellung von Verbindungen der allgemeinen Formel I, in der A wie eingangs erwähnt definiert ist und F eine Carboxygruppe darstellt oder F mit Ausnahme der Carboxygruppe wie eingangs erwähnt definiert ist und A eine Aminoalkylgruppe mit 1 bis 5 Kohlenstoffatomen, eine Amino-, Amidino- oder Guanidinogruppe, wobei in jeder der vorstehend erwähnten Gruppen jeweils an einem der Stickstoffatome ein oder zwei Wasserstoffatome jeweils durch eine Alkylgruppe ersetzt sein können, oder auch, falls A an ein Stickstoffatom der Reste B oder C gebunden ist, das nicht Teil einer Lactamgruppe ist, ein Wasserstoffatom darstellt:

Umwandlung einer Verbindung der allgemeinen Formel

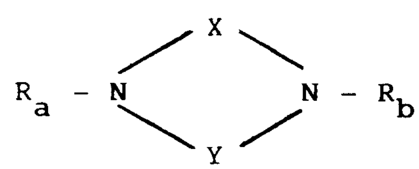

,(II)

in der

$R_a$, $R_b$, X und Y mit der Maßgabe wie eingangs definiert sind, daß einer der Reste $R_a$ bis $R_d$ eine F'-E-D-Gruppe und der andere der Reste $R_a$ bis $R_d$ eine A-B-C-Gruppe oder einer der Reste $R_a$ bis $R_d$ eine F-E-D-Gruppe und der andere der Reste $R_a$ bis $R_d$ eine A'-B-C-Gruppe oder einer der Reste $R_a$ bis $R_d$ eine F'-E-D-Gruppe und der andere der Reste eine A'-B-C-Gruppe darstellen, in denen

A bis F wie eingangs definiert sind,

A' eine mittels Hydrolyse, Behandeln mit Säure, Thermolyse oder Hydrogenolyse in eine Aminoalkyl-, Amino-, Amidino- oder Guanidinogruppe überführbare Gruppe, wobei in jeder der vorstehend erwähnten Gruppen jeweils an einem der Stickstoffatome ein oder zwei Wasserstoffatome jeweils durch eine Alkylgruppe ersetzt sein können, oder auch, falls A' an ein Stickstoffatom der Reste B oder C gebunden ist, das nicht Teil einer Lactamgruppe ist,

eine mittels Hydrolyse, Behandeln mit Säure, Thermolyse oder Hydrogenolyse durch ein Wasserstoffatom ersetzbare Gruppe und

F' eine mittels Hydrolyse, Behandeln mit Säure, Thermolyse oder Hydrogenolyse in eine Carboxygruppe überführbare Gruppe darstellen,

in eine Verbindung der allgemeinen Formel I, in der A wie eingangs erwähnt definiert ist und F eine Carboxygruppe darstellt oder F mit Ausnahme der Carboxygruppe wie eingangs erwähnt definiert ist und A eine Aminoalkylgruppe mit 1 bis 5 Kohlenstoffatomen, eine Amino-, Amidino- oder Guanidinogruppe, wobei in jeder der vorstehend erwähnten Gruppen jeweils an einem der Stickstoffatome ein oder zwei Wasserstoffatome jeweils durch eine Alkylgruppe ersetzt sein können, oder auch, falls A an ein Stickstoffatom der Reste B oder C gebunden ist, das nicht Teil einer Lactamgruppe ist, ein Wasserstoffatom darstellt.

Beispielsweise können Derivate der Carboxylgruppe wie deren unsubstituierte oder substituierte Amide, Ester, Thioester, Trimethylsilylester, Orthoester, Iminoester, Amidine oder Anhydride, oder die Nitrilgruppe mittels Hydrolyse in eine Carboxylgruppe, Ester mit tertiären Alkoholen, z.B. der tert. Butylester, mittels Behandlung mit einer Säure oder Thermolyse in eine Carboxylgruppe und Ester mit Aralkanolen, z.B. der Benzylester, mittels Hydrogenolyse in eine Carboxylgruppe sowie

Iminogruppen, die durch einen Schutzrest wie die Formyl-, Acetyl-, Trifluoracetyl-, Ethoxycarbonyl- oder Methoxycarbonylgruppe substituiert sind, mittels Hydrolyse oder

Iminogruppen, die durch einen Schutzrest wie die tert.-Butyloxycarbonylgruppe mittels Behandlung mit einer Säure oder Thermolyse oder

Iminogruppen, die durch einen Schutzrest wie die Benzyloxycarbonyl-, Benzyl-, Methoxybenzyl- oder 2,4-Dimethoxybenzylgruppe substituiert sind, mittels Hydrogenolyse in die entsprechende NH-Verbindung übergeführt werden.

Die Hydrolyse wird zweckmäßigerweise entweder in Gegenwart einer Säure wie Salzsäure, Schwefelsäure, Phosphorsäure, Essigsäure, Trichloressigsäure, Trifluoressigsäure oder deren Gemische oder in Gegenwart einer Base wie Lithiumhydroxid, Natriumhydroxid oder Kaliumhydroxid in einem geeigneten Lösungsmittel wie Wasser, Wasser/Methanol, Wasser/Äthanol, Wasser/Isopropanol, Methanol, Äthanol, Wasser/Tetrahydrofuran oder Wasser/Dioxan bei Temperaturen zwischen -10 und 120°C, z.B. bei Temperaturen zwischen Raumtemperatur und der Siedetemperatur des Reaktionsgemisches, durchgeführt.

Bedeutet F' in einer Verbindung der Formel II eine Cyano- oder Aminocarbonylgruppe, so können diese Gruppen auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen O und 50°C in die Carboxylgruppe übergeführt werden.

Enthält A' oder bedeutet F' in einer Verbindung der Formel II beispielsweise die tert. Butyloxycarbonylgruppe, so kann die tert. Butylgruppe auch durch Behandlung mit einer Säure wie Trifluoressigsäure, Ameisensäure, p-Toluolsulfonsäure, Schwefelsäure, Salzsäure, Phosphorsäure oder Polyphosphorsäure gegebenenfalls in einem inerten Lösungsmittel wie Methylenchlorid, Chloroform, Benzol, Toluol, Diethylether, Tetrahydrofuran oder Dioxan vorzugsweise bei Temperaturen zwischen -10 und 120°C, z.B. bei Temperaturen zwischen 0 und 60°C, oder auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 und 120°C, abgespalten werden, wobei eine gegebenenfalls im Reaktionsgemisch so erhaltene N-Carboxy-imino-Verbindung gleichzeitig decarboxyliert wird.

Enthält A' oder bedeutet F' in einer Verbindung der Formel II beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Ethanol, Ethanol/Wasser, Eisessig, Essigsäureäthylester, Dioxan oder Dimethylformamid vorzugsweise bei Temperaturen zwischen 0 und 80°C, z.B. bei Raumtemperatur, und einem Wasserstoffdruck von 1 bis 5 bar abgespalten werden, wobei eine gegebenenfalls im Reaktionsgemisch so erhaltene N-Carboxy-imino-Verbindung gleichzeitig decarboxyliert wird.

b) Zur Herstellung von Verbindungen der allgemeinen Formel I, in der A eine $H_2N-C(=NH)$-Gruppe darstellt, in welcher ein Stickstoffatom durch eine oder zwei Alkylgruppen mit jeweils 1 bis 3 Kohlenstoffatomen substituiert sein kann:

Umsetzung einer gegebenenfalls im Reaktionsgemisch gebildeten Verbindung der allgemeinen Formel

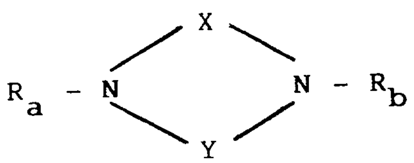

,(III)

in der

$R_a$, $R_b$, X und Y mit der Maßgabe wie eingangs definiert sind, daß einer der Reste $R_a$ bis $R_d$ eine Gruppe der Formel

$Z_1$ - C(=NH) - B - C -

darstellt, in der
B und C wie eingangs definiert sind und
$Z_1$ eine Alkoxy- oder Aralkoxygruppe wie die Methoxy-, Ethoxy-, n-Propoxy-, Isopropoxy- oder Benzyloxygruppe oder eine Alkylthio- oder Aralkylthiogruppe wie die Methylthio-, Ethylthio-, n-Propylthio- oder Benzylthiogruppe oder eine Aminogruppe darstellt, mit einem Amin der allgemeinen Formel

$R_{12}$ - NH - $R_{13}$ ,(IV)

in der
$R_{12}$ und $R_{13}$, die gleich oder verschieden sein können, Wasserstoffatome oder Alkylgruppen mit jeweils 1 bis 3 Kohlenstoffatomen bedeuten, oder mit deren Säureadditionssalzen.

Die Umsetzung wird zweckmäßigerweise in einem Lösungsmittel wie Methanol, Ethanol, n-Propanol, Wasser, Methanol/Wasser, Tetrahydrofuran oder Dioxan bei Temperaturen zwischen -10 und 150 °C, vorzugsweise bei Temperaturen zwischen 0 und 120 °C, mit einem entsprechenden freien Amin oder mit einem entsprechenden Säureadditionssalz wie beispielsweise den entsprechenden Ammoniumcarbonaten, -acetaten oder -chloriden durchgeführt.

Eine Verbindung der allgemeinen Formel III erhält man beispielsweise durch Umsetzung eines entsprechenden Nitrils mit einem entsprechenden Alkohol wie Methanol, Ethanol, n-Propanol, Isopropanol oder Benzylalkohol in Gegenwart einer Säure wie Salzsäure oder durch Umsetzung eines entsprechenden Amids mit einem Trialkyloxoniumsalz wie Triethyloxonium-tetrafluorborat in einem Lösungsmittel wie Methylenchlorid, Tetrahydrofuran oder Dioxan bei Temperaturen zwischen -10 und 50 °C, vorzugsweise jedoch bei Temperaturen zwischen 0 und 30 °C, oder eines entsprechenden Nitrils mit Schwefelwasserstoff zweckmäßigerweise in einem Lösungsmittel wie Pyridin oder Dimethylformamid und in Gegenwart einer Base wie Triethylamin und anschließender Alkylierung des gebildeten Thioamids mit einem entsprechenden Alkyl- oder Aralkylhalogenid oder durch Umsetzung eines entsprechenden Nitrils mit einem Alkoholat wie Natriummethylat in einem Lösungsmittel wie Dioxan oder Tetrahydrofuran, vorzugsweise jedoch in dem entsprechenden Alkohol. Bei den Umsetzungen mit einem Alkohol kann gleichzeitig eine vorhandene Estergruppe umgeestert werden.

c) Zur Herstellung von 4H-1,2,4-Triazol-3-onen der allgemeinen Formel I:
Cyclisierung einer Verbindung der allgemeinen Formel

($R_{14}CZ_2$) = N - $NR_{15}$ - CO - HN - $R_{16}$ ,(V)

in der
$R_{14}$ die für $R_c$ oder $R_d$ eingangs erwähnten Bedeutungen besitzt,
einer der Reste $R_{15}$ oder $R_{16}$ die für $R_a$ eingangs erwähnten Bedeutungen besitzt und
der andere der Reste $R_{15}$ oder $R_{16}$ die für $R_b$ eingangs erwähnten Bedeutungen besitzt und
$Z_2$ eine nukleophile Austrittsgruppe wie ein Halogenatom, eine Hydroxy-, Alkoxy-, Alkylsulfenyl-, Amino-, Alkylamino- oder Dialkylaminogruppe, z. B. ein Chlor-, Brom- oder Jodatom, eine Methoxy-, Ethoxy- oder Methylsulfenylgruppe, darstellt.

Die Umsetzung wird gegebenenfalls in einem Lösungsmittel wie Toluol, Xylol, Decalin, Dioxan, Dimethylformamid, Methylenchlorid, Methanol, Ethanol, Isopropanol, Pyridin, Essigsäure oder Trifluoressigsäure bei Temperaturen zwischen 20 und 250 °C, gegebenenfalls in Gegenwart einer Base oder einer Säure wie Trifluoressigsäure oder eines wasserentziehenden Mittels wie Phosphoroxychlorid, Phosphorpentachlorid oder N,N'-Dicyclohexylcarbodiimid durchgeführt. Die Umsetzung wird jedoch bevorzugt ohne Lösungsmittel durchgeführt.

Bedeutet $Z_2$ ein Halogenatom, so wird die Umsetzung gegebenenfalls in einem der vorstehend erwähnten Lösungsmittel vorzugsweise in Gegenwart einer Base wie Kaliumkarbonat, Natriumhydrid, Kalium-tert.butylat oder Triethylamin bei Temperaturen zwischen 20 und 60 °C durchgeführt, oder
bedeutet $Z_2$ eine Hydroxy-, Alkoxy-, Alkylsulfenyl-, Amino-, Alkylamino- oder Dialkylaminogruppe, so wird die Umsetzung gegebenenfalls in Gegenwart einer Säure wie Trifluoressigsäure, welche gleichzeitig auch als Lösungsmittel dienen kann, bei Temperaturen zwischen 20 und 120 °C, vorzugsweise jedoch ohne Lösungsmittel bei Temperaturen zwischen 100 und 220 °C durchgeführt.

d) Zur Herstellung von Verbindungen der allgemeinen Formel I, in der X eine Carbonylgruppe und Y eine der eingangs erwähnten Ethylen- oder Vinylengruppen darstellen:
Cyclisierung einer gegebenenfalls im Reaktionsgemisch gebildeten Verbindung der allgemeinen Formel

$R_{17}$ - CO - $CHR_{18}$ - $NR_{15}$ - CO - $NHR_{16}$ ,(VI)

in der
einer der Reste $R_{15}$ oder $R_{16}$ die für $R_a$ eingangs erwähnten Bedeutungen besitzt und
der andere der Reste $R_{15}$ oder $R_{16}$ die für $R_b$ eingangs erwähnten Bedeutungen besitzt,
einer der Reste $R_{17}$ oder $R_{18}$ die für $R_c$ eingangs erwähnten Bedeutungen besitzt und
der andere der Reste $R_{17}$ oder $R_{18}$ die für $R_d$ eingangs erwähnten Bedeutungen besitzt, und gegebenenfalls anschließende Hydrierung.

Die Cyclisierung wird vorzugsweise in einem Lösungsmittel wie Methylenchlorid, Chloroform, Essigsäure, Benzol, Toluol oder Dioxan gegebenenfalls in Gegenwart einer Säure wie Trifluoressigsäure, p-Toluolsulfonsäure oder Salzsäure und gegebenenfalls in Gegenwart eines wasserentziehenden Mittels wie Phosphoroxychlorid, Phosphorpentachlorid oder N,N'-Dicyclohexylcarbodiimid bei Temperaturen zwischen 20 und 150°C, vorzugsweise bei Temperaturen zwischen 20°C und der Siedetemperatur des verwendeten Lösungsmittels, durchgeführt.

Die gegebenenfalls anschließende Hydrierung erfolgt vorzugsweise mit Wasserstoff in Gegenwart eines Katalysators wie Palladium/Kohle oder Platin in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

e) Zur Herstellung von Verbindungen der allgemeinen Formel I, in der X eine Carbonylgruppe und Y eine der eingangs erwähnten Ethylen- oder Vinylengruppen darstellen:
Umsetzung einer Verbindung der allgemeinen Formel

$R_{15}$ - NH - $CHR_{18}$ - $R_{17}C(OR_{19})_2$, (VII)

mit einem Isocyanat der allgemeinen Formel

O = C = N - $R_{16}$ ,(VIII)

in denen
einer der Reste $R_{15}$ oder $R_{16}$ die für $R_a$ eingangs erwähnten Bedeutungen besitzt und
der andere der Reste $R_{15}$ oder $R_{16}$ die für $R_b$ eingangs erwähnten Bedeutungen besitzt,
einer der Reste $R_{17}$ oder $R_{18}$ die für $R_c$ eingangs erwähnten Bedeutungen besitzt und
der andere der Reste $R_{17}$ oder $R_{18}$ die für $R_d$ eingangs erwähnten Bedeutungen besitzt und
$R_{19}$ jeweils eine Alkylgruppe mit 1 bis 4 Kohlenstoffatomen darstellt, und gegebenenfalls anschließende Hydrierung.

Die Umsetzung wird gegebenenfalls in einem inerten Lösungsmittel wie Dioxan oder Toluol bei Temperaturen zwischen 20 und 200°C, vorzugsweise bei Temperaturen zwischen 20 und 160°C, durchgeführt. Die Umsetzung kann jedoch auch ohne Lösungsmittel durchgeführt werden.

Ein gegebenenfalls bei der Umsetzung einer Verbindung der allgemeinen Formel VII und mit einem Isocyanat der allgemeinen Formel VIII als Zwischenprodukt erhaltener offenkettiger Harnstoff wird anschließend gegebenenfalls in Gegenwart einer Säure wie Essigsäure, Trifluoressigsäure, p-Toluolsäure oder Salzsäure in die gewünschte Verbindung übergeführt.

Die gegebenenfalls anschließende Hydrierung erfolgt vorzugsweise mit Wasserstoff in Gegenwart eines Katalysators wie Palladium/Kohle oder Platin in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

f) Zur Herstellung von Verbindungen der allgemeinen Formel I, in der X eine Carbonylgruppe und Y eine der eingangs erwähnten $-COCH_2-$, $-CH_2CO-$, -CONH- oder -NHCO-Gruppen darstellt:
Cyclisierung einer Verbindung der allgemeinen Formel

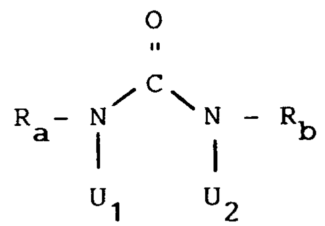

,(IX)

in der

$R_a$ und $R_b$ wie eingangs definiert sind,

einer der Reste $U_1$ oder $U_2$ ein Wasserstoffatom und

der andere der Reste $U_1$ oder $U_2$ entweder eine gegebenenfalls durch $R_c$ oder $R_d$ oder $R_c$ und $R_d$ substituierte $Z_3$-CO-$CH_2$-Gruppe darstellt, in welcher

$Z_3$ eine nukleophile Austrittsgruppe wie ein Halogenatom, eine Hydroxy-, Alkoxy- oder Sulfonyloxygruppe, z.B. ein Chlor-, Brom- oder Jodatom, eine Methoxy-, Ethoxy-, Isopropyloxy-, Methansulfonyloxy- oder p-Toluolsulfonyloxygruppe, darstellt,

oder eine gegebenenfalls durch $R_c$ oder $R_d$ substituierte $Z_3$'-CONH-Gruppe darstellt, in welcher

$Z_3$' eine nukleophile Austrittsgruppe wie ein Halogenatom oder eine Alkoxygruppe, z. B. ein Chlor- oder Bromatom oder eine Methoxygruppe, oder

$Z_3$' zusammen mit dem Wasserstoffatom der NH-Gruppe eine weitere Kohlenstoff-Stickstoff-Bindung darstellt.

Die Umsetzung wird gegebenenfalls in einem Lösungsmittel wie Ethanol, Isopropanol, Methylenchlorid, Dioxan, Toluol, Dimethylformamid oder Dimethylsulfoxid gegebenenfalls in Gegenwart einer Base wie Pyridin, Triethylamin, Natriumhydrid oder Kalium-tert.butylat und gegebenenfalls in Gegenwart eines wasserentziehenden Mittels wie N,N'-Dicyclohexylcarbodiimid bei Temperaturen zwischen 20 und 200°C durchgeführt. Die Umsetzung kann jedoch auch ohne Lösungsmittel durchgeführt werden.

Bedeutet $Z_3$ oder $Z_3$' eine nukleophile Austrittsgruppe wie ein Halogenatom oder eine Sulfonestergruppe, so wird die Umsetzung vorzugsweise in Gegenwart einer Base wie Kaliumcarbonat, Natriumhydrid, Kalium-tert.butylat, Pyridin oder Triethylamin bei Temperaturen zwischen 20 und 60°C durchgeführt, bedeutet $Z_3$ oder $Z_3$' eine Alkoxygruppe, so wird die Umsetzung vorzugsweise ohne Lösungsmittel bei Temperaturen zwischen 50 und 250°C durchgeführt, oder bedeutet $Z_3$ eine Hydroxygruppe, so wird die Umsetzung vorzugsweise in Gegenwart eines wasserentziehenden Mittels wie Triphenylphosphin/Tetrachlorkohlenstoff, N,N'-Dicyclohexylcarbodiimid oder N,N'-Carbonyldiimidazol durchgeführt.

g) Zur Herstellung von Verbindungen der allgemeinen Formel I, in der A eine durch eine Alkoxycarbonylgruppe, durch eine Benzyloxycarbonylgruppe oder durch eine $R_1$-CO-O-($R_2CR_3$)-O-CO-Gruppe substituierte Amino-, Aminoalkyl-, Amidino- oder Guanidinogruppe oder eine $R_1$-CO-O-($R_2CR_3$)-O-CO-Gruppe darstellt:

Umsetzung einer Verbindung der allgemeinen Formel

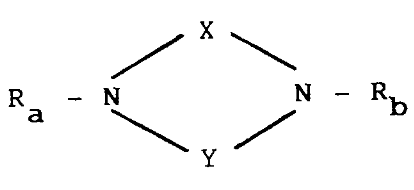

,(X)

in der

$R_a$, $R_b$, X und Y mit der Maßgabe wie eingangs definiert sind, daß einer der Reste $R_a$ bis $R_d$ eine A'' - B - C -Gruppe darstellt, in der

B und C wie eingangs definiert sind und

A'' eine $H_2N$-$C_{1-5}$alkyl-, $H_2N$-C(=NH)- oder $H_2N$-C(=NH)-NH-oder $H_2N$-Gruppe oder ein Wasserstoffatom darstellt,

mit einer Verbindung der allgemeinen Formel

$Z_4$ - $R_{20}$ ,(XI)

in der
$R_{20}$ eine Alkoxycarbonylgruppe mit insgesamt 2 bis 5 Kohlenstoffatomen, eine $R_1$-CO-O-($R_2CR_3$)-O-CO- oder Benzyloxycarbonylgruppe und
$Z_4$ eine nukleophile Austrittsgruppe wie ein Halogenatom, eine Aryloxy-, Arylthio-, Alkoxycarbonyloxy-, Aralkoxycarbonyloxy- oder N-Imidazolylgruppe, z. B. ein Chlor- oder Bromatom oder eine 4-Nitrophenoxygruppe, darstellen.

Die Acylierung wird zweckmäßigerweise in einem Lösungsmittel wie Tetrahydrofuran, Methylenchlorid, Chloroform, Dimethylformamid, Wasser oder Gemischen aus diesen Lösungsmitteln gegebenenfalls in Gegenwart einer Base wie Natriumcarbonat, Kaliumcarbonat oder Natronlauge oder in Gegenwart einer tertiären organischen Base wie Triethylamin, N-Ethyl-diisopropylamin, N-Methyl-morpholin oder Pyridin, welche gleichzeitig als Lösungsmittel dienen können, bei Temperaturen zwischen -30 und 100 °C, vorzugsweise jedoch bei Temperaturen zwischen -10 und 60 °C, durchgeführt.

h) Zur Herstellung von Verbindungen der allgemeinen Formel I, in der F eine durch eine Alkoxygruppe mit 1 bis 6 Kohlenstoffatomen, durch eine Phenylalkoxygruppe oder durch eine $R_7$O-Gruppe substituierte Carbonylgruppe darstellt:
Umsetzung einer Verbindung der allgemeinen Formel

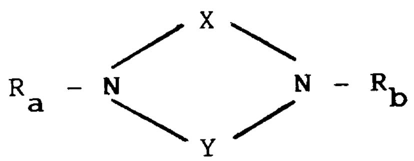
,(XII)

in der
$R_a$, $R_b$, X und Y mit der Maßgabe wie eingangs definiert sind, daß einer der Reste $R_a$ bis $R_d$ eine F'' - E - D - Gruppe darstellt, in der
E und D wie eingangs definiert sind und
F'' eine Carboxy- oder Alkoxycarbonylgruppe darstellt,
mit einem Alkohol der allgemeinen Formel

HO - $R_{21}$ ,(XIII)

in der
$R_{21}$ die für $R_7$ eingangs erwähnten Bedeutungen aufweist oder auch eine Alkylgruppe mit 1 bis 6 Kohlenstoffatomen oder eine Phenylalkylgruppe darstellt.

Die Umsetzung einer Carboxyverbindung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan oder besonders vorteilhaft in einem entsprechenden Alkohol der allgemeinen Formel XIII gegebenenfalls in Gegenwart einer Säure wie Salzsäure oder in Gegenwart eines wasserentziehenden Mittels, z.B. in Gegenwart von Chlorameisensäureisobutylester, Thionylchlorid, Trimethylchlorsilan, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid oder 1-Hydroxy-benztriazol und gegebenenfalls zusätzlich in Gegenwart von 4-Dimethylamino-pyridin, N,N'-Carbonyldiimidazol oder Triphenylphosphin/Tetrachlorkohlenstoff, zweckmäßigerweise bei Temperaturen zwischen 0 und 150 °C, vorzugsweise bei Temperaturen zwischen 0 und 80 °C, durchgeführt.

Die Umsetzung einer entsprechende Alkoxycarbonylverbindung mit einem Alkohol der allgemeinen Formel XIII wird vorzugsweise in einem entsprechenden Alkohol als Lösungsmittel gegebenenfalls in Gegenwart eines weiteren Lösungsmittels wie Methylenchlorid oder Ether vorzugsweise in Gegenwart einer Säure wie Salzsäure bei Temperaturen zwischen 0 und 150 °C, vorzugsweise bei Temperaturen zwischen 50 und 100 °C, durchgeführt.

i) Zur Herstellung von Verbindungen der allgemeinen Formel I, in der A eine $H_2N$-$CH_2$-V-Gruppe, in der V eine Bindung oder eine geradkettige oder verzweigte Alkylengruppe mit 1 bis 4 Kohlenstoffatomen bedeutet, darstellt:
Reduktion einer Verbindung der allgemeinen Formel

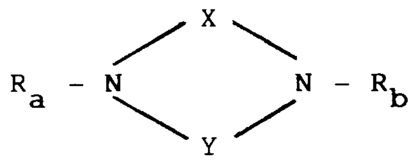

,(XIV)

in der

$R_a$, $R_b$, X und Y mit der Maßgabe wie eingangs definiert sind, daß einer der Reste $R_a$ bis $R_d$ eine NC - V - B - C - Gruppe darstellt, in der

B und C wie eingangs definiert sind und

V eine Bindung oder eine geradkettige oder verzweigte Alkylengruppe mit 1 bis 4 Kohlenstoffatomen darstellt.

Die Reduktion wird vorzugsweise in einem geeigneten Lösungsmittel wie Methanol, Methanol/Wasser, Methanol/Wasser/Ammoniak, Ethanol, Ether, Tetrahydrofuran, Dioxan oder Dimethylformamid gegebenenfalls unter Zusatz einer Säure wie Salzsäure in Gegenwart von katalytisch angeregtem Wasserstoff, z.B. von Wasserstoff in Gegenwart von Raney-Nickel, Platin oder Palladium/Kohle, oder in Gegenwart eines Metallhydrids wie Natriumborhydrid, Lithiumborhydrid oder Lithiumaluminiumhydrid bei Temperaturen zwischen 0 und 100 °C, vorzugsweise bei Temperaturen zwischen 20 und 80 °C, durchgeführt.

j) Zur Herstellung von Verbindungen der allgemeinen Formel I, in der A eine Aminoalkylgruppe darstellt, in der die Aminogruppe nicht an ein quartäres Kohlenstoffatom gebunden ist, oder eine Aminogruppe darstellt, die an eine CH- oder $CH_2$-Gruppe des Restes B oder C gebunden ist:

Reduktion einer Verbindung der allgemeinen Formel

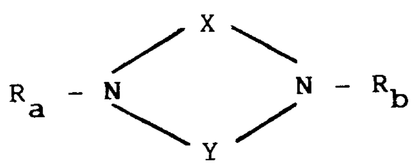

,(XV)

in der

$R_a$, $R_b$, X und Y mit der Maßgabe wie eingangs definiert sind, daß einer der Reste $R_a$ bis $R_d$ eine A - B - C -Gruppe darstellt, in der

A, B und C mit der Maßgabe wie eingangs definiert sind, daß eine im Rest A, im Rest A und B zusammen oder im Rest A und C zusammen vorhandene $H_2N$-CH- oder $H_2N$-$CH_2$-Gruppe durch eine $HO-N=C<$ oder HO-N=CH-Gruppe ersetzt ist.

Die Reduktion wird vorzugsweise in einem geeigneten Lösungsmittel wie Methanol, Methanol/Wasser, Methanol/Wasser/Ammoniak, Ethanol, Ether, Tetrahydrofuran, Dioxan oder Dimethylformamid, gegebenenfalls unter Zusatz einer Säure wie Salzsäure in Gegenwart von katalytisch angeregtem Wasserstoff, z.B. von Wasserstoff in Gegenwart von Raney-Nickel, Platin oder Palladium/Kohle, bei Temperaturen zwischen 0 und 100 °C, vorzugsweise bei Temperaturen zwischen 20 und 80 °C, durchgeführt.

k) Zur Herstellung von Verbindungen der allgemeinen Formel I, in der X eine durch eine Cyanogruppe substituierte Carbiminogruppe, eine Carbonyl-, Thiocarbonyl- oder Sulfonylgruppe darstellt:

Umsetzung einer Verbindung der allgemeinen Formel

$R_a$ - NH - Y' - NH - $R_b$ ,(XVI)

in der

$R_a$ und $R_b$ wie eingangs definiert sind und

Y' eine gegebenenfalls durch $R_c$ oder $R_d$ oder $R_c$ und $R_d$ substituierte geradkettige Alkylengruppe mit 2 oder 3 Kohlenstoffatomen, wobei eine Methylengruppe in einer Ethylengruppe zusätzlich durch eine Carbonylgruppe ersetzt sein kann, oder

eine gegebenenfalls durch $R_c$ oder $R_d$ substituierte -CO-NH-, -NH-CO-, -CH=N- oder -N=CH-Gruppe darstellt, mit einer Verbindung der allgemeinen Formel

$Z_5$ - X' - $Z_6$ ,(XVII)

in der
X' eine durch eine Cyanogruppe substituierte Carbiminogruppe, eine Carbonyl-, Thiocarbonyl- oder Sulfonylgruppe,
$Z_5$ und $Z_6$, die gleich oder verschieden sein können, nukleophile Austrittsgruppen wie Halogenatome, Alkoxy- oder Aryloxygruppen, z.B. jeweils ein Chloratom oder eine Methoxy-, Ethoxy-, Phenyloxy- oder N-Imidazolylgruppe, darstellen.

Die Umsetzung wird vorzugsweise in einem Lösungsmittel wie Methylenchlorid, Chloroform, Toluol oder Dioxan gegebenenfalls in Gegenwart einer Base wie Natriumhydrid, Triethylamin oder Pyridin bei Temperaturen zwischen 0 und 100 °C, vorzugsweise bei Temperaturen zwischen 20 und 60 °C, durchgeführt.

l) Zur Herstellung von Verbindungen der allgemeinen Formel I, in der X eine durch eine Cyanogruppe substituierte Carbiminogruppe, eine Carbonyl- oder Sulfonylgruppe und Y eine gegebenenfalls durch $R_c$ oder $R_d$ oder $R_c$ und $R_d$ substituierte geradkettige Alkylengruppe mit 2 oder 3 Kohlenstoffatomen darstellt:
Cyclisierung einer Verbindung der allgemeinen Formel

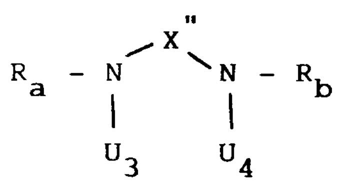
,(XVIII)

in der
$R_a$ und $R_b$ wie eingangs definiert sind,
X'' eine durch eine Cyanogruppe substituierte Carbiminogruppe, eine Carbonyl- oder Sulfonylgruppe,
einer der Reste $U_3$ oder $U_4$ ein Wasserstoffatom und der andere der Reste $U_3$ oder $U_4$ eine gegebenenfalls durch $R_c$ oder $R_d$ oder $R_c$ und $R_d$ substituierte geradkettige Alkylengruppe mit 2 oder 3 Kohlenstoffatomen, die zusätzlich endständig durch eine nukleophile Austrittsgruppe wie ein Halogenatom, eine Hydroxy- oder Sulfonsäureestergruppe, z. B. durch ein Chlor-, Brom- oder Jodatom, durch eine Methansulfonyloxy- oder p-Toluolsulfonyloxygruppe, substituiert ist.

Die Umsetzung wird vorzugsweise in einem Lösungsmittel wie Methylenchlorid, Acetonitril, Tetrahydrofuran, Toluol, Dimethylformamid oder Dimethylsulfoxid gegebenenfalls in Gegenwart einer Base wie Natriumhydrid, Kaliumkarbonat, Kalium-tert.butylat oder N-Ethyl-diisopropylamin und gegebenenfalls in Gegenwart eines wasserentziehenden Mittels wie Triphenylphospin/Azodicarbonsäure-diethylester bei Temperaturen zwischen -20 und 100 °C, vorzugsweise bei Temperaturen zwischen 0 und 60 °C, durchgeführt.

m) Zur Herstellung von Verbindungen der allgemeinen Formel I, in der X eine Carbonyl- oder Sulfonylgruppe darstellt:
Umsetzung einer Verbindung der allgemeinen Formel

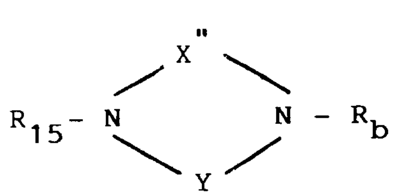
,(XIX)

mit einer Verbindung der allgemeinen Formel

$Z_7$ - $R_{16}$ ,(XX)

in denen
Y wie eingangs definiert ist,

X'' eine durch eine Cyanogruppe substituierte Carbiminogruppe, eine Carbonyl- oder Sulfonylgruppe, einer der Reste $R_{15}$ oder $R_{16}$ die für $R_a$ eingangs erwähnten Bedeutungen besitzt und der andere der Reste $R_{15}$ oder $R_{16}$ die für $R_b$ eingangs erwähnten Bedeutungen besitzt und $Z_7$ eine nukleophile Austrittsgruppe wie ein Halogenatom, eine Hydroxy- oder Sulfonsäureestergruppe, z. B. ein Fluor-, Chlor-, Brom- oder Jodatom, eine Methansulfonyloxy- oder p-Toluolsulfonyloxygruppe, bedeuten.

Die Umsetzung wird vorzugsweise in einem Lösungsmittel wie Methylenchlorid, Acetonitril, Tetrahydrofuran, Toluol, Pyridin, Dimethylformamid, Dimethylsulfoxid oder N-Methyl-pyrrolidon gegebenenfalls in Gegenwart einer Base wie Natriumhydrid, Kaliumkarbonat, Kalium-tert.butylat, N-Ethyl-diisopropylamin oder N,N,N',N'-Tetramethylethylendiamin und gegebenenfalls in Gegenwart eines wasserentziehenden Mittels wie Triphenylphosphin/Azodicarbonsäure-diethylester und gegebenenfalls in Gegenwart von Kupferpulver oder eines Kupfersalzes wie Kupfer(I)jodid als Reaktionsbeschleuniger bei Temperaturen zwischen -20 und 220°C, vorzugsweise jedoch bei Temperaturen zwischen 0 und 60°C, falls $Z_7$ an ein aliphatisches Kohlenstoffatom gebunden ist, oder bei Temperaturen zwischen 60 und 180°C, falls $Z_7$ an ein aromatisches Kohlenstoffatom gebunden ist, wobei in diesem Falle $Z_7$ nur ein Halogenatom darstellen kann.

n) Zur Herstellung von Verbindungen der allgemeinen Formel I, in der A eine Alkylgruppe darstellt: Umsetzung einer Verbindung der allgemeinen Formel

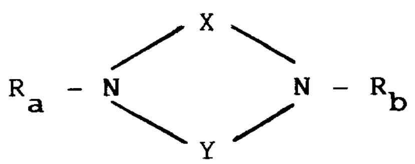

,(XXI)

in der

$R_a$, $R_b$, X und Y mit der Maßgabe wie eingangs definiert sind, daß einer der Reste $R_a$ bis $R_d$ eine A''' - B - C -Gruppe darstellt, in der

B und C wie eingangs definiert sind und

A''' ein Wasserstoffatom darstellt,

mit einer Verbindung der allgemeinen Formel

$Z_8$ - $R_{22}$ ,(XXII)

in der

$R_{22}$ eine Alkylgruppe mit 1 bis 3 Kohlenstoffatomen und $Z_8$ eine nukleophile Austrittsgruppe wie ein Halogenatom, z.B. ein Chlor-, Brom- oder Jodatom, oder eine Sulfonsäureestergruppe, z.B. eine Methansulfonyloxy- oder p-Toluolsulfonyloxygruppe, oder

$Z_8$ zusammen mit einem benachbarten Wasserstoffatom des Restes $R_{22}$ ein Sauerstoffatom bedeuten.

Die Alkylierung mit einer Verbindung der Formel XXII, in der $Z_8$ eine nukleophile Austrittsgruppe darstellt, wird zweckmäßigerweise in einem Lösungsmittel wie Methylenchlorid, Tetrahydrofuran, Dioxan, Dimethylsulfoxid oder Dimethylformamid gegebenenfalls in Gegenwart einer Base wie Natriumcarbonat, Kaliumcarbonat oder Natronlauge oder in Gegenwart einer tertiären organischen Base wie N-Ethyl-diisopropylamin oder N-Methyl-morpholin, welche gleichzeitig als Lösungsmittel dienen können, bei Temperaturen zwischen -30 und 100°C, vorzugsweise jedoch bei Temperaturen zwischen -10 und 80°C, durchgeführt.

Die reduktive Alkylierung mit einer Carbonylverbindung der allgemeinen Formel XXII wird in Gegenwart eines komplexen Metallhydrids wie Natriumborhydrid, Lithiumborhydrid oder Natriumcyanborhydrid zweckmäßigerweise bei einem pH-Wert von 6-7 und bei Raumtemperatur oder in Gegenwart eines Hydrierungskatalysators, z.B. mit Wasserstoff in Gegenwart von Palladium/Kohle, bei einem Wasserstoffdruck 1 bis 5 bar durchgeführt. Die Methylierung wird jedoch vorzugsweise in Gegenwart von Ameisensäure als Reduktionsmittel bei erhöhten Temperaturen, z. B. bei Temperaturen zwischen 60 und 120°C, durchgeführt.

o) Zur Herstellung von Verbindungen der allgemeinen Formel I, in der mindestens einer der Reste B, C, D oder E eine gegebenenfalls durch eine Alkylgruppe substituierte Cyclohexylengruppe darstellt: Hydrierung einer Verbindung der allgemeinen Formel

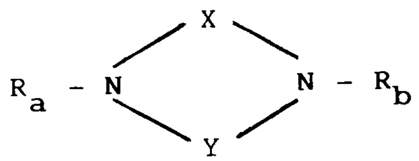

,(XXIII)

in der

$R_a$, $R_b$, X und Y mit der Maßgabe wie eingangs definiert sind, daß mindestens einer der Reste B, C, D oder E eine gegebenenfalls durch eine Alkylgruppe substituierte Phenylengruppe darstellt.

Die katalytische Hydrierung wird vorzugsweise in einem geeigneten Lösungsmittel wie Methanol, Methanol/Wasser, Essigsäure, Essigester, Ethanol, Ether, Tetrahydrofuran, Dioxan oder Dimethylformamid, gegebenenfalls unter Zusatz einer Säure wie Salzsäure in Gegenwart von katalytisch angeregtem Wasserstoff, z.B. von Wasserstoff in Gegenwart von Raney-Nickel, Platin, Rhodium oder Palladium/Kohle, bei Temperaturen zwischen 0 und 100 °C, vorzugsweise bei Temperaturen zwischen 20 und 80 °C, durchgeführt.

p) Zur Herstellung von Verbindungen der allgemeinen Formel I, in der A eine Cyanogruppe, B eine Bindung und C eine gegebenenfalls durch eine Alkylgruppe substituierte 4- bis 7-gliedrige Cycloalkylengruppe oder B eine gegebenenfalls durch eine Alkylgruppe substituierte Cyclohexylengruppe darstellen:
Umsetzung einer Verbindung der allgemeinen Formel

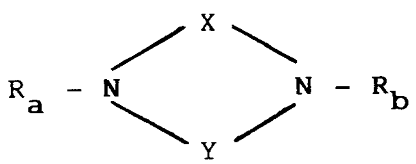

,(XXIV)

in der

$R_a$, $R_b$, X und Y mit der Maßgabe wie eingangs definiert sind, daß einer der Reste $R_a$ bis $R_d$ eine A-B-C-Gruppe darstellt, in der A zusammen mit einer -CH-Gruppe in einem der für B oder C eingangs erwähnten 4- bis 7-gliedrigen Cycloalkylgruppen eine Carbonylgruppe darstellt, mit einer Verbindung der allgemeinen Formel

$Z_9$ - $CH_2$ - NC ,(XXV)

in der $Z_9$ eine nukleophile Austrittsgruppe wie die p-Toluolsulfonylgruppe darstellt.

Die Umsetzung wird vorzugsweise in einem Lösungsmittel wie Tetrahydrofuran, Ethylenglykoldimethylether, tert.Butanol oder Ethylenglykoldimethylether/tert.Butanol in Gegenwart einer Base wie Kalium-tert.butylat bei Temperaturen zwischen -25 und 50 °C, vorzugsweise bei Temperaturen zwischen -20 °C und Raumtemperatur, durchgeführt.

q) Zur Herstellung von Verbindungen der allgemeinen Formel I, in der F eine Carbonylgruppe darstellt, die durch eine Alkoxygruppe mit 1 bis 6 Kohlenstoffatomen, eine Phenylalkoxygruppe oder $R_8$-CO-O-$CHR_9$-O-Gruppe substituiert ist, darstellt:
Umsetzung einer Verbindung der allgemeinen Formel

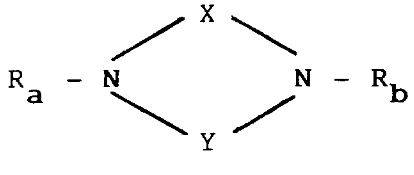

,(XXVI)

in der

$R_a$, $R_b$, X und Y mit der Maßgabe wie eingangs definiert sind, daß einer der Reste $R_a$ bis $R_d$ eine F''' - E - D -Gruppe darstellt, in der

E und D wie eingangs definiert sind und

F''' eine Carboxylgruppe darstellt,
mit einer Verbindung der allgemeinen Formel

$Z_{10} - R_{23}$ ,(XXVII)

in der
$R_{23}$ eine Alkylgruppe mit 1 bis 6 Kohlenstoffatomen, eine Phenylalkyl- oder $R_8$-CO-O-$CHR_9$-Gruppe darstellt, wobei $R_8$ und $R_9$ wie eingangs definiert sind, und
$Z_{10}$ eine nukleophile Austrittsgruppe wie ein Halogenatom oder eine Sulfonsäureestergruppe, z. B. ein Chlor- oder Bromatom, eine Methansulfonyloxy- oder p-Toluolsulfonyloxygruppe, bedeuten.

Die Umsetzung wird vorzugsweise in einem Lösungsmittel wie Methylenchlorid, Tetrahydrofuran, Dioxan, Dimethylsulfoxid oder Dimethylformamid gegebenenfalls in Gegenwart eines Reaktionsbeschleunigers wie Natrium- oder Kaliumjodid und vorzugsweise in Gegenwart einer Base wie Natriumkarbonat, Kaliumkarbonat oder Natronlauge oder in Gegenwart einer tertiären organischen Base wie N-Ethyl-diisopropylamin oder N-Methyl-morpholin, welche gleichzeitig auch als Lösungsmittel dienen können, oder gegebenenfalls in Gegenwart von Silberkarbonat oder Silberoxid bei Temperaturen zwischen -30 und 100 °C, vorzugsweise jedoch bei Temperaturen zwischen -10 und 80 °C, durchgeführt.

r) Zur Herstellung von Verbindungen der allgemeinen Formel I, in der A zusammen mit B eine Cyanoalkoxygruppe mit 1 bis 10 Kohlenstoffatomen im Alkoxyteil darstellt, die über das Sauerstoffatom mit einem Kohlenstoffatom der Gruppe C verknüpft ist:
Umsetzung einer Verbindung der allgemeinen Formel

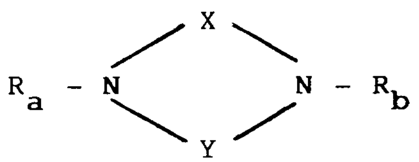
,(XXVIII)

in der
$R_a$, $R_b$, X und Y mit der Maßgabe wie eingangs definiert sind, daß einer der Reste $R_a$ bis $R_d$ eine HO-C-Gruppe darstellt, wobei die Hydroxygruppe an ein Kohlenstoffatom des Restes C gebunden ist, mit einer Verbindung der allgemeinen Formel

$Z_{11} - R_{24}$ ,(XXIX)

in der
$R_{24}$ eine Cyanoalkylgruppe mit 1 bis 10 Kohlenstoffatomen im Alkylteil und
$Z_{11}$ eine nukleophile Austrittsgruppe wie ein Halogenatom, z.B. ein Chlor-, Brom- oder Jodatom, oder eine Sulfonsäureestergruppe, z.B. eine Methansulfonyloxy- oder p-Toluolsulfonyloxygruppe bedeuten.

Die Umsetzung wird zweckmäßigerweise in einem Lösungsmittel wie Methylenchlorid, Tetrahydrofuran, Dioxan, Dimethylsulfoxid oder Dimethylformamid vorzugsweise in Gegenwart einer Base wie Natriumcarbonat, Kaliumcarbonat oder Natronlauge oder in Gegenwart einer tertiären organischen Base wie N-Ethyl-diisopropylamin oder N-Methyl-morpholin, welche gleichzeitig als Lösungsmittel dienen können, bei Temperaturen zwischen -30 und 100 °C, vorzugsweise jedoch bei Temperaturen zwischen -10 und 80 °C, durchgeführt.

Erhält man erfindungsgemäß eine Verbindung der allgemeinen Formel I, die eine Cyanogruppe enthält, so kann diese durch Behandlung mit einer wässrigen Säure oder Base in eine Aminocarbonyl- oder Carboxygruppe übergeführt werden oder
eine Verbindung der allgemeinen Formel I, die eine Kohlenstoff-Kohlenstoff-Doppelbindung enthält, so kann diese durch Reduktion in eine entsprechende gesättigte Verbindung übergeführt werden.

Die nachträgliche Umwandlung einer Cyanogruppe in eine Aminocarbonylgruppe wird zweckmäßigerweise in Schwefelsäure in Gegenwart von Wasser oder mit Natriumcarbonat oder Kaliumcarbonat in Gegenwart von wässriger Wasserstoffperoxidlösung gegebenenfalls unter Verwendung eines Lösungsmittels wie Dimethylsulfoxid, vorzugsweise jedoch in 85%iger Schwefelsäure bei Raumtemperatur, durchgeführt.

Die nachträgliche Umwandlung einer Cyanogruppe in eine Carboxygruppe wird zweckmäßigerweise mit wässriger Schwefelsäure oder Natron- oder Kalilauge bei erhöhten Temperaturen, vorzugsweise jedoch mit

wässriger Schwefelsäure bei Temperaturen zwischen 80 °C und der Siedetemperatur des Reaktionsgemisches, durchgeführt.

Die nachträgliche Reduktion einer Kohlenstoff-Kohlenstoff-Doppelbindung erfolgt vorzugsweise durch katalytische Hydrierung in einem geeigneten Lösungsmittel wie Methanol, Methanol/Wasser, Essigsäure, Essigester, Ethanol, Ether, Tetrahydrofuran, Dioxan oder Dimethylformamid, gegebenenfalls unter Zusatz einer Säure wie Salzsäure in Gegenwart von katalytisch angeregtem Wasserstoff, z.B. von Wasserstoff in Gegenwart von Raney-Nickel, Platin, Rhodium oder Palladium/Kohle, oder in Gegenwart eines Wasserstoffdonators wie 1,3-Cyclohexadien oder Ammoniumformiat in Gegenwart eines Katalysators wie Palladium/Kohle, Platinoxid oder Raney-Nickel in einem Lösungsmittel wie Methanol, Ethanol, Wasser, Dioxan, Essigsäure oder Essigester bei Temperaturen zwischen 0 und 100 °C, vorzugsweise bei Temperaturen zwischen 20 und 80 °C.

Bei den vorstehend beschriebenen Umsetzungen können gegebenenfalls vorhandene reaktive Gruppen wie Hydroxy-, Carboxy-, Phosphono-, O-Alkyl-phosphono-, Amino-, Alkylamino-, Imino- oder Amidinogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, tert.Butyl-, Trityl-, Benzyl- oder Tetrahydropyranylgruppe,
als Schutzreste für eine Carboxylgruppe die Trimethylsilyl-, Methyl-, Ethyl-, tert.Butyl-, Benzyl- oder Tetrahydropyranylgruppe,
als Schutzreste für eine Phosphonogruppe eine Alkylgruppe wie die Methyl-, Ethyl-, Isopropyl- oder n-Butylgruppe, die Phenyl- oder Benzylgruppe,
als Schutzrest für eine gegebenenfalls durch eine Alkylgruppe substituierte Amidinogruppe die Benzyloxycarbonylgruppe,
als Schutzrest für eine Amino-, Alkylamino- oder Iminogruppe die Formyl-, Acetyl-, Trifluoracetyl-, Ethoxycarbonyl-, tert.Butoxycarbonyl-, Benzyloxycarbonyl-, Benzyl-, Methoxybenzyl-oder 2,4-Dimethoxybenzylgruppe und für die Aminogruppe zusätzlich die Phthalylgruppe und
als Schutzrest für das Stickstoffatom einer 1-Aza-bicycloalkylgruppe wie der Chinuclidinylgruppe die Benzylgruppe oder Boran in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen 0 und 120 °C, vorzugsweise bei Temperaturen zwischen 10 und 100 °C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 100 °C, vorzugsweise jedoch bei Temperaturen zwischen 20 und 60 °C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Ether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120 °C oder durch Behandlung mit Natronlauge gegebenenfalls in Gegenwart eines Lösungsmittels wie Tetrahydrofuran bei Temperaturen zwischen 0 und 50 °C.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50 °C.

Die Spaltung des Komplexes einer 1-Aza-bicycloalkylgruppe wie der Chinuclidinylgruppe mit Boran erfolgt vorzugsweise durch Behandlung mit einer Säure wie Salzsäure und gegebenenfalls in Gegenwart eines Lösungsmittels wie Methanol, Ethanol, Essigsäure oder Dioxan bei Temperaturen zwischen 0 °C und der Siedetemperatur des Reaktionsgemisches. Bei dieser Umsetzung kann eine gegebenenfalls vorhandene Estergruppe gleichzeitig in die entsprechende Carboxygruppe übergeführt werden.

Die Abspaltung nur eines Alkylrestes von einer O,O'-Dialkylphosphonogruppe erfolgt beispielsweise mit Natriumjodid in einem Lösungsmittel wie Aceton, Ethylmethylketon, Acetonitril oder Dimethylformamid bei

Temperaturen zwischen 40 und 150 ° C, vorzugsweise jedoch bei Temperaturen zwischen 60 und 100 ° C.

Die Abspaltung beider Alkylreste von einer O,O'-Dialkylphosphonogruppe erfolgt beispielsweise mit Jodtrimethylsilan, Bromtrimethylsilan oder Chlortrimethylsilan/Natriumjodid in einem Lösungsmittel wie Methylenchlorid, Chloroform oder Acetonitril bei Temperaturen zwischen O ° C und der Siedetemperatur des Reaktionsgemisches, vorzugsweise jedoch bei Temperaturen zwischen 20 und 60 ° C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestes 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)- oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxy-, Phosphono-, O-Alkylphosphono- oder 5-Tetrazolylgruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Arginin, Cyclohexylamin, Äthanolamin, Diäthanolamin und Triäthanolamin in Betracht.

Die als Ausgangsstoffe verwendeten Verbindungen sind teilweise literaturbekannt oder man erhält diese nach literaturbekannten Verfahren (siehe Beispiele).

So wird beispielsweise in "The Organic Chemistry of Heterocyclic Compounds", Band 37, von C. Temple, Jr., Verlag John Wiley & Sons, 1981, in den Kapiteln 13, 14 und 19 die Herstellung von entsprechenden Triazol-Verbindungen beschrieben.

In Houben-Weyl, "Methoden der Organischen Chemie", Band E4, von H. Hagemann, Georg Thieme Verlag, 1983, wird beispielsweise ab Seite 368 die Herstellung von entsprechenden cyclischen Harnstoffverbindungen beschrieben. Außerdem wird im gleichen Band ab Seite 355 beispielsweise auch die Herstellung von entsprechenden gegebenenfalls als Ausgangsverbindungen benötigten offenkettigen Harnstoffverbindungen beschrieben.

So erhält man beispielsweise ein entsprechendes cyclisches Harnstoffderivat durch Cyclisierung eines entsprechend substituierten Harnstoffs, welcher seinerseits durch Umsetzung eines entsprechenden Amins mit einem entsprechenden Isocyanat erhalten wird, oder durch Umsetzung eines entsprechend substituierten Diamins mit einem Kohlensäurederivat wie Phosgen oder

ein entsprechendes Triazolonderivat durch Cyclisierung eines entsprechenden Semicarbazids, welches seinerseits durch Umsetzung eines entsprechenden Isocyanats mit einem entsprechenden Hydrazid erhalten wird.

In den so erhaltenen cyclischen Harnstoffderivaten kann gegebenenfalls anschließend eine Carbonylgruppe in eine entsprechende Thiocarbonyl- oder Carbiminogruppe mittels bekannten Methoden übergeführt werden.

In den so erhaltenen cyclischen Ausgangsverbindungen bzw. bereits in den für ihre Herstellung erforderlichen Ausgangsverbindungen kann
eine gegebenenfalls vorhandene Estergruppe mittels Hydrolyse in eine Carboxygruppe,
eine gegebenenfalls vorhandene Carboxygruppe in eine Ester- oder Amidgruppe,
eine gegebenenfalls vorhandene Aminocarbonylgruppe mittels Dehydratisierung in eine Cyanogruppe,
eine gegebenenfalls vorhandene Cyanogruppe in eine Amidinogruppe,
eine gegebenenfalls vorhandene Carbonylgruppe in ihr Oxim,
eine gegebenenfalls vorhandene funktionelle Gruppe wie eine Hydroxy-, Amino- oder 5-Tetrazolylgruppe mittels Alkylierung, Sulfonylierung, Acylierung oder Tritylierung in das entsprechende Derivat,
eine gegebenenfalls vorhandene Cyanogruppe in eine Tetrazolylgruppe oder
ein gegebenenfalls vorhandenes reaktives Bromatom mittels eines Metallcyanids in die Cyanogruppe übergeführt werden.

Wie bereits eingangs erwähnt, weisen die neuen cyclischen Harnstoffderivate der allgemeinen Formel I und deren Salze, insbesondere deren physiologisch verträgliche Salze mit anorganischen oder organischen Säuren oder Basen, wertvolle Eigenschaften auf. So weisen die neuen Verbindungen der allgemeinen Formel I, in denen A, A zusammen mit B, A zusammen mit C oder A zusammen mit B und C eine basische Gruppe oder eine gegebenenfalls in vivo in eine basische Gruppe überführbare Gruppe enthält und F eine Carboxyl-, Phosphono-, O-Alkyl-phosphono- oder 5-Tetrazolylgruppe oder eine gegebenenfalls in vivo in eine Carboxyl-, Phosphono-, O-Alkyl-phosphono- oder 5-Tetrazolylgruppe überführbare Gruppe, z.B. eine durch eine Alkoxy- oder Cycloalkoxygruppe substituierte Carbonylgruppe, darstellt, wertvolle pharmakologische Eigenschaften auf, neben einer entzündungshemmenden und den Knochenabbau hemmenden Wirkung insbesondere antithrombotische, antiaggregatorische und tumor- bzw. metastasenhemmende Wirkungen.

Die Verbindungen der allgemeinen Formel I, in denen A eine Cyano- oder Cyanoalkylgruppe darstellt, stellen wertvolle Zwischenprodukte zur Herstellung der entsprechenden Aminoalkyl- und Amidinoverbindungen der allgemeinen Formel I dar. Ferner stellen die Verbindungen der allgemeinen Formel I, in denen A eine gegebenenfalls im Phenylteil durch 1 bis 2 Methoxygruppen substituierte Benzylgruppe, eine Formyl-, Acetyl-, Trifluoracetyl-, Benzyloxycarbonyl- oder Alkoxycarbonylgruppe darstellt, wertvolle Zwischenprodukte zur Herstellung der entsprechenden Iminoverbindungen der allgemeinen Formel I dar, in denen A ein Wasserstoffatom darstellt.

Außerdem stellen Verbindungen der allgemeinen Formel I, in denen A zusammen mit B oder A zusammen mit B und C eine am Stickstoffatom durch Boran komplexierte oder durch eine im Phenylkern gegebenenfalls durch 1 oder 2 Methoxygruppen substituierte Benzylgruppe quartärnisierte Azabicycloalkylgruppe darstellt, wertvolle Zwischenprodukte zur Herstellung der entsprechenden am Stickstoffatom nicht komplexierten und nicht quartärnisierten Verbindungen der allgemeinen Formel I dar.

Beispielsweise wurden die Verbindungen der allgemeinen Formel I auf ihre biologischen Wirkungen wie folgt untersucht:

1. <u>Hemmung der Bindung von $^{3}$H-BIBU 52 an Humanthrombozyten:</u>

Eine Suspension von Humanthrombozyten in Plasma wird mit $^{3}$H-BIBU 52 [= (3S,5S)-5-((4'-Amidino-4-biphenylyl)oxymethyl]-3-[(carboxyl)methyl]-2-pyrrolidinon[3-$^{3}$H-4-biphenylyl]], das den literaturbekannten Liganden $^{125}$J-Fibrinogen ersetzt, (siehe deutsche Patentanmeldung P 42 14 245.8 der gleichen Anmelderin vom 30. 04. 1992, internes Zeichen: Case 5/1093-FL) und verschiedenen Konzentrationen der zu testenden Substanz inkubiert. Der freie und gebundene Ligand wird durch Zentrifugation getrennt und durch Szintillationszählung quantitativ bestimmt. Aus den Meßwerten wird die Hemmung der $^{3}$H-BIBU 52-Bindung durch die Testsubstanz bestimmt.

Hierzu wird aus einer Antikubitalvene Spenderblut entnommen und mit Trinatriumzitrat antikoaguliert (Endkonzentration 13 mM). Das Blut wird 10 Minuten bei 170 x g zentrifugiert und das überstehende plättchenreiche Plasma (PRP) abgenommen. Das Restblut wird zur Gewinnung von Plasma nocheinmal scharf abzentrifugiert. Das PRP wird mit autologem Plasma 1:10 verdünnt. 750 µl werden mit 50 µl physiologischer Kochsalzlösung, 100 µl Testsubstanzlösung, 50 µl $^{14}$C-Sucrose (3.700 Bq) und 50 µl $^{3}$H-BIBU 52 (Endkonzentration: 5 nM) bei Raumtemperatur 20 Minuten inkubiert. Zur Messung der unspezifischen Bindung wird anstelle der Testsubstanz 5 µl BIBU 52 (Endkonzentration: 30 µM) eingesetzt. Die Proben werden 20 Sekunden bei 10000 x g zentrifugiert und der Überstand abgezogen. 100 µl hiervon werden zur Bestimmung des freien Liganden gemessen. Das Pellet wird in 500 µl 0,2N NaOH gelöst, 450 µl werden mit 2 ml Szintillator und 25 µl 5N HCl versetzt und gemessen. Das im Pellet noch verbliebene

Restplasma wird aus dem $^{14}C$-Gehalt bestimmt, der gebundene Ligand aus der $^{3}H$-Messung. Nach Abzug der unspezifischen Bindung wird die Pelletaktivität gegen die Konzentration der Testsubstanz aufgetragen und die Konzentration für eine 50%ige Hemmung der Bindung ermittelt.

2. Antithrombotische Wirkung:

Methodik: Die Thrombozytenaggregation wird nach der Methode von Born und Cross (J. Physiol. 170, 397 (1964)) in plättchenreichem Plasma gesunder Versuchspersonen gemessen. Zur Gerinnungshemmung wird das Blut mit Natriumcitrat 3,14 % im Volumenverhältnis 1:10 versetzt.

Collagen-induzierte Aggregation

Der Verlauf der Abnahme der optischen Dichte der Plättchensuspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der "optical density".

Die Collagen-Menge wird möglichst gering gewählt, aber doch so, daß sich eine irreversibel verlaufende Reaktionskurve ergibt. Verwendet wird das handelsübliche Collagen der Firma Hormonchemie, München.

Vor der Collagen-Zugabe wird das Plasma jeweils 10 Minuten mit der Substanz bei 37 °C inkubiert.

Aus den erhaltenen Meßzahlen wird graphisch eine $EC_{50}$ bestimmt, die sich auf eine 50%ige Änderung der "optical density" im Sinne einer Aggregationshemmung bezieht.

Die nachfolgende Tabelle enthält die gefundenen Ergebnisse:

| Substanz (Beispiel Nr.) | Fibrinogen-Bindungstest $IC_{50}$[nM] | Hemmung der Plättchenaggregation $EC_{50}$[nM] |
|---|---|---|
| 1(1) | 0,8 | 30 |
| 1(2) | 1,2 | 70 |
| 1(3) | 64,0 | 270 |
| 1(4) | 280,0 | 500 |
| 1(6) | 18,0 | 100 |
| 1(8) | 760,0 | 1.200 |
| 1(21) | 1,0 | 36 |
| 1(39) | 25,0 | 86 |
| 1(44) | - | 380 |
| 1(50) | 65,0 | 170 |
| 2(5) | 3.500,0 | 3.200 |
| 4 | 94,0 | 130 |
| 4(4) | 2,9 | 30 |
| 4(5) | 4.100,0 | 3.300 |
| 4(13) | 250,0 | 300 |
| 5(2) | 720,0 | 840 |
| 6(14) | 250,0 | 40 |
| 6(34) | 21.000,0 | 350 |
| 6(35) | 5.700,0 | 630 |
| 6(37) | 7.100,0 | 380 |
| 6(40) | 2.500,0 | 240 |
| 15 | 12.000,0 | 4.300 |
| 17 | 16,0 | 43 |
| 18(2) | - | 110 |
| 24(1) | 150,0 | 940 |
| 24(2) | - | 71 |
| 26 | 26,0 | 84 |
| 30(4) | 200,0 | 490 |

Außerdem hemmt beispielsweise die Verbindung des Beispiels 6(14) die durch Collagen-induzierte Thrombozytenaggregation ex vivo am Rhesusaffen nach oraler Gabe von 1 mg/kg bis zu 4 Stunden.

Die erfindungsgemäßen Verbindungen sind gut verträglich, da beispielsweise nach intravenöser Gabe von 30 mg/kg der Verbindungen der Beispiele 1(1), 1(4), 1(6), 1(39), 17 und 26 an der Maus keines der 3 getesteten Tiere verstarb.

Auf Grund ihrer Hemmwirkung auf Zell-Zell- bzw. Zell-Matrix-Wechselwirkungen eignen sich die neuen cyclischen Harnstoffderivate der allgemeinen Formel I und ihre physiologisch verträglichen Salze zur Bekämpfung bzw. Verhütung von Krankheiten, bei denen kleinere oder größere Zell-Aggregate auftreten oder Zell-Matrixinteraktionen eine Rolle spielen, z.B. bei der Bekämpfung bzw. Verhütung von venösen und arteriellen Thrombosen, von zerebrovasculären Erkrankungen, von Lungenembolien, des Herzinfarktes, der Arteriosklerose, der Osteoporose und der Metastasierung von Tumoren und der Therapie genetisch bedingter oder auch erworbener Störungen der Interaktionen von Zellen untereinander oder mit soliden Strukturen. Weiterhin eignen sich diese zur Begleittherapie bei der Thrombolyse mit Fibrinolytica oder Gefäßinterventionen wie transluminaler Angioplastie oder auch bei der Therapie von Schockzuständen, der Psoriasis, des Diabetes und von Entzündungen.

Für die Bekämpfung bzw. Verhütung der vorstehend erwähnten Krankheiten liegt die Dosis zwischen 0,1 $\mu$g und 30 mg/kg Körpergewicht, vorzugsweise bei 1 $\mu$g bis 15 mg/kg Körpergewicht, bei bis zu 4 Gaben pro Tag. Hierzu lassen sich die erfindungsgemäß hergestellten Verbindungen der Formel I, gegebenenfalls in Kombination mit anderen Wirksubstanzen wie Thromboxan-Rezeptor-Antagonisten und Thromboxansynthesehemmer oder deren Kombinationen, Serotonin-Antagonisten, $\alpha$-Rezeptorantagonisten, Alkylnitrate wie Glycerintrinitrat, Phosphodiesterasehemmer, Prostacyclin und deren Analoga, Fibrinolytica wie tPA, Prourokinase, Urokinase, Streptokinase, oder Antikoagulantien wie Heparin, Dermatansulfat, aktiviertes Protein C, Vitamin K-Antagonisten, Hirudin, Inhibitoren des Thrombins oder anderer aktivierter Gerinnungsfaktoren, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Stearylalkohol, Carboxymethylcellulose oder fetthaltigen Substanze wie Hartfett oder deren geeigneten Gemischen, in übliche galenische Zubereitungen wie Tabletten, Dragees, Kapseln, Pulver, Suspensionen, Lösungen, Sprays oder Zäpfchen einarbeiten.

Die nachfolgenden Beispiele sollen die Erfindung näher erläutern:

Beispiel I

1-Acetyl-2-(4-cyanophenyl)-4-[4-(1-ethoxycarbonyl-2-propyl)phenyl]-semicarbazid

2,79 g 1-Acetyl-2-(4-cyanophenyl)-hydrazin und 4,13 g [4-(1-Ethoxycarbonyl-2-propyl)phenyl]isocyanat werden unter Stickstoff 2 Stunden bei 100 °C gerührt. Danach wird abgekühlt. Das Produkt wird ohne weitere Reinigung in Beispiel 3 weiter umgesetzt.
Ausbeute: 6,5 g (94 % der Theorie),
$R_f$-Wert: 0,37 (Kieselgel; Methylenchlorid/Essigester/Cyclohexan = 20:1:1)
Analog Beispiel I werden folgende Verbindungen erhalten:

(1) 1-Acetyl-2-(4-cyanophenyl)-4-[4-(2-ethoxycarbonyl-1-propyl)phenyl]-semicarbazid
$R_f$-Wert: 0,32 (Kieselgel; Methylenchlorid/Essigester = 20:1)

(2) 1-Acetyl-2-(4-cyanophenyl)-4-[cis-4-[2-(methoxycarbonyl)ethyl]cyclohexyl]-semicarbazid
Schmelzpunkt: 138-140 °C
$R_f$-Wert: 0,54 (Kieselgel; Methylenchlorid/Essigester = 4:1)

(3) 1-Acetyl-2-(4-cyanophenyl)-4-[trans-4-[2-(methoxycarbonyl)ethyl]cyclohexyl]-semicarbazid
Schmelzpunkt: 132-134 °C
$R_f$-Wert: 0,54 (Kieselgel; Methylenchlorid/Essigester = 4:1)

(4) 1-Acetyl-2-(4-cyanophenyl)-4-[3-[2-(methoxycarbonyl)ethyl]phenyl]-semicarbazid
$R_f$-Wert: 0,55 (Kieselgel; Methylenchlorid/Essigester = 9:1)

(5) 1-Trifluoracetyl-2-(4-cyanophenyl)-4-[4-[2-(methoxycarbonyl)ethyl]phenyl]-semicarbazid
$R_f$-Wert: 0,60 (Kieselgel; Methylenchlorid/Methanol = 20:1)

(6) 1-Acetyl-2-(4-cyanophenyl)-4-[4-[2-(methoxycarbonyl)ethyl]phenyl]-semicarbazid
$R_f$-Wert: 0,42 (Kieselgel; Methylenchlorid/Essigester = 9:1)

(7) 1-Benzoyl-2-(4-cyanophenyl)-4-[4-[2-(methoxycarbonyl)ethyl]phenyl]-semicarbazid
Schmelzpunkt: 76-81 °C
$R_f$-Wert: 0,32 (Kieselgel; Methylenchlorid/Essigester = 20:1)

(8) 1-Acetyl-4-[4-[2-(methoxycarbonyl)ethyl]phenyl]-semicarbazid
Schmelzpunkt: 159-165 °C

$R_f$-Wert: 0,37 (Kieselgel; Methylenchlorid/Methanol = 9:1)
(9) 2-(4-Cyanophenyl)-1-formyl-4-[trans-4-[2-(methoxycarbonyl)ethyl]cyclohexyl]-semicarbazid
Schmelzpunkt: 140-142 °C
$R_f$-Wert: 0,57 (Kieselgel; Methylenchlorid/Essigester = 4:1)
(10) 1-Acetyl-4-[4-[2-(methoxycarbonyl)-1-pentyl]phenyl]-semicarbazid
Schmelzpunkt: 109-111 °C

| | | | | | | |
|---|---|---|---|---|---|---|
| Ber.:<br>Gef.: | C | 60,88<br>60,81 | H | 7,51<br>7,51 | N | 12,53<br>12,30 |

(11) 1-Acetyl-4-[4-[2-(methoxycarbonyl)-3-methyl-1-butyl]phenyl]-semicarbazid
(12) 1-Acetyl-4-[4-[2-(methoxycarbonyl)-1-butyl]phenyl]-semicarbazid
(13) 1-Acetyl-4-[4-[2-(methoxycarbonyl)-1-propyl]phenyl]-semicarbazid
(14) 1-Acetyl-2-(4-cyanophenyl)-4-[4-[2-(methoxycarbonyl)-1-pentyl]phenyl]-semicarbazid
(15) 1-Formyl-4-[4-[2-(methoxycarbonyl)ethyl]phenyl]-semicarbazid
Schmelzpunkt: 146-148 °C
$R_f$-Wert: 0,34 (Kieselgel; Methylenchlorid/Methanol = 100:5)
(16) 1-Formyl-4-[trans-4-(methoxycarbonyl)cyclohexyl]-semicarbazid
Schmelzpunkt: 183 °C
$R_f$-Wert: 0,48 (Kieselgel; Methylenchlorid/Methanol = 5:1)
(17) 1-Acetyl-4-[trans-4-(methoxycarbonyl)cyclohexyl]-semicarbazid
Schmelzpunkt: 191 °C
$R_f$-Wert: 0,50 (Kieselgel; Methylenchlorid/Methanol = 5:1)

Beispiel II

3-(4-Bromphenyl)-1-[4-(2-carboxyethyl)phenyl]-imidazolidin-2-on

12,7 g 3-(4-Bromphenyl)-1-[4-[2-(ethoxycarbonyl)ethyl]phenyl]-imidazolidin-2-on, 300 ml Tetrahydrofuran, 100 ml Wasser und 23,6 ml 4N Natronlauge werden über Nacht bei Raumtemperatur gerührt. Der Niederschlag wird abgesaugt, in 1 l Wasser suspendiert, mit konzentrierter Salzsäure angesäuert und 2 Stunden gerührt. Anschließend wird abgesaugt, mit Wasser gewaschen und bei 60 °C getrocknet.
Ausbeute: 7,8 g (66 % der Theorie),
Schmelzpunkt: 244-246 °C

| | | | | | | | | |
|---|---|---|---|---|---|---|---|---|
| Ber.:<br>Gef.: | C | 55,54<br>55,65 | H | 4,40<br>4,48 | N | 7,20<br>7,24 | Br | 20,53<br>20,37 |

Beispiel III

1-(4-Bromphenyl)-3-[4-[2-(ethoxycarbonyl)ethyl]phenyl]-imidazolidin-2-on

Zu einer Lösung von 13,5 g N-(4-Bromphenyl)-N'-[4-[2-(ethoxycarbonyl)ethyl]phenyl]-N'-(2-hydroxyethyl)-harnstoff in 40 ml Methylenchlorid werden 3,7 g Methansulfonylchlorid gegeben und dann unter Kühlung in einem Eis/Methanol-Bad 3,8 g Triethylamin in 10 ml Methylenchlorid zugetropft. Nach einer Stunde Rühren ohne Kühlung wird Wasser zugesetzt, die Phasen getrennt und die wäßrige Phase mit Methylenchlorid ausgeschüttelt. Die vereinigten organischen Phasen werden mit Wasser gewaschen, getrocknet und eingeengt. Der Rückstand wird mit 9,3 g Natriumjodid in 250 ml Aceton 3 Stunden am Rückfluß erhitzt. Es wird zur Trockne eingeengt, der Rückstand in 100 ml Dimethylformamid gelöst und 3,7 g Kalium-tert.butylat in 25 ml Dimethylformamid unter Eiskühlung zugegeben. Nach 30 Minuten Rühren bei Raumtemperatur wird eingeengt, der Rückstand zwischen Wasser und Methylenchlorid verteilt und die wäßrige Phase mit Methylenchlorid extrahiert. Die vereinigten organischen Phasen werden mit Wasser gewaschen, getrocknet und einrotiert. Der Rückstand wird über Nacht mit Ethanol gerührt, es wird abgekühlt und das Produkt abgesaugt, mit Ethanol gewaschen und getrocknet.
Ausbeute: 8,9 g (69 % der Theorie),

$R_f$-Wert: 0,40 (Kieselgel; Methylenchlorid)

| | | | | | | | | |
|---|---|---|---|---|---|---|---|---|
| Ber.:<br>Gef.: | C | 57,57<br>57,34 | H | 5,07<br>4,99 | N | 6,71<br>6,83 | Br | 19,15<br>19,27 |

Beispiel IV

1-[4-[2-(Methoxycarbonyl)ethyl]phenyl]-imidazolidin-2-on

Zu 11,3 g N-(2-Chlorethyl)-N'-[4-[2-(methoxycarbonyl)ethyl]phenyl]-harnstoff in 15 ml Dimethylformamid wird eine Lösung von 4,45 g Kalium-tert.butylat in 15 ml Dimethylformamid ohne Kühlung zugetropft. Nach 2 Stunden Rühren wird das Reaktionsgemisch auf 600 ml Wasser gegossen. Der Niederschlag wird abgesaugt, mit Wasser gewaschen und bei 100°C getrocknet.
Ausbeute: 8,84 g (89 % der Theorie),
Schmelzpunkt: 171-172°C,
$R_f$-Wert: 0,46 (Kieselgel; Methylenchlorid/Methanol = 20:1)
Analog Beispiel IV werden folgende Verbindungen erhalten:
(1) 1-[4-[2-(Methoxycarbonyl)-1-pentyl]phenyl]-imidazolidin-2-on
(2) 1-[4-(1-Trifluoracetyl-4-piperidinyl)phenyl]-imidazolidin-2-on
Schmelzpunkt: 198-201°C,
$R_f$-Wert: 0,51 (Kieselgel; Essigester)
(3) 1-[4-(4-Cyano-1-trifluoracetyl-4-piperidinyl)phenyl]-imidazolidin-2-on
$R_f$-Wert: 0,45 (Kieselgel; Cyclohexan/Essigester = 3:2)
(4) 1-(4-Brom-2-fluor-phenyl)-imidazolidin-2-on
Schmelzpunkt: 134-136°C
(5) 1-(4-Brom-2-trifluormethyl-phenyl)-imidazolidin-2-on
Schmelzpunkt: 139-141°C
(6) 1-(4-Brom-2-methyl-phenyl)-imidazolidin-2-on
Schmelzpunkt: 184-186°C
(7) 1-[4-[2-(Methoxycarbonyl)ethenyl]phenyl]-imidazolidin-2-on
Schmelzpunkt: 233-234°C
(8) 1-[4-[2-(Ethoxycarbonyl)-1-phenyl-ethyl]phenyl]-imidazolidin-2-on
Schmelzpunkt: 167-169°C

Beispiel V

4-[2-(n-Butylsulfonylamino)-2-(methoxycarbonyl)-ethyl]-N-(2-hydroxyethyl)-anilin

5,0 g 4-[2-(n-Butylsulfonylamino)-2-(methoxycarbonyl)-ethyl]-anilin in 50 ml Acetonitril werden mit 1N Salzsäure versetzt bis ein pH-Wert von 6-7 resultiert. In diesem Gemisch werden 1,02 g Glycolaldehyd (dimer) gelöst und dazu 1,13 g Natriumcyanoborhydrid portionsweise zugegeben. Nach einer Stunde Rühren bei Raumtemperatur wird eingeengt und der Rückstand zwischen Eiswasser und Essigester verteilt. Die wäßrige Phase wird alkalisch gestellt und mit Essigester extrahiert. Die vereinigten organischen Phasen werden mit Kochsalzlösung gewaschen, getrocknet und eingeengt. Der Rückstand wird durch Chromatographie über eine Kieselgelsäule mit Cyclohexan/Essigester (1:1 bis 3:7) gereinigt.
Ausbeute: 1,6 g (29 % der Theorie),
$R_f$-Wert: 0,31 (Kieselgel; Cyclohexan/Essigester = 3:7)
Analog Beispiel V wird folgende Verbindung erhalten:
(1) 1-Benzyl-4-[(2-hydroxyethyl)amino]-piperidin
$R_f$-Wert: 0,33 (Kieselgel; Methylenchlorid/Methanol/Cyclohexan/konz. wäßriges Ammoniak = 7:1,5:1,5:0,2)

Beispiel VI

1-(1-Hydroxyimino-1,2,3,4-tetrahydronaphthalin-6-yl)-3-[4-[2-(methoxycarbonyl)ethyl]phenyl]-imidazolidin-2-on

Zu einer siedenden Lösung aus 2,35 g 1-(1-Oxo-1,2,3,4-tetrahydronaphthalin-6-yl)-3-[4-[2-(methoxycarbonyl)ethyl]phenyl]-imidazolidin-2-on in einem Gemisch aus 100 ml Methanol, 50 ml Dioxan und 1,5 ml Pyridin werden 460 mg Hydroxylamin-hydrochlorid gegeben und 2 Stunden unter Rückfluß erhitzt. Nach dem Abkühlen über Nacht wird abgesaugt, mit Methanol, Wasser und nochmals Methanol gewaschen und getrocknet.
Ausbeute: 2,3 g (94 % der Theorie),
$R_f$-Wert: 0,42 (Kieselgel; Methylenchlorid/Methanol = 95:5)

| | | | | | | |
|---|---|---|---|---|---|---|
| Ber.:<br>Gef.: | C | 67,80<br>67,70 | H | 6,18<br>6,04 | N | 10,31<br>10,21 |

Beispiel VII

1-(1-Oxo-1,2,3,4-tetrahydronaphthalin-6-yl)-3-[4-[2-(methoxycarbonyl)ethyl]phenyl]-imidazolidin-2-on

Zu einer Lösung von 3,7 g Triphenylphosphin und 5,5 g N-(1-Oxo-1,2,3,4-tetrahydronaphthalin-6-yl)-N'-(2-hydroxyethyl)-N'-[4-[2-(methoxycarbonyl)ethyl]phenyl]-harnstoff in 40 ml Acetonitril werden bei 40-45 °C 2,84 g Azodicarbonsäure-diethylester in 10 ml Acetonitril zugetropft. Nach 1,5 Stunden wird abgekühlt, das Produkt abgesaugt und mit wenig Aceton und Diethylether gewaschen.
Ausbeute: 4,27 g (81 % der Theorie),
Schmelzpunkt: 180-182 °C

| | | | | | | |
|---|---|---|---|---|---|---|
| Ber.:<br>Gef.: | C | 70,39<br>70,43 | H | 6,16<br>6,13 | N | 7,14<br>7,14 |

Analog Beispiel VII wird folgende Verbindung erhalten:
(1) 1-[4-[2-(Methoxycarbonyl)ethyl]phenyl]-4-methyl-imidazolidin-2-on
$R_f$-Wert: 0,16 (Kieselgel; Cyclohexan/Essigester = 1:1)

Beispiel VIII

4-[2-(n-Butylsulfonylamino)-2-(methoxycarbonyl)-ethyl]-anilin

19 g 4-[2-(n-Butylsulfonylamino)-2-(methoxycarbonyl)-ethyl]-nitrobenzol(Schmelzpunkt: 102-104 °C; hergestellt aus $\beta$-(4-Nitrophenyl)-D,L-alanin-methylester durch Umsetzung mit n-Butylsulfonsäurechlorid in Gegenwart von N-Ethyl-diisopropylamin) werden in 200 ml Essigester bei Raumtemperatur und einem Wasserstoffdruck von 3,4 bar in Gegenwart von 2 g Palladium auf Aktivkohle (10 % Palladium) 1,5 Stunden hydriert. Der Katalysator wird abgesaugt und das Filtrat eingeengt.
Ausbeute: 17,8 g (100 % der Theorie),
$R_f$-Wert: 0,45 (Kieselgel; Cyclohexan/Essigester = 1:1)
Analog Beispiel VIII werden folgende Verbindungen erhalten:
(1) 4-(4-Cyano-1-trifluoracetyl-4-piperidinyl)-anilin
$R_f$-Wert: 0,28 (Kieselgel; Cyclohexan/Essigester = 2:1)
(2) 4-(1-Trifluoracetyl-4-piperidinyl)-anilin
Schmelzpunkt: 111-112 °C
$R_f$-Wert: 0,25 (Kieselgel; Cyclohexan/Essigester = 2:1)
(3) 2-[[4-[2-(Methoxycarbonyl)ethyl]phenyl]amino]-anilin
Das Ausgangsmaterial 2-[[4-[2-(Methoxycarbonyl)ethyl]phenyl]-amino]-nitrobenzol (Schmelzpunkt: 68-70 °C) wird durch Umsetzung von 2-Fluor-nitrobenzol mit 3-(4-Aminophenyl)propionsäure-methylester erhalten.

$R_f$-Wert: 0,25 (Kieselgel; Methylenchlorid/Essigester = 100:1)
(4) 4-[2-(O,O'-Diethylphosphono)ethyl]-anilin
Das Ausgangsmaterial 4-[2-(O,O'-Diethylphosphono)ethenyl]-nitrobenzol (Schmelzpunkt: 102-104 °C) wird durch Umsetzung von Methandiphosphonsäure-tetraethylester/Kalium-tert.butylat mit 4-Nitrobenzaldehyd erhalten.
$R_f$-Wert: 0,55 (Kieselgel; Essigester/Ethanol = 15:1)
(5) 4-(4-Aminophenyl)-chinuclidin
Durchführung in 1N Salzsäure, Isolierung der Base.
$R_f$-Wert: 0,89 (Reversed Phase Kieselgel; Methanol/5%ige wäßrige Kochsalzlösung = 6:4)
(6) 4-(4-Aminophenyl)-4-methyl-1-trifluoracetyl-piperidin
$R_f$-Wert: 0,33 (Kieselgel; Cyclohexan/Essigester = 7:3)
(7) 3-(4-Aminophenyl)-3-phenyl-propionsäure-ethylester-hydrochlorid
Durchführung in Ethanol in Gegenwart von ethanolischer Salzsäure. Das Ausgangsmaterial 3-(4-Nitrophenyl)-3-phenylacrylsäure-ethylester [$R_f$-Wert: 0,36 (Kieselgel; Cyclohexan/Methylenchlorid = 1:1)] wird durch Umsetzung von 4-Nitrobenzophenon mit Phosphonoessigsäure-triethylester erhalten.
$R_f$-Wert: 0,40 (Reversed Phase Kieselgel; Methanol/5%ige wäßrige Kochsalzlösung = 6:4)

Beispiel IX

4-[4-[2-(Methoxycarbonyl)ethyl]phenyl]-5-methyl-4H-1,2,4-triazol-3-on

8,1 g 1-Acetyl-4-[4-[2-(methoxycarbonyl)ethyl]phenyl]-semicarbazid werden mit 60 ml 1N Natronlauge 1,5 Stunden auf dem Dampfbad erhitzt. Anschließend wird etwas abgekühlt, filtriert und das Filtrat mit Zitronensäure schwach angesäuert. Es wird filtriert und das Filtrat mit konzentrierter Salzsäure versetzt. Der Niederschlag wird filtriert, mit Wasser gewaschen und getrocknet. Das Zwischenprodukt wird über Nacht in Methanol mit etwas methanolischer Salzsäure gerührt. Das Reaktionsgemisch wird eingeengt und der Rückstand mit tert.-Butyl-methylether verrieben, abgesaugt und getrocknet.
Ausbeute: 4,98 g (65 % der Theorie),
$R_f$-Wert: 0,40 (Kieselgel; Toluol/Dioxan/Ethanol/Eisessig = 90:10:10:6)

| | | | | | | |
|---|---|---|---|---|---|---|
| Ber.:<br>Gef.: | C | 59,76<br>59,54 | H | 5,79<br>5,86 | N | 16,08<br>16,05 |

Analog Beispiel IX werden folgende Verbindungen erhalten:
(1) 4-[4-[2-(Methoxycarbonyl)-1-pentyl]phenyl]-5-methyl-4H-1,2,4-triazol-3-on
Schmelzpunkt: 135-137 °C

| | | | | | | |
|---|---|---|---|---|---|---|
| Ber.:<br>Gef.: | C | 63,35<br>63,39 | H | 6,98<br>7,04 | N | 13,85<br>13,83 |

(2) 4-[4-[2-(Methoxycarbonyl)-3-methyl-1-butyl]phenyl]-5-methyl-4H-1,2,4-triazol-3-on
(3) 4-[4-[2-(Methoxycarbonyl)-1-butyl]phenyl]-5-methyl-4H-1,2,4-triazol-3-on
(4) 4-[4-[2-(Methoxycarbonyl)-1-propyl]phenyl]-5-methyl-4H-1,2,4-triazol-3-on
(5) 4-[4-[2-(Methoxycarbonyl)ethyl]phenyl]-4H-1,2,4-triazol-3-on
Schmelzpunkt: 174-176 °C
$R_f$-Wert: 0,57 (Kieselgel; Toluol/Dioxan/Ethanol/Eisessig = 90:10:10:6)
(6) 4-[trans-4-(Methoxycarbonyl)cyclohexyl]-4H-1,2,4-triazol-3-on
Schmelzpunkt: 204-206 °C

| | | | | | | |
|---|---|---|---|---|---|---|
| Ber.:<br>Gef.: | C | 53,32<br>53,29 | H | 6,71<br>6,66 | N | 18,66<br>18,83 |

(7) 4-[trans-4-(Methoxycarbonyl)cyclohexyl]-5-methyl-4H-1,2,4-triazol-3-on
Schmelzpunkt: 203-204 °C

| | | | | | | |
|---|---|---|---|---|---|---|
| Ber.:<br>Gef.: | C | 55,22<br>55,14 | H | 7,15<br>7,23 | N | 17,56<br>17,32 |

Beispiel X

1-tert.Butyloxycarbonyl-4-[2-(methansulfonyloxy)ethyl]-piperidin

Eine Lösung von 1,3 g 1-tert.Butyloxycarbonyl-4-(2-hydroxyethyl)-piperidinin 30 ml Methylenchlorid werden mit 710 mg Methansulfonylchlorid versetzt und im Eisbad abgekühlt. Dazu werden 640 mg Triethylamin langsam zugetropft. Nach 1 Stunde Rühren wird mit Eiswasser versetzt, das Gemisch 15 Minuten gerührt und anschließend die organische Phase abgetrennt und eingeengt. Der Rückstand wird mit wenig Diisopropylether und Petrolether gerührt, das Produkt abgesaugt, mit Petrolether gewaschen und getrocknet.
Ausbeute: 1,37 g (80 % der Theorie),
Schmelzpunkt: 81-84 °C
$R_f$-Wert: 0,43 (Kieselgel; Cyclohexan/Essigester = 1:1)
Analog Beispiel X werden folgende Verbindungen erhalten:

(1) 1-tert.Butyloxycarbonyl-4-[2-(methansulfonyloxy)ethyl]-1-azacycloheptan
Das als Ausgangsmaterial eingesetzte 1-tert.Butyloxycarbonyl-4-(2-hydroxyethyl)-1-azacycloheptan wird ausgehend von 1-Aza-4-cycloheptylessigsäureethylester durch Umstzung mit Pyrokohlensäure-di-tert.butylester und anschließender Reduktion mit Lithiumborhydrid erhalten.
$R_f$-Wert: 0,48 (Kieselgel; Cyclohexan/Essigester = 1:1)

(2) 4-[2-(Methansulfonyloxy)ethyl]-chinuclidin x $BH_3$
Schmelzpunkt: 83-86 °C
$R_f$-Wert: 0,44 (Kieselgel; Cyclohexan/Essigester = 3:7)

(3) 1-tert.Butyloxycarbonyl-3-[2-(methansulfonyloxy)ethyl]-pyrrolidin
Das Ausgangsmaterial, 1-tert.Butyloxycarbonyl-3-(2-hydroxyethyl)-pyrrolidin[$R_f$-Wert: 0,30 (Kieselgel; Cyclohexan/Essigester = 1:1)], wird durch Umsetzung von 3-(2-Hydroxyethyl)-pyrrolidin mit Pyrokohlensäure-di-tert.butylester erhalten.
$R_f$-Wert: 0,36 (Kieselgel; Cyclohexan/Essigester = 1:1)

Beispiel XI

N-(1-Oxo-1,2,3,4-tetrahydronaphthalin-6-yl)-N'-(2-hydroxyethyl)-N'-[4-[2-(methoxycarbonyl)ethyl]phenyl]-harnstoff

Zu 4,1 g N-(2-Hydroxyethyl)-4-[2-(methoxycarbonyl)ethyl]-anilin in 10 ml Dioxan werden 3,9 g (1-Oxo-1,2,3,4-tetrahydronaphthalin-6-yl)-isocyanat in 10 ml Dioxan gegeben und das Gemisch über Nacht gerührt. Das Reaktionsgemisch wird eingeengt, der Rückstand in Essigester aufgenommen und mit verdünnter Zitronensäurelösung und Kochsalzlösung gewaschen. Die organische Phase wird eingeengt und durch Chromatographie über eine Kieselgelsäule mit Cyclohexan/Essigester (3:7) gereinigt.
Ausbeute: 5,6 g (73 % der Theorie),
$R_f$-Wert: 0,21 (Kieselgel; Cyclohexan/Essigester = 3:7)
Analog Beispiel XI werden folgende Verbindungen erhalten:

(1) N-(2,2-Diethoxyethyl)-N-(1,4-dioxaspiro[4.5]decan-8-yl)-N'-[4-[2-(methoxycarbonyl)ethyl]phenyl]-harnstoff
$R_f$-Wert: 0,34 (Kieselgel; Cyclohexan/Essigester = 1:1)

(2) N-(4-Cyanophenyl)-N'-(2-hydroxyethyl)-N'-[4-[2-(methoxycarbonyl)ethyl]phenyl]-harnstoff
Schmelzpunkt: 98-101 °C

(3) N-(4-Bromphenyl)-N'-[4-[2-(ethoxycarbonyl)ethyl]phenyl]-N'-(2-hydroxyethyl)-harnstoff
$R_f$-Wert: 0,38 (Kieselgel; Cyclohexan/Essigester = 6:4)

(4) N-(4-Cyanophenyl)-N-(methoxycarbonylmethyl)-N'-[trans-4-[2-(methoxycarbonyl)ethyl]cyclohexyl]-harnstoff
Schmelzpunkt: 108-110 °C
$R_f$-Wert: 0,62 (Kieselgel; Methylenchlorid/Essigester = 4:1)

(5) N-(2-Chlorethyl)-N'-[4-[2-(methoxycarbonyl)ethyl]phenyl]-harnstoff
Schmelzpunkt: 124-125 °C
(6) N-(2-Chlorethyl)-N'-[4-[2-(methoxycarbonyl)-1-pentyl]-phenyl]-harnstoff
(7) N-(1-Benzyl-4-piperidinyl)-N-(2-hydroxyethyl)-N'-[4-[4-(methoxycarbonyl)butyl]phenyl]-harnstoff
Schmelzpunkt: 118-120 °C
$R_f$-Wert: 0,67 (Kieselgel; Methylenchlorid/Cyclohexan/Methanol/konz. wäßriges Ammoniak = 7:1,5:1,5:0,2)
(8) N-[trans-4-(Ethoxycarbonyl)cyclohexyl]-N-(2-hydroxyethyl)-N'-[4-(1-trifluoracetyl-4-piperidinyl)phenyl]-harnstoff
Umsetzung des Isocyanats mit dem cis/trans-Gemisch der Aminoverbindung und Isolierung der trans-Verbindung durch Chromatographie über Kieselgel.
Schmelzpunkt: 158-160 °C
$R_f$-Wert: 0,32 (Kieselgel; Cyclohexan/Essigester = 1:1)
(9) N-(2-Chlorethyl)-N'-[4-(1-trifluoracetyl-4-piperidinyl)phenyl]-harnstoff
$R_f$-Wert: 0,68 (Kieselgel; Essigester/Cyclohexan = 7:3)
(10) N-[4-[2-(n-Butylsulfonylamino)-2-(methoxycarbonyl)ethyl]phenyl]-N'-4-(cyanophenyl)-N-(2-hydroxyethyl)-harnstoff
$R_f$-Wert: 0,26 (Kieselgel; Cyclohexan/Essigester = 3:7)
(11) N-[4-(Ethoxycarbonyl)phenyl]-N-(2-hydroxyethyl)-N'-[4-(1-trifluoracetyl-4-piperidinyl)phenyl]-harnstoff
Schmelzpunkt: 105-108 °C
$R_f$-Wert: 0,23 (Kieselgel; Cyclohexan/Essigester = 1:1)
(12) N-[4-(4-Cyano-1-trifluoracetyl-4-piperidinyl)phenyl]-N'-(2-hydroxyethyl)-N'-[trans-4-(methoxycarbonyl)cyclohexyl]-harnstoff
Das als Ausgangsmaterial eingesetzte 4-(4-Cyano-1-trifluoracetyl-4-piperidinyl)phenylisocyanat wird aus dem entsprechenden Amin durch Behandlung mit Phosgen hergestellt.
Schmelzpunkt: ab 189 °C (Zers.)
$R_f$-Wert: 0,58 (Kieselgel; Essigester/Cyclohexan = 1:1)
(13) N-(2-Chlorethyl)-N'-4-[4-(4-cyano-1-trifluoracetyl-4-piperidinyl)phenyl]-harnstoff
$R_f$-Wert: 0,66 (Kieselgel; Methylenchlorid/Methanol = 96:4)
(14) N-(2-Hydroxyethyl)-N-[trans-4-(methoxycarbonyl)cyclohexyl]-N'-[4-(1-trifluoracetyl-4-piperidinyl)-phenyl]-harnstoff
Das als Ausgangsmaterial eingesetzte 4-(1-Trifluoracetyl-4-piperidinyl)phenylisocyanat wird aus dem entsprechenden Amin durch Behandlung mit Phosgen hergestellt.
Schmelzpunkt: 162-163 °C
$R_f$-Wert: 0,50 (Kieselgel; Essigester/Cyclohexan = 5:1)
(15) N-[2-(1-tert.Butyloxycarbonyl-1-aza-4-cycloheptyl)ethyl]-N-(2-hydroxyethyl)-N'-[4-[2-(methoxycarbonyl)ethyl]phenyl]-harnstoff
Das als Aminkomponente eingesetzte N-[2-(1-tert.Butyloxycarbonyl-1-aza-4-cycloheptyl)ethyl]-ethanolamin wird durch Umsetzung von 1-tert.Butyloxycarbonyl-4-[2-(methansulfonyloxy)ethyl]-1-azacycloheptan mit Ethanolamin erhalten.
$R_f$-Wert: 0,27 (Kieselgel; Cyclohexan/Essigester = 3:7)
(16) N-[(Ethoxycarbonyl)methyl]-N'-[4-[2-(methoxycarbonyl)ethyl]phenyl]-harnstoff
Schmelzpunkt: 142-144 °C
$R_f$-Wert: 0,71 (Kieselgel; Cyclohexan/Essigester = 2:8)
(17) N-[4-[2-(Methoxycarbonyl)ethyl]phenyl]-N'-(1-hydroxy-2-propyl)-harnstoff
Schmelzpunkt: 132-134 °C
$R_f$-Wert: 0,42 (Kieselgel; Essigester)
(18) N-(4-Brom-2-fluor-phenyl)-N'-(2-chlorethyl)-harnstoff
Schmelzpunkt: 165-167 °C
(19) N-(4-Brom-2-trifluormethyl-phenyl)-N'-(2-chlorethyl)-harnstoff
Schmelzpunkt: 175-177 °C
(20) N-(4-Brom-2-methyl-phenyl)-N'-(2-chlorethyl)-harnstoff
Schmelzpunkt: 180-182 °C
(21) N-(2-Chlorethyl)-N'-[4-[2-(methoxycarbonyl)ethenyl]phenyl]-harnstoff
$R_f$-Wert: 0,41 (Kieselgel; Cyclohexan/Essigester = 1:1)
(22) N-(4-Cyano-bicyclo[2.2.2]octan-1-yl)-N'-(2,2-dimethoxyethyl)-N'-[4-[2-(methoxycarbonyl)ethyl]-phenyl]-harnstoff
Das als Ausgangsmaterial eingesetzte (4-Cyano-bicyclo[2.2.2]octan-1-yl)-isocyanat wird aus den entsprechenden Amin-hydrochlorid durch Umsetzung mit Phosgen hergestellt.
Das als Aminokomponente eingesetzte N-[4-[2-(Methoxycarbonyl)ethyl]phenyl]-aminoacetaldehyd-dimethylacetal [$R_f$-Wert: 0,63 (Kieselgel; Cyclohexan/Essigester = 3:2)] wird durch Umsetzung von 3-(4-Aminophenyl)-propionsäure-methylester mit Bromacetaldehyd-dimethylacetal in Gegenwart von N-Ethyl-diisopropylamin erhalten.
Schmelzpunkt: 93-94 °C
$R_f$-Wert: 0,40 (Kieselgel; Cyclohexan/Essigester = 1:1)
(23) N-(2-Chlorethyl)-N'-[4-[2-(ethoxycarbonyl)-1-phenylethyl]-phenyl]-harnstoff
Schmelzpunkt: 109-111 °C
$R_f$-Wert: 0,37 (Kieselgel; Cyclohexan/Essigester = 6:4)
(24) N-[2-(1-Benzyl-1-azoniabicyclo[2.2.2]octan-4-yl)ethyl]-N-(2,2-dimethoxyethyl)-N'-[4-[2-(methoxycarbonyl)ethyl]phenyl]-harnstoff-chlorid
Das als Aminkomponente eingesetzte N-[2-(1-Benzyl-1-azoniabicyclo[2.2.2]octan-4-yl)ethyl]-aminoacetaldehyd-dimethylacetal-chlorid-hydrochlorid [Schmelzpunkt: 202-204 °C (Zers.); $R_f$-Wert: 0,62 (Reversed Phase Kieselgel; Methanol/5%ige wässrige Kochsalzlösung = 6:4)] wird durch Umsetzung von 1-Benzyl-4-(2-chlorethyl)-1-azoniabicyclo[2.2.2]octan-chlorid mit Aminoacetaldehyd-dimethylacetal erhalten.
$R_f$-Wert: 0,43 (Reversed Phase Kieselgel; Methanol/5%ige wässrige Kochsalzlösung = 6:4)
(25) N-[2-(1-tert.Butyloxycarbonyl-4-piperidinyl)ethyl]-N-(2-hydroxyethyl)-N'-[2-cyano-4-[2-(methoxycarbonyl)ethyl]-phenyl]-harnstoff
Das als Ausgangsprodukt eingesetzte [2-Cyano-4-[2-(methoxycarbonyl)ethyl]-phenyl]-isocyanat wird durch Umsetzung des entsprechenden Amins mit Phosgen erhalten.
$R_f$-Wert: 0,36 (Kieselgel; Essigester/Cyclohexan = 2:1)

## Beispiel XII

### 1-(4-Cyanophenyl)-3-[4-[2-(N-trityl-5-tetrazolyl)ethyl]phenyl]-imidazolidin-2-on

Unter Stickstoff werden 0,9 g 1-(4-Bromphenyl)-3-[4-[2-(N-trityl-5-tetrazolyl)ethyl]phenyl]-imidazolidin-2-on, 140 mg Kaliumcyanid, 62 mg Palladium-(II)-acetat, 142 mg Triphenylphosphin, 18 mg Calciumhydroxid und 7 ml trockenes Dimethylformamid eine Stunde bei 100 °C gerührt. Nach dem Abkühlen wird mit Eiswasser versetzt und mehrmals mit Essigester extrahiert. Die vereinigten organischen Phasen werden mit Kochsalzlösung gewaschen, getrocknet und eingeengt. Der Rückstand wird durch Chromatographie über eine Kieselgelsäule mit Cyclohexan/Essigester (1:1) gereinigt.
Ausbeute: 270 mg (32 % der Theorie),
Schmelzpunkt: 192-194 °C (Zers.)
$R_f$-Wert: 0,65 (Kieselgel; Cyclohexan/Essigester = 3:7)

## Beispiel XIII

### 1-(4-Bromphenyl)-3-[4-[2-(N-trityl-5-tetrazolyl)ethyl]phenyl]-imidazolidin-2-on

Zu 3,1 g 1-(4-Bromphenyl)-3-[4-[2-(5-tetrazolyl)ethyl]phenyl]-imidazolidin-2-on in 80 ml Methylenchlorid werden 0,91 g Triethylamin und 2,51 g Tritylchlorid zugegeben und 1,5 Stunden bei Raumtemperatur gerührt. Das Reaktionsgemisch wird mit Wasser gewaschen, die organische Phase abgetrennt, getrocknet und eingeengt. Der Rückstand wird mit 70 ml Essigester kurz aufgekocht, etwas abgekühlt und das Produkt abgesaugt, mit Essigester gewaschen und getrocknet.
Ausbeute: 3,38 g (68 % der Theorie),
Schmelzpunkt: 194-197 °C (Zers.)
$R_f$-Wert: 0,67 (Kieselgel; Cyclohexan/Essigester = 1:1)

## Beispiel XIV

### 1-(4-Bromphenyl)-3-[4-[2-(5-tetrazolyl)ethyl]phenyl]-imidazolidin-2-on

7,12 g 1-(4-Bromphenyl)-3-[4-[2-(2-cyanoethyl)phenyl]-imidazolidin-2-on und 7,7 g Tributylzinnazid werden in 33 ml Dimethylformamid 9 Stunden bei 120-130 °C gerührt. Es werden nochmals 3 g Tributylzinnazid zugegeben und weitere 50 Stunden erhitzt. Das Reaktionsgemisch wird auf Eiswasser gegossen und das Gemisch gerührt. Der Niederschlag wird abgesaugt, mit etwas Methanol gewaschen und in Methylenchlorid aufgenommen. Diese Lösung wird mit Kaliumfluorid-Lösung gewaschen, die organische Phase wird abgetrennt, getrocknet und eingeengt. Der Rückstand wird durch Chromatographie über eine Kieselgelsäule mit Methylenchlorid/Methanol (95:5) gereinigt. Das Produkt wird noch mit Methanol gerührt, abgesaugt und getrocknet.
Ausbeute: 4,15 g (52 % der Theorie),
Schmelzpunkt: 232-236 °C (Zers.)
$R_f$-Wert: 0,29 (Kieselgel; Methylenchlorid/Methanol = 95:5)

Beispiel XV

1-(4-Bromphenyl)-3-[4-(2-cyanoethyl)phenyl]-imidazolidin-2-on

Zu 750 mg 1-(4-Bromphenyl)-3-[4-[2-(aminocarbonyl)ethyl]phenyl]-imidazolidin-2-on und 320 mg Pyridin in 10 ml Tetrahydrofuran werden bei -10 °C unter Rühren 460 mg Trifluoressigsäure-anhydrid in 1 ml Tetrahydrofuran zugetropft. Nach Rühren über Nacht bei Raumtemperatur wird das Reaktionsgemisch mit Eiswasser verdünnt und der ausgefallene Niederschlag abgesaugt, gewaschen und getrocknet.
Ausbeute: 700 mg (98 % der Theorie),
Schmelzpunkt: 188-192 °C

Beispiel XVI

3-[4-[2-(Methoxycarbonyl)ethyl]phenyl]-1-(4-oxocyclohexyl)-3H-imidazol-2-on

Zu einer Lösung von 12,8 g N-(2,2-Diethoxyethyl)-N-(1,4-dioxa-spiro[4.5]decan-8-yl)-N'-[4-[2-(methoxycarbonyl)ethyl]phenyl]-harnstoff in 20 ml Dioxan werden 5,6 ml 3N Salzsäure gegeben und über Nacht bei Raumtemperatur gerührt. Das Reaktionsgemisch wird eingeengt und der Rückstand zwischen Methylenchlorid und Wasser verteilt. Die wäßrige Phase wird abgetrennt und mit Methylenchlorid extrahiert. Die vereinigten organischen Phasen werden mit Wasser gewaschen, getrocknet und eingeengt. Der Rückstand wird durch Chromatographie über eine Kieselgelsäule mit Cyclohexan/Essigester (35:65) gereinigt.
Ausbeute: 2,3 g (25 % der Theorie),
Schmelzpunkt: 113-115 °C
$R_f$-Wert: 0,25 (Kieselgel; Cyclohexan/Essigester = 25:75)

Beispiel XVII

1-[4-[2-(Aminocarbonyl)ethyl]phenyl]-3-(4-bromphenyl)-imidazolidin-2-on

9,2 g 3-(4-Bromphenyl)-1-[4-(2-carboxyethyl)phenyl]-imidazolidin-2-on werden in einem Gemisch aus 50 ml Tetrahydrofuran und 25 ml Dimethylformamid mit 3,8 g N,N'-Carbonyldiimidazol versetzt und 2,5 Stunden bei 80 °C gerührt. Nach dem Abkühlen wird auf ein Gemisch aus 20 ml konzentriertes wäßriges Ammoniak und 100 g Eis gegeben und 15 Minuten gerührt. Das Produkt wird abgesaugt und getrocknet.
Ausbeute: 8,8 g (97 % der Theorie),
Schmelzpunkt: 248-253 °C

Beispiel XVIII

4-(4-Cyano-1-trifluoracetyl-4-piperidinyl)-nitrobenzol

Zu 1,6 g 4-Cyano-4-phenyl-1-trifluoracetyl-piperidin (hergestellt durch Umsetzung von 4-Cyano-4-phenyl-piperidin mit Trifluoressigsäureanhydrid in Gegenwart von N-Ethyl-diisopropylamin) in 5 ml konz. Schwefelsäure werden unter Kühlung in einem Eis/Aceton Kältebad 590 mg Kaliumnitrat, gelöst in 5 ml konz. Schwefelsäure, zugetropft. Nach 1,5 Stunden Rühren bei Raumtemperatur wird auf Eis gegossen, der Niederschlag wird abgesaugt, in Essigester gelöst und die Essigesterlösung mit Wasser und Kochsalzlösung gewaschen, getrocknet und eingedampft. Der Rückstand wird mit tert.Butyl-methylether verrührt, abgesaugt und mit tert.Butylmethylether gewaschen.
Ausbeute: 500 mg (26 % der Theorie),
Schmelzpunkt: 135-138 °C
Analog Beispiel XVIII werden folgende Verbindungen erhalten:

(1) 4-(1-Trifluoracetyl-4-piperidinyl)-nitrobenzol
Die Nitrierung wird in Eisessig/Acetanhydrid mit rauchender Salpetersäure durchgeführt.
Schmelzpunkt: 96-100 °C
$R_f$-Wert: 0,50 (Kieselgel, Cyclohexan/Essigester = 2:1).
(2) 4-(4-Nitrophenyl)-chinuclidin
Schmelzpunkt: 130-135 °C
$R_f$-Wert: 0,38 (Kieselgel, Methylenchlorid/Methanol/konz. wäßriges Ammoniak = 9:1:0,3)
(3) 4-Methyl-4-(4-nitrophenyl)-1-trifluoracetyl-piperidin
Die Nitrierung wird in Eisessig/Acetanhydrid mit rauchender Salpetersäure durchgeführt. Das Ausgangsmaterial [$R_f$-Wert: 0,72 (Kieselgel; Cyclohexan/Essigester = 2:1)] wird durch Umsetzung von 4-Methyl-4-phenyl-piperidin mit Trifluoressigsäureanhydrid in Gegenwart von N-Ethyl-diisopropylamin erhalten.
$R_f$-Wert: 0,52 (Kieselgel, Cyclohexan/Essigester = 7:3)

Beispiel XIX

trans-4-[(2-Hydroxyethyl)amino]-cyclohexancarbonsäure-methylester

13 g trans-4-[N-Benzyl-N-(2-hydroxyethyl)-amino]-cyclohexancarbonsäure-methylester werden in 150 ml Methanol mit 3,5 g Palladium auf Aktivkohle (10 % Palladium) unter einem Wasserstoffdruck von 50 psi 20 Minuten bei 50 °C hydriert. Der Katalysator wird abgesaugt und das Filtrat zur Trockene eingeengt (Das Ausgangsmaterial wird aus trans-4-Amino-cyclohexancarbonsäure-methylester durch Umsetzung mit Benzaldehyd und Wasserstoff in Gegenwart von Raney-Nickel und nachfolgender Umsetzung mit 2-Bromethanol in Gegenwart von N-Ethyl-diisopropylamin erhalten).
Ausbeute; 8,3 g (93 % der Theorie),
Schmelzpunkt: 66-68 °C
$R_f$-Wert: 0,60 (Kieselgel; Methylenchlorid/Methanol/konz. wäßriges Ammoniak = 4:1:0,2).

Analog Beispiel XIX werden folgende Verbindungen erhalten:
(1) trans-4-[(2,2-Diethoxyethyl)amino]-cyclohexancarbonsäuremethylester
Das Ausgangsmaterial wird aus trans-4-Amino-cyclohexancarbonsäure-methylester durch Umsetzung mit Benzaldehyd und Wasserstoff in Gegenwart von Raney-Nickel und nachfolgender Umsetzung mit Bromacetaldehyd-diethylacetal in Gegenwart von N-Ethyl-diisopropylamin erhalten.
$R_f$-Wert: 0,65 (Kieselgel; Methylenchlorid/Methanol/konz. wäßriges Ammoniak = 9:1:0,1).
(2) 4-Methyl-4-phenyl-piperidin
Die Debenzylierung wird in Gegenwart von Palladiumhydrochlorid auf Kohle durchgeführt.
$R_f$-Wert: 0,60 (Reversed Phase Kieselgel; Methanol/5%ige wäßrige Kochsalzlösung = 6:4).
(3) 1-(4-Hydroxyphenyl)-3-[trans-4-(methoxycarbonyl)cyclohexyl]-imidazolidin-2-on
Durchführung in Dioxan.
Das Ausgangsmaterial, 1-[4-(Benzyloxy)phenyl]-3-[trans-4-(methoxycarbonyl)cyclohexyl]-3H-imidazol-2-on [Schmelzpunkt: 183-185 °C, $R_f$-Wert: 0,49; (Kieselgel; Cyclohexan/Essigester = 1:1)], wird durch Behandlung von N-[4-(Benzyloxy)phenyl]-N'-(2,2-diethoxyethyl)-N'-[trans-4-(methoxycarbonyl)-cyclohexyl]-harnstoff mit Trifluoressigsäure in Methylenchlorid bei Raumtemperatur erhalten.
Schmelzpunkt: 184-186 °C
$R_f$-Wert: 0,26 (Kieselgel; Cyclohexan/Essigester = 1:1)
(4)(trans-4-Aminocyclohexyl)oxyessigsäure-tert.butylester
Das Ausgangsmaterial, [trans-(4-Dibenzylamino)cyclohexyl]oxyessigsäure-tert.butylester [$R_f$-Wert: 0,51; (Kieselgel; Cyclohexan/Essigester = 4:1)], wird durch Umsetzung von trans-4-(Dibenzylamino)-cyclohexanol mit Bromessigsäure-tert.-butylester in Toluol/50%ige Natronlauge in Gegenwart von Tetrabutylammonium-hydrogensulfat erhalten.
$R_f$-Wert: 0,56 (Reversed Phase Kieselgel; Methanol/5%ige wäßrige Kochsalzlösung = 6:4).

Beispiel XX

N-[2-(1-tert.Butyloxycarbonyl-4-piperidinyl)ethyl]-N'-[4-[2-(n-butylsulfonylamino)-2-(methoxycarbonyl)-ethyl]-phenyl]-N-(2-hydroxyethyl)-harnstoff

3,25 g N,N'-Carbonyldiimidazol und 2,3 g Imidazol werden in 60 ml Tetrahydrofuran gelöst und unter Stickstoff auf 0 °C abgekühlt. Unter Rühren werden 6,15 g 4-[2-(n-Butylsulfonylamino)-2-(methoxycarbonyl)-ethyl]-anilin in 30 ml Tetrahydrofuran rasch zugetropft und 4 Minuten unter Kühlung weitergerührt. Dann

werden 6 g N-[2-(1-tert.Butyloxycarbonyl-4-piperidinyl)ethyl]-ethanolamin (hergestellt durch Umsetzung von 1-tert.Butyloxycarbonyl-4-[2-(methansulfonyloxy)ethyl]-piperidin mit Ethanolamin) in 30 ml Tetrahydrofuran zugetropft. Nach Rühren über Nacht bei Raumtemperatur wird eingeengt, in tert.Butyl-methylether aufgenommen und mit verdünnter Zitronensäure und Kochsalzlösung gewaschen. Die organische Phase wird getrocknet, eingeengt und der Rückstand durch Chromatographie über eine Kieselgelsäule gereinigt.
Ausbeute: 1,6 g (13 % der Theorie),
$R_f$-Wert: 0,31 (Kieselgel; Methylenchlorid/Methanol = 95:5).

Analog Beispiel XX werden folgende Verbindungen erhalten:

(1) N-[2-(1-tert.Butyloxycarbonyl-4-piperidinyl)ethyl]-N-(2-hydroxyethyl)-N'-[4-[2-(O,O'-diethylphosphono)-ethyl]phenyl]-harnstoff
$R_f$-Wert: 0,28 (Kieselgel; Essigester/Methanol = 15:1).

(2) N-(2-Cyano-1,2,3,4-tetrahydro-6-naphthyl)-N'-(2,2-diethoxyethyl)-N'-[trans-4-(methoxycarbonyl)-cyclohexyl]-harnstoff
$R_f$-Wert: 0,55 (Kieselgel; Cyclohexan/Essigester = 1:1).

(3) N-[2-(1-tert.Butyloxycarbonyl-4-piperidinyl)ethyl]-N-(2,2-dimethoxyethyl)-N'-(2-methoxycarbonyl-6-naphthyl)-harnstoff
Das als Ausgangsmaterial eingesetzte N-[2-(1-tert.Butyloxycarbonyl-4-piperidinyl)ethyl]-aminoacetaldehyd-dimethylacetal wird durch Umsetzung von 1-tert.Butyloxycarbonyl-4-[2-(methansulfonyloxy)ethyl]-piperidin mit Aminoacetaldehyd-dimethylacetal erhalten.
$R_f$-Wert: 0,33 (Kieselgel; Cyclohexan/Essigester = 1:1).

(4) N-[2-(1-tert.Butyloxycarbonyl-4-piperidinyl)ethyl]-N-(2,2-dimethoxyethyl)-N'-(2-methoxycarbonyl-1,2,3,4-tetrahydro-6-naphthyl)-harnstoff
Das als Ausgangsmaterial eingesetzte 6-Amino-2-methoxycarbonyl-1,2,3,4-tetrahydro-naphthalin-hydrochlorid (Schmelzpunkt: 260-261 °C) wird aus 2-Cyano-6-amino-1,2,3,4-tetrahydro-naphthalin durch Rückflußerhitzen in halbkonzentrierter Salzsäure und anschließender Veresterung mit Thionylchlorid/Methanol erhalten.
$R_f$-Wert: 0,40 (Kieselgel; Cyclohexan/Essigester = 1:1).

(5) N-[4-(4-Chinuclidinyl)phenyl]-N'-(2,2-diethoxyethyl)-N'-[trans-4-(methoxycarbonyl)cyclohexyl]-harnstoff
$R_f$-Wert: 0,22 (Reversed Phase Kieselgel; Methanol/5%ige wäßrige Kochsalzlösung = 6:4).

(6) N-(2,2-Diethoxyethyl)-N-[trans-4-(methoxycarbonyl)cyclohexyl]-N'-[4-(4-methyl-1-trifluoracetyl-4-piperidinyl)phenyl]-harnstoff
$R_f$-Wert: 0,70 (Kieselgel; Methylenchlorid/Essigester = 4:1).

(7) N-[4-(Benzyloxy)phenyl]-N'-(2,2-diethoxyethyl)-N'-[trans-4-(methoxycarbonyl)cyclohexyl]-harnstoff
$R_f$-Wert: 0,35 (Kieselgel; Methylenchlorid/Essigester = 100:5).

## Beispiel XXI

### 1-Cyan-3-[2-(1-tert.butyloxycarbonyl-4-piperidinyl)ethyl]-3-(2-hydroxyethyl)-4-[4-[2-(methoxycarbonyl)ethyl]-phenyl]-guanidin

7,1 g Cyanimino-[4-[2-(methoxycarbonyl)ethyl]phenyl]-phenoxymethan und 7,2 g N-[2-(1-tert.Butyloxycarbonyl-4-piperidinyl)ethyl]-ethanolamin (hergestellt durch Umsetzung von 1-tert.Butyloxycarbonyl-4-[2-(methansulfonyloxy)ethyl]-piperidin mit Ethanolamin) werden in 150 ml Isopropanol 20 Stunden am Rückfluß erhitzt. Das Reaktionsgemisch wird eingeengt und der Rückstand durch Chromatographie über eine Kieselgelsäule mit Methylenchlorid/Essigester (4:6) gereinigt.
Ausbeute: 4,6 g (46 % der Theorie),
$R_f$-Wert: 0,26 (Kieselgel, Methylenchlorid/Essigester = 4:6).

## Beispiel XXII

### Cyanimino-[4-[2-(methoxycarbonyl)ethyl]phenyl]-phenoxy-methan

7,3 g Cyancarbimidsäure-diphenylester und 5,0 g 3-(4-Aminophenyl)propionsäure-methylester werden in 100 ml Isopropanol 20 Stunden bei Raumtemperatur gerührt. Der Feststoff wird abgesaugt, mit Isopropanol und Petrolether gewaschen und bei 50 °C getrocknet.
Ausbeute: 7,3 g (81 % der Theorie),
Schmelzpunkt: 156-158 °C
$R_f$-Wert: 0,62 (Kieselgel, Methylenchlorid/Essigester = 9:1).

Beispiel XXIII

4-(2-Hydroxyethyl)-chinuclidin x $BH_3$

Zu 2,65 g 4-(2-Methoxyvinyl)-chinuclidin in 20 ml Toluol werden unter Rühren tropfenweise 7 g 70%ige Perchlorsäure zugetropft. Nach 1,5 Stunden Rühren bei Raumtemperatur wird mit Toluol verdünnt und mit gesättigter Kaliumcarbonat-Lösung alkalisch gestellt. Die organische Phase wird abdekantiert und die wäßrige Phase dreimal mit tert.Butyl-methylether ausgerührt. Die vereinigten organischen Phasen werden eingeengt. Der Rückstand wird in 20 ml Wasser gelöst und mit 360 mg Natriumborhydrid versetzt. Nach Stehen über Nacht wird mit Citronensäure angesäuert, erneut alkalisch gestellt und mehrmals mit tert.Butyl-methylether, Essigester und Methyl-ethylketon extrahiert. Die vereinigten organischen Phasen werden getrocknet und eingeengt. Der Rückstand wird in 20 ml Tetrahydrofuran gelöst und unter Stickstoff im Trockeneisbad mit 15 ml 1 M Boran in Tetrahydrofuran versetzt. Nach Stehen bei Raumtemperatur über Nacht wird 1 ml Methanol zugetropft und eingeengt. Der Rückstand wird in tert.Butyl-methylether aufgenommen, mit Wasser und Kochsalzlösung gewaschen, getrocknet und eingeengt. Der Rückstand wird durch Chromatographie über eine Kieselgelsäule mit Cyclohexan/Essigester (1:1) gereinigt.
Ausbeute: 0,80 g (30 % der Theorie),
$R_f$-Wert: 0,15 (Kieselgel; Cyclohexan/Essigester = 1:1).

Beispiel XXIV

4-(2-Methoxyvinyl)-chinuclidin

Zu 9,93 g Methoxymethyl-triphenylphosphoniumchlorid in 45 ml Tetrahydrofuran werden unter Rühren bei Raumtemperatur 17,6 ml 1,6 M n-Butyllithium in Hexan zugetropft. Nach 10 Minuten wird bei 0°C eine Lösung von 3,4 g Chinuclidin-4-aldehyd in 20 ml Tetrahydrofuran zugetropft. Nach 3 Stunden Rühren bei Raumtemperatur wird eingeengt, der Rückstand im Eisbad abgekühlt und mit 20 ml 2N Zitronensäure-Lösung und etwas Eiswasser versetzt. Es wird dreimal mit Chloroform extrahiert. Die wäßrige Phase wird etwas eingeengt, alkalisch gestellt und mit tert.Butyl-methylether extrahiert. Die vereinigten Extrakte werden getrocknet und eingeengt.
Ausbeute: 2,4 g (58 % der Theorie),
$R_f$-Wert: 0,27 und 0,36 (Kieselgel; Methylenchlorid/Methanol/konz. wäßriges Ammoniak = 80:20:3)

Beispiel XXV

4-(4-Jodphenyl)-1-trifluoracetyl-piperidin

24,6 g 4-Phenyl-1-trifluoracetyl-piperidin (hergestellt durch Umsetzung von 4-Phenylpiperidin mit Trifluoressigsäureanhydrid in Gegenwart von N-Ethyl-diisopropylamin), 9,7 g Jod, 4,5 g Perjodsäure-dihydrat, 47,9 ml Eisessig, 9,6 ml Wasser und 1,4 ml konz. Schwefelsäure werden 6 Stunden bei 70°C gerührt. Nach Stehen über Nacht bei Raumtemperatur wird zur Trockene eingeengt, mit Toluol versetzt und erneut eingedampft. Der Rückstand wird mit 600 ml tert.Butyl-methylether und 300 ml 15%iger Natriumdisulfit-Lösung 5 Minuten heftig gerührt. Es wird abgesaugt, die organische Phase wird abgetrennt und zweimal mit Natriumdisulfit-Lösung ausgeschüttelt. Die organische Phase wird abgetrennt, getrocknet und eingeengt. Der Rückstand wird durch Chromatographie über eine Kieselgelsäule mit Cyclohexan/Essigester (3:1) gereinigt.
Ausbeute: 18,3 g (50 % der Theorie, enthält ca. 10 % Ausgangsmaterial),
$R_f$-Wert: 0,43 (Kieselgel; Cyclohexan/Essigester = 4:1)

Beispiel XXVI

3-[4-[2-(Methoxycarbonyl)ethyl]phenyl]-hydantoin

22,1 g N-[(Ethoxycarbonyl)methyl]-N'-[4-[2-(methoxycarbonyl)ethyl]phenyl]-harnstoff in 200 ml Toluol werden in der Siedehitze mit 100 mg Kalium-tert.butylat versetzt und unter Rückfluß erhitzt. Nach 30 Minuten werden 200 mg Kalium-tert.butylat zugesetzt und 30 Minuten weiter erhitzt. Das Reaktionsgemisch wird abgekühlt, mit 0,3 ml Eisessig versetzt und eingeengt. Der Rückstand wird zwischen Wasser und Essigester verteilt, die organische Phase wird abgetrennt, mit Kochsalzlösung gewaschen, getrocknet und

eingeengt. Der Rückstand wird durch Chromatographie über eine Kieselgelsäule mit Cyclohexan/Essigester (3:7) gereinigt.
Ausbeute: 14,4 g (77 % der Theorie),
Schmelzpunkt: 110-112 °C
$R_f$-Wert: 0,37 (Kieselgel; Cyclohexan/Essigester = 2:8)
Analog Beispiel XXVI wird folgende Verbindung erhalten:

(1) 1-[4-[2-(Methoxycarbonyl)ethyl]phenyl]-hydantoin Hergestellt aus N-[(Aminocarbonyl)methyl]-N-(methoxycarbonyl)-4-[2-(methoxycarbonyl)ethyl]-anilin in Gegenwart von 1 Moläquivalent Kalium-tert.butylat.
Schmelzpunkt: 228-233 °C
$R_f$-Wert: 0,42 (Kieselgel; Methylenchlorid/Methanol = 95:5)

Beispiel XXVII

1-[4-[2-(Methoxycarbonyl)ethyl]phenyl]-4,5-dimethyl-3H-imidazol-2-on

2,4 g N-[4-[2-(Methoxycarbonyl)ethyl]phenyl]-harnstoff [Schmelzpunkt: 152-154 °C (sintert ab 145 °C); hergestellt aus 3-(4-Aminophenyl)propionsäure-methylester durch Umsetzung mit Kaliumcyanat in Gegenwart von Eisessig] und 2,9 g Acetoin werden eine Stunde unter Stickstoff auf 180 °C erhitzt. Das Reaktionsgemisch wird abgekühlt, mit Eiswasser verrührt und der Feststoff abgesaugt. Das Produkt wird mit Wasser und Diethylether nachgewaschen und bei 100 °C getrocknet.
Ausbeute: 1,6 g (54 % der Theorie),
Schmelzpunkt: 176-181 °C
$R_f$-Wert: 0,11 (Kieselgel; Cyclohexan/Essigester = 2:8)

Beispiel XXVIII

N-[(Aminocarbonyl)methyl]-N-(methoxycarbonyl)-4-[2-(methoxycarbonyl)ethyl]-anilin

Zu 21,8 g N-(Carboxymethyl)-N-(methoxycarbonyl)-4-[2-(methoxycarbonyl)ethyl]-anilin [$R_f$-Wert: 0,57 (Kieselgel; Toluol/Dioxan/Ethanol/Eisessig = 90:10:10:6), hergestellt durch Hydrierung von N-[(Benzyloxycarbonyl)methyl]-N-(methoxycarbonyl)-4-[2-(methoxycarbonyl)ethyl]-anilin in Gegenwart von Palladium/Aktivkohle] in 70 ml Methylenchlorid werden rasch 7,3 ml Thionylchlorid zugetropft und ein Tropfen Dimethylformamid zugegeben. Es wird 4 Stunden unter Rückfluß erhitzt, abgekühlt, über Nacht stehen gelassen und dann eingeengt. Es wird mit Toluol versetzt und erneut einrotiert. Der Rückstand wird in 200 ml Dioxan aufgenommen und bis zur ammoniakalischen Reaktion Ammoniak über die Lösung geleitet. Das Reaktionsgemisch wird eingeengt, der Rückstand wird mit Eiswasser verrührt und der Feststoff abgesaugt. Das Produkt wird mit Wasser und mit tert.Butyl-methylether gewaschen und getrocknet.
Ausbeute: 16,2 g (75 % der Theorie),
$R_f$-Wert: 0,30 (Kieselgel; Toluol/Dioxan/Ethanol/Eisessig = 90:10:10:6)

Beispiel XXIX

N-[(Benzyloxycarbonyl)methyl]-N-(methoxycarbonyl)-4-[2-(methoxycarbonyl)ethyl]-anilin

Zu 29,5 g N-[(Benzyloxycarbonyl)methyl]-4-[2-(methoxycarbonyl)ethyl]-anilin [$R_f$-Wert: 0,42 (Kieselgel; Cyclohexan/Essigester = 7:3), hergestellt durch Umsetzung von 3-(4-Aminophenyl)propionsäure-methylester mit Bromessigsäure-benzylester in Gegenwart von N-Ethyl-diisopropylamin] und 0,3 g 4-Dimethylaminopyridin in 200 ml Pyridin werden unter Eiskühlung 27 ml Chlorameisensäure-methylester zugetropft. Danach wird über Nacht bei Raumtemperatur gerührt. Es werden weitere 14 ml Chlorameisensäure-methylester zugetropft und nochmals über Nacht gerührt. Das Reaktionsgemisch wird eingeengt, der Rückstand zwischen Wasser und Essigester verteilt und die organische Phase abgetrennt. Nach dem Waschen mit Zitronensäurelösung, Wasser und Kochsalzlösung wird getrocknet und eingeengt. Der Rückstand wird durch Chromatographie über eine Kieselgelsäule mit Cyclohexan/Essigester (75:25) gereinigt.
Ausbeute: 31,0 g (89 % der Theorie),
$R_f$-Wert: 0,31 (Kieselgel; Cyclohexan/Essigester = 7:3)

Beispiel XXX

1-[4-[2-(Methoxycarbonyl)ethyl]phenyl]-3H-benzimidazol-2-on

Zu einer Lösung von 1,2 g 2-[[4-[2-(Methoxycarbonyl)ethyl]phenyl]amino]-anilin in 5 ml Methylenchlorid werden 0,94 g N,N'-Carbonyl-diimidazol gegeben und über Nacht bei Raumtemperatur gerührt. Das Reaktionsgemisch wird mit Methylenchlorid verdünnt, mit Zitronensäurelösung und Wasser gewaschen, getrocknet und einrotiert. Der Rückstand wird durch Chromatographie über eine Kieselgelsäule mit Methylenchlorid/Essigester (75:25) gereinigt.
Ausbeute: 0,9 g (69 % der Theorie),
Schmelzpunkt: 168-170 °C
$R_f$-Wert: 0,31 (Kieselgel; Cyclohexan/Essigester = 1:1)

Beispiel XXXI

1-[2-(1-tert.Butyloxycarbonyl-4-piperidinyl)ethyl]-imidazolidin-2-on

Zu 8,9 g Imidazolidin-2-on in 150 ml Dimethylformamid werden 10,4 g Kalium-tert.butylat gegeben und 30 Minuten auf 70 °C erwärmt. Dazu werden 3,5 g 1-tert.Butyloxycarbonyl-4-(2-jodethyl)-piperidin in 5 ml Dimethylformamid gegeben und über Nacht bei Raumtemperatur gerührt. Anschließend werden 5,3 ml Eisessig zugetropft, das Reaktionsgemisch eingeengt und der Rückstand zweimal mit Toluol eingedampft. Der Rückstand wird zwischen Essigester und Wasser verteilt, die wäßrige Phase wird noch viermal mit Essigester extrahiert und die vereinigten organischen Phasen mit Wasser gewaschen, getrocknet und einrotiert. Der Rückstand wird durch Chromatographie über eine Kieselgelsäule mit Essigester/Methanol (100:3) gereinigt.
Ausbeute: 1,9 g (61 % der Theorie),
Schmelzpunkt: 111-113 °C
$R_f$-Wert: 0,30 (Kieselgel; Methylenchlorid/Methanol = 100:4)

Beispiel XXXII

2-Cyano-6-amino-1,2,3,4-tetrahydro-naphthalin

Zu 4,0 g Natriumhydroxid in 25 ml Wasser werden unter Eiskühlung 0,92 ml Brom langsam zugetropft. Es wird 10 Minuten gerührt, 3,0 g fein pulverisiertes 2-Cyano-6-aminocarbonyl-1,2,3,4-tetrahydro-naphthalin (Schmelzpunkt: 180-182 °C, hergestellt aus 2-Cyano-1,2,3,4-tetrahydro-naphthalin-6-carbonsäure durch Umsetzung mit Thionylchlorid und nachfolgender Behandlung mit konzentriertem wäßrigem Ammoniak in Gegenwart von Dioxan) zugegeben und das Kühlbad entfernt. Nach 4 Stunden wird 2,5 g Natriumdisulfit zugegeben und mit Salzsäure unter Eiskühlung einen pH-Wert von 1-2 eingestellt. Es wird 15 Minuten gerührt, auf Raumtemperatur erwärmt und abfiltriert. Die Mutterlauge wird unter Eiskühlung mit Natronlauge alkalisch gestellt, der Niederschlag wird abgesaugt, mit Eiswasser gewaschen und getrocknet.
Ausbeute: 0,87 g (34 % der Theorie),
Schmelzpunkt: 120-122 °C
$R_f$-Wert: 0,50 (Kieselgel; Cyclohexan/Essigester = 1:1)

Beispiel XXXIII

1-[4-[2-(Methoxycarbonyl)ethenyl]-2-fluor-phenyl]-imidazolidin-2-on

19,55 g 1-(4-Brom-2-fluor-phenyl)-imidazolidin-2-on, 1,96 g Palladium(II)acetat, 2,61 g Tri-o-tolylphosphin, 21,7 ml Acrylsäuremethylester, 54,5 ml Triethylamin und 32,6 ml Dimethylformamid werden bei einer Badtemperatur von 100 °C 2,5 Tage gerührt. Es wird abgekühlt, eingeengt und der Rückstand durch Chromatographie über eine Kieselgelsäule mit Methylenchlorid/Methanol (100:1) gereinigt.
Ausbeute: 9,07 g (46 % der Theorie),
Schmelzpunkt: 182-184 °C
$R_f$-Wert: 0,58 (Kieselgel; Methylenchlorid/Methanol = 20:1)
Analog Beispiel XXXIII werden folgende Verbindungen erhalten:

(1) 1-[4-[2-(Methoxycarbonyl)ethenyl]-2-trifluormethyl-phenyl]-imidazolidin-2-on
Schmelzpunkt: 145-150 °C
(2) 1-[4-[2-(Methoxycarbonyl)ethenyl]-2-methyl-phenyl]-imidazolidin-2-on
Schmelzpunkt: 149-151 °C
(3) 3-(4-Amino-3-cyano-phenyl)acrylsäure-methylester
Das Ausgangsmaterial, 4-Brom-2-cyano-anilin-hydrobromid, wird durch Umsetzung von 2-Cyano-anilin mit Brom in Eisessig erhalten.
$R_f$-Wert: 0,48 (Kieselgel; Cyclohexan/Essigester = 2:1) Das Produkt wird durch Hydrierung in Essigester in Gegenwart von Palladium auf Aktivkohle in 3-(4-Amino-3-cyano-phenyl)-propionsäure-methylester überführt.
$R_f$-Wert: 0,55 (Kieselgel; Cyclohexan/Essigester = 2:1)

Beispiel XXXIV

1-Benzyl-4-methyl-4-phenyl-piperidin

Zu 15,3 g 1-Benzyl-4-phenyl-1,2,3,6-tetrahydro-pyridin in 160 ml Tetrahydrofuran werden bei -10 bis -15 °C 38,4 ml 1,6 M Butyllithium in Hexan zugetropft. Es wird 15 Minuten gerührt, auf -50 °C abgekühlt und bei dieser Temperatur eine Lösung von 10,7 g Methyljodid in 140 ml Tetrahydrofuran zugetropft. Es wird innerhalb einer Stunde auf 15 °C erwärmt, 80 ml Wasser zugesetzt und die Phasen getrennt. Die organische Phase wird mit Wasser und gesättigter Kochsalzlösung gewaschen, getrocknet und eingedampft. Der Rückstand wird in 150 ml Ethanol in Gegenwart von 2,0 g Palladium auf Kohle (10 % Palladium) bei Raumtemperatur und einem Wasserstoffdruck von 50 psi 8 Stunden hydriert. Es wird abfiltriert, das Filtrat wird eingeengt und der Rückstand durch Chromatographie über eine Kieselgelsäule mit Methylenchlorid/Ethanol/konz. wäßriges Ammoniak (100:3:0,4) gereinigt.
Ausbeute: 4,1 g (23 % der Theorie),
$R_f$-Wert: 0,37 (Kieselgel; Methylenchlorid/Ethanol/konz. wäßriges Ammoniak = 100:3:0,4)

Beispiel XXXXV

1-Amino-4-cyano-bicyclo[2.2.2]octan-hydrochlorid

Zu einer Lösung von 4,9 g 1-Carboxy-4-cyano-bicyclo[2.2.2]octan und 3,8 ml Triethylamin in 130 ml Chloroform werden bei -10 °C rasch 2,6 ml Chlorameisensäure-ethylester zugetropft. Nach 15 Minuten Rühren wird 10 Minuten lang Ammoniakgas eingeleitet. Es wird 10 Minuten bei 0 °C gerührt, auf Raumtemperatur erwärmt und nach einer Stunde zur Trockene eingedampft. Der Rückstand wird mit Wasser in der Hitze gerührt, es wird abgekühlt, abgesaugt, mit Wasser gewaschen und getrocknet. Das erhaltene 1-Aminocarbonyl-4-cyano-bicyclo[2.2.2]octan [4,2 g (86 % der Theorie); $R_f$-Wert: 0,78 (Kieselgel; Methylenchlorid/Methanol/konz.wäßriges Ammoniak = 4:1:0,2)] wird in 100 ml Acetonitril/Wasser (1:1) suspendiert, mit 15,0 g Bis(trifluoracetoxy)jodbenzol versetzt und 18 Stunden bei Raumtemperatur gerührt. Das Reaktionsgemisch wird auf die Hälfte eingeengt, zweimal mit Essigester ausgeschüttelt und die wäßrige Phase auf einen pH von 12 bis 13 eingestellt. Es wird dreimal mit Essigester extrahiert und die vereinigten organischen Phasen mit gesättigter Kochsalzlösung gewaschen und getrocknet. Der Rückstand wird in Essigester und etwas Aceton gelöst und mit etherischer Salzsäure versetzt. Es wird im Eisbad gekühlt, abgesaugt und das Produkt mit Essigester gewaschen.
Ausbeute: 2,5 g (58 % der Theorie),
Schmelzpunkt: >250 °C
$R_f$-Wert: 0,85 (Reversed Phase Kieselgel; Methanol/5%ige wäßrige Kochsalzlösung = 6:4)

Beispiel XXXVI

(trans-4-Aminocyclohexyl)oxyessigsäure-methylester-hydrochlorid

Über eine im Eisbad gekühlte Lösung von 59,4 g (trans-4-Aminocyclohexyl)oxyessigsäure-tert.butylester in 500 ml Methanol wird eine Stunde lang Salzsäuregas geleitet und dann über Nacht bei Raumtemperatur gerührt. Es wird zur Trockene eingeengt, der Rückstand mit Aceton verrieben und der Feststoff abgesaugt und getrocknet.
Ausbeute: 34,3 g (59 % der Theorie),

Schmelzpunkt: 157-160 °C

Beispiel XXXVII

3-(2-Hydroxyethyl)-pyrrolidin

a) 1-Benzyloxycarbonyl-2-pyrrolidinon

Hergestellt durch Behandlung von 2-Pyrrolidinon mit Kalium-tert.butylat und nachfolgender Umsetzung mit Chlorameisensäure-benzylester.
Siedepunkt: 148-155 °C (0,2 mbar)
$R_f$-Wert: 0,36 (Kieselgel; Cyclohexan/Essigester = 1:1)

b) 1-Benzyloxycarbonyl-3-(tert.butyloxycarbonylmethyl)-2-pyrrolidinon

Hergestellt durch Behandlung von 1-Benzyloxycarbonyl-2-pyrrolidinon mit Lithium-bis-(trimethylsilylamid) und nachfolgender Umsetzung mit Bromessigsäure-tert.butylester bei -70 °C.
$R_f$-Wert: 0,64 (Kieselgel; Cyclohexan/Essigester = 1:1)

c) 3-(tert.Butyloxycarbonylmethyl)-2-pyrrolidinon

Hergestellt durch katalytische Hydrierung von 1-Benzyloxycarbonyl-3-(tert.butyloxycarbonylmethyl)-2-pyrrolidinon in Essigester in Gegenwart von Palladium/Aktivkohle.
$R_f$-Wert: 0,20 (Kieselgel; Cyclohexan/Essigester = 1:1)

d) 3-(2-Hydroxyethyl)-pyrrolidin

Hergestellt aus 3-(tert.Butyloxycarbonylmethyl)-2-pyrrolidinon durch Umsetzung mit Lithiumaluminiumhydrid.
$R_f$-Wert: 0,89 (Reversed Phase Kieselgel; Methanol/15%ige wäßrige Kochsalzlösung = 6:4)

Beispiel XXXVIII

1-Benzyl-4-(2-chlorethyl)-1-azoniabicyclo[2.2.2]octan-chlorid

a) 1-Benzyl-4,4-bis-(ethoxycarbonylmethyl)-piperidin

Hergestellt analog S.M. McElvain und R.E. Lyle, Jr., J. Am. Chem. Soc. 72, 384 (1950) aus 1-Benzyl-4-piperidinon.
$R_f$-Wert: 0,48 (Kieselgel; Methylenchlorid/Methanol = 95:5)

b) 1-Benzyl-4,4-bis-(2-hydroxyethyl)-piperidin

Hergestellt analog M.E. Freed und L.M. Rice, J. Heterocyclic Chem. 2, 214 (1965) aus 1-Benzyl-4,4-bis-(ethoxycarbonylmethyl)-piperidin.
$R_f$-Wert: 0,18 (Kieselgel; Methylenchlorid/Methanol = 9:1)

c) 1-Benzyl-4,4-bis-(2-chlorethyl)-piperidin-hydrochlorid

Hergestellt analog M.E. Freed und L.M. Rice, J. Heterocyclic Chem. 2, 214 (1965) aus 1-Benzyl-4,4-bis-(2-hydroxyethyl)-piperidin.
Schmelzpunkt: 168-170 °C
$R_f$-Wert: 0,45 (Reversed Phase Kieselgel; Methanol/5%ige wäßrige Kochsalzlösung = 6:4)

d) 1-Benzyl-4-(2-chlorethyl)-1-azoniabicyclo[2.2.2]octanchlorid

10,5 g 1-Benzyl-4,4-bis-(2-chlorethyl)-piperidin-hydrochlorid in 50 ml tert.Butyl-methylether werden unter starkem Rühren mit 31 ml 1N Natronlauge versetzt. Nach 15 Minuten Rühren wird die organische

Phase eingeengt. Der Rückstand wird in 30 ml Acetonitril aufgenommen, es wird 15 Minuten auf 80°C erhitzt, abgekühlt und eingeengt. Der Rückstand wird über Nacht in Aceton gerührt, der Feststoff wird abgesaugt, mit Aceton und Ether gewaschen und im Exsikkator getrocknet. Ausbeute: 7,16 g (77 % der Theorie),
Schmelzpunkt: 199-201°C
$R_f$-Wert: 0,55 (Reversed Phase Kieselgel; Methanol/5%ige wäßrige Kochsalzlösung = 6:4)

Beispiel 1

2-(4-Amidinophenyl)-4-[4-(1-carboxy-2-propyl)phenyl]-5-methyl-4H-1,2,4-triazol-3-on x 0,25 Wasser

0,5 g 2-(4-Amidinophenyl)-4[-4(1-methoxycarbonyl-2-propyl)phenyl]-5-methyl-4H-1,2,4-triazol-3-on-hydrochlorid werden in einer Mischung aus 5 ml halbkonzentrierter Salzsäure und 1 ml Eisessig bei Raumtemperatur gerührt. Nach 30, 70 und 100 Minuten werden jeweils nochmals 1 ml Eisessig zugesetzt und über Nacht weitergerührt. Es wird zur Trockene eingeengt, mit 5 ml Wasser versetzt und unter Rühren mit 1N Natronlauge ein pH-Wert von 6 eingestellt. Der Niederschlag wird abgesaugt, mit wenig Eiswasser gewaschen und getrocknet.
Ausbeute: 0,41 g (92 % der Theorie),
$R_f$-Wert: 0,55 (Reversed Phase Kieselgel; Methanol/10%ige wäßrige Kochsalzlösung = 3:1)

| | | | | | | |
|---|---|---|---|---|---|---|
| Ber.:<br>Gef.: | C | 62,57<br>62,70 | H | 5,65<br>5,71 | N | 18,24<br>18,00 |

Analog Beispiel 1 werden folgende Verbindungen erhalten:
(1) 2-(4-Amidinophenyl)-4-[4-(2-carboxy-1-propyl)phenyl]-5-methyl-4H-1,2,4-triazol-3-on x 0,5 Wasser
$R_f$-Wert: 0,55 (Reversed Phase Kieselgel; Methanol/10%ige wäßrige Kochsalzlösung = 3:1)

| | | | | | | |
|---|---|---|---|---|---|---|
| Ber.:<br>Gef.: | C | 61,85<br>61,97 | H | 5,71<br>5,77 | N | 18,03<br>18,05 |

(2) 2-(4-Amidinophenyl)-4-[4-(2-carboxyethyl)phenyl]-5-phenyl-4H-1,2,4-triazol-3-on x 2 Wasser
$R_f$-Wert: 0,47 (Reversed Phase Kieselgel; Methanol/10%ige wäßrige Kochsalzlösung = 3:1)

| | | | | | | |
|---|---|---|---|---|---|---|
| Ber.:<br>Gef.: | C | 62,47<br>62,23 | H | 5,46<br>5,18 | N | 14,88<br>15,18 |

(3) 1-(1-Amino-1,2,3,4-tetrahydronaphthalin-6-yl)-3-[4-(2-carboxyethyl)phenyl]-imidazolidin-2-on-hydrochlorid Schmelzpunkt: 236-238°C
$R_f$-Wert: 0,39 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 63,53<br>63,81 | H | 6,30<br>6,50 | N | 10,10<br>10,00 | Cl | 8,52<br>8,21 |

(4) 4-[4-(2-Carboxyethyl)phenyl]-5-methyl-2-[2-(4-piperidinyl)ethyl]-4H-1,2,4-triazol-3-on-toluolsulfonat x Wasser Das Ausgangsmaterial lag als Toluolsulfonat vor.
$R_f$-Wert: 0,59 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 56,92<br>56,82 | H | 6,61<br>6,74 | N | 10,21<br>9,95 | S | 5,84<br>5,88 |

(5) 1-(4-Amidinophenyl)-3-[trans-4-(2-carboxyethyl)cyclohexyl]-imidazolidin-2,4-dion
$R_f$-Wert: 0,45 (Reversed Phase Kieselgel; Methanol/5%ige wäßrige Kochsalzlösung = 6:4)

(6) 1-[4-(2-Carboxyethyl)phenyl]-3-[2-(4-piperidinyl)ethyl]-imidazolidin-2-on-hydrochlorid x Wasser
Schmelzpunkt: 216-225 °C
$R_f$-Wert: 0,54 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 57,06<br>57,27 | H | 7,56<br>7,55 | N | 10,51<br>10,61 | Cl | 8,86<br>9,14 |

(7) 1-[4-(2-Carboxy-1-pentyl)phenyl]-3-[2-(4-piperidinyl)ethyl]-imidazolidin-2-on-hydrochlorid
(8) 4-[4-(2-Carboxy-1-pentyl)phenyl]-5-methyl-2-[2-(4-piperidinyl)ethyl]-4H-1,2,4-triazol-3-on-hydrochlorid
$R_f$-Wert: 0,44 (Reversed Phase Kieselgel; Methanol/5%ige wäßrige Kochsalzlösung = 6:4)
(9) 4-[4-(2-Carboxy-3-methyl-1-butyl)phenyl]-5-methyl-2-[2-(4-piperidinyl)ethyl]-4H-1,2,4-triazol-3-on-hydrochlorid
(10) 4-[4-(2-Carboxy-1-butyl)phenyl]-5-methyl-2-[2-(4-piperidinyl)ethyl]-4H-1,2,4-triazol-3-on-hydrochlorid
(11) 4-[4-(2-Carboxy-1-propyl)phenyl]-5-methyl-2-[2-(4-piperidinyl)ethyl]-4H-1,2,4-triazol-3-on-hydrochlorid
(12) 2-(4-Amidinophenyl)-4-[4-(2-carboxy-1-pentyl)phenyl]-5-methyl-4H-1,2,4-triazol-3-on
(13) 4-(4-Amidinophenyl)-2-[trans-4-(2-carboxyethyl)cyclohexyl]-4H-1,2,4-triazol-3-on
(14) 1-[4-(2-Carboxyethyl)phenyl]-3-[2-(4-piperidinyl)ethyl]-3,4,5,6-tetrahydro-1H-pyrimidin-2-on-hydrochlorid
$R_f$-Wert: 0,60 (Reversed Phase Kieselgel; Methanol/5%ige wäßrige Kochsalzlösung = 6:4)
Massenspektrum: $(M+H)^+$ = 360
(15) 1-(4-Amidinophenyl)-3-[trans-4-[(carboxymethyl)oxy]-cyclohexyl]-imidazolidin-2-on
(16) 2-[4-(Aminomethyl)phenyl]-4-[4-(2-carboxy-1-propyl)phenyl]-5-methyl-4H-1,2,4-triazol-3-on-hydrochlorid
$R_f$-Wert: 0,69 (Reversed Phase Kieselgel; Methanol/10%ige wäßrige Kochsalzlösung = 3:1)
(17) 4-[4-(2-Carboxyethyl)phenyl]-5-methyl-2-[(1-piperazinyl)carbonylmethyl]-4H-1,2,4-triazol-3-on-hydrochlorid
(18) 4-[4-(2-Carboxyethyl)phenyl]-5-methyl-2-[2-(1-aza-4-cycloheptyl)ethyl]-4H-1,2,4-triazol-3-on-hydrochlorid
(19) 4-[4-(2-Carboxyethyl)phenyl]-5-methyl-2-[2-(1,4-diaza-1-cycloheptyl)ethyl]-4H-1,2,4-triazol-3-on-dihydrochlorid
(20) 1-[4-(4-Carboxybutyl)phenyl]-3-(4-piperidinyl)-imidazolidin-2-on-hydrochlorid
Schmelzpunkt: 223-225 °C
$R_f$-Wert: 0,46 (Reversed Phase Kieselgel; Methanol/5%ige wäßrige Kochsalzlösung = 6:4)
(21) 1-(4-Amidinophenyl)-3-[4-[2-(n-butylsulfonylamino)-2-carboxy-ethyl]phenyl]-imidazolidin-2-on-hydrochlorid
$R_f$-Wert: 0,43 (Reversed Phase Kieselgel; Methanol/5%ige wäßrige Kochsalzlösung = 6:4)
(22) 4-[4-(2-(n-Butylsulfonylamino)-2-carboxy-ethyl]phenyl]-5-methyl-2-[2-(4-piperidinyl)ethyl]-4H-1,2,4-triazol-3-on-hydrochlorid
(23) 1-(4-Amidinophenyl)-3-[4-[2-carboxy-2-(n-hexanoylamino)-ethyl]phenyl]-imidazolidin-2-on-hydrochlorid
(24) 1-(4-Amidinophenyl)-3-[4-[2-carboxy-2-(3-phenylpropionylamino)-ethyl]phenyl]-imidazolidin-2-on-hydrochlorid
(25) 1-(4-Amidinophenyl)-3-[4-[2-(benzylsulfonylamino)-2-carboxy-ethyl]phenyl]-imidazolidin-2-on-hydrochlorid
(26) 4-[4-[2-Carboxy-2-(methansulfonylamino)-ethyl]phenyl]-5-methyl-2-[2-(4-piperidinyl)ethyl]-4H-1,2,4-triazol-3-on-hydrochlorid
(27) 4-[4-[2-(Acetylamino)-2-carboxy-ethyl]phenyl]-5-methyl-2-[2-(4-piperidinyl)ethyl]-4H-1,2,4-triazol-3-on-hydrochlorid
(28) 4-[4-(2-Carboxy-1-octyl)phenyl]-5-methyl-2-[2-(4-piperidinyl)ethyl]-4H-1,2,4-triazol-3-on-hydrochlorid
(29) 4-[4-(2-Carboxyethyl)phenyl]-2-[2-(4-piperidinyl)ethyl]-4H-1,2,4-triazol-3-on x 1,05 HCl x 0,25 $H_20$
Schmelzpunkt: 252-255 °C
$R_f$-Wert: 0,65 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 55,84<br>55,73 | H | 6,65<br>6,73 | N | 14,47<br>14,49 | Cl | 9,61<br>9,60 |

(30) 2-[4-(2-Carboxyethyl)phenyl]-5-[2-(4-piperidinyl)ethyl]-3,4-dihydro-2H,5H-1,2,5-thiadiazol-1,1-dioxid
$R_f$-Wert: 0,71 (Reversed Phase Kieselgel; Methanol/5%ige wäßrige Kochsalzlösung = 6:4)
(31) 1-[4-(2-Carboxyethyl)phenyl]-3-[(1-piperazinyl)carbonylmethyl]-imidazolidin-2-on-hydrochlorid
$R_f$-Wert: 0,58 (Reversed Phase Kieselgel; Methanol/5%ige wäßrige Kochsalzlösung = 6:4)
Massenspektrum: $M^+$ = 360
(32) 1-[4-[2-(n-Butylsulfonylamino)-2-carboxy-ethyl]phenyl]-3-[2-(4-piperidinyl)ethyl]-imidazolidin-2-on-hydrochlorid
$R_f$-Wert: 0,51 (Reversed Phase Kieselgel; Methanol/5%ige wäßrige Kochsalzlösung = 6:4)
(33) 4-[4-(2-Carboxyethyl)phenyl]-2-[2-(1-piperazinyl)ethyl]-4H-1,2,4-triazol-3-on x 2,1 HCl x 0,15 $H_2O$
$R_f$-Wert: 0,69 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 48,08<br>48,28 | H | 6,03<br>6,11 | N | 16,49<br>16,45 | Cl | 17,53<br>17,40 |

Massenspektrum: $M^+$ = 345
(34) 1-[4-(2-Carboxyethyl)phenyl]-3-[2-(4-piperidinyl)ethyl]-3H-benzimidazol-2-on x 1,1 HCl x 0,4 $H_2O$
$R_f$-Wert: 0,38 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 62,67<br>63,06 | H | 6,61<br>6,58 | N | 9,53<br>9,48 | Cl | 8,85<br>8,51 |

(35) 1-[4-(Aminomethyl)-bicyclo[2.2.2]octan-1-yl]-3-[4-(2-carboxyethyl)phenyl]-imidazolidin-2-on-hydrochlorid
Schmelzpunkt: 334-336 °C
$R_f$-Wert: 0,43 (Reversed Phase Kieselgel; Methanol/5%ige wäßrige Kochsalzlösung = 6:4)
(36) 1-[4-(trans-4-Carboxycyclohexyl)phenyl]-3-(4-piperidinyl)-imidazolidin-2-on-hydrochlorid
(37) 2-[4-(2-Carboxyethyl)phenyl]-5-methyl-4-[2-(4-piperidinyl)ethyl]-4H-1,2,4-triazol-3-on-hydrochlorid
(38) 1-[2-(1-Aza-4-cycloheptyl)ethyl]-3-[4-(2-carboxyethyl)phenyl]-imidazolidin-2-on-hydrochlorid
Schmelzpunkt: 205-207 °C
$R_f$-Wert: 0,52 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 60,67<br>60,37 | H | 7,64<br>7,85 | N | 10,61<br>10,73 | Cl | 8,96<br>9,05 |

(39) 1-[trans-4-(2-Carboxyethyl)cyclohexyl]-3-[2-(4-piperidinyl)ethyl]-imidazolidin-2-on-hydrochlorid
$R_f$-Wert: 0,55 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 58,82<br>58,52 | H | 8,83<br>9,04 | N | 10,83<br>10,65 | Cl | 9,14<br>9,02 |

(40) 1-[4-(2-Carboxyethyl)phenyl]-3-[2-(2,2,6,6-tetramethyl-4-piperidinyl)ethyl]-imidazolidin-2-on-hydrochlorid
Schmelzpunkt: >250 °C
$R_f$-Wert: 0,44 (Reversed Phase Kieselgel; Methanol/5%ige wäßrige Kochsalzlösung = 6:4)
(41) 1-[4-(2-Carboxyethyl)phenyl]-3-[2-(4-piperidinyl)ethyl]-hydantoin-hydrochlorid x 0,6 $H_2O$
Schmelzpunkt: 226-230 °C (sintern ab 220 °C)
$R_f$-Wert: 0,56 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 56,11<br>55,72 | H | 6,74<br>6,71 | N | 10,33<br>10,32 | Cl | 8,71<br>9,11 |

(42) 3-[4-(2-Carboxyethyl)phenyl]-1-[2-(4-piperidinyl)ethyl]-hydantoin-hydrochlorid
Schmelzpunkt: 186-189 °C

$R_f$-Wert: 0,66 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 57,64<br>57,52 | H | 6,62<br>6,81 | N | 10,61<br>10,39 | Cl | 8,96<br>8,86 |

(43) 1-[4-(2-Carboxyethyl)phenyl]-3-[2-(4-piperidinyl)ethyl]-cis-4,5-dimethyl-imidazolidin-2-on-hydrochlorid
Schmelzpunkt: 157-163 °C
$R_f$-Wert: 0,53 (Reversed Phase Kieselgel; Methanol/5%ige wäßrige Kochsalzlösung = 6:4)
(44) 1-(2-Aminomethyl-1,2,3,4-tetrahydro-6-naphthyl)-3-(trans-4-carboxycyclohexyl)-imidazolidin-2-on-hydrochlorid
$R_f$-Wert: 0,55 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 61,83<br>61,86 | H | 7,41<br>7,44 | N | 10,30<br>10,30 | Cl | 8,69<br>8,80 |

(45) 1-(trans-4-Carboxycyclohexyl)-3-[4-(3-oxo-4-chinuclidinyl)phenyl]-imidazolidin-2-on-hydrochlorid
(46) 1-(5-Aminopentyl)-3-[4-(2-carboxyethyl)phenyl]-imidazolidin-2-on-hydrochlorid x 0,2 $H_2O$
Schmelzpunkt: 227-229 °C

| | | | | | | | | |
|---|---|---|---|---|---|---|---|---|
| Ber.:<br>Gef.: | C | 56,80<br>56,62 | H | 7,40<br>7,42 | N | 11,69<br>11,98 | Cl | 9,86<br>9,46 |

(47) 1-[3-(cis-1-Amino-2-cyclopentyl)propyl]-3-[4-(2-carboxyethyl)phenyl]-imidazolidin-2-on-hydrochlorid
Schmelzpunkt: 224-226 °C
(48) 1-[2-[(cis-1-Amino-2-cyclopentyl)oxy]ethyl]-3-[4-(2-carboxyethyl)phenyl]-imidazolidin-2-on-hydrochlorid
(49) 1-(2-Carboxy-1,2,3,4-tetrahydro-6-naphthyl)-3-[2-(4-piperidinyl)ethy]-imidazolidin-2-on-hydrochlorid x 1 $H_2O$
Schmelzpunkt: 274-278 °C

| | | | | | | | | |
|---|---|---|---|---|---|---|---|---|
| Ber.:<br>Gef.: | C | 59,21<br>59,33 | H | 7,57<br>7,49 | N | 9,86<br>9,87 | Cl | 8,32<br>8,27 |

Massenspektrum: $M^+$ = 371
(50) 1-(trans-4-Carboxycyclohexyl)-3-[4-(4-chinuclidinyl)phenyl]-imidazolidin-2-on-hydrochlorid x 1,2 $H_2O$
$R_f$-Wert: 0,55 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 60,63<br>60,47 | H | 7,61<br>7,60 | N | 9,22<br>9,36 | Cl | 7,78<br>7,95 |

Massenspektrum: $M^+$ = 397
(51) 1-(trans-4-Carboxycyclohexyl)-3-[4-[(2-aminoethyl)oxy]phenyl]-imidazolidin-2-on-hydrochlorid
$R_f$-Wert: 0,58 (Reversed Phase Kieselgel; Methanol/5%ige wäßrige Kochsalzlösung = 6:4)
(52) 1-[3-(trans-1-Amino-2-cyclopentyl)propyl]-3-[4-(2-carboxyethyl)phenyl]-imidazolidin-2-on-hydrochlorid
Schmelzpunkt: 219-221 °C
(53) 1-[trans-4-(2-Carboxyethyl)cyclohexyl]-3-[2-(4-chinuclidinyl)ethyl]-imidazolidin-2-on-hydrochlorid
$R_f$-Wert: 0,52 (Reversed Phase Kieselgel; Methanol/5%ige wäßrige Kochsalzlösung = 6:4)

Beispiel 2

2-(4-Amidinophenyl)-4-[4-(1-methoxycarbonyl-2-propyl)phenyl]-5-methyl-4H-1,2,4-triazol-3-on-hydrochlorid

3,2 g 2-(4-Cyanophenyl)-4-[4-(1-ethoxycarbonyl-2-propyl)phenyl]-5-methyl-4H-1,2,4-triazol-3-on, suspendiert in 75 ml trockenem Methanol, werden zu 120 ml trockenem Methanol gegeben, das bei 0°C mit Salzsäuregas gesättigt wurde. Das Gemisch wird bei 0°C mit Salzsäuregas gesättigt, es wird etwas Petrolether zugesetzt und über Nacht bei Raumtemperatur gerührt. Das Reaktionsgemisch wird eingeengt, mit 50 ml trockenem Methanol versetzt und erneut eingeengt. Der Rückstand wird in 120 ml Methanol gelöst, es wird mit methanolischem Ammoniak ein pH-Wert von 8-9 eingestellt, 2,28 g Ammoniumacetat zugegeben und 2 Stunden am Rückfluß erhitzt. Nach dem Einengen wird der Rückstand durch Chromatographie über eine Kieselgelsäule mit Methylenchlorid/Methanol (4:1) gereinigt. Das Produkt wird in Methanol/Methylenchlorid gelöst und unter Rühren mit 5 ml methanolischer Salzsäure versetzt. Die Lösung wird fast zur Trockene eingeengt, erneut Toluol zugesetzt und zur Trockene eingeengt.
Ausbeute: 2,01 g (57 % der Theorie),
$R_f$-Wert: 0,40 (Reversed Phase Kieselgel; Methanol/10%ige wäßrige Kochsalzlösung = 3:1)
Analog Beispiel 2 werden folgende Verbindungen erhalten:

(1) 2-(4-Amidinophenyl)-4-[4-(2-methoxycarbonyl-1-propyl)phenyl]-5-methyl-4H-1,2,4-triazol-3-on x 1,1 Chlorwasserstoff
$R_f$-Wert: 0,40 (Reversed Phase Kieselgel; Methanol/10%ige wäßrige Kochsalzlösung = 3:1)

| | | | | | | | | |
|---|---|---|---|---|---|---|---|---|
| Ber.:<br>Gef.: | C | 58,18<br>57,90 | H | 5,37<br>5,65 | N | 16,15<br>16,24 | Cl | 9,00<br>9,14 |

(2) 1-(4-Amidinophenyl)-3-[trans-4-[2-(methoxycarbonyl)ethyl]cyclohexyl]-3H-imidazol-2-on-hydrochlorid
$R_f$-Wert: 0,59 (Reversed Phase Kieselgel; Methanol/10%ige wäßrige Kochsalzlösung = 3:1)
(3) 1-(4-Amidinophenyl)-3-[cis-4-[2-(methoxycarbonyl)ethyl]cyclohexyl]-3H-imidazol-2-on-hydrochlorid
$R_f$-Wert: 0,46 (Reversed Phase Kieselgel; Methanol/5%ige wäßrige Kochsalzlösung = 6:4)
(4) 2-(4-Amidinophenyl)-4-[4-[2-(methoxycarbonyl)ethyl]phenyl]-5-phenyl-4H-1,2,4-triazol-3-on-hydrochlorid
$R_f$-Wert: 0,37 (Reversed Phase Kieselgel; Methanol/10%ige wäßrige Kochsalzlösung = 3:1)
(5) 1-(4-Amidinophenyl)-3-[4-[2-(5-tetrazolyl)ethyl]phenyl]-imidazolidin-2-on
$R_f$-Wert: 0,50 (Reversed Phase Kieselgel; Methanol/5%ige wäßrige Kochsalzlösung = 6:4)
(6) 2-(4-Amidinophenyl)-4-[trans-4-[2-(methoxycarbonyl)ethyl]cyclohexyl]-4H-1,2,4-triazol-3-on-hydrochlorid x $H_2O$
Schmelzpunkt: 247°C (Zers.)
$R_f$-Wert: 0,35 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 53,58<br>53,80 | H | 6,62<br>6,81 | N | 16,44<br>16,44 | Cl | 8,32<br>8,17 |

(7) 1-(4-Amidinophenyl)-3-[trans-4-[2-(methoxycarbonyl) ethyl]cyclohexyl]-imidazolidin-2,4-dion-hydrochlorid
$R_f$-Wert: 0,32 (Reversed Phase Kieselgel; Methanol/5%ige wäßrige Kochsalzlösung = 6:4)
(8) 2-(4-Amidinophenyl)-4-[4-[2-(methoxycarbonyl)-1-pentyl]phenyl]-5-methyl-4H-1,2,4-triazol-3-on-hydrochlorid
(9) 4-(4-Amidinophenyl)-2-[trans-4-[2-(methoxycarbonyl)ethyl]cyclohexyl]-4H-1,2,4-triazol-3-on-hydrochlorid
(10) 1-(4-Amidinophenyl)-3-[trans-4-[(methoxycarbonylmethyl)oxy]cyclohexyl]-imidazolidin-2-on-hydrochlorid
(11) 1-(4-Amidinophenyl)-3-[4-[2-(n-butylsulfonylamino)-2-(methoxycarbonyl)-ethyl]phenyl]-imidazolidin-2-on-acetat
Das Produkt wird als Acetat isoliert.
Schmelzpunkt: 220°C (Zers.)
$R_f$-Wert: 0,34 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 55,60<br>55,64 | H | 6,28<br>6,26 | N | 12,47<br>12,31 | S | 5,71<br>5,61 |

(12) 1-(4-Amidinophenyl)-3-[4-[2-(n-hexanoylamino)-2-(methoxycarbonyl)-ethyl]phenyl]-imidazolidin-2-on-hydrochlorid

(13) 1-(4-Amidinophenyl)-3-[4-[2-(methoxycarbonyl)-2-(3-phenylpropionylamino)-ethyl]phenyl]-imidazolidin-2-on-hydrochlorid

(14) 1-(4-Amidinophenyl)-3-[4-[2-(benzylsulfonylamino)-2-(methoxycarbonyl)-ethyl]phenyl]-imidazolidin-2-on-hydrochlorid

(15) 1-(4-Amidinophenyl)-3-[4-[2-(methoxycarbonyl)ethyl]phenyl]-imidazolidin-2-on-hydrochlorid
$R_f$-Wert: 0,45 (Reversed Phase Kieselgel; Methanol/5%ige wäßrige Kochsalzlösung = 3:2)

| | | | | | | | | |
|---|---|---|---|---|---|---|---|---|
| Ber.:<br>Gef.: | C | 59,63<br>59,59 | H | 5,75<br>5,85 | N | 13,91<br>13,70 | Cl | 8,80<br>8,55 |

(16) 2-(4-Amidinophenyl)-4-[4-[2-(methoxycarbonyl)ethyl]phenyl]-5-methyl-4H-1,2,4-triazol-3-on-acetat
Das Produkt wird als Acetat isoliert.
$R_f$-Wert: 0,32 (Reversed Phase Kieselgel; Methanol/5%ige wäßrige Kochsalzlösung = 3:2)

| | | | | | | |
|---|---|---|---|---|---|---|
| Ber.:<br>Gef.: | C | 59,59<br>59,79 | H | 5,69<br>5,72 | N | 15,79<br>16,01 |

(17) 2-(4-Amidinophenyl)-4-[4-[2-(methoxycarbonyl)ethyl]phenyl]-5-trifluormethyl-4H-1,2,4-triazol-3-on-hydrochlorid
$R_f$-Wert: 0,37 (Reversed Phase Kieselgel; Methanol/5%ige wäßrige Kochsalzlösung = 3:2)

## Beispiel 3

### 2-(4-Cyanophenyl)-4-[4-(1-ethoxycarbonyl-2-propyl)phenyl]-5-methyl-4H-1,2,4-triazol-3-on

6,5 g 1-Acetyl-2-(4-cyanophenyl)-4-[4-(1-ethoxycarbonyl-2-propyl)phenyl]-semicarbazid werden in einem auf 200 °C vorgeheizten Ölbad 1,5 Stunden im Vakuum erhitzt. Nach dem Abkühlen wird das Reaktionsgemisch durch Chromatographie über eine Kieselgelsäule mit Methylenchlorid/Essigester/Cyclohexan (20:1:1) gereinigt.
Ausbeute: 3,2 g (51 % der Theorie),
Schmelzpunkt: 134-138 °C
$R_f$-Wert: 0,51 (Kieselgel; Methylenchlorid/Essigester/Cyclohexan = 20:1:1)
Analog Beispiel 3 werden folgende Verbindungen erhalten:

(1) 2-(4-Cyanophenyl)-4-[4-(2-ethoxycarbonyl-1-propyl)phenyl]-5-methyl-4H-1,2,4-triazol-3-on
Schmelzpunkt: 124-128 °C
$R_f$-Wert: 0,77 (Kieselgel; Methylenchlorid/Essigester = 20:1)

(2) 2-(4-Cyanophenyl)-4-[cis-4-[2-(methoxycarbonyl)ethyl]cyclohexyl]-5-methyl-4H-1,2,4-triazol-3-on
Schmelzpunkt: 92-96 °C
$R_f$-Wert: 0,60 (Kieselgel; Methylenchlorid/Essigester = 9:1)

(3) 2-(4-Cyanophenyl)-4-[trans-4-[2-(methoxycarbonyl)ethyl]cyclohexyl]-5-methyl-4H-1,2,4-triazol-3-on
Schmelzpunkt: 166-167 °C
$R_f$-Wert: 0,55 (Kieselgel; Methylenchlorid/Essigester = 9:1)

(4) 2-(4-Cyanophenyl)-4-[3-[2-(methoxycarbonyl)ethyl]phenyl]-5-methyl-4H-1,2,4-triazol-3-on
Schmelzpunkt: 134-137 °C
$R_f$-Wert: 0,67 (Kieselgel; Methylenchlorid/Essigester = 9:1)

(5) 2-(4-Cyanophenyl)-4-[4-[2-(methoxycarbonyl)ethyl]phenyl]-5-phenyl-4H-1,2,4-triazol-3-on
Schmelzpunkt: 190-194 °C
$R_f$-Wert: 0,80 (Kieselgel; Methylenchlorid/Essigester = 20:1)

| | | | | | | |
|---|---|---|---|---|---|---|
| Ber.:<br>Gef.: | C | 70,74<br>70,61 | H | 4,75<br>4,81 | N | 13,20<br>13,41 |

(6) 2-(4-Cyanophenyl)-4-[trans-4-[2-(methoxycarbonyl)ethyl]cyclohexyl]-4H-1,2,4-triazol-3-on
Schmelzpunkt: 152-153 °C
(7) 2-(4-Cyanophenyl)-4-[4-[2-(methoxycarbonyl)-1-pentyl]phenyl]-5-methyl-4H-1,2,4-triazol-3-on
(8) 2-(4-Cyanophenyl)-4-[4-[2-(methoxycarbonyl)ethyl]phenyl]-5-methyl-4H-1,2,4-triazol-3-on
Schmelzpunkt: 162-165 °C
(9) 2-(4-Cyanophenyl)-4-[4-[2-(methoxycarbonyl)ethyl]phenyl]-5-trifluormethyl-4H-1,2,4-triazol-3-on
Durchführung in siedender Trifluoressigsäure
$R_f$-Wert: 0,46 (Kieselgel; Cyclohexan/Essigester = 2:1)

Beispiel 4

2-(4-Amidinophenyl)-4-[cis-4-(2-carboxyethyl)cyclohexyl]-5-methyl-4H-1,2,4-triazol-3-on

Zu einer Lösung von 400 mg 2-(4-Amidinophenyl)-4-[cis-4-[2-(methoxycarbonyl)ethyl]cyclohexyl]-5-methyl-4H-1,2,4-triazol-3-on x 1,2 Chlorwasserstoff x 1 Wasser in 4 ml Methanol werden 0,71 ml 4N Natronlauge zugegeben und 4 Stunden bei Raumtemperatur gerührt. Dann werden 178 mg Ammoniumchlorid zugegeben und eine Stunde gerührt. Der Niederschlag wird abgesaugt, mit wenig kaltem Methanol gewaschen und bei 50 °C im Vakuum getrocknet.
Ausbeute: 230 mg (65 % der Theorie),
$R_f$-Wert: 0,50 (Reversed Phase Kieselgel; Methanol/5%ige wäßrige Kochsalzlösung = 6:4)

| | | | | | | |
|---|---|---|---|---|---|---|
| Ber.:<br>Gef.: | C | 61,44<br>61,21 | H | 6,78<br>6,87 | N | 18,85<br>18,76 |

Analog Beispiel 4 werden folgende Verbindungen erhalten:
(1) 2-(4-Amidinophenyl)-4-[trans-4-(2-carboxyethyl)cyclohexyl]-5-methyl-4H-1,2,4-triazol-3-on
$R_f$-Wert: 0,50 (Reversed Phase Kieselgel; Methanol/5%ige wäßrige Kochsalzlösung = 6:4)

| | | | | | | |
|---|---|---|---|---|---|---|
| Ber.:<br>Gef.: | C | 61,44<br>61,38 | H | 6,78<br>6,93 | N | 18,55<br>18,66 |

(2) 1-(4-Amidinophenyl)-3-[trans-4-(2-carboxyethyl)cyclohexyl]-imidazolidin-2-on x 0,4 $H_2O$
$R_f$-Wert: 0,64 (Reversed Phase Kieselgel; Methanol/10%ige wäßrige Kochsalzlösung = 3:1)

| | | | | | | |
|---|---|---|---|---|---|---|
| Ber.:<br>Gef.: | C | 62,41<br>62,50 | H | 7,39<br>7,37 | N | 15,32<br>15,00 |

(3) 1-(4-Amidinophenyl)-3-[cis-4-(2-carboxyethyl)cyclohexyl]-imidazolidin-2-on x 0,25 Wasser
$R_f$-Wert: 0,64 (Reversed Phase Kieselgel; Methanol/10%ige wäßrige Kochsalzlösung = 3:1)

| | | | | | | |
|---|---|---|---|---|---|---|
| Ber.:<br>Gef.: | C | 62,88<br>63,00 | H | 7,36<br>7,32 | N | 15,44<br>15,19 |

(4) 2-(4-Amidinophenyl)-4-[trans-4-(2-carboxyethyl)cyclohexyl]-4H-1,2,4-triazol-3-on
$R_f$-Wert: 0,44 (Reversed Phase Kieselgel; Methanol/5%ige wäßrige Kochsalzlösung = 6:4)
(5) 1-[4-(Aminomethyl)cyclohexyl]-3-[4-(2-carboxyethyl)phenyl]-imidazolidin-2-on
$R_f$-Wert: 0,55 (Reversed Phase Kieselgel; Methanol/5%ige wäßrige Kochsalzlösung = 6:4)
(6) 1-[4-[2-(n-Butylsulfonylamino)-2-carboxy-ethyl]phenyl]-3-(4-cyanophenyl)-imidazolidin-2-on
Aufarbeitung mit Salzsäure.
$R_f$-Wert: 0,36 (Kieselgel; Toluol/Dioxan/Ethanol/Eisessig = 90:10:10:6)

| | | | | | | |
|---|---|---|---|---|---|---|
| Ber.: | C | 58,71 | H | 5,57 | N | 11,91 |
| Gef.: | | 58,73 | | 5,69 | | 11,82 |

(7) 2-[4-(Benzyloxycarbonylamidino)phenyl]-4-[4-(2-carboxyethyl)phenyl]-5-methyl-4H-1,2,4-triazol-3-on
(8) 2-(4-Amidinophenyl)-4-[4-(2-carboxyethyl)phenyl]-5-methyl-4H-1,2,4-triazol-3-on
$R_f$-Wert: 0,61 (Reversed Phase Kieselgel; Methanol/5%ige wäßrige Kochsalzlösung = 3:2)

| | | | | | | |
|---|---|---|---|---|---|---|
| Ber.: | C | 62,46 | H | 5,24 | N | 19,17 |
| Gef.: | | 62,21 | | 5,33 | | 19,39 |

(9) 2-(4-Amidinophenyl)-4-[4-(2-carboxyethyl)phenyl]-5-trifluormethyl-4H-1,2,4-triazol-3-on
$R_f$-Wert: 0,52 (Reversed Phase Kieselgel; Methanol/5%ige wäßrige Kochsalzlösung = 6:4)

| | | | | | | |
|---|---|---|---|---|---|---|
| Ber.: | C | 54,42 | H | 3,85 | N | 16,70 |
| Gef.: | | 54,29 | | 3,86 | | 17,06 |

(10) 1-[4-(2-Carboxyethyl)phenyl]-2-cyanimino-3-[2-(4-piperidinyl)ethyl]-imidazolidin x 0,95 HCl x 0,3 $H_2O$
$R_f$-Wert: 0,44 (Reversed Phase Kieselgel; Methanol/5%ige wäßrige Kochsalzlösung = 6:4)

| | | | | | | | | |
|---|---|---|---|---|---|---|---|---|
| Ber.: | C | 58,52 | H | 7,24 | N | 17,06 | Cl | 8,21 |
| Gef.: | | 58,84 | | 7,05 | | 16,89 | | 8,05 |

(11) 1-[4-(2-Carboxyethyl)phenyl]-3-[2-(4-piperidinyl)ethyl]-4,5-dimethyl-3H-imidazol-2-on-hydrochlorid
$R_f$-Wert: 0,43 (Reversed Phase Kieselgel; Methanol/5%ige wäßrige Kochsalzlösung = 6:4)
(12) 1-[(4-Carboxy-1-piperidinyl)carbonylmethyl]-3-[2-(4-piperidinyl)ethyl-imidazolidin-2-on
$R_f$-Wert: 0,71 (Reversed Phase Kieselgel; Methanol/5%ige wäßrige Kochsalzlösung = 6:4)
Massenspektrum: $M^+$ = 366
(13) 1-[4-(2-Carboxyethenyl)-2-fluor-phenyl]-3-[2-(4-piperidinyl)ethyl]-imidazolidin-2-on x 1,1 HCl x 1 $H_2O$
Schmelzpunkt: 253-255 °C (Zers.)

| | | | | | | | | |
|---|---|---|---|---|---|---|---|---|
| Ber.: | C | 54,39 | H | 6,51 | N | 10,02 | Cl | 9,29 |
| Gef.: | | 54,15 | | 6,43 | | 10,03 | | 9,02 |

(14) 1-(trans-4-Carboxycyclohexyl)-3-[4-(1-benzyloxycarbonyl-4-piperidinyl)phenyl]-imidazolidin-2-on
Schmelzpunkt: 194-195 °C
$R_f$-Wert: 0,54 (Kieselgel; Methylenchlorid/Methanol = 9:1)
(15) 1-(2-Carboxy-6-naphthyl)-3-[2-(4-piperidinyl)ethyl]-imidazolidin-2-on x 0,9 HCl x 1 $H_2O$
$R_f$-Wert: 0,36 (Reversed Phase Kieselgel; Methanol/5%ige wäßrige Kochsalzlösung = 6:4)

| | | | | | | | | |
|---|---|---|---|---|---|---|---|---|
| Ber.: | C | 60,25 | H | 6,72 | N | 10,05 | Cl | 7,62 |
| Gef.: | | 60,17 | | 6,69 | | 10,11 | | 7,77 |

Massenspektrum: $M^+$ = 367

Beispiel 5

2-(4-Amidinophenyl)-4-[cis-4-[2-(methoxycarbonyl)ethyl]cyclohexyl]-5-methyl-4H-1,2,4-triazol-3-on x 1,2 Chlorwasserstoff x 1 Wasser

0,9 g 2-(4-Cyanophenyl)-4-[cis-4-[2-(methoxycarbonyl)ethyl]-cyclohexyl]-5-methyl-4H-1,2,4-triazol-3-on in 10 ml trockenem Methanol werden zu 30 ml trockenem Methanol gegeben, das mit Salzsäuregas gesättigt wurde. Das Gemisch wird bei 0 °C mit Salzsäuregas gesättigt und bei Raumtemperatur über

Nacht stehengelassen. Es wird zur Trockene eingeengt, mit trockenem Methanol versetzt und erneut eingeengt. Der Rückstand wird in 20 ml Methanol aufgenommen und mit 10 ml konzentriertem wäßrigem Ammoniak versetzt und über Nacht bei Raumtemperatur gerührt. Es wird zur Trockene eingeengt und der Rückstand durch Chromatographie über eine Kieselgelsäule mit Methylenchlorid/Methanol (6:1) gereinigt.
Ausbeute: 600 mg (60 % der Theorie),
$R_f$-Wert: 0,37 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 53,71<br>53,74 | H | 6,80<br>6,87 | N | 15,65<br>15,78 | Cl | 9,51<br>9,01 |

Analog Beispiel 5 werden folgende Verbindungen erhalten:
(1) 2-(4-Amidinophenyl)-4-[trans-4-[2-(methoxycarbonyl)ethyl]cyclohexyl]-5-methyl-4H-1,2,4-triazol-3-on x 1,1 Chlorwasserstoff x 0,3 Wasser
$R_f$-Wert: 0,37 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 55,12<br>55,33 | H | 6,63<br>6,74 | N | 16,07<br>15,97 | Cl | 8,95<br>8,88 |

(2) 2-(4-Amidinophenyl)-4-[3-(2-carboxyethyl)phenyl]-5-methyl-4H-1,2,4-triazol-3-on (es wurde direkt die Amidino-Säure erhalten)
$R_f$-Wert: 0,62 (Reversed Phase Kieselgel; Methanol/5%ige wäßrige Kochsalzlösung = 6:4)

Beispiel 6

2-(4-Amidinophenyl)-4-[4-[2-(ethoxycarbonyl)ethyl]phenyl]-5-methyl-4H-1,2,4-triazol-3-on-hydrochlorid

750 mg 2-(4-Amidinophenyl)-4-[4-(2-carboxyethyl)phenyl]-5-methyl-4H-1,2,4-triazol-3-on werden 3 Stunden in ethanolischer Salzsäure gerührt. Das Reaktionsgemisch wird einrotiert, mit Toluol versetzt und erneut eingedampft. Dieser Vorgang wird einigemale wiederholt, dann wird der Rückstand mit Aceton gerührt, das Produkt abgesaugt und mit Aceton gewaschen.
Ausbeute: 770 mg (87 % der Theorie),
Schmelzpunkt: 279-283 °C (Zers.)
$R_f$-Wert: 0,39 (Reversed Phase Kieselgel; Methanol/5%ige wäßrige Kochsalzlösung = 6:4)
Analog Beispiel 6 werden folgende Verbindungen erhalten:
(1) 2-(4-Amidinophenyl)-4-[4-[2-[(3-pentyl)oxycarbonyl]ethyl]phenyl]-5-methyl-4H-1,2,4-triazol-3-on-hydrochlorid
$R_f$-Wert: 0,27 (Reversed Phase Kieselgel; Methanol/5%ige wäßrige Kochsalzlösung = 6:4)
(2) 2-(4-Amidinophenyl)-4-[4-[2-(neopentyloxycarbonyl)ethyl]phenyl]-5-methyl-4H-1,2,4-triazol-3-on-hydrochlorid
Schmelzpunkt: 295-297 °C (Zers.)
$R_f$-Wert: 0,25 (Reversed Phase Kieselgel; Methanol/5%ige wäßrige Kochsalzlösung = 6:4)
(3) 2-(4-Amidinophenyl)-4-[4-[2-(isopropyloxycarbonyl)ethyl]phenyl]-5-trifluormethyl-4H-1,2,4-triazol-3-on-hydrochlorid
Schmelzpunkt: 319-320 °C
$R_f$-Wert: 0,30 (Reversed Phase Kieselgel; Methanol/5%ige wäßrige Kochsalzlösung = 6:4)
(4) 2-(4-Amidinophenyl)-4-[trans-4-[2-(isopropyloxycarbonyl)ethyl]cyclohexyl]-5-methyl-4H-1,2,4-triazol-3-on-hydrochlorid
$R_f$-Wert: 0,29 (Reversed Phase Kieselgel; Methanol/5%ige wäßrige Kochsalzlösung = 6:4)
(5) 2-(4-Amidinophenyl)-4-[cis-4-[2-(isopropyloxycarbonyl)ethyl]cyclohexyl]-5-methyl-4H-1,2,4-triazol-3-on-hydrochlorid
$R_f$-Wert: 0,29 (Reversed Phase Kieselgel; Methanol/5%ige wäßrige Kochsalzlösung = 6:4)
(6) 1-(4-Amidinophenyl)-3-[trans-4-[2-(isopropyloxycarbonyl)ethyl]cyclohexyl]-imidazolidin-2-on-hydrochlorid
$R_f$-Wert: 0,52 (Reversed Phase Kieselgel; Methanol/10%ige wäßrige Kochsalzlösung = 3:1)
(7) 1-(4-Amidinophenyl)-3-[trans-4-[2-(ethyloxycarbonyl)ethyl]cyclohexyl]-imidazolidin-2-on-hydrochlorid x 0,5 Wasser

$R_f$-Wert: 0,55 (Reversed Phase Kieselgel; Methanol/10%ige wäßrige Kochsalzlösung = 3:1)

| | | | | | | |
|---|---|---|---|---|---|---|
| Ber.: | C | 58,39 | H | 7,47 | N | 12,97 |
| Gef.: | | 58,58 | | 7,41 | | 12,94 |

(8) 1-(4-Amidinophenyl)-3-[cis-4-[2-(isopropyloxycarbonyl)ethyl]cyclohexyl]-imidazolidin-2-on x 1,05 Chlorwasserstoff x 0,2 Wasser
$R_f$-Wert: 0,52 (Reversed Phase Kieselgel; Methanol/10%ige wäßrige Kochsalzlösung = 3:1)

| | | | | | | | | |
|---|---|---|---|---|---|---|---|---|
| Ber.: | C | 59,72 | H | 7,62 | N | 12,66 | Cl | 8,41 |
| Gef.: | | 59,81 | | 7,67 | | 12,83 | | 8,59 |

(9) 1-(4-Amidinophenyl)-3-[cis-4-[2-(ethoxycarbonyl)ethyl]cyclohexyl]-imidazolidin-2-on x 1,1 Chlorwasserstoff x 0,5 Wasser
$R_f$-Wert: 0,55 (Reversed Phase Kieselgel; Methanol/10%ige wäßrige Kochsalzlösung = 3:1)

| | | | | | | | | |
|---|---|---|---|---|---|---|---|---|
| Ber.: | C | 58,14 | H | 7,45 | N | 12,91 | Cl | 8,99 |
| Gef.: | | 58,37 | | 7,39 | | 13,08 | | 9,03 |

(10) 4-[4-[2-(Methoxycarbonyl)ethyl]phenyl]-5-methyl-2-[2-(1-methyl-4-piperidinyl)ethyl]-4H-1,2,4-triazol-3-on-hydrochlorid
(11) 1-[4-[2-(Methoxycarbonyl)ethyl]cyclohexyl]-3-[2-(4-piperidinyl)ethyl]-imidazolidin-2-on-hydrochlorid
(12) 1-[trans-4-(Methoxycarbonyl)cyclohexyl]-3-[4-(4-piperidinyl)phenyl]-imidazolidin-2-on-hydrochlorid
Verwendung von Thionylchlorid statt Salzsäuregas.
$R_f$-Wert: 0,38 (Reversed Phase Kieselgel; Methanol/10%ige wäßrige Kochsalzlösung = 6:4)
Massenspektrum: $M^+$ = 385
(13) 1-[4-(Methoxycarbonyl)butyl]-3-[4-(4-piperidinyl)phenyl]-imidazolidin-2-on-hydrochlorid
(14) 2-(4-Amidinophenyl)-4-[4-[2-(cyclohexyloxycarbonyl)ethyl]phenyl)-5-methyl-4H-1,2,4-triazol-3-on-hydrochlorid
$R_f$-Wert: 0,16 (Reversed Phase Kieselgel; Methanol/5%ige wäßrige Kochsalzlösung = 6:4)

| | | | | | | | | |
|---|---|---|---|---|---|---|---|---|
| Ber.: | C | 62,04 | H | 6,25 | N | 14,47 | Cl | 7,33 |
| Gef.: | | 61,87 | | 6,24 | | 14,50 | | 7,60 |

(15) 2-(4-Amidinophenyl)-4-[4-[2-(cyclohexylmethyloxycarbonyl)ethyl]phenyl]-5-methyl-4H-1,2,4-triazol-3-on-hydrochlorid
Schmelzpunkt: 273-275 °C (Zers.)
$R_f$-Wert: 0,10 (Reversed Phase Kieselgel; Methanol/5%ige wäßrige Kochsalzlösung = 6:4)

| | | | | | | | | |
|---|---|---|---|---|---|---|---|---|
| Ber.: | C | 62,70 | H | 6,48 | N | 14,06 | Cl | 7,12 |
| Gef.: | | 62,43 | | 6,61 | | 14,00 | | 7,27 |

(16) 2-(4-Amidinophenyl)-4-[4-[2-(cyclopentyloxycarbonyl)ethyl]phenyl]-5-methyl-4H-1,2,4-triazol-3-on-hydrochlorid
$R_f$-Wert: 0,22 (Reversed Phase Kieselgel; Methanol/5%ige wäßrige Kochsalzlösung = 6:4)

| | | | | | | | | |
|---|---|---|---|---|---|---|---|---|
| Ber.: | C | 61,34 | H | 6,01 | N | 14,90 | Cl | 7,54 |
| Gef.: | | 61,13 | | 6,03 | | 14,79 | | 7,84 |

(17) 2-(4-Amidinophenyl)-4-[4-[2-(cycloheptyloxycarbonyl)ethyl]phenyl]-5-methyl-4H-1,2,4-triazol-3-on-hydrochlorid

(18) 2-(4-Amidinophenyl)-4-[4-[2-(cyclooctyloxycarbonyl)ethyl]phenyl]-5-methyl-4H-1,2,4-triazol-3-on-hydrochlorid
(19) 2-(4-Amidinophenyl)-4-[4-[2-[(2-cyclohexylethyl)oxycarbonyl]ethyl]phenyl]-5-methyl-4H-1,2,4-triazol-3-on-hydrochlorid
(20) 2-(4-Amidinophenyl)-4-[4-[2-[(cyclopentylmethyl)oxycarbonyl]ethyl]phenyl]-5-methyl-4H-1,2,4-triazol-3-on-hydrochlorid
(21) 2-(4-Amidinophenyl)-4-[4-[2-[(4-methylcyclohexyl)oxycarbonyl]ethyl]phenyl]-5-methyl-4H-1,2,4-triazol-3-on-hydrochlorid
(22) 2-(4-Amidinophenyl)-4-[4-[2-[(3,5-dimethylcyclohexyl)oxycarbonyl]ethyl]phenyl]-5-methyl-4H-1,2,4-triazol-3-on-hydrochlorid
(23) 2-(4-Amidinophenyl)-4-[4-[2-[(4-ethylcyclohexyl)oxycarbonyl]ethyl]phenyl]-5-methyl-4H-1,2,4-triazol-3-on-hydrochlorid
(24) 2-(4-Amidinophenyl)-4-[4-[2-(menthyloxycarbonyl)ethyl]phenyl]-5-methyl-4H-1,2,4-triazol-3-on-hydrochlorid
(25) 2-(4-Amidinophenyl)-4-[4-[2-[(4-methoxycyclohexyl)oxycarbonyl]ethyl]phenyl]-5-methyl-4H-1,2,4-triazol-3-on-hydrochlorid
(26) 2-(4-Amidinophenyl)-4-[4-[2-[(2-norbornyl)oxycarbonyl]ethyl]phenyl]-5-methyl-4H-1,2,4-triazol-3-on-hydrochlorid
(27) 2-(4-Amidinophenyl)-4-[4-[2-[(3-pinanyl)oxycarbonyl]ethyl]phenyl]-5-methyl-4H-1,2,4-triazol-3-on-hydrochlorid
(28) 2-(4-Amidinophenyl)-4-[4-[2-(fenchyloxycarbonyl]ethyl]phenyl]-5-methyl-4H-1,2,4-triazol-3-on-hydrochlorid
(29) 2-(4-Amidinophenyl)-4-[4-[(2-indanyl)oxycarbonyl]ethyl]phenyl]-5-methyl-4H-1,2,4-triazol-3-on-hydrochlorid
(30) 2-(4-Amidinophenyl)-4-[4-[2-(isopropyloxycarbonyl)-1-propyl]phenyl]-5-methyl-4H-1,2,4-triazol-3-on-hydrochlorid
Schmelzpunkt: 260-262 °C
$R_f$-Wert: 0,36 (Reversed Phase Kieselgel; Methanol/5%ige wäßrige Kochsalzlösung = 6:4)
(31) 2-(4-Amidinophenyl)-4-[4-[2-(isopropyloxycarbonyl)ethyl]phenyl]-5-phenyl-4H-1,2,4-triazol-3-on-hydrochlorid
Schmelzpunkt: 295-297 °C
$R_f$-Wert: 0,24 (Reversed Phase Kieselgel; Methanol/5%ige wäßrige Kochsalzlösung = 6:4)
(32) 1-[4-[2-(Cyclohexyloxycarbonyl)ethyl]phenyl]-3-[2-(4-piperidinyl)ethyl]-imidazolidin-2-on-hydrochlorid x $H_2O$
Schmelzpunkt: 185-188 °C
$R_f$-Wert: 0,24 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 62,29<br>62,34 | H | 8,36<br>8,38 | N | 8,72<br>8,74 | Cl | 7,35<br>8,04 |

(33) 1-[2-(4-Piperidinyl)ethyl]-3-[4-[2-(isopropyloxycarbonyl)ethyl]phenyl]-imidazolidin-2-on-hydrochlorid
Schmelzpunkt: 185-188 °C
$R_f$-Wert: 0,32 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 62,32<br>62,10 | H | 8,08<br>8,04 | N | 9,91<br>9,96 | Cl | 8,36<br>8,20 |

(34) 1-[4-[2-(Ethoxycarbonyl)ethyl]phenyl]-3-[2-(4-piperidinyl)ethyl]-imidazolidin-2-on-hydrochlorid
Verwendung von Thionylchlorid statt Salzsäuregas.
Schmelzpunkt: 177-180 °C
$R_f$-Wert: 0,33 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 61,53<br>61,30 | H | 7,87<br>7,92 | N | 10,25<br>10,28 | Cl | 8,65<br>8,83 |

(35) 1-[trans-4-[2-(Ethoxycarbonyl)ethyl]cyclohexyl]-3-[2-(4-piperidinyl)ethyl]-imidazolidin-2-on-hydrochlorid
Verwendung von Thionylchlorid anstatt Salzsäuregas.
Schmelzpunkt: 188-196 °C
$R_f$-Wert: 0,34 (Reversed Phase Kieselgel; Methanol/5%ige wäßrige Kochsalzlösung = 6:4)

| | | | | | | | | |
|---|---|---|---|---|---|---|---|---|
| Ber.: | C | 60,63 | H | 9,21 | N | 10,10 | Cl | 8,52 |
| Gef.: | | 60,32 | | 9,17 | | 9,99 | | 8,79 |

(36) 1-[trans-4-[2-(Isopropyloxycarbonyl)ethyl]cyclohexyl]-3-[2-(4-piperidinyl)ethyl]-imidazolidin-2-on x 1,05 HCl
Verwendung von Thionylchlorid anstatt Salzsäuregas.
Schmelzpunkt: 194-199 °C
$R_f$-Wert: 0,26 (Reversed Phase Kieselgel; Methanol/5%ige wäßrige Kochsalzlösung = 6:4)

| | | | | | | | | |
|---|---|---|---|---|---|---|---|---|
| Ber.: | C | 61,15 | H | 9,39 | N | 9,73 | Cl | 8,67 |
| Gef.: | | 61,00 | | 9,33 | | 9,77 | | 8,75 |

(37) 1-[2-(4-Chinuclidinyl)ethyl]-3-[4-[2-(ethoxycarbonyl)ethyl]phenyl]-imidazolidin-2-on-hydrochlorid
Verwendung von Thionylchlorid anstatt Salzsäuregas.
Schmelzpunkt: 217-220 °C
$R_f$-Wert: 0,29 (Reversed Phase Kieselgel; Methanol/5%ige wäßrige Kochsalzlösung = 6:4)
(38) 1-[2-(4-Chinuclidinyl)ethyl]-3-[4-[2-(isopropyloxycarbonyl)ethyl]phenyl]-imidazolidin-2-on-hydrochlorid
Verwendung von Thionylchlorid anstatt Salzsäuregas.
$R_f$-Wert: 0,25 (Reversed Phase Kieselgel; Methanol/5%ige wäßrige Kochsalzlösung = 6:4)
(39) 1-[4-(4-Cyano-4-piperidinyl)phenyl]-3-[trans-4-(methoxycarbonyl)cyclohexyl]-imidazolidin-2-on-hydrochlorid x $H_2O$
Schmelzpunkt: >240 °C
$R_f$-Wert: 0,54 (Reversed Phase Kieselgel; Methanol/5%ige wäßrige Kochsalzlösung = 6:4)

| | | | | | | | | |
|---|---|---|---|---|---|---|---|---|
| Ber.: | C | 59,41 | H | 7,15 | N | 12,05 | Cl | 7,62 |
| Gef.: | | 59,71 | | 7,21 | | 11,95 | | 7,94 |

Massenspektrum: $M^+$ = 410
(40) 1-[trans-4-(Ethoxycarbonyl)cyclohexyl]-3-[4-(4-piperidinyl)phenyl]-imidazolidin-2-on-hydrochlorid
Verwendung von Thionylchlorid statt Salzsäuregas.
$R_f$-Wert: 0,39 (Reversed Phase Kieselgel; Methanol/5%ige wäßrige Kochsalzlösung = 6:4)

| | | | | | | | | |
|---|---|---|---|---|---|---|---|---|
| Ber.: | C | 63,36 | H | 7,86 | N | 9,64 | Cl | 8,13 |
| Gef.: | | 62,91 | | 7,86 | | 9,62 | | 8,65 |

(41) 1-[4-(4-Chinuclidinyl)phenyl)-3-[trans-4-(ethoxycarbonyl)cyclohexyl]-imidazolidin-2-on-hydrochlorid x 0,5 $H_2O$
$R_f$-Wert: 0,27 (Reversed Phase Kieselgel; Methanol/5%ige wäßrige Kochsalzlösung = 6:4)

| | | | | | | | | |
|---|---|---|---|---|---|---|---|---|
| Ber.: | C | 63,75 | H | 7,92 | N | 8,92 | Cl | 7,52 |
| Gef.: | | 63,69 | | 7,93 | | 8,82 | | 7,38 |

Massenspektrum: $M^+$ = 425
(42) 1-[trans-4-(Ethoxycarbonyl)cyclohexyl]-3-[4-(4-piperidinyl)phenyl]-3H-imidazol-2-on-hydrochlorid
Schmelzpunkt: 260-270 °C
$R_f$-Wert: 0,50 (Reversed Phase Kieselgel; Methanol/5%ige wäßrige Kochsalzlösung = 6:4)

| | | | | | | |
|---|---|---|---|---|---|---|
| Ber.: | C | 63,65 | H | 7,43 | N | 9,68 |
| Gef.: | | 63,31 | | 7,53 | | 9,58 |

Massenspektrum: $M^+$ = 397

(43) 1-[trans-4-(Ethoxycarbonyl)cyclohexyl]-3-[4-(4-methyl-4-piperidinyl)phenyl]-imidazolidin-2-on-hydrochlorid

$R_f$-Wert: 0,43 (Reversed Phase Kieselgel; Methanol/5%ige wäßrige Kochsalzlösung = 6:4)

| | | | | | | | | |
|---|---|---|---|---|---|---|---|---|
| Ber.: | C | 64,05 | H | 8,06 | N | 9,34 | Cl | 7,88 |
| Gef.: | | 63,80 | | 8,28 | | 9,24 | | 7,89 |

(44) 1-[2-(4-Chinuclidinyl)ethyl]-3-[trans-4-[2-(ethoxycarbonyl)ethyl]cyclohexyl)-imidazolidin-2-on-hydrochlorid

Schmelzpunkt: 224-226 °C

Beispiel 7

1-(4-Amidinophenyl)-3-[trans-4-[2-(methoxycarbonyl)ethyl]cyclohexyl]-imidazolidin-2-on-hydrochlorid

1,1 g 1-(4-Amidinophenyl)-3-[trans-4-[2-(methoxycarbonyl)ethyl]cyclohexyl]-3H-imidazol-2-on-hydrochlorid in 55 ml Methanol werden mit 1 g Palladium auf Aktivkohle bei Raumtemperatur unter einem Wasserstoffdruck von 51 psi 3 Stunden hydriert. Es wird vom Katalysator abgesaugt, das Filtrat eingeengt und der Rückstand mit Methylenchlorid gerührt. Das Produkt wird abgesaugt und getrocknet.

Ausbeute: 870 mg (79 % der Theorie),

$R_f$-Wert: 0,52 (Reversed Phase Kieselgel; Methanol/10%ige wäßrige Kochsalzlösung = 3:1)

| | | | | | | |
|---|---|---|---|---|---|---|
| Ber.: | C | 58,74 | H | 7,15 | N | 13,70 |
| Gef.: | | 58,54 | | 7,31 | | 13,53 |

Analog Beispiel 7 werden folgende Verbindungen erhalten:

(1) 1-(4-Amidinophenyl)-3-[cis-4-[2-(methoxycarbonyl)ethyl]cyclohexyl]-imidazolidin-2-on-hydrochlorid x 1 Wasser

$R_f$-Wert: 0,52 (Reversed Phase Kieselgel; Methanol/10%ige wäßrige Kochsalzlösung = 3:1)

| | | | | | | |
|---|---|---|---|---|---|---|
| Ber.: | C | 56,27 | H | 7,32 | N | 13,12 |
| Gef.: | | 56,46 | | 7,16 | | 13,45 |

(2) 1-[2-(1-tert.Butyloxycarbonyl-4-piperidinyl)ethyl]-3-[trans-4-[2-(methoxycarbonyl)ethyl]cyclohexyl]-imidazolidin-2-on

Schmelzpunkt: 92-94 °C

$R_f$-Wert: 0,58 (Kieselgel; Essigester)

(3) 1-[2-(1-tert.Butyloxycarbonyl-4-piperidinyl)ethyl]-3-[4-[2-(methoxycarbonyl)ethyl]phenyl]-cis-4,5-dimethyl-imidazolidin-2-on

Durchführung in Essigester bei 80 °C.

$R_f$-Wert: 0,51 (Kieselgel; Cyclohexan/Essigester = 1:1)

(4) 1-[2-(Methoxycarbonyl)-6-naphthyl]-3-[2-(4-piperidinyl)ethyl]-imidazolidin-2-on-hydrochlorid

Durchführung in Methanol/wäßrige Salzsäure

$R_f$-Wert: 0,25 (Reversed Phase Kieselgel; Methanol/5%ige wäßrige Kochsalzlösung = 6:4)

(5) 1-[2-(Methoxycarbonyl)-1,2,3,4-tetrahydro-6-naphthyl]-3-[2-(4-piperidinyl)ethyl]-imidazolidin-2-on-hydrochlorid

Durchführung in Methanol/wäßrige Salzsäure

$R_f$-Wert: 0,50 (Kieselgel; Methylenchlorid/Methanol/konz. wäßriges Ammoniak = 5:1:0,2)

(6) 1-[trans-4-(Methoxycarbonyl)cyclohexyl]-3-[4-(1-trifluoracetyl-4-piperidinyl)phenyl]-imidazolidin-2-on

Schmelzpunkt: 183-185 °C

$R_f$-Wert: 0,59 (Kieselgel; Cyclohexan/Essigester = 2:3)
(7) 1-[trans-4-(Methoxycarbonyl)cyclohexyl]-3-[4-(4-chinuclidinyl)phenyl]-imidazolidin-2-on-hydrochlorid
Durchführung in Methanol/wäßrige Salzsäure
$R_f$-Wert: 0,38 (Reversed Phase Kieselgel; Methanol/5%ige wäßrige Kochsalzlösung = 6:4)
(8) 1-[trans-4-(Methoxycarbonyl)cyclohexyl]-3-[4-(4-methyl-1-trifluoracetyl-4-piperidinyl)phenyl]-imidazolidin-2-on
Durchführung in Essigester
Schmelzpunkt: 158-161 °C
$R_f$-Wert: 0,50 (Kieselgel; Cyclohexan/Essigester = 1:1)
(9) 1-[2-(1-tert.Butyloxycarbonyl-4-piperidinyl)ethyl]-3-[trans-4-[[(methoxycarbonyl)methyl]oxy]-cyclohexyl]-imidazolidin-2-on
$R_f$-Wert: 0,42 (Kieselgel; Essigester)
(10) 1-[2-(4-Chinuclidinyl)ethyl]-3-[4-[2-(methoxycarbonyl)ethyl]phenyl]-imidazolidin-2-on-hydrochlorid
Ausgangsmaterial: Verbindung des Beispiels 36(6)
Schmelzpunkt: 194-196 °C

Beispiel 8

1-(4-Cyanophenyl)-3-[trans-4-[2-(methoxycarbonyl)ethyl]cyclohexyl]-3H-imidazol-2-on

6,8 g 4-[(2,2-Diethoxyethyl)amino]benzonitril (Schmelzpunkt: 75-78 °C, hergestellt aus 4-Aminobenzonitril durch Umsetzung mit Bromacetaldehyd-diethylacetal in Gegenwart von N-Ethyldiisopropylamin) und 6,81 g [trans-4-[2-(Methoxycarbonyl)ethyl]cyclohexyl]isocyanat (hergestellt aus dem entsprechenden Amin-hydrochlorid durch Umsetzung mit Phosgen) werden 8 Stunden bei 65 °C und dann 2 Stunden bei 100 °C gerührt. Nach dem Abkühlen wird das Reaktionsgemisch durch Chromatographie über eine Kieselgelsäule mit Cyclohexan/Essigester (1:1) gereinigt. Das Produkt wird über Nacht in einem Gemisch aus 15 ml Dioxan und 10 ml 2N Salzsäure gerührt. Der Niederschlag wird abgesaugt und getrocknet.
Ausbeute: 1,9 g (19 % der Theorie),
Schmelzpunkt: 136-142 °C
Analog Beispiel 8 werden folgende Verbindungen erhalten:
(1) 1-(4-Cyanophenyl)-3-[cis-4-[2-(methoxycarbonyl)ethyl]cyclohexyl]-3H-imidazol-2-on
$R_f$-Wert: 0,40 (Kieselgel; Cyclohexan/Essigester = 1:1)
(2) 1-[2-(1-tert.Butyloxycarbonyl-4-piperidinyl)ethyl]-3-[trans-4-[2-(methoxycarbonyl)ethyl]cyclohexyl]-3H-imidazol-2-on
Das als Amin eingesetzte N-[2-(1-tert.Butyloxycarbonyl-4-piperidinyl)ethyl]-N-(2,2-diethoxyethyl)-amin wird durch Umsetzung von 1-tert.Butyloxycarbonyl-4-[2-(methansulfonyloxy)ethyl]-piperidin mit Aminoacetaldehyd-diethylacetal erhalten.
Durchführung der Harnstoffbildung bei Raumtemperatur, Ringschluß durch trockenes Erhitzen auf 145 °C.
$R_f$-Wert: 0,34 (Kieselgel; Essigester)
(3) 1-[trans-4-(Methoxycarbonyl)cyclohexyl]-3-[4-(1-trifluoracetyl-4-piperidinyl)phenyl]-3H-imidazol-2-on
Das als Amin eingesetzte N-(2,2-Diethoxyethyl)-4-(1-trifluoracetyl-4-piperidinyl)-anilin [$R_f$-Wert: 0,90 (Kieselgel; Cyclohexan/Essigester = 1:3] wird durch Umsetzung von 4-(1-Trifluoracetyl-4-piperidinyl)-anilin mit Bromacetaldehyddiethylacetal in Gegenwart von N-Ethyl-diisopropylamin erhalten.
Das als Isocyanat eingesetzte trans-4-(Methoxycarbonyl)cyclohexyl-isocyanat wird aus dem entsprechenden Amin-hydrochlorid durch Umsetzung mit Phosgen erhalten.
Durchführung der Harnstoffbildung bei 100 °C, Ringschluß durch trockenes Erhitzen auf 125 °C.
Schmelzpunkt: 178-179 °C
$R_f$-Wert: 0,36 (Kieselgel; Cyclohexan/Essigester = 2:3)
(4) 1-[2-(1-tert.Butyloxycarbonyl-4-piperidinyl)ethyl]-3-[trans-4-[[(methoxycarbonyl)methyl]oxy]-cyclohexyl]-3H-imidazol-2-on
Das als Isocyanat eingesetzte trans-4-[[(Methoxycarbonyl)methyl]oxy]cyclohexyl-isocyanat wird aus dem entsprechenden Amin-hydrochlorid durch Umsetzung mit Phosgen erhalten.
Durchführung der Harnstoffbildung bei Raumtemperatur, Ringschluß durch trockenes Erhitzen auf 160 °C.

Beispiel 9

1-(1-Amino-1,2,3,4-tetrahydronaphthalin-6-yl)-3-[4-[2-(methoxycarbonyl)ethyl]phenyl]-imidazolidin-2-on-hydrochlorid

2,2 g 1-(1-Hydroxyimino-1,2,3,4-tetrahydronaphthalin-6-yl)-3-[4-[2-(methoxycarbonyl)ethyl]phenyl]-imidazolidin-2-on in einem Gemisch aus 100 ml Methanol und 5 ml methanolischer Salzsäure werden 5 Stunden bei 50 °C und einem Wasserstoffdruck von 3,4 bar in Gegenwart von 0,3 g Palladium auf Aktivkohle (10 % Palladium) hydriert. Der Katalysator wird abgesaugt, das Filtrat eingeengt und der Rückstand mit wenig Methanol gerührt. Das Produkt wird abgesaugt, mit wenig Methanol und Diethylether gewaschen und getrocknet.
Ausbeute: 2,1 g (90 % der Theorie),
$R_f$-Wert: 0,73 (Kieselgel; Toluol/Dioxan/Methanol/konz.wäßriges Ammoniak = 20:50:20:5)

Beispiel 10

4-[4-[2-(Methoxycarbonyl)ethyl]phenyl]-5-methyl-2-[2-(4-piperidinyl)ethyl]-4H-1,2,4-triazol-3-on-toluolsulfonat x Wasser

Zu 1,3 g 2-[2-(1-tert.Butyloxycarbonyl-4-piperidinyl)ethyl]-4-[2-(methoxycarbonyl)ethyl]phenyl]-5-methyl-4H-1,2,4-triazol-3-on in 13 ml Methylenchlorid werden 4 ml Trifluoressigsäure gegeben und 2 Stunden bei Raumtemperatur gerührt. Das Reaktionsgemisch wird eingeengt und der Rückstand in Methylenchlorid aufgenommen. Es wird mit Eiswasser versetzt, mit Natronlauge leicht alkalisch gestellt und die organische Phase abgetrennt. Die organische Phase wird eingeengt, der Rückstand in Aceton aufgenommen und mit einer Lösung von 500 mg p-Toluolsulfonsäure-hydrat in Aceton versetzt. Dann wird Diethylether zugegeben, die Mischung wird unter Eiskühlung gerührt und der Niederschlag abgesaugt und mit Aceton gewaschen.
Ausbeute: 760 mg (49 % der Theorie),
$R_f$-Wert: 0,19 (Kieselgel; Toluol/Dioxan/Methanol/konz.waßriges Ammoniak = 20:50:20:5)

| | | | | | | | | |
|---|---|---|---|---|---|---|---|---|
| Ber.:<br>Gef.: | C | 57,63<br>57,84 | H | 6,81<br>6,77 | N | 9,96<br>9,78 | S | 5,70<br>6,04 |

Analog Beispiel 10 werden folgende Verbindungen erhalten:
(1) 1-[4-[2-(Methoxycarbonyl)ethyl]phenyl]-3-[2-(4-piperidinyl)ethyl]-imidazolidin-2-on-hydrochlorid
Isolierung als Hydrochlorid
$R_f$-Wert: 0,38 (Reversed Phase Kieselgel; Methanol/5%ige wäßrige Kochsalzlösung = 6:4)
(2) 1-[4-[2-(Methoxycarbonyl)-1-pentyl]phenyl]-3-[2-(4-piperidinyl)ethyl]-imidazolidin-2-on-hydrochlorid
(3) 4-[4-[2-(Methoxycarbonyl)-1-pentyl]phenyl]-5-methyl-2-[2-(4-piperidinyl)ethyl]-4H-1,2,4-triazol-3-on-hydrochlorid x 0,5 Wasser
Schmelzpunkt: 132-140 °C

| | | | | | | | | |
|---|---|---|---|---|---|---|---|---|
| Ber.:<br>Gef.: | C | 60,05<br>60,04 | H | 7,89<br>7,82 | N | 12,18<br>12,16 | Cl | 7,71<br>8,25 |

(4) 4-[4-[2-(Methoxycarbonyl)-3-methyl-1-butyl]phenyl]-5-methyl-2-[2-(4-piperidinyl)ethyl]-4H-1,2,4-triazol-3-on-hydrochlorid
(5) 4-[4-[2-(Methoxycarbonyl)-1-butyl]phenyl]-5-methyl-2-[2-(4-piperidinyl)ethyl]-4H-1,2,4-triazol-3-on-hydrochlorid
(6) 4-[4-[2-(Methoxycarbonyl)-1-propyl]phenyl]-5-methyl-2-[2-(4-piperidinyl)ethyl]-4H-1,2,4-triazol-3-on-hydrochlorid
(7) 1-[4-[2-(Methoxycarbonyl)ethyl]phenyl]-3-[2-(4-piperidinyl)ethyl]-3,4,5,6-tetrahydro-1H-pyrimidin-2-on-hydrochlorid
(8) 4-[4-[2-(Methoxycarbonyl)ethyl]phenyl]-5-methyl-2-[(1-piperazinyl)carbonylmethyl]-4H-1,2,4-triazol-3-on-hydrochlorid
(9) 4-[4-[2-(Methoxycarbonyl)ethyl]phenyl]-5-methyl-2-[2-(1-aza-4-cycloheptyl)ethyl]-4H-1,2,4-triazol-3-on-hydrochlorid

(10) 4-[4-[2-(Methoxycarbonyl)ethyl]phenyl]-5-methyl-2-(2-(1,4-diaza-1-cycloheptyl)ethyl]-4H-1,2,4-triazol-3-on-dihydrochlorid
(11) 4-[4-[2-(n-Butylsulfonylamino)-2-(methoxycarbonyl)ethyl]phenyl]-5-methyl-2-[2-(4-piperidinyl)ethyl]-4H-1,2,4-triazol-3-on-hydrochlorid
(12) 4-[4-[2-(Methansulfonylamino)-2-(methoxycarbonyl)ethyl]phenyl]-5-methyl-2-[2-(4-piperidinyl)ethyl]-4H-1,2,4-triazol-3-on-hydrochlorid
(13) 4-[4-[2-(Acetylamino)-2-(methoxycarbonyl)-ethyl]phenyl]-5-methyl-2-[2-(4-piperidinyl)ethyl]-4H-1,2,4-triazol-3-on-hydrochlorid
(14) 4-[4-[2-(Methoxycarbonyl)-1-octyl]phenyl]-5-methyl-2-[2-(4-piperidinyl)ethyl]-4H-1,2,4-triazol-3-on-hydrochlorid
(15) 4-[4-[2-(Methoxycarbonyl)ethyl]phenyl]-2-[2-(4-piperidinyl)ethyl]-4H-1,2,4-triazol-3-on-hydrochlorid x 0,5 $H_2O$
Durchführung mit methanolischer Salzsäure.
Schmelzpunkt: 188-191 °C
$R_f$-Wert: 0,52 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 56,50<br>56,79 | H | 6,99<br>6,93 | N | 13,87<br>13,76 | Cl | 8,78<br>8,83 |

(16) 2-[4-[2-(Methoxycarbonyl)ethyl]phenyl]-5-[2-(4-piperidinyl)ethyl]-3,4-dihydro-2H,5H-1,2,5-thiadiazol-1,1-dioxidhydrochlorid
Durchführung mit methanolischer Salzsäure.
$R_f$-Wert: 0,63 (Reversed Phase Kieselgel; Methanol/5%ige wäßrige Kochsalzlösung = 6:4)
(17) 1-[4-[2-(Methoxycarbonyl)ethyl]phenyl]-3-[(1-piperazinyl)carbonylmethyl]-imidazolidin-2-on-hydrochlorid
Durchführung mit methanolischer Salzsäure (Die erhaltene Verbindung wurde direkt in die Verbindung des Beispiels 1(31) übergeführt).
(18) 1-[4-[2-(n-Butylsulfonylamino)-2-(methoxycarbonyl)ethyl]phenyl]-3-[2-(4-piperidinyl)ethyl]-imidazolidin-2-on-hydrochlorid
Durchführung mit methanolischer Salzsaure.
Schmelzpunkt: 208 °C (Zers.)
$R_f$-Wert: 0,43 (Reversed Phase Kieselgel; Methanol/5%ige wäßrige Kochsalzlösung = 6:4)
(19) 4-[4-[2-(Methoxycarbonyl)ethyl]phenyl]-2-[2-(1-piperazinyl)ethyl]-4H-1,2,4-triazol-3-on x 2,1 HCl x 0,3 $H_2O$
Durchführung mit methanolischer Salzsäure.
Schmelzpunkt: 210-213 °C
$R_f$-Wert: 0,61 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 48,98<br>48,96 | H | 6,32<br>6,36 | N | 15,87<br>15,88 | Cl | 16,87<br>16,99 |

Massenspektrum: $M^+$ = 359
(20) 2-Cyanimino-1-[4-[2-(methoxycarbonyl)ethyl]phenyl]-3-[2-(4-piperidinyl)ethyl]-imidazolidin x HCl x 0,5 $H_2O$
Durchführung mit Trimethyljodsilan in Methylenchlorid und Isolierung als Hydrochlorid.
Schmelzpunkt: 162-165 °C
$R_f$-Wert: 0,34 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 58,80<br>58,70 | H | 7,28<br>7,22 | N | 16,33<br>16,18 | Cl | 8,27<br>8,51 |

(21) 1-[2-(1-Aza-4-cycloheptyl)ethyl]-3-[4-[2-(methoxycarbonyl)ethyl]phenyl]-imidazolidin-2-on-hydrochlorid x 0,15 Wasser
Durchführung mit methanolischer Salzsäure.
Schmelzpunkt: 134-136 °C

$R_f$-Wert: 0,38 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 61,13<br>61,18 | H | 7,89<br>7,89 | N | 10,18<br>10,29 | Cl | 8,59<br>8,38 |

(22) 1-[trans-4-[2-(Methoxycarbonyl)ethyl]cyclohexyl]-3-[2-(4-piperidinyl)ethyl]-imidazolidin-2-on-hydrochlorid
Durchführung mit methanolischer Salzsäure in Gegenwart von Orthokohlensäure-tetramethylester.
Schmelzpunkt: 190-195 °C (Zers.)
$R_f$-Wert: 0,36 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 59,76<br>59,47 | H | 9,03<br>9,11 | N | 10,45<br>10,58 | Cl | 8,82<br>8,84 |

(23) 3-[4-[2-(Methoxycarbonyl)ethyl]phenyl]-1-[2-(4-piperidinyl)ethyl]-hydantoin-hydrochlorid
Durchführung mit methanolischer Salzsäure.
Schmelzpunkt: 203-205 °C (Zers.)
$R_f$-Wert: 0,57 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 58,60<br>58,40 | H | 6,88<br>6,95 | N | 10,25<br>10,14 | Cl | 8,65<br>8,55 |

(24) 1-[4-[2-(Methoxycarbonyl)ethyl]phenyl]-3-[2-(4-piperidinyl)ethyl]-4,5-dimethyl-3H-imidazol-2-on-hyrochlorid
Durchführung mit methanolischer Salzsäure.
$R_f$-Wert: 0,33 (Reversed Phase Kieselgel; Methanol/5%ige wäßrige Kochsalzlösung = 6:4)
(25) 1-[4-[2-(Methoxycarbonyl)ethyl]phenyl]-3-[2-(4-piperidinyl)ethyl]-cis-4,5-dimethyl-imidazolidin-2-on-hyrochlorid
Durchführung mit methanolischer Salzsäure.
$R_f$-Wert: 0,30 (Reversed Phase Kieselgel; Methanol/5%ige wäßrige Kochsalzlösung = 6:4)
(26) 1-[4-[2-(Methoxycarbonyl)ethyl]phenyl]-3-[2-(4-piperidinyl)ethyl]-hydantoin-hyrochlorid
Durchführung mit methanolischer Salzsäure.
Schmelzpunkt: 186-188 °C
$R_f$-Wert: 0,41 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 58,60<br>58,27 | H | 6,88<br>7,10 | N | 10,25<br>10,22 | Cl | 8,65<br>8,70 |

(27) 1-[4-[2-(Methoxycarbonyl)ethyl]phenyl]-3-[2-(4-piperidinyl)ethyl]-3H-benzimidazol-2-on-hyrochlorid
Durchführung mit methanolischer Salzsäure.
$R_f$-Wert: 0,33 (Reversed Phase Kieselgel; Methanol/5%ige wäßrige Kochsalzlösung = 6:4)
(28) 1-[4-[2-(O-Ethyl-phosphono)ethyl]phenyl]-3-[2-(4-piperidinyl)ethyl]-imidazolidin-2-on x 0,9 HCl x 1,1 $H_2O$
Durchführung mit ethanolischer Salzsäure.
$R_f$-Wert: 0,42 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 51,98<br>51,74 | H | 7,65<br>7,43 | N | 9,09<br>9,08 | Cl | 6,90<br>6,51 |

Massenspektrum: $(M+H)^+$ = 410
(29) 1-[[4-(Ethoxycarbonyl)-1-piperidinyl]carbonylmethyl]-3-[2-(4-piperidinyl)ethyl]-imidazolidin-2-on-trifluoracetat
$R_f$-Wert: 0,57 (Reversed Phase Kieselgel; Methanol/5%ige wäßrige Kochsalzlösung = 6:4)

(30) 1-[4-[2-(Methoxycarbonyl)ethenyl]-2-fluor-phenyl]-3-[2-(4-piperidinyl)ethyl]-imidazolidin-2-on-hydrochlorid
Durchführung mit methanolischer Salzsäure.
Schmelzpunkt: 175-180 °C
$R_f$-Wert: 0,30 (Reversed Phase Kieselgel; Methanol/5%ige wäßrige Kochsalzlösung = 6:4)

Beispiel 11

1-[4-(Aminomethyl)cyclohexyl]-3-[4-[2-(methoxycarbonyl)ethyl]phenyl]-imidazolidin-2-on-hydrochlorid

500 mg 1-(4-Cyanocyclohexyl]-3-(4-[2-(methoxycarbonyl)ethyl]phenyl]-3H-imidazol-2-on werden in einem Gemisch aus 20 ml Methanol und 1 ml methanolischer Salzsäure in Gegenwart von 500 mg Palladium auf Aktivkohle (10 % Palladium) bei Raumtemperatur und einem Wasserstoffdruck von 5 bar hydriert. Der Katalysator wird abfiltriert und das Filtrat eingeengt. Der Rückstand wird mit Aceton verrieben, abgesaugt und mit Aceton und Petrolether gewaschen.
Ausbeute: 110 mg (20 % der Theorie),
$R_f$-Wert: 0,46 (Reversed Phase Kieselgel; Methanol/5%ige wäßrige Kochsalzlösung = 6:4)
Analog Beispiel 11 werden folgende Verbindungen erhalten:
(1) 2-[4-(Aminomethyl)phenyl]-4-[4-[2-(ethoxycarbonyl)-1-propyl]phenyl]-5-methyl-4H-1,2,4-triazol-3-on-hydrochlorid
Durchführung in Ethanol
Schmelzpunkt: 194-197 °C
$R_f$-Wert: 0,54 (Reversed Phase Kieselgel; Methanol/10%ige wäßrige Kochsalzlösung = 3:1)
(2) 1-(2-Aminomethyl-1,2,3,4-tetrahydro-6-naphthyl)-3-[trans-4-(methoxycarbonyl)cyclohexyl]-imidazolidin-2-on-hydrochlorid
Schmelzpunkt: 323-327 °C (Zers.)
$R_f$-Wert: 0,35 (Reversed Phase Kieselgel; Methanol/5%ige wäßrige Kochsalzlösung = 6:4)
(3) 1-[4-(Aminomethyl-bicyclo[2.2.2]octan-1-yl]-3-[4-[2-(methoxycarbonyl)ethyl]phenyl]-imidazolidin-2-on-hydrochlorid
Schmelzpunkt: 303-306 °C (Zers.)
$R_f$-Wert: 0,27 (Reversed Phase Kieselgel; Methanol/5%ige wäßrige Kochsalzlösung = 6:4)
(4) 1-(5-Aminopentyl)-3-[4-[2-(methoxycarbonyl)ethyl]phenyl]-imidazolidin-2-on-hydrochlorid
Schmelzpunkt: 234-236 °C

| | | | | | | | | |
|---|---|---|---|---|---|---|---|---|
| Ber.:<br>Gef.: | C | 58,45<br>58,17 | H | 7,63<br>7,58 | N | 11,36<br>11,50 | Cl | 9,58<br>9,66 |

(5) 1-[4-[(2-Aminoethyl)oxy]phenyl]-3-[trans-4-(methoxycarbonyl)cyclohexyl]-imidazolidin-2-on-hydrochlorid
Durchführung in Methanol/0,1N HCl
$R_f$-Wert: 0,47 (Reversed Phase Kieselgel; Methanol/5%ige wäßrige Kochsalzlösung = 6:4)

Beispiel 12

1-(4-Cyanocyclohexyl]-3-[4-[2-(methoxycarbonyl)ethyl]phenyl]-3H-imidazol-2-on

Zu 2,7 g 3-[4-[2-(Methoxycarbonyl)ethyl]phenyl]-1-(4-oxocyclohexyl)-3H-imidazol-2-on und 2,0 g p-Toluolsulfonyl-methylisocyanid in 60 ml Ethylenglykoldimethylether werden bei -12 bis -16 °C 1,8 g Kalium-tert.butylat in 50 ml tert.Butanol zugetropft. Nach 45 Minuten wird das Kühlbad entfernt und weitere 2 Stunden gerührt. Das Reaktionsgemisch wird mit Salzsäure neutralisiert und dann eingeengt. Der Rückstand wird in Chloroform gelöst, es wird dreimal mit Wasser gewaschen, getrocknet und die organische Phase eingeengt. Der Rückstand wird durch Chromatographie über eine Kieselgelsäule mit Cyclohexan/Essigester (1:1) gereinigt.
Ausbeute: 500 mg (18 % der Theorie),
Schmelzpunkt: 177-180 °C
$R_f$-Wert: 0,24 (Kieselgel; Cyclohexan/Essigester = 3:7)

Beispiel 13

1-(4-Amidinophenyl)-3-[4-[2-(isopropyloxycarbonyl)ethyl]phenyl]-imidazolidin-2-on x 1,1 Chlorwasserstoff

In ein Gemisch aus 400 mg 1-(4-Amidinophenyl)-3-[4-[2-(methoxycarbonyl)ethyl]phenyl]-imidazolidin-2-on-hydrochlorid und 300 ml Isopropanol wird Salzsäuregas eingeleitet und dann 1,5 Stunden am Rückfluß erhitzt. Nach dem Abkühlen wird eingeengt und der Rückstand mit Aceton verrieben und abgesaugt. Das Produkt wird mit Aceton und Diethylether gewaschen und getrocknet.
Ausbeute: 400 mg (92 % der Theorie),
$R_f$-Wert: 0,32 (Reversed Phase Kieselgel; Methanol/5%ige wäßrige Kochsalzlösung = 3:2)

| | | | | | | | | |
|---|---|---|---|---|---|---|---|---|
| Ber.:<br>Gef.: | C | 60,80<br>60,73 | H | 6,29<br>6,29 | N | 12,89<br>12,86 | Cl | 8,97<br>8,86 |

Analog Beispiel 13 werden folgende Verbindungen erhalten:
(1) 1-(4-Amidinophenyl)-3-[4-[2-(cyclohexyloxycarbonyl)ethyl]phenyl]-imidazolidin-2-on-hydrochlorid
$R_f$-Wert: 0,07 (Reversed Phase Kieselgel; Methanol/5%ige wäßrige Kochsalzlösung = 6:4)
(2) 1-(4-Amidinophenyl)-3-[trans-4-[2-(cyclohexyloxycarbonyl)ethyl]cyclohexyl]-imidazolidin-2,4-dion-hydrochlorid x Cyclohexanol
$R_f$-Wert: 0,11 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 62,98<br>63,05 | H | 8,01<br>7,97 | N | 9,48<br>9,79 | Cl | 6,00<br>6,40 |

(3) 2-(4-Amidinophenyl)-4-[4-[2-(cyclohexyloxycarbonyl)-1-propyl]phenyl]-5-methyl-4H-1,2,4-triazol-3-on-hydrochlorid
$R_f$-Wert: 0,12 (Reversed Phase Kieselgel; Methanol/5%ige wäßrige Kochsalzlösung = 6:4)
(4) 1-(4-Amidinophenyl)-3-[4-[2-(n-butylsulfonylamino)-2-(cyclohexyloxycarbonyl)-ethyl]phenyl]-imidazolidin-2-on-hydrochlorid

Beispiel 14

1-(4-Cyanophenyl)-3-[trans-4-[2-(methoxycarbonyl)ethyl]cyclohexyl]-imidazolidin-2,4-dion

5,5 g N-(4-Cyanophenyl)-N-(methoxycarbonylmethyl)-N'-[trans-4-[2-(methoxycarbonyl)ethyl]cyclohexyl]-harnstoff werden im Vakuum 4,5 Stunden auf 180 °C erhitzt. Nach dem Abkühlen werden 10 ml Essigester zugegeben, es wird kurz zum Sieden erhitzt und dann im Eisbad abgekühlt. Der Feststoff wird abgesaugt und mit etwas Essigester gewaschen.
Ausbeute: 3,8 g (75 % der Theorie),
Schmelzpunkt: 207-208 °C,
$R_f$-Wert: 0,75 (Kieselgel; Methylenchlorid/Essigester = 3:1)

Beispiel 15

1-[4-(2-Carboxyethyl)phenyl]-3-[2-(1-methyl-4-piperidinyl)ethyl]-imidazolidin-2-on x 1,1 HCl

900 mg 1-[4-[2-(Methoxycarbonyl)ethyl]phenyl]-3-[2-(4-piperidinyl)ethyl]-imidazolidin-2-on-hydrochlorid, 600 mg Ameisensäure und 250 mg wäßrige Formaldehydlösung (37 %) werden 4 Stunden bei 70-80 °C gerührt. Es werden 1,1 ml 2N Natronlauge und 0,2 ml wäßrige Formaldehydlösung (37 %) zugegeben und weitere 3 Stunden bei 70-80 °C gerührt. Es wird eingeengt, der Rückstand wird mit 3 ml Salzsäure versetzt und 1 Stunde bei 70 °C gerührt. Es wird abgekühlt, Aceton zugegeben und vom Niederschlag abfiltriert. Das Filtrat wird eingeengt und mit Aceton verrührt. Der Feststoff wird abgesaugt und getrocknet. Der Rückstand wird mit 70 ml Methylenchlorid ausgekocht, es wird abfiltriert und das Filtrat eingeengt. Dieser Vorgang wird mit 70 ml Chloroform wiederholt. Die vereinigten Eindampfrückstände werden mit Aceton verrührt, der Feststoff wird abgesaugt, gewaschen und getrocknet.
Ausbeute: 400 mg (46 % der Theorie),

Schmelzpunkt: 220 ° C (Zers.)
$R_f$-Wert: 0,45 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 60,12<br>60,04 | H | 7,59<br>7,64 | N | 10,52<br>10,30 | Cl | 9,76<br>9,63 |

Analog Beispiel 15 werden folgende Verbindungen erhalten:

(1) 4-[4-(2-Carboxyethyl)phenyl]-5-methyl-2-[2-(1-methyl-4-piperidinyl)ethyl]-4H-1,2,4-triazol-3-on-hydrochlorid

(2) 1-(trans-4-Carboxycyclohexyl)-3-[4-(1-methyl-4-piperidinyl)phenyl]-imidazolidin-2-on x 0,95 HCl x 0,5 $H_2O$
Die Reaktion wird mit der Carbonsäure durchgeführt.
$R_f$-Wert: 0,49 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 61,57<br>61,67 | H | 7,74<br>7,77 | N | 9,79<br>9,92 | Cl | 7,84<br>7,70 |

Beispiel 16

1-[4-[4-(Methoxycarbonyl)butyl]phenyl]-3-(4-piperidinyl)-imidazolidin-2-on-hydrochlorid

1,7 g 1-(1-Benzyl-4-piperidinyl)-3-[4-[4-(methoxycarbonyl)butyl]phenyl]-imidazolidin-2-on (enthält noch Triphenylphosphinoxid und Hydrazin-1,2-dicarbonsäure-diethylester) werden in 50 ml Methanol in Gegenwart von 0,5 g Palladium auf Aktivkohle (10 % Palladium) bei Raumtemperatur und einem Wasserstoffdruck von 50 psi hydriert. Nach 4,5 Stunden werden nochmal 0,5 g Palladiumkatalysator zugegeben und weitere 8 Stunden hydriert. Es wird vom Katalysator abfiltriert, eingedampft, mit Aceton und etherischer Salzsäure versetzt und erneut eingedampft. Nach Verrühren mit Aceton wird das Produkt abgesaugt und getrocknet.
Ausbeute: 150 mg,
$R_f$-Wert: 0,22 (Reversed Phase Kieselgel; Methanol/5%ige wäßrige Kochsalzlösung = 6:4)

Beispiel 17

1-(trans-4-Carboxycyclohexyl)-3-[4-(4-piperidinyl)phenyl]-imidazolidin-2-on-hydrochlorid

940 mg 1-[trans-4-(Ethoxycarbonyl)cyclohexyl]-3-[4-(1-trifluoracetyl-4-piperidinyl)phenyl]-imidazolidin-2-on (enthält noch Hydrazin-1,2-dicarbonsäure-diethylester), 10 ml Eisessig, 10 ml Wasser und 10 ml konzentrierte Salzsäure werden 5,5 Stunden auf 90 ° C erhitzt. Anschließend wird abgekühlt, 2 Tage bei Raumtemperatur stehen gelassen, eingedampft, 2 x mit Wasser versetzt und jeweils erneut eingedampft und dann mit 10 ml Wasser im Eisbad 1,5 Stunden lang gerührt. Der erhaltene Niederschlag wird abgesaugt, getrocknet und über Nacht mit 20 ml tert.Butyl-methylether gerührt. Das Produkt wird abgesaugt, mit tert.Butyl-methylether gewaschen und getrocknet.
Ausbeute: 47 mg,
Schmelzpunkt: 315-320 ° C (Zers.)
$R_f$-Wert: 0,55 (Reversed Phase Kieselgel; Methanol/5%ige wäßrige Kochsalzlösung = 6:4)
Massenspektrum: $M^+$ = 371
Analog Beispiel 17 werden folgende Verbindungen erhalten:

(1) 1-(4-Carboxybutyl)-3-[4-(4-piperidinyl)phenyl]-imidazolidin-2-on-hydrochlorid
Schmelzpunkt: 245-248 ° C (Zers.)
$R_f$-Wert: 0,49 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 59,75<br>59,38 | H | 7,39<br>7,41 | N | 11,00<br>10,84 | Cl | 9,28<br>9,02 |

(2) 1-(4-Carboxyphenyl)-3-[4-(4-piperidinyl)phenyl]-imidazolidin-2-on-hydrochlorid x $H_2O$
Schmelzpunkt: >250 °C

| | | | | | | | | |
|---|---|---|---|---|---|---|---|---|
| Ber.:<br>Gef.: | C | 60,09<br>60,31 | H | 6,24<br>6,25 | N | 10,01<br>10,27 | Cl | 8,44<br>8,52 |

Massenspektrum: $M^+$ = 365

Beispiel 18

1-[4-(2-Carboxyethyl)cyclohexyl]-3-[2-(4-piperidinyl)ethyl]-imidazolidin-2-on-hydrochlorid

400 mg 1-[4-(2-Carboxyethyl)phenyl]-3-[2-(4-piperidinyl)ethyl]-imidazolidin-2-on-hydrochlorid in 10 ml Eisessig werden mit 50 mg Platin-(IV)-oxid unter einem Wasserstoffdruck von 3,4 bar 13 Stunden bei 60 °C hydriert. Das Reaktionsgemisch wird filtriert, das Filtrat eingeengt, in einem Gemisch aus verdünnter Salzsäure und Tetrahydrofuran aufgenommen, filtriert und erneut eingeengt. Das Produkt wird mit Tetrahydrofuran und dann mit Aceton und Diethylether verrührt, abgesaugt, mit Aceton und Diethylether gewaschen und getrocknet.
Ausbeute: 244 mg (58 % der Theorie),
$R_f$-Wert: 0,49 (Reversed Phase Kieselgel; Methanol/5%ige wäßrige Kochsalzlösung = 6:4)
Analog Beispiel 18 werden folgende Verbindungen erhalten:
(1) 1-[4-(2-Carboxyethyl)cyclohexyl]-3-[2-(4-piperidinyl)ethyl]-3a,4,5,6,7,7a-hexahydro-benzimidazol-2-on x 1,6 HCl x 1,8 $H_2O$
$R_f$-Wert: 0,40 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 55,66<br>55,64 | H | 8,98<br>8,69 | N | 8,47<br>8,66 | Cl | 11,43<br>11,46 |

(2) 1-(trans-4-Carboxycyclohexyl)-3-[4-(4-piperidinyl)cyclohexyl]-imidazolidin-2-on-hydrochlorid
Durchführung in Wasser mit Rhodium/Platin-Katalysator
$R_f$-Wert: 0,55 (Reversed Phase Kieselgel; Methanol/5%ige wäßrige Kochsalzlösung = 6:4)
Massenspektrum: $M^+$ 377

Beispiel 19

1-[4-[2-(n-Butylsulfonylamino)-2-(methoxycarbonyl)-ethyl]phenyl]-3-(4-cyanophenyl)-imidazolidin-2-on

Zu 1,3 g N-[4-[2-(n-Butylsulfonylamino)-2-(methoxycarbonyl)-ethyl]phenyl]-N'-4-(cyanophenyl)-N-(2-hydroxyethyl)-harnstoff und 0,71 g Triphenylphosphin in 8 ml Acetonitril werden bei Raumtemperatur 495 mg Azodicarbonsäure-diethylester in 2 ml Acetonitril rasch zugetropft und dann 3,5 Stunden bei Raumtemperatur gerührt. Der Niederschlag wird abgesaugt, mit wenig Acetonitril und Methanol gewaschen und dann getrocknet.
Ausbeute: 1,05 g (84 % der Theorie),
Schmelzpunkt: 181-183 °C
Analog Beispiel 19 werden folgende Verbindungen erhalten:
(1) 1-(4-Cyanophenyl)-3-[4-[2-(methoxycarbonyl)ethyl]phenyl]-imidazolidin-2-on
Schmelzpunkt: 162-164 °C
(2) 1-[trans-4-(Ethoxycarbonyl)cyclohexyl]-3-[4-(1-trifluoracetyl-4-piperidinyl)phenyl]-imidazolidin-2-on
Enthält noch Hydrazin-1,2-dicarbonsäure-diethylester
$R_f$-Wert: 0,60 (Kieselgel; Cyclohexan/Essigester = 2:1)
(3) 1-[4-(Ethoxycarbonyl)phenyl]-3-[4-(1-trifluoracetyl-4-piperidinyl)phenyl]-imidazolidin-2-on
Schmelzpunkt: 235-239 °C (Zers.)
(4) 1-[4-(4-Cyano-1-trifluoracetyl-4-piperidinyl)phenyl]-3-[trans-4-(methoxycarbonyl)cyclohexyl]-imidazolidin-2-on
Durchführung in Dimethylformamid bei 50 °C.
$R_f$-Wert: 0,70 (Kieselgel; Essigester/Cyclohexan = 5:1)

(5) 1-[trans-4-(Methoxycarbonyl)cyclohexyl]-3-[4-(1-trifluoracetyl-4-piperidinyl)phenyl]-imidazolidin-2-on
Durchführung in Dimethylformamid bei 50 °C.
$R_f$-Wert: 0,77 (Kieselgel; Essigester/Cyclohexan = 5:1)
(6) 1-[2-(1-tert.Butyloxycarbonyl-4-piperidinyl)ethyl]-3-[4-[2-(n-butylsulfonylamino)-2-(methoxycarbonyl)-ethyl]phenyl]-imidazolidin-2-on
Schmelzpunkt: 124-126 °C
$R_f$-Wert: 0,42 (Kieselgel; Methylenchlorid/Essigester = 7:3)
(7) 1-[2-(1-tert.Butyloxycarbonyl-4-piperidinyl)ethyl]-2-cyanimino-3-[4-[2-(methoxycarbonyl)ethyl]phenyl]-imidazolidin
$R_f$-Wert: 0,27 (Kieselgel; Cyclohexan/Essigester = 3:7)
(8) 1-[2-(1-tert.Butyloxycarbonyl-1-aza-4-cycloheptyl)ethyl]-3-[4-[2-(methoxycarbonyl)ethyl]phenyl]-imidazolidin-2-on
$R_f$-Wert: 0,38 (Kieselgel; Cyclohexan/Essigester = 1:1)
(9) 1-(1-Benzyl-4-piperidinyl)-3-[4-[4-(methoxycarbonyl)butyl]phenyl]-imidazolidin-2-on
Enthält noch Triphenylphosphinoxid und Hydrazin-1,2-dicarbonsäure-diethylester.
$R_f$-Wert: 0,46 (Kieselgel; Methylenchlorid/Essigester/Methanol = 20:0,5:1,5)
(10) 1-[4-[2-(O,O'-Diethylphosphono)ethyl]phenyl]-3-[2-(1-tert.butyloXycarbonyl-4-piperidinyl)ethyl]-imidazolidin-2-on
$R_f$-Wert: 0,35 (Kieselgel; Methylenchlorid/Essigester/Methanol = 20:1:1)
(11) 1-[2-(1-tert.Butyloxycarbonyl-4-piperidinyl)ethyl]-3-[2-cyano-4-[2-(methoxycarbonyl)ethyl]phenyl]-imidazolidin-2-on
$R_f$-Wert: 0,63 (Kieselgel; Methylenchlorid/Essigester = 1:1)

## Beispiel 20

### 1-[4-[(1-Acetoxyethyl)oxycarbonylamidino]phenyl]-3-[4-[2-(isopropyloxycarbonyl)ethyl]phenyl]-imidazolidin-2-on

220 mg 1-(4-Amidinophenyl)-3-[4-[2-(isopropyloxycarbonyl)ethyl]phenyl]-imidazolidin-2-on-hydrochlorid, 190 mg Kohlensäure-(1-acetoxyethyl)-(4-nitrophenyl)-ester und 168 mg N-Ethyl-diisopropylamin werden in 20 ml Methylenchlorid 4 Stunden bei Raumtemperatur gerührt. Das Reaktionsgemisch wird mit etwas Methylenchlorid verdünnt und dann mit Eiswasser, zweimal mit 0,2N Natronlauge und wieder mit Eiswasser gewaschen. Die organische Phase wird getrocknet, eingeengt und der Rückstand mit tert.Butylmethylether gerührt. Das Produkt wird abgesaugt, mit etwas tert.Butylmethylether gewaschen und getrocknet.
Ausbeute: 250 mg (95 % der Theorie),
$R_f$-Wert: 0,47 (Kieselgel; Methylenchlorid/Methanol = 95:5)

| | | | | | | |
|---|---|---|---|---|---|---|
| Ber.: | C | 61,82 | H | 6,15 | N | 10,68 |
| Gef.: | | 61,69 | | 6,21 | | 10,63 |

Analog Beispiel 20 werden folgende Verbindungen erhalten:
(1) 1-[4-[(Acetoxymethyl)oxycarbonylamidino]phenyl]-3-[4-[2-(isopropyloxycarbonyl)ethyl]phenyl]-imidazolidin-2-on
(2) 2-[4-[(1-Acetoxyethyl)oxycarbonylamidino]phenyl]-4-[4-[2-(cyclohexyloxycarbonyl)ethyl]phenyl]-5-methyl-4H-1,2,4-triazol-3-on
(3) 2-[4-[(Acetoxymethyl)oxycarbonylamidino]phenyl]-4-[4-[2-(cyclohexyloxycarbonyl)ethyl]phenyl]-5-methyl-4H-1,2,4-triazol-3-on
(4) 4-[4-[2-(Methoxycarbonyl)ethyl]phenyl]-5-methyl-2-[4-[(pivaloyloxymethyl)oxycarbonylamidino]-phenyl]-4H-1,2,4-triazol-3-on
(5) 4-[4-[2-(Cyclohexyloxycarbonyl)ethyl]phenyl]-2-[4-(methoxycarbonylamidino)phenyl]-5-methyl-4H-1,2,4-triazol-3-on
(6) 4-[4-[2-(Cyclohexyloxycarbonyl)ethyl]phenyl]-2-[4-(ethoxycarbonylamidino)phenyl]-5-methyl-4H-1,2,4-triazol-3-on
(7) 2-[4-(Benzyloxycarbonylamidino)phenyl]-4-[4-[2-(methoxycarbonyl)ethyl)phenyl]-5-methyl-4H-1,2,4-triazol-3-on
(8) 2-[4-[(1-Acetoxyethyl)oxycarbonylamidino]phenyl]-4-[4-[2-(isopropyloxycarbonyl)ethyl]phenyl]-5-methyl-4H-1,2,4,triazol-3-on

Schmelzpunkt: ab 167 ° C (Zers.)
$R_f$-Wert: 0,39 (Kieselgel; Methylenchlorid/Methanol = 95:5)

| | | | | | | |
|---|---|---|---|---|---|---|
| Ber.:<br>Gef.: | C | 60,33<br>60,25 | H | 5,81<br>5,86 | N | 13,03<br>13,25 |

(9) 2-[4-[(Acetoxymethyl)oxycarbonylamidino]phenyl]-4-[4-[2-(isopropyloxycarbonyl)ethyl]phenyl]-5-methyl-4H-1,2,4,triazol-3-on
Schmelzpunkt: 152-153 ° C (Zers.)
$R_f$-Wert: 0,45 (Kieselgel; Methylenchlorid/Methanol = 95:5)

| | | | | | | |
|---|---|---|---|---|---|---|
| Ber.:<br>Gef.: | C | 59,65<br>59,25 | H | 5,58<br>5,65 | N | 13,38<br>13,51 |

(10) 1-[trans-4-(Methoxycarbonyl)cyclohexyl]-3-[4-(1-benzyloxycarbonyl-4-piperidinyl)phenyl]-imidazolidin-2-on
Schmelzpunkt: 145-148 ° C
$R_f$-Wert: 0,75 (Kieselgel; Methylenchlorid/Methanol = 9:1)
(11) 1-(trans-4-Carboxycyclohexyl)-3-[4-(1-benzyloxycarbonyl-4-cyano-4-piperidinyl)phenyl]-imidazolidin-2-on
Durchführung in Wasser mit Natronlauge als Base.
Schmelzpunkt: 238-240 ° C
$R_f$-Wert: 0,42 (Kieselgel; Methylenchlorid/Methanol = 9:1)

Beispiel 21

1-[trans-4-[[1-(Cyclohexyloxycarbonyloxy)ethyl]oxycarbonyl]-cyclohexyl]-3-[4-(1-benzyloxycarbonyl-4-piperidinyl)phenyl]-imidazolidin-2-on

2,0 g 1-(trans-4-Carboxycyclohexyl)-3-[4-(1-benzyloxycarbonyl-4-piperidinyl)phenyl]-imidazolidin-2-on, 4,5 g Kohlensäure-(1-chlorethyl)-cyclohexylester und 0,2 g Natriumjodid in 10 ml Dimethylsulfoxid werden eine Stunde bei Raumtemperatur gerührt. Es wird 1,1 g Kaliumcarbonat zugegeben und 16 Stunden bei 60 ° C gerührt. Nach dem Abkühlen wird das Reaktionsgemisch auf 100 ml Wasser gegeben, dem 1 ml Eisessig zugefügt worden ist. Es wird dreimal mit Essigester extrahiert, die vereinigten organischen Phasen werden mit Kochsalzlösung gewaschen, getrocknet und eingeengt. Der Rückstand wird durch Chromatographie über eine Kieselgelsäule mit Cyclohexan/Essigester (1:1) gereinigt.
Ausbeute: 0,8 g (30 % der Theorie),
$R_f$-Wert: 0,54 (Kieselgel; Cyclohexan/Essigester = 1:1)
Massenspektrum: $M^+$ = 675
Analog Beispiel 21 werden folgende Verbindungen erhalten:
(1) 2-[4-(Benzyloxycarbonylamidino)phenyl]-5-methyl-4-[4-[2-[(pivaloyloxymethyl)oxycarbonyl]ethyl]-phenyl]-4H-1,2,4-triazol-3-on
Alkylierungsmittel: Pivalinsäure-chlormethylester
(2) 2-[4-(Benzyloxycarbonylamidino)phenyl]-4-[4-[2-[[1-(cyclohexyloxycarbonyloxy)ethyl]oxycarbonyl]-ethyl]phenyl]-5-methyl-4H-1,2,4-triazol-3-on
(3) 1-[4-(1-Benzyloxycarbonyl-4-piperidinyl)phenyl]-3-[trans-4-[(pivaloyloxymethyl)oxycarbonyl]-cyclohexyl]-imidazolidin-2-on
Alkylierungsmittel: Pivalinsäure-chlormethylester
$R_f$-Wert: 0,50 (Kieselgel; Cyclohexan/Essigester = 1:1)
Massenspektrum: $M^+$ = 619
(4) 1-[4-(1-Benzyloxycarbonyl-4-piperidinyl)phenyl]-3-[trans-4-[[1-(ethyloxycarbonyloxy)ethyl]-oxycarbonyl]cyclohexyl]-imidazolidin-2-on
Alkylierungsmittel: Kohlensäure-(1-chlorethyl)-ethylester
$R_f$-Wert: 0,40 (Kieselgel; Cyclohexan/Essigester = 1:1)

Beispiel 22

1-[trans-4-(5-Indanyloxycarbonyl)cyclohexyl]-3-[4-(4-piperidinyl)phenyl]-imidazolidin-2-on x $H_2O$ x 0,85 HCl

1,0 g 1-[trans-4-(5-Indanyloxycarbonyl)cyclohexyl]-3-[4-(1-benzyloxycarbonyl-4-piperidinyl)phenyl]-imidazolidin-2-on werden in einem Gemisch aus 20 ml Tetrahydrofuran, 2 ml Wasser und 1,6 ml 1N Salzsäure in Gegenwart von 0,2 g Palladium auf Aktivkohle bei Raumtemperatur und einem Wasserstoffdruck von 50 psi 2,5 Stunden hydriert. Es werden noch 0,2 g Katalysator und 1 ml 1N Salzsäure zugegeben und weitere 2 Stunden hydriert. Anschließend wird vom Katalysator abgesaugt und mehrmals mit Tetrahydrofuran/Wasser/1N Salzsäure (20:1:1) nachgewaschen. Die vereinigten Filtrate werden mit 1N Natronlauge auf einen pH von 7 eingestellt und eingeengt. Die erhaltene Suspension wird eine Stunde im Eisbad gerührt. Der Feststoff wird abgesaugt, mit wenig Eiswasser gewaschen und getrocknet.
Ausbeute: 460 mg (54 % der Theorie),
$R_f$-Wert: 0,40 (Kieselgel; Methylenchlorid/Methanol/konz. wäßriges Ammoniak = 4:1:0,2)

| | | | | | | | | |
|---|---|---|---|---|---|---|---|---|
| Ber.:<br>Gef.: | C | 67,14<br>67,29 | H | 7,48<br>7,59 | N | 7,83<br>7,90 | Cl | 5,62<br>5,81 |

Massenspektrum: $M^+$ = 487
Analog Beispiel 22 werden folgende Verbindungen erhalten:

(1) 2-(4-Amidinophenyl)-5-methyl-4-[4-[2-[(pivaloyloxymethyl)oxycarbonyl]ethyl]phenyl]-4H-1,2,4-triazol-3-on-hydrochlorid

(2) 2-(4-Amidinophenyl)-4-[4-[2-[[1-(cyclohexyloxycarbonyloxy)ethyl]oxycarbonyl]ethyl]phenyl]-5-methyl-4H-1,2,4-triazol-3-on-hydrochlorid

(3) 1-[4-(4-Piperidinyl)phenyl]-3-[trans-4-[(pivaloyloxymethyl)oxycarbonyl]cyclohexyl]-imidazolidin-2-on-hydrochlorid
$R_f$-Wert: 0,52 (Kieselgel; Methylenchlorid/Methanol/konz. wäßriges Ammoniak = 4:1:0,2)
Massenspektrum: $M^+$ = 485

(4) 1-[trans-4-[[1-(Ethyloxycarbonyloxy)ethyl]oxycarbonyl]cyclohexyl]-3-[4-(4-piperidinyl)phenyl]-imidazolidin-2-on-hydrochlorid
$R_f$-Wert: 0,50 (Kieselgel; Methylenchlorid/Methanol/konz. wäßriges Ammoniak = 4:1:0,2)

(5) 1-[trans-4-[[1-(Cyclohexyloxycarbonyloxy)ethyl]oxycarbonyl]cyclohexyl]-3-[4-(4-piperidinyl)phenyl]-imidazolidin-2-on-hydrochlorid
$R_f$-Wert: 0,45 (Kieselgel; Methylenchlorid/Methanol/konz. wäßriges Ammoniak = 4:1:0,2)
Massenspektrum: $M^+$ = 541

(6) 1-[trans-4-(5-Indanyloxycarbonyl)cyclohexyl]-3-[4-(4-cyano-4-piperidinyl)phenyl]-imidazolidin-2-on
$R_f$-Wert: 0,30 (Kieselgel; Methylenchlorid/Methanol/konz. wäßriges Ammoniak = 4:1:0,2)
Massenspektrum: $M^+$ = 512

Beispiel 23

2-[2-(1-tert.Butyloxycarbonyl-4-piperidinyl)ethyl]-4-[4-[2-(methoxycarbonyl)ethyl]phenyl]-5-methyl-4H-1,2,4-triazol-3-on

Zu 1,1 g 4-[4-[2-(Methoxycarbonyl)ethyl]phenyl]-5-methyl-4H-1,2,4,-triazol-3-on in 6 ml Dimethylformamid werden 500 mg Kalium-tert.butylat gegeben und 30 Minuten bei Raumtemperatur gerührt. Danach wird im Eisbad gekühlt und mit 1,3 g 1-tert.-Butyloxycarbonyl-4-[2-(methansulfonyloxy)ethyl]-piperidin versetzt. Danach wird 2 Stunden bei Raumtemperatur gerührt. Das Reaktionsgemisch wird mit Eiswasser versetzt und mit Zitronensäurelösung neutral gestellt. Es wird abdekantiert und der Rückstand mit Diethylether digeriert. Das Produkt wird abgesaugt, mit Wasser gewaschen und getrocknet.
Ausbeute: 1,38 g (69 % der Theorie),
$R_f$-Wert: 0,61 (Kieselgel; Essigester)
Analog Beispiel 23 werden folgende Verbindungen erhalten:

(1) 1-[2-(1-tert.Butyloxycarbonyl-4-piperidinyl)ethyl]-3-[4-[2-(methoxycarbonyl)ethyl]phenyl]-imidazolidin-2-on
Durchführung in Dimethylformamid mit Natriumhydrid
$R_f$-Wert: 0,41 (Kieselgel; Cyclohexan/Essigester = 1:1)

(2) 1-[2-(1-tert.Butyloxycarbonyl-4-piperidinyl)ethyl]-3-[4-[2-(methoxycarbonyl)-1-pentyl]phenyl]-imidazolidin-2-on
(3) 2-[2-(1-tert.Butyloxycarbonyl-4-piperidinyl)ethyl]-4-[4-[2-(methoxycarbonyl)-1-pentyl]phenyl]-5-methyl-4H-1,2,4-triazol-3-on
$R_f$-Wert: 0,73 (Kieselgel; Essigester)

| | | | | | | |
|---|---|---|---|---|---|---|
| Ber.:<br>Gef.: | C | 65,34<br>65,54 | H | 8,22<br>8,32 | N | 10,89<br>10,75 |

(4) 2-[2-(1-tert.Butyloxycarbonyl-4-piperidinyl)ethyl]-4-[4-[2-(methoxycarbonyl)-3-methyl-1-butyl]phenyl]-5-methyl-4H-1,2,4-triazol-3-on
(5) 2-[2-(1-tert.Butyloxycarbonyl-4-piperidinyl)ethyl]-4-[4-[2-(methoxycarbonyl)-1-butyl]phenyl]-5-methyl-4H-1,2,4-triazol-3-on
(6) 2-[2-(1-tert.Butyloxycarbonyl-4-piperidinyl)ethyl]-4-[4-[2-(methoxycarbonyl)-1-propyl]phenyl]-5-methyl-4H-1,2,4-triazol-3-on
(7) 1-[4-(Methoxycarbonyl)butyl]-3-[4-(1-trifluoracetyl-4-piperidinyl)phenyl]-imidazolidin-2-on
Durchführung in Dimethylformamid mit Natriumhydrid.
Schmelzpunkt: 115-117 ° C
$R_f$-Wert: 0,73 (Kieselgel; Essigester/Cyclohexan = 4:1)
(8) 1-[4-(4-Cyano-1-trifluoracetyl-4-piperidinyl)phenyl]-3-[4-(methoxycarbonyl)butyl]-imidazolidin-2-on
Durchführung in Dimethylformamid mit Natriumhydrid.
$R_f$-Wert: 0,49 (Kieselgel; Essigester/Cyclohexan = 7:3)
(9) 2-[2-(1-tert.Butyloxycarbonyl-4-piperidinyl)ethyl]-4-[4-[2-(methoxycarbonyl)ethyl]phenyl]-4H-1,2,4-triazol-3-on
Schmelzpunkt: 139-141 ° C
$R_f$-Wert: 0,58 (Kieselgel; Essigester/Cyclohexan = 7:3)
(10) 2-[2-(1-tert.Butyloxycarbonyl-4-piperidinyl)ethyl]-5-[4-[2-(methoxycarbonyl)ethyl]phenyl]-3,4-dihydro-2H,5H-1,2,5-thiadiazol-1,1-dioxid
Durchführung in Dimethylformamid mit Natriumhydrid.
$R_f$-Wert: 0,41 (Kieselgel; Cyclohexan/Essigester = 1:1)
(11) 1-[(1-tert.Butyloxycarbonyl-4-piperazinyl)carbonylmethyl]-3-[4-[2-(methoxycarbonyl)ethyl]phenyl]-imidazolidin-2-on
Durchführung in Dimethylformamid mit Natriumhydrid.
(Das als Alkylierungsmittel eingesetzte (1-tert.Butyloxycarbonyl-4-piperazinyl)carbonylmethylchlorid wird aus 1-Benzylpiperazin durch Umsetzung mit Pyrokohlensäure-di-tert.butylester, anschließender Hydrierung mit Palladium auf Aktivkohle und nachfolgender Umsetzung mit Chloracetylchlorid erhalten).
Schmelzpunkt: 185-187 ° C
$R_f$-Wert: 0,44 (Kieselgel; Methylenchlorid/Essigester = 1:1)

| | | | | | | |
|---|---|---|---|---|---|---|
| Ber.:<br>Gef.: | C | 60,74<br>60,70 | H | 7,22<br>7,31 | N | 11,81<br>11,87 |

(12) 1-[2-(1-tert.Butyloxycarbonyl-4-piperidinyl)ethyl]-3-[4-[2-(methoxycarbonyl)ethyl]phenyl]-3,4,5,6-tetrahydro-1H-pyrimidin-2-on
Durchführung in Dimethylformamid mit Natriumhydrid.
$R_f$-Wert: 0,36 (Kieselgel; Methylenchlorid/Methanol = 200:3)
(13) 2-[2-(1-tert.Butyloxycarbonyl-4-piperazinyl)ethyl]-4-[4-[2-(methoxycarbonyl)ethyl]phenyl]-4H-1,2,4-triazol-3-on
Das eingesetzte Alkylierungsmittel (Schmelzpunkt: 238-240 ° C) wird aus 1-(2-Hydroxyethyl)piperazin durch Umsetzung mit Pyrokohlensäure-di-tert.butylester und nachfolgender Umsetzung mit Methansulfonsäurechlorid in Gegenwart von Triethylamin erhalten.
Schmelzpunkt: 128-130 ° C
$R_f$-Wert: 0,35 (Kieselgel; Essigester)
(14) 1-[4-[2-(Methoxycarbonyl)ethyl]phenyl]-3-[2-(2,2,6,6-tetramethyl-4-piperidinyl)ethyl]-imidazolidin-2-on
Durchführung in Dimethylformamid mit Natriumhydrid.
Schmelzpunkt: 127-129 ° C

$R_f$-Wert: 0,32 (Reversed Phase Kieselgel; Methanol/5%ige wäßrige Kochsalzlösung = 6:4)

| | | | | | | |
|---|---|---|---|---|---|---|
| Ber.:<br>Gef.: | C | 69,36<br>69,21 | H | 8,97<br>9,07 | N | 10,11<br>10,03 |

(15) 1-[4-[2-(Methoxycarbonyl)ethyl]phenyl]-3-[2-(4-chinuclidinyl)ethyl]-imidazolidin-2-on x $BH_3$
Durchführung in Dimethylformamid mit Natriumhydrid.
Schmelzpunkt: 173-176 °C
$R_f$-Wert: 0,56 (Kieselgel; Cyclohexan/Essigester = 2:8)
(16) 1-[2-(1-tert.Butyloxycarbonyl-4-piperidinyl)ethyl]-3-[4-[2-(methoxycarbonyl)ethyl]phenyl]-hydantoin
Durchführung in Dimethylformamid mit Natriumhydrid.
Schmelzpunkt: 104-106 °C
$R_f$-Wert: 0,30 (Kieselgel; Cyclohexan/Essigester = 1:1)

| | | | | | | |
|---|---|---|---|---|---|---|
| Ber.:<br>Gef.: | C | 63,41<br>63,66 | H | 7,45<br>7,49 | N | 8,87<br>8,95 |

(17) 3-[2-(1-tert.Butyloxycarbonyl-4-piperidinyl)ethyl]-1-[4-[2-(methoxycarbonyl)ethyl]phenyl]-4,5-dimethyl-3H-imidazol-2-on
Durchführung in Dimethylformamid mit Natriumhydrid.
Schmelzpunkt: 123-125 °C
$R_f$-Wert: 0,39 (Kieselgel; Cyclohexan/Essigester = 2:8)
(18) 3-[2-(1-tert.Butyloxycarbonyl-4-piperidinyl)ethyl]-1-[4-[2-(methoxycarbonyl)ethyl]phenyl]-hydantoin
Durchführung in Dimethylformamid mit Natriumhydrid.
Schmelzpunkt: 97-99 °C
$R_f$-Wert: 0,46 (Kieselgel; Cyclohexan/Essigester = 1:1)
(19) 1-[4-[2-(Methoxycarbonyl)ethyl]phenyl]-3-[2-(1-tert.butyloxycarbonyl-4-piperidinyl)ethyl]-3H-benzimidazol-2-on
Durchführung in Dimethylformamid mit Natriumhydrid.
$R_f$-Wert: 0,33 (Kieselgel; Cyclohexan/Essigester = 6:4)
(20) 1-[4-[2-(Methoxycarbonyl)ethyl]phenyl]-3-[2-(1-tert.butyloxycarbonyl-4-piperidinyl)ethyl]-4-methyl-imidazolidin-2-on
Durchführung in Dimethylformamid mit Natriumhydrid.
$R_f$-Wert: 0,58 (Kieselgel; Cyclohexan/Essigester = 1:1)
(21) 1-[2-(1-tert.Butyloxycarbonyl-4-piperidinyl)ethyl]-3-[[4-(ethoxycarbonyl)-1-piperidinyl]carbonylmethyl]-imidazolidin-2-on
Durchführung in Dimethylformamid mit Natriumhydrid.
Das Alkylierungsmittel [[4-(Ethoxycarbonyl)-1-piperidinyl]carbonyl]-methylchlorid [$R_f$-Wert: 0,35 (Kieselgel; Cyclohexan/Essigester = 1:1)] wird durch Umsetzung von Piperidin-4-carbonsäure-ethylester mit Chloracetylchlorid in Gegenwart von Triethylamin erhalten.
(22) 1-[2-(1-tert.Butyloxycarbonyl-4-piperidinyl)ethyl]-3-[4-[2-(methoxycarbonyl)ethenyl]-2-fluor-phenyl]-imidazolidin-2-on
Schmelzpunkt: 120-122 °C
$R_f$-Wert: 0,70 (Kieselgel; Cyclohexan/Essigester = 1:3)
(23) 1-[2-(1-tert.Butyloxycarbonyl-4-piperidinyl)ethyl]-3-[4-[2-(methoxycarbonyl)ethenyl]-2-trifluormethyl-phenyl]-imidazolidin-2-on
$R_f$-Wert: 0,54 (Kieselgel; Cyclohexan/Essigester = 1:2)
(24) 1-[2-(1-tert.Butyloxycarbonyl-4-piperidinyl)ethyl]-3-[4-[2-(methoxycarbonyl)ethenyl]-2-methyl-phenyl]-imidazolidin-2-on
$R_f$-Wert: 0,60 (Kieselgel; Cyclohexan/Essigester = 1:2)
(25) 1-[2-(1-tert.Butyloxycarbonyl-4-piperidinyl)ethyl]-3-[4-[2-(methoxycarbonyl)ethenyl]phenyl]-imidazolidin-2-on
Schmelzpunkt: 148-149 °C
$R_f$-Wert: 0,82 (Kieselgel; Methylenchlorid/Essigester = 1:1)
(26) 1-[2-(1-tert.Butyloxycarbonyl-4-piperidinyl)ethyl]-3-[4-[2-(ethoxycarbonyl)-1-phenyl-ethyl]phenyl]-imidazolidin-2-on

Schmelzpunkt: 100-102 °C
$R_f$-Wert: 0,35 (Kieselgel; Methylenchlorid/Essigester = 9:1)
(27) 1-(4-Cyanobutyl)-3-[4-[2-(methoxycarbonyl)ethyl]phenyl]-imidazolidin-2-on
Schmelzpunkt: 99-101 °C
$R_f$-Wert: 0,48 (Kieselgel; Essigester/Cyclohexan = 7:3)
(28) 1-[2-(1-tert.Butyloxycarbonyl-3-pyrrolidinyl)ethyl]-3-[4-[2-(methoxycarbonyl)ethyl]phenyl]-imidazolidin-2-on
Schmelzpunkt: 118-119 °C
$R_f$-Wert: 0,50 (Kieselgel; Essigester/Cyclohexan = 7:3)
(29) 1-[2-(1-Benzyl-1-azoniabicyclo[2.2.2]octan-4-yl)ethyl]-3-[4-[2-(methoxycarbonyl)ethyl]phenyl]-imidazolidin-2-on
$R_f$-Wert: 0,16 (Kieselgel; Methylenchlorid/Methanol = 9:1)

Beispiel 24

1-(trans-4-Carboxycyclohexyl)-3-[4-(4-cyano-4-piperidinyl)phenyl]-imidazolidin-2-on

Zu 1,4 g 1-[4-(4-Cyano-1-trifluoracetyl-4-piperidinyl)phenyl]-3-[trans-4-(methoxycarbonyl)cyclohexyl]-imidazolidin-2-on in 25 ml Tetrahydrofuran und 6 ml Wasser werden 2,8 ml 2N Natronlauge gegeben und über Nacht gerührt. Es werden 5,6 ml 1N Salzsäure zugegeben, das Tetrahydrofuran wird abrotiert und der Rückstand mit Natronlauge auf einen pH-Wert von 7,0 gebracht. Es wird eine Stunde im Eisbad gerührt, das Produkt wird abgesaugt, mit wenig Eiswasser und mit Aceton gewaschen und dann getrocknet.
Ausbeute: 1,0 g (91 % der Theorie),
$R_f$-Wert: 0,61 (Reversed Phase Kieselgel; Methanol/5%ige wäßrige Kochsalzlösung = 6:4)
Massenspektrum: $M^+$ = 396
Analog Beispiel 24 werden folgende Verbindungen erhalten:
(1) 1-(4-Carboxybutyl)-3-[4-(4-cyano-4-piperidinyl)phenyl]-imidazolidin-2-on
$R_f$-Wert: 0,62 (Reversed Phase Kieselgel; Methanol/5%ige wäßrige Kochsalzlösung = 6:4)
Massenspektrum: $M^+$ = 370
(2) 1-(trans-4-Carboxycyclohexyl)-3-[4-(4-methyl-4-piperidinyl)phenyl]-imidazolidin-2-on
$R_f$-Wert: 0,58 (Reversed Phase Kieselgel; Methanol/5%ige wäßrige Kochsalzlösung = 6:4)
Massenspektrum: $M^+$ = 385
(3) 1-[4-(1-Aza-4-cycloheptyl)phenyl]-3-(trans-4-carboxycyclohexyl)-imidazolidin-2-on
(4) 1-(4-Carboxybicyclo[2.2.2]octan-1-yl)-3-[4-(4-piperidinyl)phenyl]-imidazolidin-2-on
(5) 4-(trans-4-Carboxycyclohexyl)-2-[4-(4-piperidinyl)phenyl]-4H-1,2,4-triazol-3-on x 1,1 $H_2O$
Schmelzpunkt: >220 °C
$R_f$-Wert: 0,59 (Reversed Phase Kieselgel; Methanol/5%ige wäßrige Kochsalzlösung = 6:4)

| | | | | | | |
|---|---|---|---|---|---|---|
| Ber.:<br>Gef.: | C | 61,55<br>61,88 | H | 7,28<br>7,28 | N | 14,36<br>14,06 |

(6) 4-(trans-4-Carboxycyclohexyl)-2-[4-(4-piperidinyl)phenyl]-5-methyl-4H-1,2,4-triazol-3-on x $H_2O$
Schmelzpunkt: >220 °C
$R_f$-Wert: 0,54 (Reversed Phase Kieselgel; Methanol/5%ige wäßrige Kochsalzlösung = 6:4)

| | | | | | | |
|---|---|---|---|---|---|---|
| Ber.:<br>Gef.: | C | 62,67<br>62,85 | H | 7,51<br>7,73 | N | 13,92<br>12,65 |

(7) 1-(trans-4-Carboxycyclohexyl)-3-[4-(4-piperidinyl)phenyl]-3H-imidazol-2-on x 1,3 $H_2O$
$R_f$-Wert: 0,58 (Reversed Phase Kieselgel; Methanol/5%ige wäßrige Kochsalzlösung = 6:4)

| | | | | | | |
|---|---|---|---|---|---|---|
| Ber.:<br>Gef.: | C | 64,20<br>64,13 | H | 7,59<br>7,56 | N | 10,69<br>10,78 |

(8) 1-(trans-4-Carboxycyclohexyl)-3-[4-(4-piperidinyl)phenyl]-imidazolidin-2-on x 0,2 $H_2O$
Schmelzpunkt: 327-330 °C (Zers.)

| | | | | | | |
|---|---|---|---|---|---|---|
| Ber.:<br>Gef.: | C | 67,25<br>67,19 | H | 7,90<br>7,95 | N | 11,20<br>11,50 |

Beispiel 25

1-[4-(4-Aminocarbonyl-4-piperidinyl)phenyl]-3-(trans-4-carboxycyclohexyl)-imidazolidin-2-on x 0,8 $H_2SO_4$

200 mg 1-(trans-4-Carboxycyclohexyl)-3-[4-(4-cyano-4-piperidinyl)phenyl]-imidazolidin-2-on werden mit 2 ml 85 %iger Schwefelsäure 4 Tage bei Raumtemperatur gerührt. Es wird unter Kühlung mit Wasser versetzt, der Niederschlag wird abgesaugt, mit Eiswasser gewaschen und bei 90 °C im Vakuum getrocknet.
Ausbeute: 154 mg (54 % der Theorie),
$R_f$-Wert: 0,65 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 53,60<br>53,68 | H | 6,46<br>6,76 | N | 11,36<br>11,23 | S | 5,20<br>5,89 |

Massenspektrum: $(M+H)^+$ = 415

Beispiel 26

1-[4-(2-Carboxyethyl)phenyl]-3-[2-(4-chinuclidinyl)ethyl]-imidazolidin-2-on-hydrochlorid

Über eine Mischung aus 1,05 g 1-[4-[2-(Methoxycarbonyl)ethyl]phenyl]-3-[2-(4-chinuclidinyl)ethyl]-imidazolidin-2-on x $BH_3$ und 1 ml Orthokohlensäure-tetramethylester in 35 ml Methanol wird unter Rühren Chlorwasserstoff geleitet. Es wird 2 1/2 Tage bei Raumtemperatur gerührt, eingeengt und der Rückstand mit 10 ml halbkonzentrierter Salzsäure versetzt. Es wird 3 Stunden auf dem Dampfbad erhitzt, abgekühlt und zur Trockene eingedampft. Der Rückstand wird mit Aceton verrührt, abgesaugt, mit Aceton und Ether gewaschen und bei 100 °C getrocknet.
Ausbeute: 0,90 g (84 % der Theorie),
Schmelzpunkt: >250 °C
$R_f$-Wert: 0,49 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 61,83<br>61,56 | H | 7,41<br>7,34 | N | 10,30<br>10,38 | Cl | 8,69<br>8,42 |

Analog Beispiel 26 werden folgende Verbindungen erhalten:
(1) 1-[2-(1-Azabicyclo[2.2.1]heptan-4-yl)ethyl]-3-[4-(2-carboxyethyl)phenyl]-imidazolidin-2-on-hydrochlorid
(2) 1-(trans-4-Carboxycyclohexyl)-3-[4-(4-chinuclidinyl)phenyl]-imidazolidin-2-on-hydrochlorid

Beispiel 27

4-[trans-4-(Methoxycarbonyl)cyclohexyl]-2-[4-(1-trifluoracetyl-4-piperidinyl)phenyl]-5-methyl-4H-1,2,4-triazol-3-on

8,0 g 4-(4-Jodphenyl)-1-trifluoracetyl-piperidin (ca. 90%ig), 4,5 g 4-[trans-4-(Methoxycarbonyl)-cyclohexyl]-5-methyl-4H-1,2,4-triazol-3-on, 5,19 g Kaliumcarbonat, 0,59 ml Tris-[2-(2-methoxyethoxy)ethyl]-amin, 0,42 g Kupfer(I)-chlorid, 0,42 g Kupfer(I)-jodid und 80 ml N-Methyl-pyrrolidinon werden unter Stickstoff eine Stunde bei 170 °C gerührt. Es wird abgekühlt, abgesaugt und mit Dimethylformamid nachgewaschen. Das Filtrat wird auf eine Kieselgelsäule gegeben und mit Cyclohexan/Essigester (1:1) eluiert. Die Produktfraktionen werden eingeengt und der Rückstand über Nacht mit Wasser verrührt. Der Niederschlag wird abgesaugt und bei 100 °C im Vakuum getrocknet.

Ausbeute: 650 mg (7 % der Theorie),
$R_f$-Wert: 0,30 (Kieselgel; Cyclohexan/Essigester = 1:1)
Analog Beispiel 27 wird folgende Verbindung erhalten:

(1) 4-[trans-4-(Methoxycarbonyl)cyclohexyl]-2-[4-(1-trifluoracetyl-4-piperidinyl)phenyl]-4H-1,2,4-triazol-3-on
Schmelzpunkt: 184-186 °C
$R_f$-Wert; 0,26 (Kieselgel; Cyclohexan/Essigester = 1:1)

## Beispiel 28

### 1-[trans-4-(5-Indanyloxycarbonyl)cyclohexyl]-3-[4-(1-benzyloxycarbonyl-4-piperidinyl)phenyl]-imidazolidin-2-on

1,5 g 1-(trans-4-Carboxycyclohexyl)-3-[4-(1-benzyloxycarbonyl-4-piperidinyl)phenyl]-imidazolidin-2-on, 1,3 ml Thionylchlorid und ein Tropfen Dimethylformamid werden 2 Stunden bei Raumtemperatur gerührt. Danach wird zur Trockene einrotiert, der Rückstand wird in 5 ml Methylenchlorid gelöst und unter Eiskühlung mit einer Lösung von 0,4 g 5-Indanol, einer Spatelspitze 4-Dimethylaminopyridin und 0,7 ml Triethylamin in 5 ml Methylenchlorid versetzt. Nach 5 Stunden Rühren bei Raumtemperatur werden erneut 0,8 g 5-Indanol und 2,1 ml Triethylamin zugesetzt und über Nacht gerührt. Das Reaktionsgemisch wird mit 50 ml Methylenchlorid verdünnt und dreimal mit Wasser ausgeschüttelt. Die organische Phase wird abgetrennt, getrocknet und einrotiert. Der Rückstand wird durch Chromatographie über eine Kieselgelsäule mit Methylenchlorid/Essigester/Cyclohexan (1:1:1) gereinigt.
Ausbeute: 1,57 g (85 % der Theorie),
Schmelzpunkt: 172-174 °C
$R_f$-Wert; 0,40 (Kieselgel; Cyclohexan/Essigester/Methylenchlorid = 2:1:1)
Analog Beispiel 28 wird folgende Verbindung erhalten:

(1) 1-[trans-4-(5-Indanyloxycarbonyl)cyclohexyl]-3-[4-(1-benzyloxycarbonyl-4-cyano-4-piperidinyl)phenyl]-imidazolidin-2-on
Schmelzpunkt: 194-196 °C
$R_f$-Wert; 0,27 (Kieselgel; Cyclohexan/Essigester/Methylenchlorid = 2:1:1)

## Beispiel 29

### 1-[4-[2-(O-Ethylphosphono)ethyl]phenyl]-3-[2-(1-tert.butyloxycarbonyl-4-piperidinyl)ethyl]-imidazolidin-2-on

2,0 g 1-[4-[2-(O,O'-Diethylphosphono)ethyl]phenyl]-3-[2-(1-tert.butyloxycarbonyl-4-piperidinyl)ethyl]-imidazolidin-2-on und 0,56 g Natriumjodid werden in 20 ml Methyl-ethylketon 4 Tage unter Rückfluß erhitzt. Es wird abgekühlt, der Feststoff wird abgesaugt, mit Methyl-ethylketon gewaschen und getrocknet. Der Feststoff wird in 15 ml Wasser gelöst und die Lösung mit 10%iger Kaliumhydrogensulfat-Lösung versetzt bis ein pH von 2 resultiert. Das Gemisch wird im Eisbad gekühlt, der Niederschlag wird abgesaugt, mit Eiswasser gewaschen und getrocknet.
Ausbeute: 1,1 g (58 % der Theorie),
$R_f$-Wert; 0,16 (Reversed Phase Kieselgel; Methanol/5%ige wäßrige Kochsalzlösung = 6:4)

## Beispiel 30

### 1-[4-(2-Carboxyethyl)phenyl]-3-[2-(4-piperidinyl)ethyl]-4-methyl-imidazolidin-2-on x 1,1 HCl x 0,2 $H_2O$

260 mg 1-[4-[2-(Methoxycarbonyl)ethyl]phenyl]-3-[2-(1-tert.-butyloxycarbonyl-4-piperidinyl)ethyl]-4-methyl-imidazolidin-2-on werden mit 2,2 ml 3N Salzsäure 6,5 Stunden auf 90 °C erhitzt. Das Reaktionsgemisch wird eingeengt, der Rückstand zweimal mit Toluol eingedampft und dann mit Aceton verrieben. Der Feststoff wird abgesaugt, mit Aceton und Diethylether gewaschen und bei 60 °C getrocknet.
Ausbeute: 200 mg (90 % der Theorie),
Schmelzpunkt: 180-183 °C
$R_f$-Wert: 0,46 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 59,58<br>59,49 | H | 7,63<br>7,76 | N | 10,42<br>10,38 | Cl | 9,67<br>9,64 |

Analog Beispiel 30 werden folgende Verbindungen erhalten:

(1) 1-(1-Carboxymethyl-5-indanyl)-3-[2-(4-piperidinyl)ethyl]-imidazolidin-2-on-hydrochlorid

(2) 1-[4-(2-Carboxyethyl)phenyl]-3-methyl-4-[2-(4-piperidinyl)ethyl]-imidazolidin-2-on-hydrochlorid

(3) 1-[4-(2-Carboxy-1-phenyl-ethyl)phenyl]-3-[2-(4-piperidinyl)ethyl]-imidazolidin-2-on-hydrochlorid

$R_f$-Wert: 0,43 (Reversed Phase Kieselgel; Methanol/5%ige wäßrige Kochsalzlösung = 6:4)

(4) 1-[trans-4-[(Carboxymethyl)oxy]cyclohexyl]-3-[2-(4-piperidinyl)ethyl]-imidazolidin-2-on-hydrochlorid x 0,3 $H_2O$

Schmelzpunkt: 207-209 °C (Zers.)

$R_f$-Wert: 0,64 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 54,69<br>54,39 | H | 8,31<br>8,21 | N | 10,63<br>10,63 | Cl | 8,97<br>9,33 |

(5) 1-[4-(2-Carboxyethyl)-2-methyl-phenyl]-3-[2-(4-piperidinyl)ethyl]-imidazolidin-2-on-hydrochlorid

$R_f$-Wert: 0,57 (Reversed Phase Kieselgel; Methanol/5%ige wäßrige Kochsalzlösung = 6:4)

Massenspektrum: $M^+$ = 359

(6) 1-[4-(2-Carboxyethyl)-3-chlor-phenyl]-3-[2-(4-piperidinyl)ethyl]-imidazolidin-2-on-hydrochlorid

(7) 1-(2-Carboxy-1,2,3,4-tetrahydro-6-naphthyl)-3-[2-(4-piperidinyl)ethyl]-imidazolidin-2-on-hydrochlorid

(8) 1-[4-(2-Carboxyethyl)-2-fluor-phenyl]-3-[2-(4-piperidinyl)ethyl]-imidazolidin-2-on-hydrochlorid x 0,5 $H_2O$

Schmelzpunkt: 198-200 °C

$R_f$-Wert: 0,58 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 55,80<br>55,86 | H | 6,90<br>6,68 | N | 10,27<br>10,56 | Cl | 8,67<br>9,01 |

Massenspektrum: $M^+$ = 363

(9) 1-[4-(2-Carboxyethyl)-2-trifluormethyl-phenyl]-3-[2-(4-piperidinyl)ethyl]-imidazolidin-2-on-hydrochlorid

$R_f$-Wert: 0,50 (Reversed Phase Kieselgel; Methanol/5%ige wäßrige Kochsalzlösung = 6:4)

(10) 1-(trans-4-Carboxycyclohexyl)-3-[4-[(2-aminoethyl)oxy]phenyl]-imidazolidin-2-on-hydrochlorid

(11) 1-[4-(2-Carboxyethyl)phenyl]-3-methyl-4-[(4-piperidinyl)oxymethyl]-imidazolidin-2-on-hydrochlorid

(12) 1-[4-(2-Carboxyethyl)phenyl]-3-[2-(2-oxo-1-piperazinyl)ethyl]-imidazolidin-2-on-hydrochlorid

(13) 1-[4-(2-Carboxyethyl)phenyl]-3-[2-(3-pyrrolidinyl)ethyl]-imidazolidin-2-on-hydrochlorid x 0,25 $H_2O$

Schmelzpunkt: 200-203 °C

$R_f$-Wert: 0,57 (Reversed Phase Kieselgel; Methanol/5%ige wäßrige Kochsalzlösung = 6:4)

| | | | | | | | | |
|---|---|---|---|---|---|---|---|---|
| Ber.:<br>Gef.: | C | 58,06<br>57,97 | H | 7,17<br>7,12 | N | 11,28<br>11,28 | Cl | 9,52<br>9,73 |

(14) 1-[4-(2-Carboxyethyl)phenyl]-3-[3-(3-piperidinyl)propyl]-imidazolidin-2-on-hydrochlorid

(15) 1-[4-(2-Carboxyethenyl)phenyl]-3-[2-(4-piperidinyl)ethyl]-imidazolidin-2-on-hydrochlorid x 1 $H_2O$

Schmelzpunkt: 307-310 °C (Zers.)

$R_f$-Wert: 0,50 (Reversed Phase Kieselgel; Tetrahydrofuran/Wasser = 5:4)

| | | | | | | |
|---|---|---|---|---|---|---|
| Ber.:<br>Gef.: | C | 57,35<br>57,49 | H | 7,09<br>7,15 | N | 10,56<br>10,60 |

Massenspektrum: $M^+$ = 343

(16) 1-[4-(2-Carboxyethyl)-2-cyano-phenyl]-3-[2-(4-piperidinyl)ethyl]-imidazolidin-2-on-hydrochlorid

$R_f$-Wert: 0,71 (Reversed Phase Kieselgel; Methanol/5%ige wäßrige Kochsalzlösung = 6:4)

(17) 1-[1-(2-Carboxyethyl)piperidin-4-yl]-3-[2-(4-piperidinyl)ethyl]-imidazolidin-2-on-dihydrochlorid
$R_f$-Wert: 0,75 (Reversed Phase Kieselgel; Methanol/5%ige wäßrige Kochsalzlösung = 6:4)
Massenspektrum: $M^+$ = 352

Beispiel 31

1-[4-(2-Phosphonoethyl)phenyl]-3-[2-(4-piperidinyl)ethyl]-imidazolidin-2-on x 1,5 $H_2O$

450 mg 1-[4-[2-(O-Ethyl-phosphono)ethyl]phenyl]-3-[2-(4-piperidinyl)ethyl]-imidazolidin-2-on x 0,9 HCl x 1,1 $H_2O$, 22,5 ml Methylenchlorid und 0,9 ml Bromtrimethylsilan werden 3 Stunden unter Rückfluß erhitzt. Es wird zur Trockne eingeengt, der erhaltene Rückstand wird in 15 ml Wasser gelöst und mit 1N Natronlauge auf pH 7 eingestellt. Der erhaltene Niederschlag wird abgesaugt, mit Wasser gewaschen und getrocknet.
Ausbeute: 260 mg (70 % der Theorie),
$R_f$-Wert: 0,58 (Reversed Phase Kieselgel; Methanol/5%ige wäßrige Kochsalzlösung = 6:4)

| | | | | | | |
|---|---|---|---|---|---|---|
| Ber.:<br>Gef.: | C | 52,93<br>52,80 | H | 7,65<br>7,48 | N | 10,28<br>10,34 |

Beispiel 32

1-(2-Cyano-1,2,3,4-tetrahydro-6-naphthyl)-3-[trans-4-(methoxycarbonyl)cyclohexyl]-3H-imidazol-2-on

2,5 g N-(2-Cyano-1,2,3,4-tetrahydro-6-naphthyl)-N'-(2,2-diethoxyethyl)-N'-[trans-4-(methoxycarbonyl)-cyclohexyl]-harnstoff werden in 20 ml Trifluoressigsäure gelöst und 2,5 Stunden am Rückfluß gekocht. Anschließend wird abgekühlt, zur Trockene eingeengt, der Rückstand mit 75 ml Wasser versetzt und mit 2N Natronlauge neutralisiert. Der erhaltende Feststoff wird abgesaugt, mit Wasser gewaschen, getrocknet und durch Chromatographie über eine Kieselgelsäule mit Essigester/Methylenchlorid/Cyclohexan (1:1:1) gereinigt.
Ausbeute: 1,4 g (70% der Theorie),
Schmelzpunkt: 175-176 °C
$R_f$-Wert: 0,34 (Kieselgel; Essigester/Cyclohexan = 3:2)
Analog Beispiel 32 werden folgende Verbindungen erhalten:
(1) 1-[2-(Methoxycarbonyl)-6-naphthyl]-3-[2-(4-piperidinyl)ethyl]-3H-imidazol-2-on-trifluoracetat
Unter gleichzeitiger Abspaltung der N-tert.Butyloxycarbonyl-Schutzgruppe
Schmelzpunkt: 148-151 °C
$R_f$-Wert: 0,38 (Reversed Phase Kieselgel; Methanol/5%ige wäßrige Kochsalzlösung = 6:4)

| | | | | | | |
|---|---|---|---|---|---|---|
| Ber.:<br>Gef.: | C | 58,41<br>58,47 | H | 5,31<br>5,39 | N | 8,51<br>8,51 |

(2) 1-[2-(Methoxycarbonyl)-1,2,3,4-tetrahydro-6-naphthyl]-3-[2-(4-piperidinyl)ethyl]-3H-imidazol-2-on
Unter gleichzeitiger Abspaltung der N-tert.Butyloxycarbonyl-Schutzgruppe; Isolierung der Base
Schmelzpunkt: 116-118 °C
$R_f$-Wert: 0,50 (Reversed Phase Kieselgel; Methanol/5%ige wäßrige Kochsalzlösung = 6:4)
(3) 1-[trans-4-(Methoxycarbonyl)cyclohexyl]-3-[4-(4-chinuclidinyl)phenyl]-3H-imidazol-2-on
Isolierung der Base
Schmelzpunkt: 223-225 °C
$R_f$-Wert: 0,47 (Reversed Phase Kieselgel; Methanol/5%ige wäßrige Kochsalzlösung = 6:4)
Massenspektrum: $M^+$ = 409
(4) 1-[trans-4-(Methoxycarbonyl)cyclohexyl]-3-[4-(4-methyl-1-trifluoracetyl-4-piperidinyl)phenyl]-3H-imidazol-2-on
Schmelzpunkt: 182-184 °C
$R_f$-Wert: 0,44 (Kieselgel; Methylenchlorid/Essigester = 4:1)

(5) 1-(4-Cyano-bicyclo[2.2.2]octan-1-yl)-3-[4-[2-(methoxycarbonyl)ethyl]phenyl]-imidazolidin-2-on
Schmelzpunkt: 195-197°C
$R_f$-Wert: 0,68 (Kieselgel; Methylenchlorid/Essigester = 4:1)
(6) 1-[2-(1-Benzyl-1-azoniabicyclo[2.2.2]octan-4-yl)ethyl]-3-[-4-[2-(methoxycarbonyl)ethyl]phenyl]-3H-imidazol-2-on-hydrochlorid
$R_f$-Wert: 0,19 (Kieselgel; Methylenchlorid/Methanol = 100:7)

Beispiel 33

1-[2-(1-tert.Butyloxycarbonyl-4-piperidinyl)ethyl]-3-[4-[2-(methoxycarbonyl)ethyl]-2-fluor-phenyl]-imidazolidin-2-on

2,0 g 1-[2-(1-tert.Butyloxycarbonyl-4-piperidinyl)ethyl]-3-[4-[2-(methoxycarbonyl)ethenyl]-2-fluor-phenyl]-imidazolidin-2-on werden mit 0,5 g 10%igem Palladium auf Kohle in 20 ml Essigester bei Raumtemperatur und einem Wasserstoffdruck von 50 psi 15 Minuten hydriert. Es wird abgesaugt und das Filtrat zur Trockene eingeengt.
Ausbeute: 2,0 g (100% der Theorie),
$R_f$-Wert: 0,65 (Kieselgel; Essigester/Cyclohexan = 3:1)
Analog Beispiel 33 werden folgende Verbindungen erhalten:
(1) 1-[2-(1-tert.Butyloxycarbonyl-4-piperidinyl)ethyl]-3-[4-[2-(methoxycarbonyl)ethyl]-2-trifluormethyl-phenyl]-imidazolidin-2-on
$R_f$-Wert: 0,50 (Kieselgel; Essigester/Cyclohexan = 2:1)
(2) 1-[2-(1-tert.Butyloxycarbonyl-4-piperidinyl)ethyl]-3-[4-[2-(methoxycarbonyl)ethyl]-2-methyl-phenyl]-imidazolidin-2-on
$R_f$-Wert: 0,60 (Kieselgel; Essigester/Cyclohexan = 2:1)

Beispiel 34

1-[4-[(Cyanomethyl)oxy]phenyl]-3-[trans-4-(methoxycarbonyl)cyclohexyl]-imidazolidin-2-on

Zu einer Lösung von 500 mg 1-(4-Hydroxyphenyl)-3-[trans-4-(methoxycarbonyl)cyclohexyl]-imidazolidin-2-on in 3 ml Dimethylformamid werden 270 mg Kaliumcarbonat und 210 mg Bromacetonitril gegeben und 5,5 Stunden auf 60°C erhitzt. Es wird eingeengt, der Rückstand mit Wasser versetzt, mit 2 N Zitronensäure neutralisiert und 4 x mit Essigester extrahiert. Die vereinigten organischen Phasen werden mit Wasser gewaschen, getrocknet und im Vakuum eingeengt. Der Rückstand wird durch Chromatographie über eine Kieselgelsäule mit Methylenchlorid/Essigester (100:8) gereinigt.
Ausbeute: 370 mg (65 % der Theorie),
Schmelzpunkt: 136-138°C
$R_f$-Wert: 0,33 (Kieselgel; Essigester/Cyclohexan = 1:1)

Beispiel 35

Trockenampulle mit 2,5 mg Wirkstoff pro 1 ml

| Zusammensetzung: | |
|---|---|
| Wirkstoff | 2,5 mg |
| Mannitol | 50,0 mg |
| Wasser für Injektionszwecke ad | 1,0 ml |

Herstellung:

Wirkstoff und Mannitol werden in Wasser gelöst. Nach Abfüllung wird gefriergetrocknet.
Die Auflösung zur gebrauchsfertigen Lösung erfolgt mit Wasser für Injektionszwecke.

Beispiel 36

Trockenampulle mit 35 mg Wirkstoff pro 2 ml

| Zusammensetzung: | |
|---|---|
| Wirkstoff | 35,0 mg |
| Mannitol | 100,0 mg |
| Wasser für Injektionszwecke ad | 2,0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel 37

Tablette mit 50 mg Wirkstoff

| Zusammensetzung: | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| | 215,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 9 mm.

Beispiel 38

Tablette mit 350 mg Wirkstoff

| Zusammensetzung: | |
|---|---|
| (1) Wirkstoff | 350,0 mg |
| (2) Milchzucker | 136,0 mg |
| (3) Maisstärke | 80,0 mg |
| (4) Polyvinylpyrrolidon | 30,0 mg |
| (5) Magnesiumstearat | 4.0 mg |
| | 600,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger

Facette und einseitiger Teilkerbe. Durchmesser der Tabletten: 12 mm.

Beispiel 39

Kapseln mit 50 mg Wirkstoff

| Zusammensetzung: | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2.0 mg |
| | 160,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 3 abgefüllt.

Beispiel 40

Kapseln mit 350 mg Wirkstoff

| Zusammensetzung: | |
|---|---|
| (1) Wirkstoff | 350,0 mg |
| (2) Maisstärke getrocknet | 46,0 mg |
| (3) Milchzucker pulverisiert | 30,0 mg |
| (4) Magnesiumstearat | 4.0 mg |
| | 430,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 0 abgefüllt.

**Patentansprüche**

**1.** Cyclische Harnstoffderivate der allgemeinen Formel I mit Ausnahme von
1-(4-Amidino-phenyl)-3-[4-(2-carboxy-ethyl)-cyclohexyl]-imidazolidin-2-on,
1-(4-Amidino-phenyl)-3-[4-(2-methoxycarbonyl-ethyl)-cyclohexyl]-imidazolidin-2-on,
1-(4-Cyano-phenyl)-3-[4-(2-methoxycarbonyl-ethyl)-cyclohexyl]-imidazolidin-2-on,
1-(4-Amidino-phenyl)-3-[4-[2-(5-tetrazolyl)-ethyl]-phenyl]-imidazolidin-2-on,
1-(4-Aminomethyl-cyclohexyl)-3-[4-(2-carboxy-ethyl)-phenyl]-imidazolidin-2-on,
1-(4-Aminomethyl-cyclohexyl)-3-[4-(2-methoxycarbonyl-ethyl)-phenyl]-imidazolidin-2-on,
4-(4'-Amidino-4-biphenylyl)-1-(2-carboxy-ethyl)-3-phenyl-3H-imidazol-2-on,
4-(4'-Amidino-4-biphenylyl)-1-(2-methoxycarbonyl-ethyl)-3-phenyl-3H-imidazol-2-on,
4-(4'-Cyano-4-biphenylyl)-1-(2-methoxycarbonyl-ethyl)-3-phenyl-3H-imidazol-2-on,
4-[4'-Amidino-4-biphenylyl)-1-(2-carboxy-ethyl)-3-phenyl-imidazolidin-2-on,

4-(4'-Amidino-4-biphenylyl)-1-(2-methoxycarbonyl-ethyl)-3-phenyl-imidazolidin-2-on,
1-(4'-Amidino-4-biphenylyl)-3-(2-ethoxycarbonyl-ethyl)-3H-imidazol-2-on,
1-(4'-Cyano-4-biphenylyl)-3-(2-ethoxycarbonyl-ethyl)-3H-imidazol-2-on,
1-(4'-Amidino-4-biphenylyl)-3-(2-ethoxycarbonyl-ethyl)-imidazolidin-2,4-dion,
1-(4'-Cyano-4-biphenylyl)-3-(2-ethoxycarbonyl-ethyl)-imidazolidin-2,4-dion,
1-(4'-Amidino-4-biphenylyl)-3-(2-ethoxycarbonyl-ethyl)-3,4,5,6-tetrahydro-1H-pyrimidin-2-on,
1-(4'-Cyano-4-biphenylyl)-3-(2-ethoxycarbonyl-ethyl)-3,4,5,6-tetrahydro-1H-pyrimidin-2-on,
2-(4-Amidino-phenyl)-4-[4-(2-isopropyloxycarbonyl-ethyl)-phenyl]-5-methyl-4H-1,2,4-triazol-3-on,
4-[4-(2-Isopropyloxycarbonyl-ethyl)-phenyl]-2-(4-methoxycarbonylamidino-phenyl)-5-methyl-4H-1,2,4-triazol-3-on,
1-(4-Amidino-phenyl)-3-[4-(2-ethoxycarbonyl-ethyl)-phenyl]-3H-imidazol-2-on,
1-(4-Ethoxycarbonylamidino-phenyl)-3-[4-(2-ethoxycarbonyl-ethyl)-phenyl]-3H-imidazol-2-on,
1-(4-Ethoxycarbonylamidino-phenyl)-3-[4-(2-ethoxycarbonyl-ethyl)-phenyl]-imidazolidin-2-on,
1-(4-Methoxycarbonylamidino-phenyl)-3-[4-(2-methoxycarbonyl-ethyl)-cyclohexyl]-imidazolidin-2-on,
4-[4-(2-Isobutyloxycarbonyl-ethyl)-phenyl]-2-(4-methoxycarbonylamidino-phenyl)-5-methyl-4H-1,2,4-triazol-3-on,
2-(4-Amidino-phenyl)-4-[4-(2-isobutyloxycarbonyl-ethyl)-phenyl]-5-methyl-4H-1,2,4-triazol-3-on,
1-(1-Amino-5-indanyl)-3-[4-(2-carboxy-ethyl)-phenyl]-imidazolidin-2-on,
1-(1-Amino-5-indanyl)-3-[4-(2-methoxycarbonyl-ethyl)-phenyl]-imidazolidin-2-on,
2-(4-Amidino-phenyl)-5-ethyl-4-[4-(2-isopropyloxycarbonyl-ethyl)-phenyl]-4H-1,2,4-triazol-3-on,
1-(4-Amidino-phenyl)-3-[4-(2-isopropyloxycarbonyl-ethyl)-phenyl]-4-methyl-3H-imidazol-2-on,
1-(4-Amidino-phenyl)-3-[4-(2-isopropyloxycarbonyl-ethyl)-phenyl]-3H-imidazol-2-on,
1-(4-Amidino-phenyl)-3-[4-(2-isopropyloxycarbonyl-ethyl)-phenyl]-imidazolidin-2,4-dion,
2-(4-Amidino-phenyl)-4-[4-(2-carboxy-ethyl)-phenyl]-5-trifluormethyl-4H-1,2,4-triazol-3-on und
3-(4-Cyano-phenyl)-1-(4-ethoxycarbonyl-butyl)-3H-imidazo[4,5-b]pyridin-2-on,

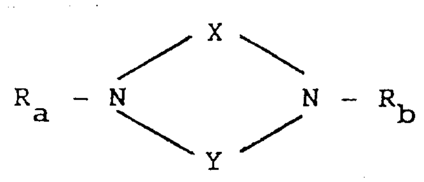

,(I)

in der mit den Maßgaben, daß

(i) X eine am Stickstoffatom durch eine Cyanogruppe substituierte Carbiminogruppe, eine 1-Nitro-ethen-2,2-diyl- oder 1,1-Dicyano-ethen-2,2-diyl-Gruppe,

(ii) Y eine gegebenenfalls durch $R_c$ oder $R_d$ oder $R_c$ und $R_d$ substituierte 1,2-Cycloalkylengruppe mit 5 bis 7 Kohlenstoffatomen,

(iii) A eine Aminoalkylgruppe mit 1 bis 5 Kohlenstoffatomen, eine Amino-, Amidino- oder Guanidinogruppe, wobei in jeder der vorstehend erwähnten Gruppen jeweils an einem der Stickstoffatome ein Wasserstoffatom durch eine $R_1$-CO-O-($R_2CR_3$)-O-CO-Gruppe ersetzt ist,

(iv) B eine gegebenenfalls durch eine oder zwei Alkylgruppen substituierte Cycloalkylengruppe mit 3 bis 7 Kohlenstoffatomen,

eine gegebenenfalls durch einen Rest $R_e$ substituierte Cycloalkylengruppe mit 4 oder 5 Kohlenstoffatomen, welche zusätzlich durch 1 bis 4 Alkylgruppen substituiert sein kann und in der im Cycloalkylenteil jeweils eine >CH-Einheit durch ein Stickstoffatom ersetzt ist,

eine gegebenenfalls durch einen Rest $R_e$ substituierte Cycloalkylengruppe mit 6 bis 8 Kohlenstoffatomen, welche zusätzlich durch 1 bis 4 Alkylgruppen substituiert sein kann und in der im Cycloalkylenteil jeweils eine >CH-Einheit durch ein Stickstoffatom ersetzt ist und zusätzlich eine weitere in 4-Stellung befindliche >CH-Einheit durch ein Stickstoffatom ersetzt sein kann, wobei außerdem in den vorstehend erwähnten 6- bis 8-gliedrigen Ringen jeweils eine oder zwei zu einem Stickstoffatom benachbarte Methylengruppen jeweils durch eine Carbonylgruppe ersetzt sein können,

eine gegebenenfalls durch eine oder zwei Alkylgruppen substituierte Cycloalkenylengruppe mit 5 bis 7 Kohlenstoffatomen, welche zusätzlich durch eine oder zwei Methylgruppen substituiert sein kann und in der im Cycloalkenylenteil jeweils eine >CH-Einheit durch ein Stickstoffatom ersetzt ist,

eine Cycloalkylengruppe mit 5 bis 7 Kohlenstoffatomen, in der eine >CH-Einheit durch ein Stickstoffatom, welches mit dem Rest A verknüpft ist, ersetzt ist und zusätzlich zwei Wasserstoffatome am Kohlenstoffgerüst durch eine geradkettige Alkylenbrücke ersetzt sind, wobei diese Brücke 2 bis 6 Kohlenstoffatome enthält, wenn die zwei Wasserstoffatome sich am selben Kohlenstoffatom befinden, oder 1 bis 5 Kohlenstoffatome enthält, wenn sie zwei Wasserstoffatome an benachbarten Kohlenstoffatomen ersetzt, oder 1 bis 4 Kohlenstoffatome enthält, wenn sie zwei Wasserstoffatome an Kohlenstoffatomen ersetzt, die durch ein weiteres Atom getrennt sind, oder 1 bis 3 Kohlenstoffatome enthält, wenn sie zwei Wasserstoffatome an Kohlenstoffatomen ersetzt, die durch zwei Atome getrennt sind, wobei die vorstehenden Gruppen jeweils durch eine oder zwei Alkylgruppen substituiert sein können,

eine über den Rest W mit dem Rest C verknüpfte Alkylengruppe mit 1 bis 6 Kohlenstoffatomen, oder

B zusammen mit A eine gegebenenfalls durch 1 oder 2 Alkylgruppen substituierte Pyridylgruppe oder eine Piperidinylgruppe, in der das Wasserstoffatom in 1-Stellung zusammen mit einem Wasserstoffatom in 2-Stellung durch eine geradkettige Alkylengruppe mit 3 oder 4 Kohlenstoffatomen ersetzt ist, oder eine Piperidinylgruppe, in der das Wasserstoffatom in 1-Stellung zusammen mit einem Wasserstoffatom in 3-Stellung durch eine geradkettige Alkylengruppe mit 2 bis 4 Kohlenstoffatomen ersetzt ist, oder eine Piperidinylgruppe, in der das Wasserstoffatom in 1-Stellung zusammen mit einem Wasserstoffatom in 4-Stellung durch eine geradkettige Alkylengruppe mit 1 bis 3 Kohlenstoffatomen ersetzt ist, wobei die an die 4-Stellung gebundene Methylengruppe einer Ethylenkette durch eine Hydroxymethylen- oder Carbonylgruppe ersetzt sein kann und zusätzlich das Stickstoffatom dieser vorstehend erwähnten bicyclischen Gruppen, welche im Kohlenstoffgerüst durch eine oder zwei Alkylgruppen substituiert sein können, durch Boran komplexiert oder durch eine im Phenylkern gegebenenfalls durch 1 bis 2 Methoxygruppen substituierte Benzylgruppe quarternisiert sein kann,

(v) C eine Alkylengruppe mit 1 bis 6 Kohlenstoffatomen, in der eine Methylengruppe durch ein Sauerstoff- oder Schwefelatom oder durch eine Sulfinyl-, Sulfonyl- oder $-NR_4$-Gruppe ersetzt ist oder in der eine Ethylengruppe durch eine $-CONR_5-$ oder $-NR_5CO$-Gruppe ersetzt ist,

eine gegebenenfalls durch eine oder zwei Alkylgruppen substituierte 3- bis 7-gliedrige Cycloalkylengruppe,

eine Cyclohexylengruppe, in der zwei durch drei Bindungen getrennte Kohlenstoffatome zusätzlich durch eine geradkettige Alkylengruppe mit 1 bis 3 Kohlenstoffatomen verknüpft sind, wobei diese bicyclischen Gruppen jeweils außerdem durch ein bis zwei Alkylgruppen substituiert sein können,

eine Indanylen-, Naphthylen-, 1,2,3,4-Tetrahydronaphthylen- oder Benzosuberanylengruppe, wobei die gesättigten Ringe jeweils durch 1 oder 2 Alkylgruppen substituiert sein können und die aromatischen Ringe jeweils durch ein Fluor-, Chlor-, Brom- oder Jodatom, durch eine Alkyl-, Trifluormethyl-, Hydroxy-, Alkoxy-, Alkylsulfenyl-, Alkylsulfinyl-, Alkylsulfonyl- oder Cyanogruppe substituiert sein können und in denen zusätzlich eine oder zwei Methingruppen jeweils durch ein Stickstoffatom ersetzt sein können,

eine gegebenenfalls durch den Rest $R_e$ substituierte Cycloalkylengruppe mit 4 oder 5 Kohlenstoffatomen, welche zusätzlich durch 1 bis 4 Alkylgruppen substituiert sein kann und in denen im Cycloalkylenteil jeweils eine $>$CH-Einheit durch ein Stickstoffatom ersetzt ist,

eine gegebenenfalls durch den Rest $R_e$ substituierte Cycloalkylengruppe mit 6 bis 8 Kohlenstoffatomen, welche zusätzlich durch 1 bis 4 Alkylgruppen substituiert sein kann und in denen im Cycloalkylenteil jeweils eine $>$CH-Einheit durch ein Stickstoffatom ersetzt ist und zusätzlich eine weitere in 4-Stellung befindliche $>$CH-Einheit durch ein Stickstoffatom ersetzt sein kann, wobei außerdem in den vorstehend erwähnten 6- bis 8-gliedrigen Ringen jeweils eine oder zwei zu einem Stickstoffatom benachbarte Methylengruppen jeweils durch eine Carbonylgruppe ersetzt sein können,

eine gegebenenfalls durch eine oder zwei Alkylgruppen substituierte Cycloalkenylengruppe mit 5 bis 7 Kohlenstoffatomen, welche zusätzlich durch eine oder zwei Methylgruppen substituiert sein kann und in denen im Cycloalkenylenteil jeweils eine $>$CH-Einheit durch ein Stickstoffatom ersetzt ist,

eine Cycloalkylengruppe mit 5 bis 7 Kohlenstoffatomen, in der eine $>$CH-Einheit durch ein Stickstoffatom, welches mit dem Rest A verknüpft ist, ersetzt ist und zusätzlich zwei Wasserstoffatome am Kohlenstoffgerüst durch eine geradkettige Alkylenbrücke ersetzt sind, wobei diese Brücke 2 bis 6 Kohlenstoffatome enthält, wenn die zwei Wasserstoffatome sich am selben Kohlenstoffatom befinden, oder 1 bis 5 Kohlenstoffatome enthält, wenn sie zwei Wasserstoffatome an benachbarten Kohlenstoffatomen ersetzt, oder 1 bis 4 Kohlenstoffatome enthält, wenn sie zwei Wasserstoffatome an Kohlenstoffatomen ersetzt, die durch ein weiteres Atom getrennt sind, oder 1 bis 3 Kohlenstoffatome enthält, wenn sie zwei Wasserstoffatome an Kohlenstoffatomen ersetzt, die durch zwei Atome

getrennt sind, wobei die vorstehenden Gruppen jeweils durch eine oder zwei Alkylgruppen substituiert sein können, oder
C zusammen mit A und B eine gegebenenfalls jeweils durch eine oder zwei Alkylgruppen substituierte Pyridyl- oder 1-(4-Pyridyl)-piperidinylgruppe oder eine Piperidinylgruppe, in der das Wasserstoffatom in 1-Stellung zusammen mit einem Wasserstoffatom in 2-Stellung durch eine geradkettige Alkylengruppe mit 3 oder 4 Kohlenstoffatomen ersetzt ist, oder eine Piperidinylgruppe, in der das Wasserstoffatom in 1-Stellung zusammen mit einem Wasserstoffatom in 3-Stellung durch eine geradkettige Alkylengruppe mit 2 bis 4 Kohlenstoffatomen ersetzt ist, oder eine Piperidinylgruppe, in der das Wasserstoffatom in 1-Stellung zusammen mit einem Wasserstoffatom in 4-Stellung durch eine geradkettige Alkylengruppe mit 1 bis 3 Kohlenstoffatomen ersetzt ist, wobei die an die 4-Stellung gebundene Methylengruppe einer Ethylenkette durch eine Hydroxymethylen- oder Carbonylgruppe ersetzt sein kann und zusätzlich das Stickstoffatom dieser vorstehend erwähnten bicyclischen Gruppen, welche im Kohlenstoffgerüst durch eine oder zwei Alkylgruppen substituiert sein können, durch Boran komplexiert oder durch eine im Phenylkern gegebenenfalls durch 1 bis 2 Methoxygruppen substituierte Benzylgruppe quartärnisiert sein kann, oder
C eine Alkylengruppe mit 1 bis 6 Kohlenstoffatomen und B eine Bindung,
(vi) D eine 1,3-Arylengruppe,
eine Indanylen-, Naphthylen-, 1,2,3,4-Tetrahydronaphthylen- oder Benzosuberanylengruppe, wobei die gesättigten Ringe jeweils durch 1 oder 2 Alkylgruppen substituiert sein können und die aromatischen Ringe jeweils durch ein Fluor-, Chlor-, Brom- oder Jodatom, durch eine Alkyl-, Trifluormethyl-, Hydroxy-, Alkoxy-, Alkylsulfenyl-, Alkylsulfinyl-, Alkylsulfonyl- oder Cyanogruppe substituiert sein können und in denen zusätzlich eine oder zwei Methingruppen jeweils durch ein Stickstoffatom ersetzt sein können,
eine gegebenenfalls durch eine oder zwei Alkylgruppen substituierte Cycloalkylengruppe mit 3 bis 7 Kohlenstoffatomen oder
eine Cyclohexylengruppe, in der zwei durch drei Bindungen getrennte Kohlenstoffatome zusätzlich durch eine geradkettige Alkylengruppe mit 1 bis 3 Kohlenstoffatomen verknüpft sind, wobei diese bicyclischen Gruppen jeweils außerdem durch ein bis zwei Alkylgruppen substituiert sein können,
(vii) E eine geradkettige Alkylengruppe mit 1 bis 6 Kohlenstoffatomen oder eine geradkettige Alkenylengruppe mit 2 bis 6 Kohlenstoffatomen, die jeweils durch eine Alkylgruppe mit 1 bis 8 Kohlenstoffatomen, durch eine Aryl- oder Heteroarylgruppe, durch eine $HNR_6$- oder N-Phenylalkyl-$NR_6$-Gruppe oder durch eine N-Alkyl-$NR_6$-Gruppe mit 1 bis 8 Kohlenstoffatomen im Alkylteil substituiert ist,
eine gegebenenfalls durch eine oder zwei Alkylgruppen substituierte Cycloalkylengruppe mit 5 bis 7 Kohlenstoffatomen, in der eine $>$CH-Einheit durch ein Stickstoffatom ersetzt ist,
eine durch eine Hydroxy-, Amino-, Aryl- oder Heteroarylgruppe, durch eine Alkoxy- oder Alkylaminogruppe mit jeweils 1 bis 8 Kohlenstoffatomen, durch eine Dialkylaminogruppe mit insgesamt 2 bis 10 Kohlenstoffatomen, durch eine $HNR_6$- oder N-Phenylalkyl-$NR_6$-Gruppe oder durch eine N-Alkyl-$NR_6$-Gruppe mit 1 bis 8 Kohlenstoffatomen im Alkylteil substituierte Cycloalkylengruppe mit 4 bis 7 Kohlenstoffatomen im Cycloalkylenteil, oder
eine über den Rest W mit dem Rest D verknüpfte Alkylengruppe, wobei die Alkylengruppe zusätzlich durch eine oder zwei Alkylgruppen mit jeweils 1 bis 8 Kohlenstoffatomen, durch eine Hydroxy-, Amino-, Aryl- oder Heteroarylgruppe, durch eine Alkoxy- oder Alkylaminogruppe mit jeweils 1 bis 8 Kohlenstoffatomen, durch eine Dialkylaminogruppe mit insgesamt 2 bis 10 Kohlenstoffatomen, durch eine $HNR_6$- oder N-Phenylalkyl-$NR_6$-Gruppe oder durch eine N-Alkyl-$NR_6$-Gruppe mit 1 bis 8 Kohlenstoffatomen im Alkylteil substituiert ist,
(viii) F eine Alkoxycarbonylgruppe mit insgesamt 3 bis 9 Kohlenstoffatomen, eine $R_7$O-CO-, Phosphono-, O-Alkyl-phosphono-, O,O'-Dialkyl-phosphono-, Tetrazol-5-yl- oder $R_8$CO-O-CHR$_9$-O-CO-Gruppe und
(ix) der dritte der Reste $R_a$ bis $R_d$ eine Perfluoralkyl- oder Arylgruppe
darstellt, wobei mindestens eine der obigen Bedingungen (i) bis (ix) erfüllt sein müssen,
X eine gegebenenfalls am Stickstoffatom durch eine Alkyl- oder Cyanogruppe substituierte Carbiminogruppe, eine Carbonyl-, Thiocarbonyl-, Sulfonyl-, 1-Nitro-ethen-2,2-diyl- oder 1,1-Dicyano-ethen-2,2-diyl-Gruppe,
Y eine gegebenenfalls durch $R_c$ oder $R_d$ oder $R_c$ und $R_d$ substituierte geradkettige Alkylengruppe mit 2 bis 4 Kohlenstoffatomen, die zusätzlich durch eine oder zwei Alkylgruppen substituiert sein kann, und in der zusätzlich eine oder zwei Methylengruppen jeweils durch eine Carbonylgruppe ersetzt sein können,
eine gegebenenfalls durch $R_c$ oder $R_d$ oder $R_c$ und $R_d$ substituierte geradkettige Alkenylengruppe mit 2

bis 4 Kohlenstoffatomen, in der zusätzlich eine gegebenenfalls vorhandene Methylengruppe durch eine Carbonylgruppe ersetzt sein kann,
eine gegebenenfalls durch $R_c$ oder $R_d$ oder $R_c$ und $R_d$ substituierte 1,2-Cycloalkylengruppe mit 5 bis 7 Kohlenstoffatomen,
eine gegebenenfalls durch $R_c$ oder $R_d$ oder $R_c$ und $R_d$ substituierte 1,2-Cycloalkenylengruppe mit 5 bis 7 Kohlenstoffatomen,
eine 1,2-Arylengruppe,
eine 1,2-Phenylengruppe, in der eine oder zwei Methingruppen jeweils durch ein Stickstoffatom ersetzt sind oder in der eine oder zwei -CH = CH-Gruppen jeweils durch eine -CO-NH-Gruppe ersetzt sind oder in der eine Methingruppe durch ein Stickstoffatom und eine -CH = CH-Gruppe durch eine -CO-NH-Gruppe ersetzt ist, wobei die vorstehend erwähnten heterocyclischen Gruppen zusätzlich durch eine oder zwei Alkylgruppen substituiert sein können, oder
eine gegebenenfalls durch $R_c$ oder $R_d$ substituierte -CO-NH-, -NH-CO-, -CH = N- oder -N = CH-Gruppe,
der erste der Reste $R_a$ bis $R_d$ eine Gruppe der Formel

A - B - C -,

in der
A eine Aminoalkylgruppe mit 1 bis 5 Kohlenstoffatomen, eine Amino-, Amidino- oder Guanidinogruppe, wobei in jeder der vorstehend erwähnten Gruppen jeweils an einem der Stickstoffatome ein oder zwei Wasserstoffatome jeweils durch eine Alkylgruppe oder ein Wasserstoffatom durch eine Alkoxycarbonylgruppe mit insgesamt 2 bis 5 Kohlenstoffatomen, durch eine Benzyloxycarbonylgruppe oder durch eine $R_1$-CO-O-($R_2CR_3$)-O-CO-Gruppe ersetzt sein kann, wobei

$R_1$ eine Alkylgruppe mit 1 bis 8 Kohlenstoffatomen, eine Cycloalkylgruppe mit 5 bis 7 Kohlenstoffatomen, eine Phenyl- oder Phenylalkylgruppe,

$R_2$ ein Wasserstoffatom, eine Alkylgruppe, eine Cycloalkylgruppe mit 5 bis 7 Kohlenstoffatomen oder eine Phenylgruppe und

$R_3$ ein Wasserstoffatom oder eine Alkylgruppe darstellen,

eine Cyano- oder Cyanoalkylgruppe oder
auch, falls A an ein Stickstoffatom der Reste B oder C gebunden ist, das nicht Teil einer Lactamgruppe ist, ein Wasserstoffatom, eine Alkylgruppe, eine Benzylgruppe, in der der Phenylteil durch 1 bis 2 Methoxygruppen substituiert sein kann, eine Formyl-, Acetyl- oder Trifluoracetylgruppe, eine Alkoxycarbonylgruppe mit insgesamt 2 bis 5 Kohlenstoffatomen, eine Benzyloxycarbonylgruppe oder eine $R_1$-CO-O-($R_2CR_3$)-O-CO-Gruppe, wobei $R_1$ bis $R_3$ wie vorstehend erwähnt definiert sind,
B eine Bindung,
eine Alkylengruppe mit 1 bis 6 Kohlenstoffatomen,
eine Arylengruppe,
eine Pyridinylen-, Pyrimidinylen-, Pyrazinylen- oder Pyridazinylengruppe, in denen eine oder zwei -CH = N-Gruppen jeweils durch eine -CO-NH-Gruppe ersetzt sein können und eines der Stickstoffatome statt an ein Wasserstoffatom auch an den Rest C gebunden sein kann, sofern dieser nicht mit einem Heteroatom oder einer Carbonylgruppe an den Rest B anschließt, wobei die vorstehend erwähnten heterocyclischen Gruppen zusätzlich durch eine oder zwei Alkylgruppen substituiert sein können,
eine gegebenenfalls durch eine oder zwei Alkylgruppen substituierte Cycloalkylengruppe mit 3 bis 7 Kohlenstoffatomen,
eine gegebenenfalls durch einen Rest $R_e$ substituierte Cycloalkylengruppe mit 4 oder 5 Kohlenstoffatomen, welche zusätzlich durch 1 bis 4 Alkylgruppen substituiert sein kann und in der im Cycloalkylenteil jeweils eine $>$CH-Einheit durch ein Stickstoffatom, welches mit dem Rest A verknüpft ist, ersetzt ist, wobei

$R_e$ mit der Maßgabe, daß ein Heteroatom des Restes $R_e$ durch mindestens zwei Kohlenstoffatome von dem Ringstickstoffatom der cyclischen Iminogruppe getrennt ist, eine Alkyl-, Hydroxy-, Alkoxy-, Phenylalkoxy-, Alkylsulfenyl-, Alkylsulfinyl-, Alkylsulfonyl-, Cyano-, Carboxy-, Alkoxycarbonyl-, Phenylalkoxycarbonyl-, Alkylcarbonyl-, Aminocarbonyl-, Alkylaminocarbonyl-, Dialkylaminocarbonyl-, Amino-, Alkylamino-, Dialkylamino- oder Phenylgruppe darstellt,

eine gegebenenfalls durch einen Rest $R_e$ substituierte Cycloalkylengruppe mit 6 bis 8 Kohlenstoffatomen, welche zusätzlich durch 1 bis 4 Alkylgruppen substituiert sein kann und in denen im Cycloalkylenteil jeweils eine $>$CH-Einheit durch ein Stickstoffatom, welches mit dem Rest A verknüpft ist, ersetzt ist und zusätzlich eine weitere in 4-Stellung befindliche $>$CH-Einheit durch ein Stickstoffatom ersetzt sein kann, wobei außerdem in den vorstehend erwähnten 6- bis 8-gliedrigen Ringen jeweils eine oder

zwei zu einem Stickstoffatom benachbarte Methylengruppen jeweils durch eine Carbonylgruppe ersetzt sein können, wobei $R_e$ wie vorstehend erwähnt definiert ist,
eine gegebenenfalls durch eine oder zwei Alkylgruppen substituierte Cycloalkenylengruppe mit 5 bis 7 Kohlenstoffatomen, welche zusätzlich durch eine oder zwei Methylgruppen substituiert sein kann und in denen im Cycloalkenylenteil jeweils eine $>$CH-Einheit durch ein Stickstoffatom, welches mit dem Rest A verknüpft ist, ersetzt ist, wobei das Stickstoffatom durch mindestens eine gegebenenfalls mono- oder disubstituierte Methylengruppe von der Doppelbindung getrennt ist,
eine Cycloalkylengruppe mit 5 bis 7 Kohlenstoffatomen, in der eine $>$CH-Einheit durch ein Stickstoffatom, welches mit dem Rest A verknüpft ist, ersetzt ist und zusätzlich zwei Wasserstoffatome am Kohlenstoffgerüst durch eine geradkettige Alkylenbrücke ersetzt sind, wobei diese Brücke 2 bis 6 Kohlenstoffatome enthält, wenn die zwei Wasserstoffatome sich am selben Kohlenstoffatom befinden, oder 1 bis 5 Kohlenstoffatome enthält, wenn sie zwei Wasserstoffatome an benachbarten Kohlenstoffatomen ersetzt, oder 1 bis 4 Kohlenstoffatome enthält, wenn sie zwei Wasserstoffatome an Kohlenstoffatomen ersetzt, die durch ein weiteres Atom getrennt sind, oder 1 bis 3 Kohlenstoffatome enthält, wenn sie zwei Wasserstoffatome an Kohlenstoffatomen ersetzt, die durch zwei Atome getrennt sind, wobei die vorstehenden Gruppen jeweils durch eine oder zwei Alkylgruppen substituiert sein können, oder
eine über den Rest W mit dem Rest C verknüpfte Alkylengruppe mit 1 bis 6 Kohlenstoffatomen, wobei W ein Sauerstoff- oder Schwefelatom, eine Sulfinyl-, Sulfonyl-, $-NR_4-$, $-NR_5CO-$ oder $-CONR_5$-Gruppe darstellt, wobei

$R_4$ ein Wasserstoffatom, eine Alkyl-, Alkylcarbonyl-, Alkylsulfonyl-, Arylcarbonyl-, Heteroarylcarbonyl-, Arylsulfonyl- oder Heteroarylsulfonylgruppe und

$R_5$ ein Wasserstoffatom oder eine Alkylgruppe darstellen,

oder B zusammen mit A eine gegebenenfalls durch 1 oder 2 Alkylgruppen substituierte Pyridylgruppe oder eine Piperidinylgruppe, in der das Wasserstoffatom in 1-Stellung zusammen mit einem Wasserstoffatom in 2-Stellung durch eine geradkettige Alkylengruppe mit 3 oder 4 Kohlenstoffatomen ersetzt ist, oder eine Piperidinylgruppe, in der das Wasserstoffatom in 1-Stellung zusammen mit einem Wasserstoffatom in 3-Stellung durch eine geradkettige Alkylengruppe mit 2 bis 4 Kohlenstoffatomen ersetzt ist, oder eine Piperidinylgruppe, in der das Wasserstoffatom in 1-Stellung zusammen mit einem Wasserstoffatom in 4-Stellung durch eine geradkettige Alkylengruppe mit 1 bis 3 Kohlenstoffatomen ersetzt ist, wobei die an die 4-Stellung gebundene Methylengruppe einer Ethylenkette durch eine Hydroxymethylen- oder Carbonylgruppe ersetzt sein kann und zusätzlich das Stickstoffatom dieser vorstehend erwähnten bicyclischen Gruppen, welche im Kohlenstoffgerüst durch eine oder zwei Alkylgruppen substituiert sein können, durch Boran komplexiert oder durch eine im Phenylkern gegebenenfalls durch 1 bis 2 Methoxygruppen substituierte Benzylgruppe quarternisiert sein kann, und
C eine Alkylengruppe mit 1 bis 6 Kohlenstoffatomen, in der eine Methylengruppe durch ein Sauerstoff- oder Schwefelatom oder durch eine Sulfinyl-, Sulfonyl- oder $-NR_4$-Gruppe ersetzt sein kann, oder in der eine Ethylengruppe durch eine $-CONR_5-$ oder $-NR_5CO$-Gruppe ersetzt sein kann, wobei $R_4$ und $R_5$ wie vorstehend erwähnt definiert sind,
eine Alkenylengruppe mit 2 bis 6 Kohlenstoffatomen,
eine über die Carbonylgruppe mit dem Rest B verbundene Alkylencarbonylgruppe mit insgesamt 2 bis 7 Kohlenstoffatomen,
eine Arylengruppe,
eine Pyridinylen-, Pyrimidinylen-, Pyrazinylen- oder Pyridazinylengruppe, in denen eine oder zwei $-CH=N$-Gruppen jeweils durch eine -CO-NH-Gruppe ersetzt sein können und eines der Stickstoffatome statt an ein Wasserstoffatom auch an den Rest B gebunden sein kann, sofern dieser nicht eine Bindung bedeutet oder nicht mit einem Heteroatom oder einer Carbonylgruppe an den Rest C anschließt, wobei die vorstehend erwähnten heterocyclischen Gruppen zusätzlich durch eine oder zwei Alkylgruppen substituiert sein können,
eine gegebenenfalls durch eine oder zwei Alkylgruppen substituierte 3- bis 7-gliedrige Cycloalkylengruppe oder
eine Cyclohexylengruppe, in der zwei durch drei Bindungen getrennte Kohlenstoffatome zusätzlich durch eine geradkettige Alkylengruppe mit 1 bis 3 Kohlenstoffatomen verknüpft sind, wobei diese bicyclischen Gruppen jeweils außerdem durch ein bis zwei Alkylgruppen substituiert sein können, oder
C zusammen mit A und B eine gegebenenfalls jeweils durch eine oder zwei Alkylgruppen substituierte Pyridyl- oder 1-(4-Pyridyl)-piperidinylgruppe oder eine Piperidinylgruppe, in der das Wasserstoffatom in 1-Stellung zusammen mit einem Wasserstoffatom in 2-Stellung durch eine geradkettige Alkylengruppe mit 3 oder 4 Kohlenstoffatomen ersetzt ist, oder eine Piperidinylgruppe, in der das Wasserstoffatom in 1-Stellung zusammen mit einem Wasserstoffatom in 3-Stellung durch eine geradkettige Alkylengruppe

mit 2 bis 4 Kohlenstoffatomen ersetzt ist, oder eine Piperidinylgruppe, in der das Wasserstoffatom in 1-Stellung zusammen mit einem Wasserstoffatom in 4-Stellung durch eine geradkettige Alkylengruppe mit 1 bis 3 Kohlenstoffatomen ersetzt ist, wobei die an die 4-Stellung gebundene Methylengruppe einer Ethylenkette durch eine Hydroxymethylen- oder Carbonylgruppe ersetzt sein kann und zusätzlich das Stickstoffatom dieser vorstehend erwähnten bicyclischen Gruppen, welche im Kohlenstoffgerüst durch eine oder zwei Alkylgruppen substituiert sein können, durch Boran komplexiert oder durch eine im Phenylkern gegebenenfalls durch 1 bis 2 Methoxygruppen substituierte Benzylgruppe quarternisiert sein kann, oder

C, wenn B eine Bindung darstellt, auch

(a) eine Indanylen-, Naphthylen-, 1,2,3,4-Tetrahydronaphthylen- oder Benzosuberanylengruppe, in denen jeweils einer der Ringe an den Rest A und der andere der Ringe an den cyclischen Rest der allgemeinen Formel I gebunden ist, wobei die gesättigten Ringe jeweils durch 1 oder 2 Alkylgruppen substituiert sein können und die aromatischen Ringe jeweils durch ein Fluor-, Chlor-, Brom- oder Jodatom, durch eine Alkyl-, Trifluormethyl-, Hydroxy-, Alkoxy-, Alkylsulfenyl-, Alkylsulfinyl-, Alkylsulfonyl- oder Cyanogruppe substituiert sein können und in denen zusätzlich eine oder zwei Methingruppen jeweils durch ein Stickstoffatom ersetzt sein können,

(b) eine gegebenenfalls durch den Rest $R_e$ substituierte Cycloalkylengruppe mit 4 oder 5 Kohlenstoffatomen, welche zusätzlich durch 1 bis 4 Alkylgruppen substituiert sein kann und in denen im Cycloalkylenteil jeweils eine >CH-Einheit durch ein Stickstoffatom, welches mit dem Rest A verknüpft ist, ersetzt ist, wobei $R_e$ wie vorstehend erwähnt definiert ist,

(c) eine gegebenenfalls durch den Rest $R_e$ substituierte Cycloalkylengruppe mit 6 bis 8 Kohlenstoffatomen, welche zusätzlich durch 1 bis 4 Alkylgruppen substituiert sein kann und in denen im Cycloalkylenteil jeweils eine >CH-Einheit durch ein Stickstoffatom, welches mit dem Rest A verknüpft ist, ersetzt ist und zusätzlich eine weitere in 4-Stellung befindliche >CH-Einheit durch ein Stickstoffatom ersetzt sein kann, wobei außerdem in den vorstehend erwähnten 6- bis 8-gliedrigen Ringen jeweils eine oder zwei zu einem Stickstoffatom benachbarte Methylengruppen jeweils durch eine Carbonylgruppe ersetzt sein können, wobei $R_e$ wie vorstehend erwähnt definiert ist,

(d) eine gegebenenfalls durch eine oder zwei Alkylgruppen substituierte Cycloalkenylengruppe mit 5 bis 7 Kohlenstoffatomen, welche zusätzlich durch eine oder zwei Methylgruppen substituiert sein kann und in denen im Cycloalkenylenteil jeweils eine >CH-Einheit durch ein Stickstoffatom, welches mit dem Rest A verknüpft ist, ersetzt ist, wobei das Stickstoffatom durch mindestens eine gegebenenfalls mono- oder disubstituierte Methylengruppe von der Doppelbindung getrennt ist, oder

e) eine Cycloalkylengruppe mit 5 bis 7 Kohlenstoffatomen, in der eine >CH-Einheit durch ein Stickstoffatom, welches mit dem Rest A verknüpft ist, ersetzt ist und zusätzlich zwei Wasserstoffatome am Kohlenstoffgerüst durch eine geradkettige Alkylenbrücke ersetzt sind, wobei diese Brücke 2 bis 6 Kohlenstoffatome enthält, wenn die zwei Wasserstoffatome sich am selben Kohlenstoffatom befinden, oder 1 bis 5 Kohlenstoffatome enthält, wenn sie zwei Wasserstoffatome an benachbarten Kohlenstoffatomen ersetzt, oder 1 bis 4 Kohlenstoffatome enthält, wenn sie zwei Wasserstoffatome an Kohlenstoffatomen ersetzt, die durch ein weiteres Atom getrennt sind, oder 1 bis 3 Kohlenstoffatome enthält, wenn sie zwei Wasserstoffatome an Kohlenstoffatomen ersetzt, die durch zwei Atome getrennt sind, wobei die vorstehenden Gruppen jeweils durch eine oder zwei Alkylgruppen substituiert sein können, darstellen,

der zweite der Reste $R_a$ bis $R_d$ eine Gruppe der Formel

F - E - D -,

in der

D eine Alkylengruppe mit 1 bis 6 Kohlenstoffatomen, in der eine Methylengruppe durch ein Sauerstoff- oder Schwefelatom, durch eine Sulfinyl-, Sulfonyl- oder $-NR_4$-Gruppe ersetzt sein kann, oder in der eine Ethylengruppe durch eine $-CO-NR_5-$ oder $-NR_5-CO$-Gruppe ersetzt sein kann, wobei $R_4$ und $R_5$ wie vorstehend erwähnt definiert sind,

eine Alkenylengruppe mit 2 bis 6 Kohlenstoffatomen,

eine Arylengruppe,

eine Pyridinylen-, Pyrimidinylen-, Pyrazinylen- oder Pyridazinylengruppe, in denen eine oder zwei -CH = N-Gruppen jeweils durch eine -CO-NH-Gruppe ersetzt sein können und eines der Stickstoffatome statt an ein Wasserstoffatom auch an den Rest E gebunden sein kann, sofern dieser nicht eine Bindung bedeutet oder nicht mit einem Heteroatom oder einer Carbonylgruppe an den Rest D anschließt, wobei die vorstehend erwähnten heterocyclischen Gruppen zusätzlich durch eine oder zwei Alkylgruppen

substituiert sein können,
eine Indanylen-, Naphthylen-, 1,2,3,4-Tetrahydronaphthylen- oder Benzosuberanylengruppe, in denen jeweils einer der Ringe an den Rest E und der andere der Ringe an den cyclischen Rest der allgemeinen Formel I gebunden ist, wobei die gesättigten Ringe jeweils durch 1 oder 2 Alkylgruppen substituiert sein können und die aromatischen Ringe jeweils durch ein Fluor-, Chlor-, Brom- oder Jodatom, durch eine Alkyl-, Trifluormethyl-, Hydroxy-, Alkoxy-, Alkylsulfenyl-, Alkylsulfinyl-, Alkylsulfonyl- oder Cyanogruppe substituiert sein können und in denen zusätzlich eine oder zwei Methingruppen jeweils durch ein Stickstoffatom ersetzt sein können,
eine gegebenenfalls durch eine oder zwei Alkylgruppen substituierte Cycloalkylengruppe mit 3 bis 7 Kohlenstoffatomen,
eine gegebenenfalls durch eine oder zwei Alkylgruppen substituierte Cycloalkylengruppe mit 4 bis 7 Kohlenstoffatomen, in der eine >CH-Einheit durch ein Stickstoffatom ersetzt ist, wobei außerdem in den vorstehend erwähnten 4- bis 7-gliedrigen Ringen eine und, ab einer Ringgröße von 5, jeweils eine oder zwei zu einem Stickstoffatom benachbarte Methylengruppen jeweils durch eine Carbonylgruppe ersetzt sein können,
eine gegebenenfalls durch eine oder zwei Alkylgruppen substituierte Cycloalkylengruppe mit 6 oder 7 Kohlenstoffatomen, in der zwei zueinander in 1,4-Stellung befindliche >CH-Einheiten jeweils durch ein Stickstoffatom ersetzt sind, wobei außerdem in den vorstehend erwähnten 6- oder 7-gliedrigen Ringen jeweils eine oder zwei zu einem Stickstoffatom benachbarte Methylengruppen jeweils durch eine Carbonylgruppe ersetzt sein können,
eine Cyclohexylengruppe, in der zwei durch drei Bindungen getrennte Kohlenstoffatome zusätzlich durch eine geradkettige Alkylengruppe mit 1 bis 3 Kohlenstoffatomen verknüpft sind, wobei diese bicyclischen Gruppen jeweils außerdem durch ein bis zwei Alkylgruppen substituiert sein können,
oder, wenn E eine cyclische Iminogruppe darstellt, eine Alkylencarbonylgruppe mit insgesamt 2 bis 6 Kohlenstoffatomen, wobei die Carbonylgruppe jeweils an das Stickstoffatom der cyclischen Iminogruppe der Gruppe E gebunden ist,
oder auch, falls E keine Bindung darstellt, eine Bindung,
E eine Bindung,
eine Alkylengruppe mit 1 bis 6 Kohlenstoffatomen oder eine Alkenylengruppe mit 2 bis 6 Kohlenstoffatomen, die jeweils durch eine oder zwei Alkylgruppen mit jeweils 1 bis 8 Kohlenstoffatomen, durch eine Hydroxy-, Amino-, Aryl- oder Heteroarylgruppe, durch eine Alkoxy- oder Alkylaminogruppe mit jeweils 1 bis 8 Kohlenstoffatomen, durch eine Dialkylaminogruppe mit insgesamt 2 bis 10 Kohlenstoffatomen, durch eine $HNR_6$- oder N-Phenylalkyl-$NR_6$-Gruppe oder durch eine N-Alkyl-$NR_6$-Gruppe mit 1 bis 8 Kohlenstoffatomen im Alkylteil substituiert sein können, wobei
$R_6$ eine Alkylcarbonyl- oder Alkylsulfonylgruppe mit jeweils 1 bis 8 Kohlenstoffatomen im Alkylteil, eine Alkyloxycarbonylgruppe mit insgesamt 2 bis 5 Kohlenstoffatomen, eine Cycloalkylcarbonyl- oder Cycloalkylsulfonylgruppe mit jeweils 5 bis 7 Kohlenstoffatomen im Cycloalkylteil, eine Arylalkylcarbonyl-, Arylalkylsulfonyl-, Arylalkoxycarbonyl-, Arylcarbonyl-, Arylsulfonyl-, Heteroarylalkylcarbonyl-, Heteroarylalkylsulfonyl-, Heteroarylalkoxycarbonyl-, Heteroarylcarbonyl- oder Heteroarylsulfonylgruppe darstellt,
eine Arylengruppe,
eine Pyridinylen-, Pyrimidinylen-, Pyrazinylen- oder Pyridazinylengruppe, in denen eine oder zwei -CH=N-Gruppen jeweils durch eine -CO-NH-Gruppe ersetzt sein können und eines der Stickstoffatome statt an ein Wasserstoffatom auch an den Rest D gebunden sein kann, sofern dieser nicht eine Bindung bedeutet oder nicht mit einem Heteroatom oder einer Carbonylgruppe an den Rest E anschließt, wobei die vorstehend erwähnten heterocyclischen Gruppen zusätzlich durch eine oder zwei Alkylgruppen substituiert sein können,
eine gegebenenfalls durch eine oder zwei Alkylgruppen substituierte Cycloalkylengruppe mit 5 bis 7 Kohlenstoffatomen, in der eine >CH-Einheit durch ein Stickstoffatom, welches mit einem Kohlenstoffatom des Restes D verknüpft ist, ersetzt ist,
eine gegebenenfalls durch eine oder zwei Alkylgruppen mit jeweils 1 bis 8 Kohlenstoffatomen, durch eine Hydroxy-, Amino-, Aryl- oder Heteroarylgruppe, durch eine Alkoxy- oder Alkylaminogruppe mit jeweils 1 bis 8 Kohlenstoffatomen, durch eine Dialkylaminogruppe mit insgesamt 2 bis 10 Kohlenstoffatomen, durch eine $HNR_6$- oder N-Phenylalkyl-$NR_6$-Gruppe oder durch eine N-Alkyl-$NR_6$-Gruppe mit 1 bis 8 Kohlenstoffatomen im Alkylteil substituierte Cycloalkylengruppe mit 4 bis 7 Kohlenstoffatomen im Cycloalkylenteil, wobei $R_6$ wie vorstehend definiert ist,
oder auch, falls D keine Bindung darstellt, eine über den Rest W mit dem Rest D verknüpfte Alkylengruppe, in der W wie eingangs erwähnt definiert ist und die Alkylengruppe zusätzlich durch eine

oder zwei Alkylgruppen mit jeweils 1 bis 6 Kohlenstoffatomen, durch eine Hydroxy-, Amino-, Aryl- oder Heteroarylgruppe, durch eine Alkoxy- oder Alkylaminogruppe mit jeweils 1 bis 8 Kohlenstoffatomen, durch eine Dialkylaminogruppe mit insgesamt 2 bis 10 Kohlenstoffatomen, durch eine $HNR_6$- oder N-Phenylalkyl-$NR_6$-Gruppe oder durch eine N-Alkyl-$NR_6$-Gruppe mit 1 bis 8 Kohlenstoffatomen im Alkylteil substituiert sein kann, wobei das Heteroatom des zusätzlichen Substituenten durch mindestens 2 Kohlenstoffatome von einem Heteroatom des Restes W getrennt ist und $R_6$ wie vorstehend definiert ist, sowie mit der Maßgabe, daß D zusammen mit E keine $-(CH_2)_n-CONH-CH_2CH_2$-Gruppe darstellt, wobei

n die Zahl 1,2,3 oder 4 bedeutet und der mit dem Stickstoffatom verbundene Ethylenteil gegebenenfalls wie vorstehend erwähnt substituiert sein kann, und

F eine Carbonylgruppe, die durch eine Hydroxygruppe, durch eine Alkoxygruppe mit 1 bis 8 Kohlenstoffatomen, durch eine Arylalkoxygruppe oder durch eine $R_7O$-Gruppe substituiert ist, wobei

$R_7$ eine Cycloalkylgruppe mit 4 bis 8 Kohlenstoffatomen oder eine Cycloalkylalkylgruppe mit 3 bis 8 Kohlenstoffatomen im Cycloalkylteil, in denen jeweils die Cycloalkylgruppe durch eine Alkylgruppe, durch eine Alkylgruppe und durch 1 bis 3 Methylgruppen, durch eine Alkoxy- oder Dialkylaminogruppe substituiert und zusätzlich eine Methylengruppe in einem 4 bis 8 gliedrigen Cycloalkylteil durch ein Sauerstoff- oder Schwefelatom, durch eine Sulfinyl-, Sulfonyl- oder Alkyliminogruppe ersetzt sein kann, eine Bicycloalkyl- oder Bicycloalkylalkylgruppe, in denen jeweils der Bicycloalkylteil jeweils 6 bis 10 Kohlenstoffatome enthält und zusätzlich durch 1 bis 3 Methylgruppen substituiert sein kann, eine Benzocycloalkylgruppe mit 9 bis 12 Kohlenstoffatomen oder eine Arylgruppe darstellt,

eine Phosphono-, O-Alkyl-phosphono-, O,O'-Dialkyl-phosphono-, Tetrazol-5-yl- oder $R_8CO-O-CHR_9-O-CO$-Gruppe darstellen, wobei

$R_8$ eine Alkyl- oder Alkoxygruppe mit jeweils 1 bis 8 Kohlenstoffatomen, eine Cycloalkyl- oder Cycloalkoxygruppe mit jeweils 5 bis 7 Kohlenstoffatomen im Cycloalkylteil, eine Aryl-, Aryloxy-, Arylalkyl- oder Arylalkoxygruppe und

$R_9$ ein Wasserstoffatom, eine Cycloalkylgruppe mit 5 bis 7 Kohlenstoffatomen, eine Alkyl- oder Arylgruppe darstellen,

und der kürzeste Abstand zwischen dem Rest F und dem von dem Rest F am weitesten entfernten Stickstoffatom der Gruppe A-B-C- mindestens 11 Bindungen beträgt,

der dritte der Reste $R_a$ bis $R_d$ ein Wasserstoffatom, eine Alkyl-, Perfluoralkyl-, Alkoxy-, Alkylsulfenyl-, Alkylsulfinyl-, Alkylsulfonyl-, Amino-, Alkylamino-, Dialkylamino-, Aryl-, Arylalkyl-, Heteroaryl- oder Heteroarylalkylgruppe und der vierte der Reste $R_a$ bis $R_d$ ein Wasserstoffatom oder eine Alkyl-, Aryl- oder Arylalkylgruppe bedeuten,

wobei, soweit nichts anderes erwähnt wurde,

unter den bei der Definition der vorstehenden Reste erwähnten Arylteilen eine Phenylgruppe, die jeweils durch $R_{10}$ monosubstituiert, durch $R_{11}$ mono-, di- oder trisubstituiert oder durch $R_{10}$ monosubstituiert und zusätzlich durch $R_{11}$ mono- oder disubstituiert sein kann, wobei die Substituenten gleich oder verschieden sein können und

$R_{10}$ eine Cyano-, Carboxy-, Aminocarbonyl-, Alkylaminocarbonyl-, Dialkylaminocarbonyl-, Alkoxycarbonyl-, Alkylcarbonyl-, Alkylsulfenyl-, Alkylsulfinyl-, Alkylsulfonyl-, Alkylsulfonyloxy-, Perfluoralkyl-, Trifluormethoxy-, Nitro-, Amino-, Alkylamino-, Dialkylamino-, Alkylcarbonylamino-, Phenylalkylcarbonylamino-, Phenylcarbonylamino-, Alkylsulfonylamino-, Phenylalkylsulfonylamino-, Phenylsulfonylamino-, N-Alkyl-alkylcarbonylamino-, N-Alkyl-phenylalkylcarbonylamino-, N-Alkyl-phenylcarbonylamino-, N-Alkyl-alkylsulfonylamino-, N-Alkyl-phenylalkylsulfonylamino-, N-Alkyl-phenylsulfonylamino-, Aminosulfonyl-, Alkylaminosulfonyl- oder Dialkylaminosulfonylgruppe und

$R_{11}$ eine Alkyl-, Hydroxy- oder Alkoxygruppe, ein Fluor-, Chlor-, Brom- oder Jodatom darstellen, wobei zwei Reste $R_{11}$ sofern diese an benachbarte Kohlenstoffatome gebunden sind, auch eine Alkylengruppe mit 3 bis 6 Kohlenstoffatomen, eine 1,3-Butadien-1,4-diylengruppe oder eine Methylendioxygruppe darstellen können,

unter den bei der Definition der vorstehenden Resten erwähnten Arylenteilen eine Phenylengruppe die jeweils durch $R_{10}$ monosubstituiert, durch $R_{11}$ mono- oder disubstituiert oder durch $R_{10}$ monosubstituiert und zusätzlich durch $R_{11}$ monosubstituiert sein kann, wobei die Substituenten gleich oder verschieden sein können und wie vorstehend erwähnt definiert sind,

unter den bei der Definition der vorstehenden Reste erwähnten Heteroarylteilen ein 5-gliedriger heteroaromatischer Ring, welcher ein Sauerstoff-, Schwefel- oder Stickstoffatom, ein Stickstoffatom und ein Sauerstoff-, Schwefel- oder Stickstoffatom oder zwei Stickstoffatome und ein Sauerstoff-, Schwefel- oder Stickstoffatom enthält, oder ein 6-gliedriger heteroaromatischer Ring, welcher 1, 2 oder 3 Stickstoffatome enthält und in dem zusätzlich eine oder zwei -CH=N-Gruppen jeweils durch eine -CO-

$NR_5$-Gruppe ersetzt sein können, wobei $R_5$ wie vorstehend erwähnt definiert ist, und zusätzlich die vorstehend erwähnten heteroaromatischen Ringe durch eine oder zwei Alkylgruppen oder am Kohlenstoffgerüst auch durch ein Fluor-, Chlor-, Brom- oder Jodatom, durch eine Hydroxy- oder Alkoxygruppe substituiert sein können,
zu verstehen ist, sowie, soweit nichts anderes erwähnt wurde, die vorstehend erwähnten Alkyl-, Alkylen- oder Alkoxyteile jeweils 1 bis 4 Kohlenstoffatome enthalten können und jedes Kohlenstoffatom in den vorstehend erwähnten Alkylen- und Cycloalkylenteilen höchstens mit einem Heteroatom verknüpft ist, deren Tautomere, deren Stereoisomere und deren Salze.

**2.** Cyclische Harnstoffderivate der allgemeinen Formel I gemäß Anspruch 1 mit Ausnahme von
1-[4-(4-Amidino-phenyl)-cyclohexyl]-3-(2-carboxy-ethyl)-imidazolidin-2-on,
1-[4-(4-Amidino-phenyl)-cyclohexyl]-3-(2-methoxycarbonyl-ethyl)-imidazolidin-2-on,
1-[4-(4-Amidino-phenyl)-cyclohexyl]-3-(2-methoxycarbonyl-ethyl)-3H-imidazol-2-on,
1-[4-(4-Amidino-phenyl)-cyclohexyl]-3-(2-carboxy-ethyl)-3H-imidazol-2-on,
1-[4-(4-Cyano-phenyl)-cyclohexyl]-3-(2-ethoxycarbonyl-ethyl)-3H-imidazol-2-on,
1-(4-Amidino-phenyl)-3-[4-(2-carboxy-ethyl)-cyclohexyl]-imidazolidin-2-on,
1-(4-Amidino-phenyl)-3-[4-(2-methoxycarbonyl-ethyl)-cyclohexyl]-imidazolidin-2-on,
1-(4-Cyano-phenyl)-3-[4-(2-methoxycarbonyl-ethyl)-cyclohexyl]-imidazolidin-2-on,
1-(4-Amidino-phenyl)-3-[4-(2-phosphono-ethyl)-phenyl]-imidazolidin-2-on,
1-(4-Amidino-phenyl)-3-[4-[2-(O-methyl-phosphono)-ethyl]-phenyl]-imidazolidin-2-on,
1-(4-Cyano-phenyl)-3-[4-(2-phosphono-ethyl)-phenyl]-imidazolidin-2-on,
1-(4-Cyano-phenyl)-3-[4-[2-(0-methyl-phosphono)-ethyl]-phenyl]-imidazolidin-2-on,
1-(1-Amidino-4-piperidinyl)-3-[4-(2-carboxy-ethyl)-phenyl]-imidazolidin-2-on,
1-(1-Amidino-4-piperidinyl)-3-[4-(2-methoxycarbonyl-ethyl)-phenyl]-imidazolidin-2-on,
1-(4-Aminomethyl-cyclohexyl)-3-[4-(2-carboxy-ethyl)-phenyl]-imidazolidin-2-on,
1-(4-Aminomethyl-cyclohexyl)-3-[4-(2-methoxycarbonyl-ethyl)-phenyl]-imidazolidin-2-on,
1-(4-Amidino-phenyl)-3-[4-(2-carboxy-ethyl)-phenyl]-4-phenyl-3H-imidazol-2-on,
1-(4-Amidino-phenyl)-3-[4-(2-methoxycarbonyl-ethyl)-phenyl]-4-phenyl-3H-imidazol-2-on,
1-(4-Amidino-phenyl)-3-[4-(2-carboxy-ethyl)-phenyl]-4-phenyl-imidazolidin-2-on,
1-(4-Amidino-phenyl)-3-[4-(2-methoxycarbonyl-ethyl)-phenyl]-4-phenyl-imidazolidin-2-on,
4-(4-Amidino-phenyl)-1-[4-(2-carboxy-ethyl)-phenyl]-3-phenyl-3H-imidazol-2-on,
4-(4-Amidino-phenyl)-1-[4-(2-methoxycarbonyl-ethyl)-phenyl]-3-phenyl-3H-imidazol-2-on,
4-(4-Cyano-phenyl)-1-[4-(2-methoxycarbonyl-ethyl)-phenyl]-3-phenyl-3H-imidazol-2-on,
4-(4-Amidino-phenyl)-1-[4-(2-carboxy-ethyl)-phenyl]-3-phenyl-3H-imidazol-2-thion,
4-(4-Amidino-phenyl)-1-[4-(2-methoxycarbonyl-ethyl)-phenyl]-3-phenyl-3H-imidazol-2-thion,
4-(4-Cyano-phenyl)-1-[4-(2-methoxycarbonyl-ethyl)-phenyl]-3-phenyl-3H-imidazol-2-thion,
4-(4-Amidino-phenyl)-1-[4-(2-carboxy-ethyl)-phenyl]-5-methyl-3-phenyl-3H-imidazo1-2-on,
4-(4-Amidino-phenyl)-1-[4-(2-methoxycarbonyl-ethyl)-phenyl]-5-methyl-3-phenyl-3H-imidazol-2-on,
4-(4-Cyano-phenyl)-1-[4-(2-methoxycarbonyl-ethyl)-phenyl]-5-methyl-3-phenyl-3H-imidazol-2-on,
4-(4-Amidino-phenyl)-1-[4-(2-carboxy-ethyl)-phenyl]-3-phenyl-imidazolidin-2-on,
4-(4-Amidino-phenyl)-1-[4-(2-methoxycarbonyl-ethyl)-phenyl]-3-phenyl-imidazolidin-2-on,
1-(4-Amidino-phenyl)-4-[4-(2-carboxy-ethyl)-phenyl]-3-phenyl-3H-imidazol-2-on,
1-(4-Amidino-phenyl)-4-[4-(2-methoxycarbonyl-ethyl)-phenyl]-3-phenyl-3H-imidazol-2-on,
1-(4-Cyano-phenyl)-4-[4-(2-methoxycarbonyl-ethyl)-phenyl]-3-phenyl-3H-imidazol-2-on,
1-(4-Amidino-phenyl)-4-[4-(2-carboxy-ethyl)-phenyl]-3-phenyl-imidazolidin-2-on,
1-(4-Amidino-phenyl)-4-[4-(2-methoxycarbonyl-ethyl)-phenyl]-3-phenyl-imidazolidin-2-on,
1-(4-Amidino-phenyl)-4-[4-(2-carboxy-ethyl)-phenyl]-3-phenyl-3H-imidazol-2-thion,
1-(4-Amidino-phenyl)-4-[4-(2-methoxycarbonyl-ethyl)-phenyl]-3-phenyl-3H-imidazol-2-thion,
1-(4-Cyano-phenyl)-4-[4-(2-methoxycarbonyl-ethyl)-phenyl]-3-phenyl-3H-imidazol-2-thion,
1-(4-Amidino-phenyl)-4-[4-(2-carboxy-ethyl)-phenyl]-5-methyl-3-phenyl-3H-imidazol-2-on,
1-(4-Amidino-phenyl)-4-[4-(2-methoxycarbonyl-ethyl)-phenyl]-5-methyl-3-phenyl-3H-imidazol-2-on,
1-(4-Cyano-phenyl)-4-[4-(2-methoxycarbonyl-ethyl)-phenyl]-5-methyl-3-phenyl-3H-imidazol-2-on,
4-(4'-Amidino-4-biphenylyl)-1-(2-carboxy-ethyl)-3-phenyl-3H-imidazol-2-on,
4-(4'-Amidino-4-biphenylyl)-1-(2-methoxycarbonyl-ethyl)-3-phenyl-3H-imidazol-2-on,
4-(4'-Cyano-4-biphenylyl)-1-(2-methoxycarbonyl-ethyl)-3-phenyl-3H-imidazol-2-on,
4-[4'-Amidino-4-biphenylyl)-1-(2-carboxy-ethyl)-3-phenyl-imidazolidin-2-on,
4-(4'-Amidino-4-biphenylyl)-1-(2-methoxycarbonyl-ethyl)-3-phenyl-imidazolidin-2-on,
4-(4'-Amidino-4-biphenylyl)-1-(2-carboxy-ethyl)-3-phenyl-3H-imidazol-2-thion,

4-(4'-Amidino-4-biphenylyl)-1-(2-methoxycarbonyl-ethyl)-3-phenyl-3H-imidazol-2-thion,
4-(4'-Cyano-4-biphenylyl)-1-(2-methoxycarbonyl-ethyl)-3-phenyl-3H-imidazol-2-thion,
4-(4'-Amidino-4-biphenylyl)-1-(2-carboxy-ethyl)-5-methyl-3-phenyl-3H-imidazol-2-on,
4-(4'-Amidino-4-biphenylyl)-1-(2-methoxycarbonyl-ethyl)-5-methyl-3-phenyl-3H-imidazol-2-on,
4-(4'-Cyano-4-biphenylyl)-1-(2-methoxycarbonyl-ethyl)-5-methyl-3-phenyl-3H-imidazol-2-on,
1-(4-Amidino-phenyl)-3-[4-(2-butyloxycarbonyl-ethyl)-phenyl]-imidazolidin-2-on,
1-(4'-Amidino-4-biphenylyl)-3-(2-ethoxycarbonyl-ethyl)-3H-imidazol-2-on,
1-(4'-Cyano-4-biphenylyl)-3-(2-ethoxycarbonyl-ethyl)-3H-imidazol-2-on,
1-(4'-Amidino-4-biphenylyl)-3-(2-ethoxycarbonyl-ethyl)-imidazolidin-2,4-dion,
1-(4'-Cyano-4-biphenylyl)-3-(2-ethoxycarbonyl-ethyl)-imidazolidin-2,4-dion,
1-(4'-Amidino-4-biphenylyl)-3-(2-ethoxycarbonyl-ethyl)-3,4,5,6-tetrahydro-1H-pyrimidin-2-on,
1-(4'-Cyano-4-biphenylyl)-3-(2-ethoxycarbonyl-ethyl)-3,4,5,6-tetrahydro-1H-pyrimidin-2-on,
2-(4-Amidino-phenyl)-4-[4-(2-isopropyloxycarbonyl-ethyl)-phenyl]-5-methyl-4H-1,2,4-triazol-3-on,
4-[4-(2-Isopropyloxycarbonyl-ethyl)-phenyl]-2-(4-methoxycarbonylamidino-phenyl)-5-methyl-4H-1,2,4-triazol-3-on,
1-(4-Amidino-phenyl)-3-[4-(2-ethoxycarbonyl-ethyl)-phenyl]-3H-imidazol-2-on,
1-(4-Ethoxycarbonylamidino-phenyl)-3-[4-(2-ethoxycarbonyl-ethyl)-phenyl]-3H-imidazol-2-on,
1-(4-Ethoxycarbonylamidino-phenyl)-3-[4-(2-ethoxycarbonyl-ethyl)-phenyl]-imidazolidin-2-on,
1-(4-Methoxycarbonylamidino-phenyl)-3-[4-(2-methoxycarbonyl-ethyl)-cyclohexyl]-imidazolidin-2-on,
4-[4-(2-Isobutyloxycarbonyl-ethyl)-phenyl]-2-(4-methoxycarbonylamidino-phenyl)-5-methyl-4H-1,2,4-triazol-3-on,
2-(4-Amidino-phenyl)-4-[4-(2-isobutyloxycarbonyl-ethyl)-phenyl]-5-methyl-4H-1,2,4-triazol-3-on,
1-(1-Amino-5-indanyl)-3-[4-(2-carboxy-ethyl)-phenyl]-imidazolidin-2-on,
1-(1-Amino-5-indanyl)-3-[4-(2-methoxycarbonyl-ethyl)-phenyl]-imidazolidin-2-on,
1-(1-Amino-1,2,3,4-tetrahydro-6-naphthyl)-3-[4-(2-carboxy-ethyl)-phenyl]-imidazolidin-2-on,
1-(1-Amino-1,2,3,4-tetrahydro-6-naphthyl)-3-[4-(2-methoxycarbonyl-ethyl)-phenyl]-imidazolidin-2-on,
1-(4-Amino-cyclohexyl)-3-[4-(3-carboxy-propyl)-phenyl]-imidazolidin-2-on,
1-(4-Amino-cyclohexyl)-3-[4-(3-methoxycarbonyl-propyl)-phenyl]-imidazolidin-2-on,
1-(4-Amino-cyclohexyl)-3-[4-(2-carboxy-ethyl)-phenyl]-imidazolidin-2-on,
1-(4-Amino-cyclohexyl)-3-[4-(2-methoxycarbonyl-ethyl)-phenyl]-imidazolidin-2-on,
1-(4-Aminomethyl-phenyl)-3-[3-(2-carboxy-ethyl)-phenyl]-imidazolidin-2-on,
1-(4-Aminomethyl-phenyl)-3-[3-(2-methoxycarbonyl-ethyl)-phenyl]-imidazolidin-2-on,
1-(4-Amino-cyclohexyl)-3-[4-[(2-carboxy-ethyl)-aminocarbonyl]-phenyl]-imidazolidin-2-on,
1-(4-Amino-cyclohexyl)-3-[4-[(2-methoxycarbonyl-ethyl)-aminocarbonyl]-phenyl]-imidazolidin-2-on,
1-[4-[(2-Carboxy-ethyl)-aminocarbonyl]-phenyl]-3-(4-piperidinyl)-imidazolidin-2-on,
1-[4-[(2-Methoxycarbonyl-ethyl)-aminocarbonyl]-phenyl]-3-(4-piperidinyl)-imidazolidin-2-on,
1-[4-(1-Amino-cyclopropyl)-phenyl]-3-[4-(2-carboxy-ethyl)-phenyl]-imidazolidin-2-on,
1-[4-(1-Amino-cyclopropyl)-phenyl]-3-[4-(2-methoxycarbonyl-ethyl)-phenyl]-imidazolidin-2-on,
1-[4-(1-Amino-cyclopentyl)-phenyl]-3-[4-(2-carboxy-ethyl)-phenyl]-imidazolidin-2-on,
1-[4-(1-Amino-cyclopentyl)-phenyl]-3-[4-[(2-methoxycarbonyl-ethyl)-phenyl]-imidazolidin-2-on,
2-(4-Amidino-phenyl)-5-ethyl-4-[4-(2-isopropyloxycarbonyl-ethyl)-phenyl]-4H-1,2,4-triazol-3-on,
1-(4-Amidino-phenyl)-3-[4-(2-isopropyloxycarbonyl-ethyl)-phenyl]-4-methyl-3H-imidazol-2-on,
4-(4-Amidino-phenyl)-2-[4-(2-isopropyloxycarbonyl-ethyl)-phenyl]-4H-1,2,4-triazol-3-on,
1-(4-Amidino-phenyl)-3-[4-(2-isopropyloxycarbonyl-ethyl)-phenyl]-imidazolidin-2-on,
1-(4-Amidino-phenyl)-3-[4-(2-isopropyloxycarbonyl-ethyl)-phenyl]-3H-imidazol-2-on,
1-(4-Amidino-phenyl)-3-[4-(2-isopropyloxycarbonyl-ethyl)-phenyl]-imidazolidin-2,4-dion,
1-[4-[(2-Carboxy-ethyl)-aminocarbonyl]-phenyl]-3-(1-methyl-4-piperidinyl)-imidazolidin-2-on,
2-(4-Amidino-phenyl)-4-[4-(2-phosphono-ethyl)-phenyl]-5-methyl-4H-1,2,4-triazol-3-on,
2-(4-Amidino-phenyl)-4-[4-[2-(0-methyl-phosphono)-ethyl]-phenyl]-5-methyl-4H-1,2,4-triazol-3-on,
2-(4-Amidino-phenyl)-4-[4-(2-carboxy-ethyl)-phenyl]-5-phenyl-4H-1,2,4-triazol-3-on,
2-(4-Amidino-phenyl)-4-[4-(2-methoxycarbonyl-ethyl)-phenyl]-5-phenyl-4H-1,2,4-triazol-3-on,
3-(4-Amidino-phenyl)-1-[4-(2-carboxy-ethyl)-phenyl]-4-trifluormethyl-3H-imidazol-2-on,
3-(4-Amidino-phenyl)-1-[4-(2-methoxycarbonyl-ethyl)-phenyl]-4-trifluormethyl-3H-imidazol-2-on,
2-(4-Amidino-phenyl)-4-[4-(2-carboxy-ethyl)-phenyl]-5-trifluormethyl-4H-1,2,4-triazol-3-on,
2-(4-Amidino-phenyl)-4-[4-(2-methoxycarbonyl-ethyl)-phenyl]-5-trifluormethyl-4H-1,2,4-triazol-3-on und
3-(4-Cyano-phenyl)-1-(4-ethoxycarbonyl-butyl)-3H-imidazo-[4,5-b]pyridin-2-on
sowie mit den Maßgaben, daß

(i) X eine am Stickstoffatom durch eine Cyanogruppe substituierte Carbiminogruppe,

(ii) Y eine gegebenenfalls durch $R_c$ oder $R_d$ oder $R_c$ und $R_d$ substituierte 1,2-Cycloalkylengruppe mit 5 bis 7 Kohlenstoffatomen,

(iii) A eine Aminoalkylgruppe mit 1 bis 5 Kohlenstoffatomen, eine Amino-, Amidino- oder Guanidinogruppe, wobei in jeder der vorstehend erwähnten Gruppen jeweils an einem der Stickstoffatome ein Wasserstoffatom durch eine $R_1$-CO-O-($R_2CR_3$)-O-CO-Gruppe ersetzt ist,

(iv) B eine gegebenenfalls durch eine oder zwei Alkylgruppen substituierte Cycloalkylengruppe mit 3 bis 7 Kohlenstoffatomen,

eine gegebenenfalls durch einen Rest $R_e$ substituierte Cycloalkylengruppe mit 4 oder 5 Kohlenstoffatomen, welche zusätzlich durch 1 bis 4 Alkylgruppen substituiert sein kann und in der im Cycloalkylenteil jeweils eine $>$CH-Einheit durch ein Stickstoffatom ersetzt ist,

eine gegebenenfalls durch einen Rest $R_e$ substituierte Cycloalkylengruppe mit 6 bis 8 Kohlenstoffatomen, welche zusätzlich durch 1 bis 4 Alkylgruppen substituiert sein kann und in der im Cycloalkylenteil jeweils eine $>$CH-Einheit durch ein Stickstoffatom ersetzt ist und zusätzlich eine weitere in 4-Stellung befindliche $>$CH-Einheit durch ein Stickstoffatom ersetzt sein kann, wobei außerdem in den vorstehend erwähnten 6- bis 8-gliedrigen Ringen jeweils eine oder zwei zu einem Stickstoffatom benachbarte Methylengruppen jeweils durch eine Carbonylgruppe ersetzt sein können,

eine gegebenenfalls durch eine oder zwei Alkylgruppen substituierte Cycloalkenylengruppe mit 5 bis 7 Kohlenstoffatomen, welche zusätzlich durch eine oder zwei Methylgruppen substituiert sein kann und in der im Cycloalkenylenteil jeweils eine $>$CH-Einheit durch ein Stickstoffatom ersetzt ist,

eine Piperidinylengruppe, in der zwei Wasserstoffatome am Kohlenstoffgerüst durch eine geradkettige Alkylenbrücke ersetzt sind, wobei diese Brücke 2 bis 6 Kohlenstoffatome enthält, wenn die zwei Wasserstoffatome sich am selben Kohlenstoffatom befinden, oder 1 bis 5 Kohlenstoffatome enthält, wenn sie zwei Wasserstoffatome an benachbarten Kohlenstoffatomen ersetzt, oder 1 bis 4 Kohlenstoffatome enthält, wenn sie zwei Wasserstoffatome an Kohlenstoffatomen ersetzt, die durch ein weiteres Atom getrennt sind, oder 1 bis 3 Kohlenstoffatome enthält, wenn sie zwei Wasserstoffatome an Kohlenstoffatomen ersetzt, die durch zwei Atome getrennt sind, wobei die vorstehenden Gruppen jeweils durch eine oder zwei Alkylgruppen substituiert sein können,

eine über den Rest W mit dem Rest C verknüpfte Alkylengruppe oder

B zusammen mit A eine gegebenenfalls durch 1 oder 2 Alkylgruppen substituierte Pyridylgruppe oder eine Piperidinylgruppe, in der das Wasserstoffatom in 1-Stellung zusammen mit einem Wasserstoffatom in 2-Stellung durch eine geradkettige Alkylengruppe mit 3 oder 4 Kohlenstoffatomen ersetzt ist, oder eine Piperidinylgruppe, in der das Wasserstoffatom in 1-Stellung zusammen mit einem Wasserstoffatom in 3-Stellung durch eine geradkettige Alkylengruppe mit 2 bis 4 Kohlenstoffatomen ersetzt ist, oder eine Piperidinylgruppe, in der das Wasserstoffatom in 1-Stellung zusammen mit einem Wasserstoffatom in 4-Stellung durch eine geradkettige Alkylengruppe mit 1 bis 3 Kohlenstoffatomen ersetzt ist, wobei die an die 4-Stellung gebundene Methylengruppe einer Ethylenkette durch eine Hydroxymethylen- oder Carbonylgruppe ersetzt sein kann und zusätzlich das Stickstoffatom dieser vorstehend erwähnten bicyclischen Gruppen, welche im Kohlenstoffgerüst durch eine oder zwei Alkylgruppen substituiert sein können, durch Boran komplexiert oder durch eine im Phenylkern gegebenenfalls durch 1 bis 2 Methoxygruppen substituierte Benzylgruppe quarternisiert sein kann,

(v) C eine Alkylengruppe mit 1 bis 6 Kohlenstoffatomen, in der eine Methylengruppe durch ein Sauerstoff- oder Schwefelatom oder durch eine Sulfinyl-, Sulfonyl- oder -$NR_4$-Gruppe ersetzt ist oder in der eine Ethylengruppe durch eine -$CONR_5$- oder -$NR_5CO$-Gruppe ersetzt ist,

eine gegebenenfalls durch eine oder zwei Alkylgruppen substituierte 5- bis 7-gliedrige Cycloalkylengruppe,

eine Cyclohexylengruppe, in der zwei durch drei Bindungen getrennte Kohlenstoffatome zusätzlich durch eine geradkettige Alkylengruppe mit 1 bis 3 Kohlenstoffatomen verknüpft sind, wobei diese bicyclischen Gruppen jeweils außerdem durch ein bis zwei Alkylgruppen substituiert sein können,

eine Indanylen-, Naphthylen-, 1,2,3,4-Tetrahydronaphthylen- oder Benzosuberanylengruppe, wobei die gesättigten Ringe jeweils durch 1 oder 2 Alkylgruppen substituiert sein können und die aromatischen Ringe jeweils durch ein Fluor-, Chlor-, Brom- oder Jodatom, durch eine Alkyl-, Trifluormethyl-, Hydroxy-, Alkoxy-, Alkylsulfenyl-, Alkylsulfinyl-, Alkylsulfonyl- oder Cyanogruppe substituiert sein können,

eine gegebenenfalls durch den Rest $R_e$ substituierte Cycloalkylengruppe mit 5 bis 7 Kohlenstoffatomen, welche zusätzlich durch 1 bis 4 Alkylgruppen substituiert sein kann und in denen im Cycloalkylenteil jeweils eine $>$CH-Einheit durch ein Stickstoffatom ersetzt ist,

eine Piperidinylengruppe, in der zwei Wasserstoffatome am Kohlenstoffgerüst durch eine geradkettige Alkylenbrücke ersetzt sind, wobei diese Brücke 2 bis 6 Kohlenstoffatome enthält, wenn die zwei Wasserstoffatome sich am selben Kohlenstoffatom befinden, oder 1 bis 5 Kohlenstoffatome enthält, wenn sie zwei Wasserstoffatome an benachbarten Kohlenstoffatomen ersetzt, oder 1 bis 4 Kohlenstoffatome enthält, wenn sie zwei Wasserstoffatome an Kohlenstoffatomen ersetzt, die durch ein weiteres Atom getrennt sind, oder 1 bis 3 Kohlenstoffatome enthält, wenn sie zwei Wasserstoffatome an Kohlenstoffatomen ersetzt, die durch zwei Atome getrennt sind, wobei die vorstehenden Gruppen jeweils durch eine oder zwei Alkylgruppen substituiert sein können, oder

C zusammen mit A und B eine gegebenenfalls jeweils durch eine oder zwei Alkylgruppen substituierte Pyridyl- oder 1-(4-Pyridyl)-piperidinylgruppe oder eine Piperidinylgruppe, in der das Wasserstoffatom in 1-Stellung zusammen mit einem Wasserstoffatom in 4-Stellung durch eine geradkettige Alkylengruppe mit 1 bis 3 Kohlenstoffatomen ersetzt ist, wobei die an die 4-Stellung gebundene Methylengruppe einer Ethylenkette durch eine Hydroxymethylen- oder Carbonylgruppe ersetzt sein kann und zusätzlich das Stickstoffatom dieser vorstehend erwähnten bicyclischen Gruppen, welche im Kohlenstoffgerüst durch eine oder zwei Alkylgruppen substituiert sein können, durch Boran komplexiert oder durch eine im Phenylkern gegebenenfalls durch 1 bis 2 Methoxygruppen substituierte Benzylgruppe quarternisiert sein kann, oder

C eine Alkylengruppe mit 1 bis 6 Kohlenstoffatomen und B eine Bindung,

(vi) D eine 1,3-Arylengruppe,

eine Indanylen-, Naphthylen-, 1,2,3,4-Tetrahydronaphthylen- oder Benzosuberanylengruppe, wobei die gesättigten Ringe jeweils durch 1 oder 2 Alkylgruppen substituiert sein können und die aromatischen Ringe jeweils durch ein Fluor-, Chlor-, Brom- oder Jodatom, durch eine Alkyl-, Trifluormethyl-, Hydroxy-, Alkoxy-, Alkylsulfenyl-, Alkylsulfinyl-, Alkylsulfonyl- oder Cyanogruppe substituiert sein können,

eine gegebenenfalls durch eine oder zwei Alkylgruppen substituierte Cycloalkylengruppe mit 4 bis 7 Kohlenstoffatomen oder

eine Cyclohexylengruppe, in der zwei durch drei Bindungen getrennte Kohlenstoffatome zusätzlich durch eine geradkettige Alkylengruppe mit 1 bis 3 Kohlenstoffatomen verknüpft sind, wobei diese bicyclischen Gruppen jeweils außerdem durch ein bis zwei Alkylgruppen substituiert sein können,

(vii) E eine geradkettige Alkylengruppe mit 1 bis 6 Kohlenstoffatomen, die durch eine Alkylgruppe mit 1 bis 6 Kohlenstoffatomen, durch eine Arylgruppe oder durch eine $HNR_6$- oder N-Alkyl-$NR_6$-Gruppe substituiert ist,

eine gegebenenfalls durch eine oder zwei Alkylgruppen substituierte Cycloalkylengruppe mit 5 bis 7 Kohlenstoffatomen, in der eine $>$CH-Einheit durch ein Stickstoffatom ersetzt ist,

eine durch eine Hydroxy- oder Aminogruppe, durch eine Alkoxygruppe mit 1 bis 6 Kohlenstoffatomen oder durch eine $HNR_6$- oder N-Alkyl-$NR_6$-Gruppe substituierte Cycloalkylengruppe mit 4 bis 7 Kohlenstoffatomen im Cycloalkylenteil oder

eine über den Rest W mit dem Rest D verknüpfte Alkylengruppe, wobei die Alkylengruppe zusätzlich durch eine oder zwei Alkylgruppen mit jeweils 1 bis 6 Kohlenstoffatomen, durch eine Hydroxy-, Amino- oder Arylgruppe, durch eine Alkoxygruppe mit 1 bis 6 Kohlenstoffatomen oder durch eine $HNR_6$- oder N-Alkyl-$NR_6$-Gruppe substituiert ist,

(viii) F eine Alkoxycarbonylgruppe mit insgesamt 3 bis 9 Kohlenstoffatomen, eine $R_7$O-CO-, Phosphono-, O-Alkyl-phosphono-, O,O'-Dialkyl-phosphono- oder $R_8$CO-O-$CHR_9$-O-CO-Gruppe und

(ix) der dritte der Reste $R_a$ bis $R_d$ eine Trifluormethyl- oder Arylgruppe

darstellt, wobei mindestens eine der obigen Bedingungen (i) bis (ix) erfüllt sein müssen, diejenigen in denen

X eine gegebenenfalls am Stickstoffatom durch eine Alkyl- oder Cyanogruppe substituierte Carbiminogruppe, eine Carbonyl-, Thiocarbonyl- oder Sulfonylgruppe,

Y eine gegebenenfalls durch $R_c$ oder $R_d$ oder $R_c$ und $R_d$ substituierte geradkettige Alkylengruppe mit 2 oder 3 Kohlenstoffatomen, die zusätzlich durch eine oder zwei Alkylgruppen substituiert sein kann, und in der zusätzlich eine Methylengruppe durch eine Carbonylgruppe ersetzt sein kann,

eine gegebenenfalls durch $R_c$ oder $R_d$ oder $R_c$ und $R_d$ substituierte geradkettige Alkenylengruppe mit 2 oder 3 Kohlenstoffatomen, in der zusätzlich eine gegebenenfalls vorhandene Methylengruppe durch eine Carbonylgruppe ersetzt sein kann,

eine gegebenenfalls durch $R_c$ oder $R_d$ oder $R_c$ und $R_d$ substituierte 1,2-Cycloalkylengruppe mit 5 bis 7 Kohlenstoffatomen,

eine 1,2-Cycloalkenylengruppe mit 5 bis 7 Kohlenstoffatomen,

eine 1,2-Arylengruppe,

eine 1,2-Phenylengruppe, in der eine oder zwei Methingruppen jeweils durch ein Stickstoffatom ersetzt sind, wobei die vorstehend erwähnten heterocyclischen Gruppen zusätzlich durch eine oder zwei Alkylgruppen substituiert sein können, oder
eine gegebenenfalls durch $R_c$ oder $R_d$ substituierte -CO-NH-, -NH-CO-, -CH = N- oder -N = CH-Gruppe,
der erste der Reste $R_a$ bis $R_d$ eine Gruppe der Formel

A - B - C -,

in der
A eine Aminoalkylgruppe mit 1 bis 5 Kohlenstoffatomen, eine Amino-, Amidino- oder Guanidinogruppe, wobei in jeder der vorstehend erwähnten Gruppen jeweils an einem der Stickstoffatome ein oder zwei Wasserstoffatome jeweils durch eine Alkylgruppe oder ein Wasserstoffatom durch eine Alkoxycarbonylgruppe mit insgesamt 2 bis 5 Kohlenstoffatomen, durch eine Benzyloxycarbonylgruppe oder durch eine $R_1$-CO-O-($R_2CR_3$)-O-CO-Gruppe ersetzt sein kann, wobei
$R_1$ eine Alkylgruppe mit 1 bis 8 Kohlenstoffatomen, eine Cycloalkylgruppe mit 5 bis 7 Kohlenstoffatomen, eine Phenyl- oder Phenylalkylgruppe,
$R_2$ ein Wasserstoffatom oder eine Alkylgruppe und
$R_3$ ein Wasserstoffatom darstellen,
eine Cyano- oder Cyanoalkylgruppe oder
auch, falls A an ein Stickstoffatom der Reste B oder C gebunden ist, das nicht Teil einer Lactamgruppe ist, ein Wasserstoffatom, eine Alkylgruppe, eine Benzylgruppe, in der der Phenylteil durch 1 bis 2 Methoxygruppen substituiert sein kann, eine Formyl-, Acetyl- oder Trifluoracetylgruppe, eine Alkoxycarbonylgruppe mit insgesamt 2 bis 5 Kohlenstoffatomen, eine Benzyloxycarbonylgruppe oder eine $R_1$-CO-O-($R_2CR_3$)-O-CO-Gruppe, wobei $R_1$ bis $R_3$ wie vorstehend erwähnt definiert sind,
B eine Bindung,
eine Alkylengruppe,
eine Arylengruppe,
eine gegebenenfalls durch eine oder zwei Alkylgruppen substituierte Pyridinylen-, Pyrimidinylen-, Pyrazinylen- oder Pyridazinylengruppe,
eine gegebenenfalls durch eine oder zwei Alkylgruppen substituierte Cycloalkylengruppe mit 3 bis 7 Kohlenstoffatomen,
eine gegebenenfalls durch einen Rest $R_e$ substituierte Cycloalkylengruppe mit 4 oder 5 Kohlenstoffatomen, welche zusätzlich durch 1 bis 4 Alkylgruppen substituiert sein kann und in denen im Cycloalkylenteil jeweils eine >CH-Einheit durch ein Stickstoffatom, welches mit dem Rest A verknüpft ist, ersetzt ist, wobei
$R_e$ mit der Maßgabe, daß ein Heteroatom des Restes $R_e$ durch mindestens zwei Kohlenstoffatome von dem Ringstickstoffatom der cyclischen Iminogruppe getrennt ist, eine Alkyl-, Hydroxy-, Alkoxy-, Cyano-, Carboxy-, Alkoxycarbonyl-, Alkylcarbonyl-, Aminocarbonyl-, Alkylaminocarbonyl- oder Dialkylaminocarbonylgruppe darstellt,
eine gegebenenfalls durch einen Rest $R_e$ substituierte Cycloalkylengruppe mit 6 bis 8 Kohlenstoffatomen, welche zusätzlich durch 1 bis 4 Alkylgruppen substituiert sein kann und in denen im Cycloalkylenteil jeweils eine >CH-Einheit durch ein Stickstoffatom, welches mit dem Rest A verknüpft ist, ersetzt ist und zusätzlich eine weitere in 4-Stellung befindliche >CH-Einheit durch ein Stickstoffatom ersetzt sein kann, wobei außerdem in den vorstehend erwähnten 6- bis 8-gliedrigen Ringen jeweils eine oder zwei zu einem Stickstoffatom benachbarte Methylengruppen jeweils durch eine Carbonylgruppe ersetzt sein können, wobei $R_e$ wie vorstehend erwähnt definiert ist,
eine gegebenenfalls durch eine oder zwei Alkylgruppen substituierte Cycloalkenylengruppe mit 5 bis 7 Kohlenstoffatomen, welche zusätzlich durch eine oder zwei Methylgruppen substituiert sein kann und in denen im Cycloalkenylenteil jeweils eine >CH-Einheit durch ein Stickstoffatom, welches mit dem Rest A verknüpft ist, ersetzt ist, wobei das Stickstoffatom durch mindestens eine gegebenenfalls mono- oder disubstituierte Methylengruppe von der Doppelbindung getrennt ist,
eine Piperidinylengruppe, in der zwei Wasserstoffatome am Kohlenstoffgerüst durch eine geradkettige Alkylenbrücke ersetzt sind, wobei diese Brücke 2 bis 6 Kohlenstoffatome enthält, wenn die zwei Wasserstoffatome sich am selben Kohlenstoffatom befinden, oder 1 bis 5 Kohlenstoffatome enthält, wenn sie zwei Wasserstoffatome an benachbarten Kohlenstoffatomen ersetzt, oder 1 bis 4 Kohlenstoffatome enthält, wenn sie zwei Wasserstoffatome an Kohlenstoffatomen ersetzt, die durch ein weiteres Atom getrennt sind, oder 1 bis 3 Kohlenstoffatome enthält, wenn sie zwei Wasserstoffatome an Kohlenstoffatomen ersetzt, die durch zwei Atome getrennt sind, wobei die vorstehenden Gruppen

jeweils durch eine oder zwei Alkylgruppen substituiert sein können,
eine über den Rest W mit dem Rest C verknüpfte Alkylengruppe, wobei W ein Sauerstoff- oder Schwefelatom, eine Sulfinyl-, Sulfonyl-, $-NR_4-$, $-NR_5CO-$ oder $-CONR_5$-Gruppe darstellt, wobei

$R_4$ ein Wasserstoffatom, eine Alkyl-, Alkylcarbonyl-, Alkylsulfonyl-, Arylcarbonyl- oder Arylsulfonylgruppe und

$R_5$ ein Wasserstoffatom oder eine Alkylgruppe darstellen,

oder B zusammen mit A eine gegebenenfalls durch 1 oder 2 Alkylgruppen substituierte Pyridylgruppe oder eine Piperidinylgruppe, in der das Wasserstoffatom in 1-Stellung zusammen mit einem Wasserstoffatom in 2-Stellung durch eine geradkettige Alkylengruppe mit 3 oder 4 Kohlenstoffatomen ersetzt ist, oder eine Piperidinylgruppe, in der das Wasserstoffatom in 1-Stellung zusammen mit einem Wasserstoffatom in 3-Stellung durch eine geradkettige Alkylengruppe mit 2 bis 4 Kohlenstoffatomen ersetzt ist, oder eine Piperidinylgruppe, in der das Wasserstoffatom in 1-Stellung zusammen mit einem Wasserstoffatom in 4-Stellung durch eine geradkettige Alkylengruppe mit 1 bis 3 Kohlenstoffatomen ersetzt ist, wobei die an die 4-Stellung gebundene Methylengruppe einer Ethylenkette durch eine Hydroxymethylen- oder Carbonylgruppe ersetzt sein kann und zusätzlich das Stickstoffatom dieser vorstehend erwähnten bicyclischen Gruppen, welche im Kohlenstoffgerüst durch eine oder zwei Alkylgruppen substituiert sein können, durch Boran komplexiert oder durch eine im Phenylkern gegebenenfalls durch 1 bis 2 Methoxygruppen substituierte Benzylgruppe quarternisiert sein kann, und
C eine Alkylengruppe mit 1 bis 6 Kohlenstoffatomen, in der eine Methylengruppe durch ein Sauerstoff- oder Schwefelatom oder durch eine Sulfinyl-, Sulfonyl- oder $-NR_4$-Gruppe ersetzt sein kann, oder in der eine Ethylengruppe durch eine $-CONR_5-$ oder $-NR_5CO$-Gruppe ersetzt sein kann, wobei $R_4$ und $R_5$ wie vorstehend erwähnt definiert sind,
eine Alkenylengruppe mit 2 bis 6 Kohlenstoffatomen,
eine über die Carbonylgruppe mit dem Rest B verbundene Alkylencarbonylgruppe mit insgesamt 2 bis 6 Kohlenstoffatomen,
eine Arylengruppe,
eine Pyridinylen-, Pyrimidinylen-, Pyrazinylen- oder Pyridazinylengruppe, in denen eine oder zwei -CH = N-Gruppen jeweils durch eine -CO-NH-Gruppe ersetzt sein können und eines der Stickstoffatome statt an ein Wasserstoffatom auch an den Rest B gebunden sein kann, sofern dieser nicht eine Bindung bedeutet oder nicht mit einem Heteroatom oder einer Carbonylgruppe an den Rest C anschließt, wobei die vorstehend erwähnten heterocyclischen Gruppen zusätzlich durch eine oder zwei Alkylgruppen substituiert sein können,
eine gegebenenfalls durch eine oder zwei Alkylgruppen substituierte 5- bis 7-gliedrige Cycloalkylengruppe,
eine Cyclohexylengruppe, in der zwei durch drei Bindungen getrennte Kohlenstoffatome zusätzlich durch eine geradkettige Alkylengruppe mit 1 bis 3 Kohlenstoffatomen verknüpft sind, wobei diese bicyclischen Gruppen jeweils außerdem durch ein bis zwei Alkylgruppen substituiert sein können, oder
C zusammen mit A und B eine gegebenenfalls jeweils durch eine oder zwei Alkylgruppen substituierte Pyridyl- oder 1-(4-Pyridyl)-piperidinylgruppe oder eine Piperidinylgruppe, in der das Wasserstoffatom in 1-Stellung zusammen mit einem Wasserstoffatom in 4-Stellung durch eine geradkettige Alkylengruppe mit 1 bis 3 Kohlenstoffatomen ersetzt ist, wobei die an die 4-Stellung gebundene Methylengruppe einer Ethylenkette durch eine Hydroxymethylen- oder Carbonylgruppe ersetzt sein kann und zusätzlich das Stickstoffatom dieser vorstehend erwähnten bicyclischen Gruppen, welche im Kohlenstoffgerüst durch eine oder zwei Alkylgruppen substituiert sein können, durch Boran komplexiert oder durch eine im Phenylkern gegebenenfalls durch 1 bis 2 Methoxygruppen substituierte Benzylgruppe quarternisiert sein kann, oder
C, wenn B eine Bindung darstellt, auch

(a) eine Indanylen-, Naphthylen-, 1,2,3,4-Tetrahydronaphthylen- oder Benzosuberanylengruppe, in denen jeweils einer der Ringe an den Rest A und der andere der Ringe an den cyclischen Rest der allgemeinen Formel I gebunden ist, wobei die gesättigten Ringe jeweils durch 1 oder 2 Alkylgruppen substituiert sein können und die aromatischen Ringe jeweils durch ein Fluor-, Chlor-, Brom- oder Jodatom, durch eine Alkyl-, Trifluormethyl-, Hydroxy-, Alkoxy-, Alkylsulfenyl-, Alkylsulfinyl-, Alkylsulfonyl- oder Cyanogruppe substituiert sein können,

(b) eine gegebenenfalls durch den Rest $R_e$ substituierte Cycloalkylengruppe mit 5 bis 7 Kohlenstoffatomen, welche zusätzlich durch 1 bis 4 Alkylgruppen substituiert sein kann und in denen im Cycloalkylenteil jeweils eine $>$CH-Einheit durch ein Stickstoffatom, welches mit dem Rest A verknüpft ist, ersetzt ist, wobei $R_e$ wie vorstehend erwähnt definiert ist, oder

(c) eine Piperidinylengruppe, in der zwei Wasserstoffatome am Kohlenstoffgerüst durch eine geradkettige Alkylenbrücke ersetzt sind, wobei diese Brücke 2 bis 6 Kohlenstoffatome enthält, wenn die zwei Wasserstoffatome sich am selben Kohlenstoffatom befinden, oder 1 bis 5 Kohlenstoffatome enthält, wenn sie zwei Wasserstoffatome an benachbarten Kohlenstoffatomen ersetzt, oder 1 bis 4 Kohlenstoffatome enthält, wenn sie zwei Wasserstoffatome an Kohlenstoffatomen ersetzt, die durch ein weiteres Atom getrennt sind, oder 1 bis 3 Kohlenstoffatome enthält, wenn sie zwei Wasserstoffatome an Kohlenstoffatomen ersetzt, die durch zwei Atome getrennt sind, wobei die vorstehenden Gruppen jeweils durch eine oder zwei Alkylgruppen substituiert sein können, darstellen,

der zweite der Reste $R_a$ bis $R_d$ eine Gruppe der Formel

F - E - D -,

in der

D eine Alkylengruppe mit 1 bis 6 Kohlenstoffatomen, in der eine Methylengruppe durch ein Sauerstoff- oder Schwefelatom, durch eine Sulfinyl-, Sulfonyl- oder $-NR_4$-Gruppe ersetzt sein kann, oder in der eine Ethylengruppe durch eine $-CO-NR_5-$ oder $-NR_5-CO$-Gruppe ersetzt sein kann, wobei $R_4$ und $R_5$ wie vorstehend erwähnt definiert sind,

eine Arylengruppe,

eine Pyridinylen-, Pyrimidinylen-, Pyrazinylen- oder Pyridazinylengruppe, in denen eine oder zwei -CH = N-Gruppen jeweils durch eine -CO-NH-Gruppe ersetzt sein können und eines der Stickstoffatome statt an ein Wasserstoffatom auch an den Rest E gebunden sein kann, sofern dieser nicht eine Bindung bedeutet oder nicht mit einem Heteroatom oder einer Carbonylgruppe an den Rest D anschließt, wobei die vorstehend erwähnten heterocyclischen Gruppen zusätzlich durch eine oder zwei Alkylgruppen substituiert sein können,

eine Indanylen-, Naphthylen-, 1,2,3,4-Tetrahydronaphthylen- oder Benzosuberanylengruppe, in denen jeweils einer der Ringe an den Rest E und der andere der Ringe an den cyclischen Rest der allgemeinen Formel I gebunden ist, wobei die gesättigten Ringe jeweils durch 1 oder 2 Alkylgruppen substituiert sein können und die aromatischen Ringe jeweils durch ein Fluor-, Chlor-, Brom- oder Jodatom, durch eine Alkyl-, Trifluormethyl-, Hydroxy-, Alkoxy-, Alkylsulfenyl-, Alkylsulfinyl-, Alkylsulfonyl- oder Cyanogruppe substituiert sein können,

eine gegebenenfalls durch eine oder zwei Alkylgruppen substituierte Cycloalkylengruppe mit 4 bis 7 Kohlenstoffatomen,

eine gegebenenfalls durch eine oder zwei Alkylgruppen substituierte Cycloalkylengruppe mit 5 bis 7 Kohlenstoffatomen, in der eine $>$CH-Einheit durch ein Stickstoffatom ersetzt ist, wobei außerdem in den vorstehend erwähnten 5- bis 7-gliedrigen Ringen jeweils eine zu einem Stickstoffatom benachbarte Methylengruppe durch eine Carbonylgruppe ersetzt sein kann,

eine gegebenenfalls durch eine oder zwei Alkylgruppen substituierte Piperazinylengruppe, in der jeweils eine zu einem Stickstoffatom benachbarte Methylengruppe durch eine Carbonylgruppe ersetzt sein kann,

eine Cyclohexylengruppe, in der zwei durch drei Bindungen getrennte Kohlenstoffatome zusätzlich durch eine geradkettige Alkylengruppe mit 1 bis 3 Kohlenstoffatomen verknüpft sind, wobei diese bicyclischen Gruppen jeweils außerdem durch ein bis zwei Alkylgruppen substituiert sein können,

oder, wenn E eine cyclische Iminogruppe darstellt, auch eine Alkylencarbonylgruppe mit insgesamt 2 bis 6 Kohlenstoffatomen, wobei die Carbonylgruppe jeweils an das Stickstoffatom der cyclischen Iminogruppe der Gruppe E gebunden ist,

oder auch, falls E keine Bindung darstellt, eine Bindung,

E eine Bindung,

eine Alkylengruppe mit 1 bis 6 Kohlenstoffatomen, die durch eine oder zwei Alkylgruppen mit jeweils 1 bis 6 Kohlenstoffatomen, durch eine Hydroxy-, Amino- oder Arylgruppe, durch eine Alkoxygruppe mit 1 bis 6 Kohlenstoffatomen, durch eine $HNR_6$- oder N-Alkyl-$NR_6$-Gruppe substituiert sein kann, wobei

$R_6$ eine Alkylcarbonyl- oder Alkylsulfonylgruppe mit jeweils 1 bis 6 Kohlenstoffatomen im Alkylteil, eine Alkyloxycarbonylgruppe mit insgesamt 2 bis 5 Kohlenstoffatomen, eine Cycloalkylcarbonyl- oder Cycloalkylsulfonylgruppe mit jeweils 5 bis 7 Kohlenstoffatomen im Cycloalkylteil, eine Arylalkylcarbonyl-, Arylalkylsulfonyl-, Arylalkoxycarbonyl-, Arylcarbonyl- oder Arylsulfonylgruppe darstellt,

eine Alkenylengruppe mit 2 bis 6 Kohlenstoffatomen,

eine Arylengruppe,

eine gegebenenfalls durch eine oder zwei Alkylgruppen substituierte Pyridinylen-, Pyrimidinylen-, Pyrazinylen- oder Pyridazinylengruppe,

eine gegebenenfalls durch eine oder zwei Alkylgruppen substituierte Cycloalkylengruppe mit 5 bis 7 Kohlenstoffatomen, in der eine >CH-Einheit durch ein Stickstoffatom, welches mit einem Kohlenstoffatom des Restes D verknüpft ist, ersetzt ist,
eine gegebenenfalls durch eine oder zwei Alkylgruppen mit jeweils 1 bis 6 Kohlenstoffatomen, durch eine Hydroxy-, Amino- oder Arylgruppe, durch eine Alkoxygruppe mit 1 bis 6 Kohlenstoffatomen, durch eine $HNR_6$- oder N-Alkyl-$NR_6$-Gruppe substituierte Cycloalkylengruppe mit 4 bis 7 Kohlenstoffatomen im Cycloalkylenteil, wobei $R_6$ wie vorstehend definiert ist,
oder auch, falls D keine Bindung darstellt, eine über den Rest W mit dem Rest D verknüpfte Alkylengruppe, in der W wie eingangs erwähnt definiert ist und die Alkylengruppe zusätzlich durch eine oder zwei Alkylgruppen mit jeweils 1 bis 6 Kohlenstoffatomen, durch eine Hydroxy-, Amino- oder Arylgruppe, durch eine Alkoxygruppe mit 1 bis 6 Kohlenstoffatomen, durch eine $HNR_6$- oder N-Alkyl-$NR_6$-Gruppe substituiert sein kann, wobei das Heteroatom des zusätzlichen Substituenten durch mindestens 2 Kohlenstoffatome von einem Heteroatom des Restes W getrennt ist und $R_6$ wie vorstehend definiert ist, sowie mit der Maßgabe, daß D zusammen mit E keine $-(CH_2)_n$-CONH-$CH_2CH_2$-Gruppe darstellt, wobei
n die Zahl 1,2,3 oder 4 bedeutet und der mit dem Stickstoffatom verbundene Ethylenteil gegebenenfalls wie vorstehend erwähnt substituiert sein kann, und
F eine Carbonylgruppe, die durch eine Hydroxygruppe, durch eine Alkoxygruppe mit 1 bis 8 Kohlenstoffatomen, durch eine Arylalkoxygruppe oder durch eine $R_7O$-Gruppe substituiert ist, wobei
$R_7$ eine Cycloalkylgruppe mit 5 bis 7 Kohlenstoffatomen oder eine Cycloalkylalkylgruppe mit 5 bis 7 Kohlenstoffatomen im Cycloalkylteil, in denen jeweils die Cycloalkylgruppe durch eine Alkylgruppe, durch eine Alkylgruppe und durch 1 bis 3 Methylgruppen oder durch eine Alkoxygruppe substituiert und zusätzlich eine Methylengruppe in einem 5 bis 7-gliedrigen Cycloalkylteil durch ein Sauerstoffatom oder durch eine Alkyliminogruppe ersetzt sein kann, eine Benzocycloalkylgruppe mit 9 bis 12 Kohlenstoffatomen oder eine Arylgruppe darstellt,
eine Phosphono-, O-Alkyl-phosphono-, O,O'-Dialkyl-phosphono-, Tetrazol-5-yl- oder $R_8$CO-O-$CHR_9$-O-CO-Gruppe darstellen, wobei
$R_8$ eine Alkyl- oder Alkoxygruppe mit jeweils 1 bis 8 Kohlenstoffatomen, eine Cycloalkyl- oder Cycloalkoxygruppe mit jeweils 5 bis 7 Kohlenstoffatomen im Cycloalkylteil, eine Arylalkyl- oder Arylalkoxygruppe und
$R_9$ ein Wasserstoffatom oder eine Alkylgruppe darstellen,
und der kürzeste Abstand zwischen dem Rest F und dem von dem Rest F am weitesten entfernten Stickstoffatom der Gruppe A-B-C- mindestens 11 Bindungen beträgt,
der dritte der Reste $R_a$ bis $R_d$ ein Wasserstoffatom, eine Alkyl-, Trifluormethyl-, Aryl- oder Arylalkylgruppe und
der vierte der Reste $R_a$ bis $R_d$ ein Wasserstoffatom, eine Alkyl- oder Arylgruppe bedeuten,
wobei, soweit nichts anderes erwähnt wurde,
unter den bei der Definition der vorstehenden Reste erwähnten Arylteilen eine Phenylgruppe, die jeweils durch $R_{10}$ monosubstituiert, durch $R_{11}$ mono-, di- oder trisubstituiert oder durch $R_{10}$ monosubstituiert und zusätzlich durch $R_{11}$ mono- oder disubstituiert sein kann, wobei die Substituenten gleich oder verschieden sein können und
$R_{10}$ eine Cyano-, Carboxy-, Aminocarbonyl-, Alkylaminocarbonyl-, Dialkylaminocarbonyl-, Alkoxycarbonyl-, Alkylcarbonyl-, Alkylsulfenyl-, Alkylsulfinyl-, Alkylsulfonyl-, Alkylsulfonyloxy-, Trifluormethyl-, Trifluormethoxy-, Nitro-, Amino-, Alkylamino-, Dialkylamino-, Alkylcarbonylamino-, Phenylalkylcarbonylamino-, Phenylcarbonylamino-, Alkylsulfonylamino-, Phenylalkylsulfonylamino-, Phenylsulfonylamino-, N-Alkyl-alkylcarbonylamino-, N-Alkyl-phenylalkylcarbonylamino-, N-Alkyl-phenylcarbonylamino-, N-Alkyl-alkylsulfonylamino-, N-Alkyl-phenylalkylsulfonylamino-, N-Alkyl-phenylsulfonylamino-, Aminosulfonyl-, Alkylaminosulfonyl- oder Dialkylaminosulfonylgruppe und
$R_{11}$ eine Alkyl-, Hydroxy- oder Alkoxygruppe, ein Fluor-, Chlor-, Brom- oder Jodatom darstellen, wobei zwei Reste $R_{11}$ sofern diese an benachbarte Kohlenstoffatome gebunden sind, auch eine Alkylengruppe mit 3 bis 6 Kohlenstoffatomen, eine 1,3-Butadien-1,4-diylengruppe oder eine Methylendioxygruppe darstellen können,
unter den bei der Definition der vorstehenden Resten erwähnten Arylenteilen eine Phenylengruppe die jeweils durch $R_{10}$monosubstituiert, durch $R_{11}$ mono- oder disubstituiert oder durch $R_{10}$ monosubstituiert und zusätzlich durch $R_{11}$ monosubstituiert sein kann, wobei die Substituenten gleich oder verschieden sein können und wie vorstehend erwähnt definiert sind,
zu verstehen ist, sowie, soweit nichts anderes erwähnt wurde, die vorstehend erwähnten Alkyl-, Alkylen- oder Alkoxyteile jeweils 1 bis 4 Kohlenstoffatome enthalten können, und jedes Kohlenstoffatom in den

vorstehend erwähnten Alkylen- und Cycloalkylenteilen höchstens mit einem Heteroatom verknüpft ist, deren Tautomere, deren Stereoisomere und deren Salze.

**3.** Cyclische Harnstoffderivate der allgemeinen Formel I gemäß Anspruch 1 mit Ausnahme von
1-(4-Amidino-phenyl)-3-[4-(2-carboxy-ethyl)-cyclohexyl]-imidazolidin-2-on,
1-(4-Amidino-phenyl)-3-[4-(2-methoxycarbonyl-ethyl)-cyclohexyl]-imidazolidin-2-on,
1-(4-Cyano-phenyl)-3-[4-(2-methoxycarbonyl-ethyl)-cyclohexyl]-imidazolidin-2-on,
1-(4-Amidino-phenyl)-3-[4-(2-phosphono-ethyl)-phenyl]-imidazolidin-2-on,
1-(4-Amidino-phenyl)-3-[4-[2-(O-methyl-phosphono)-ethyl]-phenyl]-imidazolidin-2-on,
1-(4-Cyano-phenyl)-3-[4-(2-phosphono-ethyl)-phenyl]-imidazolidin-2-on,
1-(4-Cyano-phenyl)-3-[4-[2-(0-methyl-phosphono)-ethyl]-phenyl]-imidazolidin-2-on,
1-(4-Aminomethyl-cyclohexyl)-3-[4-(2-carboxy-ethyl)-phenyl]-imidazolidin-2-on,
1-(4-Aminomethyl-cyclohexyl)-3-[4-(2-methoxycarbonyl-ethyl)-phenyl]-imidazolidin-2-on,
1-(4-Amidino-phenyl)-3-[4-(2-carboxy-ethyl)-phenyl]-4-phenyl-3H-imidazol-2-on,
1-(4-Amidino-phenyl)-3-[4-(2-methoxycarbonyl-ethyl)-phenyl]-4-phenyl-3H-imidazol-2-on,
1-(4-Amidino-phenyl)-3-[4-(2-carboxy-ethyl)-phenyl]-4-phenyl-imidazolidin-2-on,
1-(4-Amidino-phenyl)-3-[4-(2-methoxycarbonyl-ethyl)-phenyl]-4-phenyl-imidazolidin-2-on,
4-(4-Amidino-phenyl)-1-[4-(2-carboxy-ethyl)-phenyl]-3-phenyl-3H-imidazol-2-on,
4-(4-Amidino-phenyl)-1-[4-(2-methoxycarbonyl-ethyl)-phenyl]-3-phenyl-3H-imidazol-2-on,
4-(4-Cyano-phenyl)-1-[4-(2-methoxycarbonyl-ethyl)-phenyl]-3-phenyl-3H-imidazol-2-on,
4-(4-Amidino-phenyl)-1-[4-(2-carboxy-ethyl)-phenyl]-5-methyl-3-phenyl-3H-imidazol-2-on,
4-(4-Amidino-phenyl)-1-[4-(2-methoxycarbonyl-ethyl)-phenyl]-5-methyl-3-phenyl-3H-imidazol-2-on,
4-(4-Cyano-phenyl)-1-[4-(2-methoxycarbonyl-ethyl)-phenyl]-5-methyl-3-phenyl-3H-imidazol-2-on,
4-(4-Amidino-phenyl)-1-[4-(2-carboxy-ethyl)-phenyl]-3-phenyl-imidazolidin-2-on,
4-(4-Amidino-phenyl)-1-[4-(2-methoxycarbonyl-ethyl)-phenyl]-3-phenyl-imidazolidin-2-on,
1-(4-Amidino-phenyl)-4-[4-(2-carboxy-ethyl)-phenyl]-3-phenyl-3H-imidazol-2-on,
1-(4-Amidino-phenyl)-4-[4-(2-methoxycarbonyl-ethyl)-phenyl]-3-phenyl-3H-imidazol-2-on,
1-(4-Cyano-phenyl)-4-[4-(2-methoxycarbonyl-ethyl)-phenyl]-3-phenyl-3H-imidazol-2-on,
1-(4-Amidino-phenyl)-4-[4-(2-carboxy-ethyl)-phenyl]-3-phenyl-imidazolidin-2-on,
1-(4-Amidino-phenyl)-4-[4-(2-methoxycarbonyl-ethyl)-phenyl]-3-phenyl-imidazolidin-2-on,
1-(4-Amidino-phenyl)-4-[4-(2-carboxy-ethyl)-phenyl]-5-methyl-3-phenyl-3H-imidazol-2-on,
1-(4-Amidino-phenyl)-4-[4-(2-methoxycarbonyl-ethyl)-phenyl]-5-methyl-3-phenyl-3H-imidazol-2-on,
1-(4-Cyano-phenyl)-4-[4-(2-methoxycarbonyl-ethyl)-phenyl]-5-methyl-3-phenyl-3H-imidazol-2-on,
1-(4-Amidino-phenyl)-3-[4-(2-butyloxycarbonyl-ethyl)-phenyl]-imidazolidin-2-on,
2-(4-Amidino-phenyl)-4-[4-(2-isopropyloxycarbonyl-ethyl)-phenyl]-5-methyl-4H-1,2,4-triazol-3-on,
4-[4-(2-Isopropyloxycarbonyl-ethyl)-phenyl]-2-(4-methoxycarbonylamidino-phenyl)-5-methyl-4H-1,2,4-triazol-3-on,
1-(4-Amidino-phenyl)-3-[4-(2-ethoxycarbonyl-ethyl)-phenyl]-3H-imidazol-2-on,
1-(4-Ethoxycarbonylamidino-phenyl)-3-[4-(2-ethoxycarbonyl-ethyl)-phenyl]-3H-imidazol-2-on,
1-(4-Ethoxycarbonylamidino-phenyl)-3-[4-(2-ethoxycarbonyl-ethyl)-phenyl]-imidazolidin-2-on,
1-(4-Methoxycarbonylamidino-phenyl)-3-[4-(2-methoxycarbonyl-ethyl)-cyclohexyl]-imidazolidin-2-on,
4-[4-(2-Isobutyloxycarbonyl-ethyl)-phenyl]-2-(4-methoxycarbonylamidino-phenyl)-5-methyl-4H-1,2,4-triazol-3-on,
2-(4-Amidino-phenyl)-4-[4-(2-isobutyloxycarbonyl-ethyl)-phenyl]-5-methyl-4H-1,2,4-triazol-3-on,
1-(1-Amino-5-indanyl)-3-[4-(2-carboxy-ethyl)-phenyl]-imidazolidin-2-on,
1-(1-Amino-5-indanyl)-3-[4-(2-methoxycarbonyl-ethyl)-phenyl]-imidazolidin-2-on,
1-(1-Amino-1,2,3,4-tetrahydro-6-naphthyl)-3-[4-(2-carboxy-ethyl)-phenyl]-imidazolidin-2-on,
1-(1-Amino-1,2,3,4-tetrahydro-6-naphthyl)-3-[4-(2-methoxycarbonyl-ethyl)-phenyl]-imidazolidin-2-on,
1-(4-Amino-cyclohexyl)-3-[4-(3-carboxy-propyl)-phenyl]-imidazolidin-2-on,
1-(4-Amino-cyclohexyl)-3-[4-(3-methoxycarbonyl-propyl)-phenyl]-imidazolidin-2-on,
1-(4-Amino-cyclohexyl)-3-[4-(2-carboxy-ethyl)-phenyl]-imidazolidin-2-on,
1-(4-Amino-cyclohexyl)-3-[4-(2-methoxycarbonyl-ethyl)-phenyl]-imidazolidin-2-on,
1-(4-Aminomethyl-phenyl)-3-[3-(2-carboxy-ethyl)-phenyl]-imidazolidin-2-on,
1-(4-Aminomethyl-phenyl)-3-[3-(2-methoxycarbonyl-ethyl)-phenyl]-imidazolidin-2-on,
1-[4-(1-Amino-cyclopropyl)-phenyl]-3-[4-(2-carboxy-ethyl)-phenyl]-imidazolidin-2-on,
1-[4-(1-Amino-cyclopropyl)-phenyl]-3-[4-(2-methoxycarbonyl-ethyl)-phenyl]-imidazolidin-2-on,
1-[4-(1-Amino-cyclopentyl)-phenyl]-3-[4-(2-carboxy-ethyl)-phenyl]-imidazolidin-2-on,
1-[4-(1-Amino-cyclopentyl)-phenyl]-3-[4-[(2-methoxycarbonyl-ethyl)-phenyl]-imidazolidin-2-on,

2-(4-Amidino-phenyl)-5-ethyl-4-[4-(2-isopropyloxycarbonyl-ethyl)-phenyl]-4H-1,2,4-triazol-3-on,
1-(4-Amidino-phenyl)-3-[4-(2-isopropyloxycarbonyl-ethyl)-phenyl]-4-methyl-3H-imidazol-2-on,
4-(4-Amidino-pheny1)-2-[4-(2-isopropyloxycarbonyl-ethyl)-phenyl]-4H-1,2,4-triazol-3-on,
1-(4-Amidino-phenyl)-3-[4-(2-isopropyloxycarbonyl-ethyl)-phenyl]-imidazolidin-2-on,
1-(4-Amidino-phenyl)-3-[4-(2-isopropyloxycarbonyl-ethyl)-phenyl]-3H-imidazol-2-on,
1-(4-Amidino-phenyl)-3-[4-(2-isopropyloxycarbonyl-ethyl)-phenyl]-imidazolidin-2,4-dion,
2-(4-Amidino-phenyl)-4-[4-(2-phosphono-ethyl)-phenyl]-5-methyl-4H-1,2,4-triazol-3-on,
2-(4-Amidino-phenyl)-4-[4-[2-(0-methyl-phosphono)-ethyl]-phenyl]-5-methyl-4H-1,2,4-triazol-3-on,
2-(4-Amidino-phenyl)-4-[4-(2-carboxy-ethyl)-phenyl]-5-phenyl-4H-1,2,4-triazol-3-on,
2-(4-Amidino-phenyl)-4-[4-(2-methoxycarbonyl-ethyl)-phenyl]-5-phenyl-4H-1,2,4-triazol-3-on,
3-(4-Amidino-phenyl)-1-[4-(2-carboxy-ethyl)-phenyl]-4-trifluormethyl-3H-imidazol-2-on,
3-(4-Amidino-phenyl)-1-[4-(2-methoxycarbonyl-ethyl)-phenyl]-4-trifluormethyl-3H-imidazol-2-on,
2-(4-Amidino-phenyl)-4-[4-(2-carboxy-ethyl)-phenyl]-5-trifluormethyl-4H-1,2,4-triazol-3-on,
2-(4-Amidino-phenyl)-4-[4-(2-methoxycarbonyl-ethyl)-phenyl]-5-trifluormethyl-4H-1,2,4-triazol-3-on und
3-(4-Cyano-phenyl)-1-(4-ethoxycarbonyl-butyl)-3H-imidazo[4,5-b]pyridin-2-on
sowie mit den Maßgaben, daß

(i) X eine am Stickstoffatom durch eine Cyanogruppe substituierte Carbiminogruppe,

(ii) Y eine gegebenenfalls durch eine oder zwei Alkylgruppen substituierte 1,2-Cyclopentylen- oder 1,2-Cyclohexylengruppe,

(iii) A eine durch eine $R_1$-CO-O-($R_2CR_3$)-O-CO-Gruppe substituierte Amidinogruppe,

(iv) B eine gegebenenfalls durch eine oder zwei Alkylgruppen substituierte Pyrrolidinylengruppe, eine gegebenenfalls durch eine bis vier Alkylgruppen substituierte Piperidinylengruppe, welche zusätzlich in 4-Stellung durch eine Cyano-, Aminocarbonyl-, Alkylaminocarbonyl-, Dialkylaminocarbonyl-, Alkylcarbonyl- oder Alkoxygruppe substituiert sein kann,
eine Piperidinylengruppe, in der zwei Wasserstoffatome am Kohlenstoffgerüst durch eine geradkettige Alkylenbrücke ersetzt sind, wobei diese Brücke 2 bis 5 Kohlenstoffatome enthält, wenn die zwei Wasserstoffatome sich am selben Kohlenstoffatom befinden, oder 1 bis 4 Kohlenstoffatome enthält, wenn sie zwei Wasserstoffatome an benachbarten Kohlenstoffatomen ersetzt, oder 2 oder 3 Kohlenstoffatome enthält, wenn sie zwei Wasserstoffatome an Kohlenstoffatomen ersetzt, die durch ein weiteres Atom getrennt sind, oder 1 oder 2 Kohlenstoffatome enthält, wenn sie zwei Wasserstoffatome an Kohlenstoffatomen ersetzt, die durch zwei Atome getrennt sind,
eine gegebenenfalls durch eine oder zwei Alkylgruppen substituierte 1,2,3,6-Tetrahydro-pyridinylengruppe,
eine gegebenenfalls durch eine oder zwei Alkylgruppen substituierte Azacycloheptylengruppe,
eine gegebenenfalls durch eine oder zwei Alkylgruppen substituierte Piperazinylengruppe,
eine gegebenenfalls durch eine oder zwei Alkylgruppen substituierte 2-Oxo-piperazinylengruppe,
eine Alkylenoxygruppe,
eine gegebenenfalls durch eine oder zwei Alkylgruppen substituierte Cycloalkylengruppe mit 3 bis 6 Kohlenstoffatomen oder
B zusammen mit A eine gegebenenfalls durch eine oder zwei Alkylgruppen substituierte Pyridylgruppe oder eine Piperidinylgruppe, in der das Wasserstoffatom in 1-Stellung zusammen mit einem Wasserstoffatom in 4-Stellung durch eine Methylen-, Ethylen- oder 1,3-Propylengruppe ersetzt ist, wobei die an die 4-Stellung gebundene Methylengruppe einer Ethylenkette durch eine Carbonyl- oder Hydroxymethylengruppe ersetzt sein kann und zusätzlich das Stickstoffatom der vorstehend erwähnten bicyclischen Gruppen, welche im Kohlenstoffgerüst zusätzlich durch eine oder zwei Alkylgruppen substituiert sein können, durch Boran komplexiert oder durch eine Benzylgruppe quarternisiert sein kann,

(v) C eine Alkylenoxygruppe,
eine Cyclohexylengruppe, in der zwei durch drei Bindungen getrennte Kohlenstoffatome zusätzlich durch eine Methylen- oder Ethylengruppe verknüpft sein können,
eine gegebenenfalls durch eine oder zwei Alkylgruppen substituierte Piperidinylengruppe oder
eine Indanylen-, Naphthylen- oder 1,2,3,4-Tetrahydro-naphthylengruppe oder
C zusammen mit A und B eine gegebenenfalls jeweils durch eine oder zwei Alkylgruppen substituierte Pyridyl- oder 1-(4-Pyridyl)-piperidinylgruppe oder eine Piperidinylgruppe, in der das Wasserstoffatom in 1-Stellung zusammen mit einem Wasserstoffatom in 4-Stellung durch eine Methylen- oder Ethylengruppe ersetzt ist und zusätzlich das Stickstoffatom dieser vorstehend erwähnten bicyclischen Gruppen, welche im Kohlenstoffgerüst zusätzlich durch eine oder zwei Alkylgruppen substituiert sein können, durch Boran komplexiert oder durch eine Benzylgruppe quarternisiert sein

kann, oder

C eine Alkylengruppe mit 1 bis 6 Kohlenstoffatomen und B eine Bindung,

(vi) D eine 1,3-Arylengruppe,

eine gegebenenfalls durch eine oder zwei Alkylgruppen substituierte Cycloalkylengruppe mit 5 bis 7 Kohlenstoffatomen,

eine Cyclohexylengruppe, in der zwei durch drei Bindungen getrennte Kohlenstoffatome zusätzlich durch eine Methylen- oder Ethylengruppe verknüpft sind,

eine Indanylen-, Naphthylen- oder 1,2,3,4-Tetrahydro-naphthylengruppe,

(vii) E eine geradkettige Alkylengruppe, die durch eine Alkylgruppe mit 1 bis 6 Kohlenstoffatomen, durch eine Arylgruppe oder durch eine $HNR_6$-Gruppe substituiert ist,

eine über das Sauerstoffatom mit dem Rest D verknüpfte Alkylenoxygruppe, die durch eine Alkylgruppe mit 1 bis 6 Kohlenstoffatomen, durch eine Aryl-, Hydroxy- oder Aminogruppe, durch eine Alkoxygruppe mit 1 bis 6 Kohlenstoffatomen oder durch eine $HNR_6$-Gruppe substituiert ist, oder eine gegebenenfalls durch eine oder zwei Alkylgruppen substituierte Piperidinylengruppe,

(viii) F eine Alkoxycarbonylgruppe mit insgesamt 3 bis 7 Kohlenstoffatomen, eine $R_7O$-CO-, Phosphono-, O-Alkyl-phosphono-, O,O'-Dialkyl-phosphono- oder $R_8CO$-O-$CHR_9$-O-CO-Gruppe und

(ix) der dritte der Reste $R_a$ bis $R_d$ eine Trifluormethyl- oder Arylgruppe

darstellt, wobei mindestens eine der obigen Bedingungen (i) bis (ix) erfüllt sein müssen, diejenigen in denen

X eine am Stickstoffatom durch eine Cyanogruppe substituierte Carbiminogruppe, eine Carbonyl- oder Sulfonylgruppe,

Y eine gegebenenfalls durch $R_c$ oder $R_c$ und $R_d$ substituierte $-CH_2CH_2-$, $-CH_2CH_2CH_2-$, $-CH=CH-$, $-CH_2CO-$ oder $-COCH_2-$Gruppe,

eine gegebenenfalls durch $R_c$ substituierte -N=CH- oder -CH=N-Gruppe,

eine 1,2-Arylengruppe,

eine 1,2-Phenylengruppe, in der eine oder zwei Methingruppen jeweils durch ein Stickstoffatom ersetzt sind, wobei die vorstehend erwähnten heterocyclischen Gruppen zusätzlich durch eine oder zwei Alkylgruppen substituiert sein können, oder

eine gegebenenfalls durch eine oder zwei Alkylgruppen substituierte 1,2-Cyclopentylen- oder 1,2-Cyclohexylengruppe,

der erste der Reste $R_a$ bis $R_d$ eine Gruppe der Formel

A - B - C -,

in der

A eine gegebenenfalls durch eine Alkoxycarbonylgruppe mit insgesamt 2 oder 3 Kohlenstoffatomen, durch eine Benzyloxycarbonyl- oder $R_1CO$-O-($R_2CR_3$)-O-CO-Gruppe substituierte Amidinogruppe, wobei

$R_1$ eine Alkylgruppe,

$R_2$ ein Wasserstoffatom oder eine Methylgruppe und

$R_3$ ein Wasserstoffatom darstellen,

eine Cyano-, Cyanomethyl-, Cyanoethyl-, Amino-, Aminomethyl-, Aminoethyl- oder Aminopropylgruppe oder, falls A an ein Stickstoffatom der Reste B oder C gebunden ist, ein Wasserstoffatom, eine Methyl-, Ethyl-, Benzyl-, tert.Butyloxycarbonyl-, Benzyloxycarbonyl-, Trifluoracetyl- oder $R_1CO$-O-($R_2CR_3$)-O-CO-Gruppe, wobei $R_1$ bis $R_3$ wie vorstehend erwähnt definiert sind,

B eine Bindung,

eine gegebenenfalls durch ein oder zwei Alkylgruppen substituierte Pyrrolidinylengruppe,

eine gegebenenfalls durch eine bis vier Alkylgruppen substituierte Piperidinylengruppe, welche zusätzlich in 4-Stellung durch eine Cyano-, Aminocarbonyl-, Alkylaminocarbonyl-, Dialkylaminocarbonyl-, Alkylcarbonyl- oder Alkoxygruppe substituiert sein kann,

eine Piperidinylengruppe, in der zwei Wasserstoffatome am Kohlenstoffgerüst durch eine geradkettige Alkylenbrücke ersetzt sind, wobei diese Brücke 2 bis 5 Kohlenstoffatome enthält, wenn die zwei Wasserstoffatome sich am selben Kohlenstoffatom befinden, oder 1 bis 4 Kohlenstoffatome enthält, wenn sie zwei Wasserstoffatome an benachbarten Kohlenstoffatomen ersetzt, oder 2 oder 3 Kohlenstoffatome enthält, wenn sie zwei Wasserstoffatome an Kohlenstoffatomen ersetzt, die durch ein weiteres Atom getrennt sind, oder 1 oder 2 Kohlenstoffatome enthält, wenn sie zwei Wasserstoffatome an Kohlenstoffatomen ersetzt, die durch zwei Atome getrennt sind,

eine gegebenenfalls durch eine oder zwei Alkylgruppen substituierte 1,2,3,6-Tetrahydro-pyridinylengruppe,
eine gegebenenfalls durch eine oder zwei Alkylgruppen substituierte Azacycloheptylengruppe,
eine gegebenenfalls durch eine oder zwei Alkylgruppen substituierte Piperazinylengruppe, wobei jeweils ein Stickstoffatom der bei der Definition des Restes B vorstehend erwähnten cyclischen Iminogruppen mit dem Rest A verknüpft ist,
eine gegebenenfalls durch eine oder zwei Alkylgruppen substituierte 2-Oxo-piperazinylengruppe, in der das Stickstoffatom in 4-Stellung mit dem Rest A verknüpft ist,
eine Alkylengruppe,
eine Alkylenoxygruppe, wobei das Sauerstoffatom mit dem Rest C verknüpft ist und die Stickstoffatome des Restes A jeweils durch mindestens zwei Kohlenstoffatome von dem Sauerstoffatom getrennt sind,
eine gegebenenfalls durch eine oder zwei Alkylgruppen substituierte Cycloalkylengruppe mit 3 bis 6 Kohlenstoffatomen oder
B zusammen mit A eine gegebenenfalls durch eine oder zwei Alkylgruppen substituierte Pyridylgruppe oder eine Piperidinylgruppe, in der das Wasserstoffatom in 1-Stellung zusammen mit einem Wasserstoffatom in 4-Stellung durch eine Methylen-, Ethylen- oder 1,3-Propylengruppe ersetzt ist, wobei die an die 4-Stellung gebundene Methylengruppe einer Ethylenkette durch eine Carbonyl- oder Hydroxymethylengruppe ersetzt sein kann und zusätzlich das Stickstoffatom der vorstehend erwähnten bicyclischen Gruppen, welche im Kohlenstoffgerüst zusätzlich durch eine oder zwei Alkylgruppen substituiert sein können, durch Boran komplexiert oder durch eine Benzylgruppe quarternisiert sein kann, und
C eine Alkylengruppe mit 1 bis 6 Kohlenstoffatomen,
eine Alkylenoxygruppe, wobei das Sauerstoffatom jeweils an ein Kohlenstoffatom des Restes B oder an eine der von B zusammen mit A gebildeten bicyclischen Gruppen gebunden ist und zwischen dem Sauerstoffatom der Alkylenoxygruppe und dem Stickstoffatom des cyclischen Restes der allgemeinen Formel I jeweils mindestens zwei Kohlenstoffatome stehen,
eine Alkenylengruppe mit 2 bis 4 Kohlenstoffatomen, wobei diese jeweils nicht über den Vinylenteil mit einem Heteroatom des Restes A, des Restes B oder des cyclischen Restes der allgemeinen Formel I verknüpft sein kann,
eine Cyclohexylengruppe, in der zwei durch drei Bindungen getrennte Kohlenstoffatome zusätzlich durch eine Methylen- oder Ethylengruppe verknüpft sein können,
eine Arylengruppe oder eine gegebenenfalls durch eine oder zwei Alkylgruppen substituierte Pyridinylen-, Pyrimidinylen-, Pyrazinylen- oder Pyridazinylengruppe oder,
falls B eine gegebenenfalls durch eine oder zwei Alkylgruppen substituierte Piperazinylengruppe darstellt, auch eine Alkylencarbonylgruppe, wobei die Carbonylgruppe mit einem Stickstoffatom der gegebenenfalls durch eine oder zwei Alkylgruppen substituierten Piperazinylengruppe des Restes B verknüpft ist, oder
C zusammen mit A und B eine gegebenenfalls jeweils durch eine oder zwei Alkylgruppen substituierte Pyridyl- oder 1-(4-Pyridyl)-piperidinylgruppe oder eine Piperidinylgruppe, in der das Wasserstoffatom in 1-Stellung zusammen mit einem Wasserstoffatom in 4-Stellung durch eine Methylen- oder Ethylengruppe ersetzt ist und zusätzlich das Stickstoffatom dieser vorstehend erwähnten bicyclischen Gruppen, welche im Kohlenstoffgerüst zusätzlich durch eine oder zwei Alkylgruppen substituiert sein können, durch Boran komplexiert oder durch eine Benzylgruppe quarternisiert sein kann, oder
C, wenn B eine Bindung darstellt, auch eine gegebenenfalls durch eine oder zwei Alkylgruppen substituierte Piperidinylengruppe, in der das Stickstoffatom mit dem Rest A verknüpft ist, oder eine Indanylen-, Naphthylen- oder 1,2,3,4-Tetrahydro-naphthylengruppe, wobei jeweils einer der Ringe an den Rest A und der andere der Ringe an den cyclischen Rest der allgemeinen Formel I gebunden ist, darstellen,
der zweite der Reste $R_a$ bis $R_d$ eine Gruppe der Formel

F - E - D -,

in der
D eine Alkylengruppe,
eine Arylengruppe,
eine gegebenenfalls durch eine oder zwei Alkylgruppen substituierte Pyridinylen-, Pyrimidinylen-, Pyrazinylen- oder Pyridazinylengruppe,
eine gegebenenfalls durch eine oder zwei Alkylgruppen substituierte Cycloalkylengruppe mit 5 bis 7 Kohlenstoffatomen,

eine Cyclohexylengruppe, in der zwei durch drei Bindungen getrennte Kohlenstoffatome zusätzlich durch eine Methylen- oder Ethylengruppe verknüpft sind,
eine Indanylen-, Naphthylen- oder 1,2,3,4-Tetrahydro-naphthylengruppe, wobei jeweils einer der Ringe an den Rest E und der andere der Ringe an den cyclischen Rest der allgemeinen Formel I gebunden ist,
oder, falls E eine gegebenenfalls durch eine oder zwei Alkylgruppen substituierte Piperidinylengruppe darstellt, eine Alkylencarbonylgruppe, wobei die Carbonylgruppe mit dem Stickstoffatom der gegebenenfalls durch eine oder zwei Alkylgruppen substituierten Piperidinylengruppe des Restes E verknüpft ist,
E eine Bindung,
eine Alkenylengruppe mit 2 bis 4 Kohlenstoffatomen,
eine Alkylengruppe, die durch eine Alkylgruppe mit 1 bis 6 Kohlenstoffatomen, durch eine Aryl-, Hydroxy- oder Aminogruppe, durch eine Alkoxygruppe mit 1 bis 6 Kohlenstoffatomen oder durch eine $HNR_6$-Gruppe substituiert sein kann, wobei
$R_6$ eine Alkylcarbonyl- oder Alkylsulfonylgruppe mit jeweils 1 bis 6 Kohlenstoffatomen im Alkylteil, eine Cycloalkylcarbonyl-oder Cycloalkylsulfonylgruppe mit jeweils 5 bis 7 Kohlenstoffatomen im Cycloalkylteil, eine Arylcarbonyl-, Arylsulfonyl-, Arylalkylcarbonyl- oder Arylalkylsulfonylgruppe darstellt,
eine über das Sauerstoffatom mit dem Rest D verknüpfte Alkylenoxygruppe, die durch eine Alkylgruppe mit 1 bis 6 Kohlenstoffatomen, durch eine Aryl-, Hydroxy- oder Aminogruppe, durch eine Alkoxygruppe mit 1 bis 6 Kohlenstoffatomen oder durch eine $HNR_6$-Gruppe substituiert sein kann, wobei das Heteroatom des zusätzlichen Substituenten durch mindestens 2 Kohlenstoffatome von dem Sauerstoffatom der Alkylenoxygruppe getrennt ist und $R_6$ wie vorstehend erwähnt definiert ist,
eine Arylengruppe,
eine gegebenenfalls durch eine oder zwei Alkylgruppen substituierte Cyclohexylengruppe
oder, falls D eine Alkylencarbonylgruppe darstellt, eine gegebenenfalls durch eine oder zwei Alkylgruppen substituierte Piperidinylengruppe, wobei das Stickstoffatom mit der Carbonylgruppe der Alkylencarbonylgruppe des Restes D verknüpft ist, und
F eine Carbonylgruppe, die durch eine Hydroxygruppe, durch eine Alkoxygruppe mit 1 bis 6 Kohlenstoffatomen, durch eine Arylalkoxygruppe oder durch eine $R_7O$-Gruppe substituiert ist, wobei
$R_7$ eine Cycloalkylgruppe mit 5 bis 7 Kohlenstoffatomen, eine Cycloylkylalkylgruppe mit 5 bis 7 Kohlenstoffatomen im Cycloalkylteil, eine Benzocycloalkylgruppe mit 9 bis 11 Kohlenstoffatomen oder eine Arylgruppe darstellt,
eine Phosphono-, O-Alkyl-phosphono-, O,O'-Dialkyl-phosphono-, Tetrazol-5-yl- oder $R_8CO$-O-$CHR_9$-O-CO-Gruppe darstellen, wobei
$R_8$ eine Cycloalkyl- oder Cycloalkoxygruppe mit jeweils 5 bis 7 Kohlenstoffatomen im Cycloalkylteil, eine Alkyl- oder Alkoxygruppe mit jeweils 1 bis 6 Kohlenstoffatomen und
$R_9$ ein Wasserstoffatom oder eine Alkylgruppe darstellen,
und der kürzeste Abstand zwischen dem Rest F und dem von dem Rest F am weitesten entfernten Stickstoffatom der Gruppe A-B-C- mindestens 11 Bindungen beträgt,
der dritte der Reste $R_a$ bis $R_d$ ein Wasserstoffatom, eine Alkyl-, Trifluormethyl-, Aryl- oder Arylalkylgruppe und
der vierte der Reste $R_a$ bis $R_d$ ein Wasserstoffatom oder eine Alkylgruppe bedeuten,
wobei, soweit nichts anderes erwähnt wurde,
unter den bei der Definition der vorstehenden Reste erwähnten Arylteilen eine Phenylgruppe, die jeweils durch $R_{10}$ monosubstituiert, durch $R_{11}$ mono- oder disubstituiert oder durch $R_{10}$ monosubstituiert und zusätzlich durch $R_{11}$ monosubstituiert sein kann, wobei die Substituenten gleich oder verschieden sein können und
$R_{10}$ eine Cyano-, Aminocarbonyl-, Alkylaminocarbonyl-, Dialkylaminocarbonyl-, Alkylcarbonyl-, Alkylsulfenyl-, Alkylsulfinyl-, Alkylsulfonyl-, Alkylsulfonyloxy-, Trifluormethyl-, Nitro-, Amino-, Alkylcarbonylamino-, N-Alkyl-alkylcarbonylamino-, Alkylsulfonylamino- oder N-Alkyl-alkylsulfonylaminogruppe und
$R_{11}$ eine Alkyl-, Hydroxy- oder Alkoxygruppe, ein Fluor-, Chlor- oder Bromatom darstellen, wobei zwei Reste $R_{11}$ sofern diese an benachbarte Kohlenstoffatome gebunden sind, auch eine Alkylengruppe mit 3 oder 4 Kohlenstoffatomen, eine 1,3-Butadien-1,4-diylengruppe oder eine Methylendioxygruppe darstellen können,
unter den bei der Definition der vorstehenden Resten erwähnten Arylenteilen eine Phenylengruppe die jeweils durch $R_{10}$ monosubstituiert, durch $R_{11}$ mono- oder disubstituiert oder durch $R_{10}$ monosubstituiert und zusätzlich durch $R_{11}$ monosubstituiert sein kann, wobei die Substituenten gleich oder verschieden sein können und wie vorstehend erwähnt definiert sind,

zu verstehen ist, sowie, soweit nichts anderes erwähnt wurde, die vorstehend erwähnten Alkyl-, Alkylen- oder Alkoxyteile jeweils 1 bis 4 Kohlenstoffatome enthalten können und jedes Kohlenstoffatom in den vorstehend erwähnten Alkylen- und Cycloalkylenteilen höchstens mit einem Heteroatom verknüpft ist, deren Tautomere, deren Stereoisomere und deren Salze.

**4.** Cyclische Harnstoffderivate der allgemeinen Formel I gemäß Anspruch 1 mit Ausnahme von
1-(4-Amidino-phenyl)-3-[4-(2-carboxy-ethyl)-cyclohexyl]-imidazolidin-2-on,
1-(4-Amidino-phenyl)-3-[4-(2-methoxycarbonyl-ethyl)-cyclohexyl]-imidazolidin-2-on,
1-(4-Amidino-phenyl)-3-[4-(2-phosphono-ethyl)-phenyl]-imidazolidin-2-on,
1-(4-Amidino-phenyl)-3-[4-[2-(O-methyl-phosphono)-ethyl]-phenyl]-imidazolidin-2-on,
1-(4-Aminomethyl-cyclohexyl)-3-[4-(2-carboxy-ethyl)-phenyl]-imidazolidin-2-on,
1-(4-Aminomethyl-cyclohexyl)-3-[4-(2-methoxycarbonyl-ethyl)-phenyl]-imidazolidin-2-on,
1-(4-Amidino-phenyl)-3-[4-(2-butyloxycarbonyl-ethyl)-phenyl]-imidazolidin-2-on,
2-(4-Amidino-pheny1)-4-[4-(2-isopropyloxycarbonyl-ethyl)-phenyl]-5-methyl-4H-1,2,4-triazol-3-on,
1-(4-Amidino-pheny1)-3-[4-(2-ethoxycarbonyl-ethyl)-phenyl]-3H-imidazol-2-on,
2-(4-Amidino-phenyl)-4-[4-(2-isobutyloxycarbonyl-ethyl)-phenyl]-5-methyl-4H-1,2,4-triazol-3-on,
1-(4-Amino-cyclohexyl)-3-[4-(3-carboxy-propyl)-phenyl]-imidazolidin-2-on,
1-(4-Amino-cyclohexyl)-3-[4-(3-methoxycarbonyl-propyl)-phenyl]-imidazolidin-2-on,
1-(4-Amino-cyclohexyl)-3-[4-(2-carboxy-ethyl)-phenyl]-imidazolidin-2-on,
1-(4-Amino-cyclohexyl)-3-[4-(2-methoxycarbonyl-ethyl)-phenyl]-imidazolidin-2-on,
1-(4-Aminomethyl-phenyl)-3-[3-(2-carboxy-ethyl)-phenyl]-imidazolidin-2-on,
1-(4-Aminomethyl-phenyl)-3-[3-(2-methoxycarbonyl-ethyl)-phenyl]-imidazolidin-2-on,
1-(4-Amidino-phenyl)-3-[4-(2-isopropyloxycarbonyl-ethyl)-phenyl]-4-methyl-3H-imidazol-2-on,
4-(4-Amidino-phenyl)-2-[4-(2-isopropyloxycarbonyl-ethyl)-phenyl]-4H-1,2,4-triazol-3-on,
1-(4-Amidino-phenyl)-3-[4-(2-isopropyloxycarbonyl-ethyl)-phenyl]-imidazolidin-2-on,
1-(4-Amidino-phenyl)-3-[4-(2-isopropyloxycarbonyl-ethyl)-phenyl]-3H-imidazol-2-on,
1-(4-Amidino-phenyl)-3-[4-(2-isopropyloxycarbonyl-ethyl)-phenyl]-imidazolidin-2,4-dion,
2-(4-Amidino-phenyl)-4-[4-(2-phosphono-ethyl)-phenyl]-5-methyl-4H-1,2,4-triazol-3-on und
2-(4-Amidino-phenyl)-4-[4-[2-(0-methyl-phosphono)-ethyl]-phenyl]-5-methyl-4H-1,2,4-triazol-3-on,
sowie mit den Maßgaben, daß

(i) X eine am Stickstoffatom durch eine Cyanogruppe substituierte Carbiminogruppe,
(ii) Y eine 1,2-Cyclohexylengruppe,
(iii) A eine durch eine $R_1$-CO-O($R_2CR_3$)-O-CO-Gruppe substituierte Amidinogruppe,
(iv) B eine 1,3-Pyrrolidinylen- oder 1,3-Piperidinylengruppe,
eine gegebenenfalls durch eine bis vier Methylgruppen substituierte 1,4-Piperidinylengruppe, die zusätzlich in 4-Stellung durch eine Cyano- oder Aminocarbonylgruppe substituiert sein kann,
eine 1,4-Azacycloheptylengruppe,
eine 1,4-Piperazinylengruppe,
eine 2-Oxo-1,4-piperazinylengruppe,
eine $-CH_2CH_2O$-Gruppe,
eine 1,2-Cyclopentylengruppe oder
B zusammen mit A eine 4-Piperidinylgruppe, in der das Wasserstoffatom in 1-Stellung zusammen mit einem Wasserstoffatom in 4-Stellung durch eine Methylen- oder Ethylengruppe ersetzt ist, wobei die an die 4-Stellung gebundene Methylengruppe einer Ethylenkette durch eine Carbonylgruppe ersetzt sein kann,
(v) C eine $-O-CH_2$- oder $-O-CH_2CH_2$-Gruppe,
eine 1,4-Cyclohexylengruppe, in der ein Wasserstoffatom in 1-Stellung zusammen mit einem Wasserstoffatom in 4-Stellung durch eine Ethylengruppe ersetzt sein kann,
eine 1,4-Piperidinylengruppe,
eine 1,2,3,4-Tetrahydro-2,6-naphthylengruppe oder
C zusammen mit A und B eine 4-Piperidinylgruppe, in der das Wasserstoffatom in 1-Stellung zusammen mit einem Wasserstoffatom in 4-Stellung durch eine Methylen- oder Ethylengruppe ersetzt ist, oder
C eine Alkylengruppe mit 1 bis 4 Kohlenstoffatomen und B eine Bindung,
(vi) D eine gegebenenfalls durch ein Fluor- oder Chloratom, durch eine Methyl-, Trifluormethyl- oder Cyanogruppe substituierte 1,3-Phenylengruppe,
eine 1,4-Cyclohexylengruppe, in der ein Wasserstoffatom in 1-Stellung, zusammen mit einem Wasserstoffatom in 4-Stellung durch eine Ethylengruppe ersetzt sein kann,

eine 1,5-Indanylen-, 2,6-Naphthylen- oder 1,2,3,4-Tetrahydro-2,6-naphthylengruppe,
(vii) E eine geradkettige Alkylengruppe mit 1 bis 4 Kohlenstoffatomen, die durch eine Alkylgruppe mit 1 bis 4 Kohlenstoffatomen, durch eine Phenylgruppe oder durch eine Alkylsulfonylaminogruppe mit 1 bis 4 Kohlenstoffatomen substituiert ist, oder
eine 1,4-Piperidinylengruppe,
(viii) F eine Alkoxycarbonylgruppe mit insgesamt 3 bis 6 Kohlenstoffatomen, eine $R_7O$-CO-, $R_8CO$-O-$CHR_9$-O-CO-, Phosphono-, O-Methyl-phosphono- oder O-Ethyl-phosphono-Gruppe
darstellt, wobei mindestens eine der obigen Bedingungen (i) bis (viii) erfüllt sein müssen, in denen
X eine am Stickstoffatom durch eine Cyanogruppe substituierte Carbiminogruppe, eine Carbonyl- oder Sulfonylgruppe,
Y eine gegebenenfalls durch $R_c$ oder $R_c$ und $R_d$ substituierte $-CH_2CH_2-$, $-CH_2CH_2CH_2-$, -CH=CH-, $-CH_2CO-$ oder $-COCH_2-$Gruppe,
eine gegebenenfalls durch $R_c$ substituierte -N=CH- oder -CH=N-Gruppe,
eine 1,2-Phenylen- oder 1,2-Cyclohexylengruppe,
der erste der Reste $R_a$ bis $R_d$ eine Gruppe der Formel

A - B - C -,

in der
A eine gegebenenfalls durch eine $R_1$-CO-O-($R_2CR_3$)-O-CO-Gruppe substituierte Amidinogruppe, wobei
$R_1$ eine Methylgruppe,
$R_2$ ein Wasserstoffatom oder eine Methylgruppe und
$R_3$ ein Wasserstoffatom darstellen,
eine Amino- oder Aminomethylgruppe oder, falls A an ein Stickstoffatom der Reste B oder C gebunden ist, ein Wasserstoffatom oder eine Methylgruppe,
B eine Bindung,
eine 1,3-Pyrrolidinylen- oder 1,3-Piperidinylengruppe,
eine gegebenenfalls durch eine bis vier Methylgruppen substituierte 1,4-Piperidinylengruppe, die zusätzlich in 4-Stellung durch eine Cyano- oder Aminocarbonylgruppe substituiert sein kann,
eine 1,4-Azacycloheptylengruppe,
eine 1,4-Piperazinylengruppe, wobei jeweils ein Stickstoffatom der bei der Definition des Restes B vorstehend erwähnten Gruppen mit dem Rest A verknüpft ist,
eine 2-Oxo-1,4-piperazinylengruppe, in der das Stickstoffatom in 4-Stellung mit dem Rest A verknüpft ist,
eine $-CH_2CH_2O$-Gruppe, wobei das Sauerstoffatom mit dem Rest C verknüpft ist,
eine 1,2-Cyclopentylengruppe oder
B zusammen mit A eine 4-Piperidinylgruppe, in der das Wasserstoffatom in 1-Stellung zusammen mit einem Wasserstoffatom in 4-Stellung durch eine Methylen- oder Ethylengruppe ersetzt ist, wobei die an die 4-Stellung gebundene Methylengruppe einer Ethylenkette durch eine Carbonylgruppe ersetzt sein kann, und
C eine Alkylengruppe mit 1 bis 4 Kohlenstoffatomen,
eine $-O-CH_2-$ oder $-O-CH_2CH_2-$Gruppe, wobei das Sauerstoffatom jeweils an ein Kohlenstoffatom des Restes B oder an eine der von B zusammen mit A gebildeten bicyclischen Gruppen gebunden ist und die Methylengruppe der $-O-CH_2-$Gruppe an ein Kohlenstoffatom des cyclischen Restes der allgemeinen Formel I gebunden ist,
eine 1,4-Cyclohexylengruppe, in der ein Wasserstoffatom in 1-Stellung zusammen mit einem Wasserstoffatom in 4-Stellung durch eine Ethylengruppe ersetzt sein kann,
eine 1,4-Phenylengruppe oder
C zusammen mit A und B eine 4-Piperidinylgruppe, in der das Wasserstoffatom in 1-Stellung zusammen mit einem Wasserstoffatom in 4-Stellung durch eine Methylen- oder Ethylengruppe ersetzt ist,
oder C, falls B eine 1,4-Piperazinylengruppe darstellt, auch eine $-CO-CH_2-$Gruppe, wobei die Carbonylgruppe an ein Stickstoffatom der 1,4-Piperazinylengruppe des Restes B gebunden ist, oder
C, wenn B eine Bindung darstellt, auch eine 1,4-Piperidinylengruppe, in der das Stickstoffatom mit dem Rest A verknüpft ist, oder eine 1,2,3,4-Tetrahydro-2,6-napthylengruppe, die in 2-Stellung mit dem Rest A verknüpft ist, darstellen,
der zweite der Reste $R_a$ bis $R_d$ eine Gruppe der Formel

F - E - D -,

in der
D eine Alkylengruppe mit 1 bis 4 Kohlenstoffatomen,
eine gegebenenfalls durch ein Fluor- oder Chloratom, durch eine Methyl-, Trifluormethyl- oder Cyanogruppe substituierte 1,3- oder 1,4-Phenylengruppe,
eine 1,4-Cyclohexylengruppe, in der ein Wasserstoffatom in 1-Stellung, zusammen mit einem Wasserstoffatom in 4-Stellung durch eine Ethylengruppe ersetzt sein kann,
eine 1,5-Indanylen-, 2,6-Naphthylen- oder 1,2,3,4-Tetrahydro-2,6-naphthylengruppe,
oder, falls E eine 1,4-Piperidinylengruppe darstellt, eine $-COCH_2$-Gruppe, wobei die Carbonylgruppe mit dem Stickstoffatom der 1,4-Piperidinylengruppe des Restes E verknüpft ist,
E eine Bindung,
eine -CH = CH-Gruppe,
eine Alkylengruppe mit 1 bis 4 Kohlenstoffatomen, die durch eine Alkylgruppe mit 1 bis 4 Kohlenstoffatomen, durch eine Phenylgruppe oder durch eine Alkylsulfonylaminogruppe mit 1 bis 4 Kohlenstoffatomen substituiert sein kann,
eine $-O-CH_2$-Gruppe, wobei das Sauerstoffatom mit dem Rest D verknüpft ist,
eine 1,4-Cyclohexylengruppe
oder, falls D eine $-COCH_2$-Gruppe darstellt, eine 1,4-Piperidinylengruppe, wobei das Stickstoffatom mit der Carbonylgruppe der $-COCH_2$-Gruppe des Restes D verknüpft ist, und
F eine Carbonylgruppe, die durch eine Hydroxygruppe, durch eine Alkoxygruppe mit 1 bis 5 Kohlenstoffatomen, durch eine Phenylalkoxygruppe mit 1 bis 3 Kohlenstoffatomen im Alkoxyteil oder durch eine $R_7O$-Gruppe substituiert ist, wobei
$R_7$ eine Cycloalkylgruppe mit 5 bis 7 Kohlenstoffatomen, eine Cyclohexylmethyl- oder Indanylgruppe darstellt,
eine $R_8CO-O-CHR_9-O-CO-$, Phosphono-, O-Methyl-phosphono- oder O-Ethyl-phosphono-Gruppe, wobei
$R_8$ eine Cycloalkoxygruppe mit 5 bis 7 Kohlenstoffatomen im Cycloalkylteil, eine Alkyl- oder Alkoxygruppe mit jeweils 1 bis 4 Kohlenstoffatomen und
$R_9$ ein Wasserstoffatom oder eine Methylgruppe darstellen,
und der kürzeste Abstand zwischen dem Rest F und dem von dem Rest F am weitesten entfernten Stickstoffatom der Gruppe A-B-C- mindestens 11 Bindungen beträgt,
der dritte der Reste $R_a$ bis $R_d$ ein Wasserstoffatom, eine Methyl-, Trifluormethyl- oder Phenylgruppe und
der vierte der Reste $R_a$ bis $R_d$ ein Wasserstoffatom oder eine Methylgruppe bedeuten,
und jedes Kohlenstoffatom in den vorstehend erwähnten Alkylen- und Cycloalkylenteilen höchstens mit einem Heteroatom verknüpft ist,
deren Tautomere, deren Stereoisomere und deren Salze.

**5.** Cyclische Harnstoffderivate der allgemeinen Formel I gemäß Anspruch 1 mit Ausnahme von
1-(4-Amidino-phenyl)-3-[4-(2-phosphono-ethyl)-phenyl]-imidazolidin-2-on,
1-(4-Amidino-phenyl)-3-[4-(2-butyloxycarbonyl-ethyl)-phenyl]-imidazolidin-2-on,
2-(4-Amidino-phenyl)-4-[4-(2-isopropyloxycarbonyl-ethyl)-phenyl]-5-methyl-4H-1,2,4-triazol-3-on,
1-(4-Amidino-phenyl)-3-[4-(2-ethoxycarbonyl-ethyl)-phenyl]-3H-imidazol-2-on,
2-(4-Amidino-phenyl)-4-[4-(2-isobutyloxycarbonyl-ethyl)-phenyl]-5-methyl-4H-1,2,4-triazol-3-on,
1-(4-Aminomethyl-phenyl)-3-[3-(2-carboxy-ethyl)-phenyl]-imidazolidin-2-on,
1-(4-Aminomethyl-phenyl)-3-[3-(2-methoxycarbonyl-ethyl)-phenyl]-imidazolidin-2-on,
1-(4-Amidino-phenyl)-3-[4-(2-isopropyloxycarbonyl-ethyl)-phenyl]-4-methyl-3H-imidazol-2-on,
1-(4-Amidino-phenyl)-3-[4-(2-isopropyloxycarbonyl-ethyl)-phenyl]-imidazolidin-2-on,
1-(4-Amidino-phenyl)-3-[4-(2-isopropyloxycarbonyl-ethyl)-phenyl]-3H-imidazol-2-on,
1-(4-Amidino-phenyl)-3-[4-(2-isopropyloxycarbonyl-ethyl)-phenyl]-imidazolidin-2,4-dion und
2-(4-Amidino-phenyl)-4-[4-(2-phosphono-ethyl)-phenyl]-5-methyl-4H-1,2,4-triazol-3-on,
sowie mit den Maßgaben, daß
(i) X eine am Stickstoffatom durch eine Cyanogruppe substituierte Carbiminogruppe,
(ii) Y eine 1,2-Cyclohexylengruppe,
(iii) A eine durch eine $R_1-CO-O-(R_2CR_3)-O-CO$-Gruppe substituierte Amidinogruppe,
(iv) B eine 1,4-Azacycloheptylengruppe, eine gegebenenfalls durch 1 bis 4 Methylgruppen substituierte 1,4-Piperidinylengruppe, die zusätzlich in 4-Stellung durch eine Cyano- oder Aminocarbonylgruppe substituiert sein kann, eine 1,4-Piperazinylengruppe oder

A und B zusammen eine 2-Amino-ethoxy- oder 4-Chinuclidinylgruppe,

(v) C eine 1,4-Piperidinylen-, 1,2,3,4-Tetrahydro-2,6-naphthylen- oder 1,4-Bicyclo[2.2.2]octanylen-Gruppe,

(vi) D eine 1,3-Phenylen-, cis-1,4-Cyclohexylen- oder trans-1,4-Cyclohexylengruppe,

(vii) E eine geradkettige Alkylenkette mit 2 bis 4 Kohlenstoffatomen, die durch eine Alkylgruppe mit 1 bis 4 Kohlenstoffatomen, durch eine Phenylgruppe oder durch eine Alkylsulfonylaminogruppe mit 1 bis 4 Kohlenstoffatomen substituiert ist, oder

eine 1,4-Piperidinylengruppe,

(viii) F eine Alkoxycarbonylgruppe mit insgesamt 3 bis 6 Kohlenstoffatomen eine $R_7$O-CO-, $R_8$CO-O-CHR$_9$-O-CO-, Phosphono- oder O-Ethyl-phosphono-Gruppe darstellt, wobei mindestens eine der obigen Bedingungen (i) bis (viii) erfüllt sein müssen,

diejenigen, in denen

X eine am Stickstoffatom durch eine Cyanogruppe substituierte Carbiminogruppe, eine Carbonyl- oder Sulfonylgruppe,

Y eine gegebenenfalls durch $R_c$ oder $R_c$ und $R_d$ substituierte $-CH_2CH_2-$, $-CH_2CH_2CH_2-$ oder -CH = CH-Gruppe,

eine gegebenenfalls durch $R_c$ substituierte -N = CH-Gruppe,

eine $-CH_2CO-$ oder $-COCH_2-$Gruppe,

eine 1,2-Phenylen- oder 1,2-Cyclohexylengruppe, wobei

$R_c$ ein Wasserstoffatom, eine Methyl-, Trifluormethyl- oder Phenylgruppe und

$R_d$ ein Wasserstoffatom oder eine Methylgruppe darstellen,

$R_a$ eine Gruppe der Formel

A - B - C -,

in der

A eine gegebenenfalls durch eine $R_1$-CO-O-($R_2$C$R_3$)-O-CO-Gruppe substituierte Amidinogruppe, wobei

$R_1$ eine Methylgruppe,

$R_2$ ein Wasserstoffatom oder eine Methylgruppe und

$R_3$ ein Wasserstoffatom darstellen,

eine Aminomethylgruppe oder, falls A an ein Stickstoffatom der Reste B oder C gebunden ist, ein Wasserstoffatom oder eine Methylgruppe,

B eine Bindung, eine 1,4-Azacycloheptylengruppe, eine gegebenenfalls durch 1 bis 4 Methylgruppen substituierte 1,4-Piperidinylengruppe, die zusätzlich in 4-Stellung durch eine Cyano- oder Aminocarbonylgruppe substituiert sein kann, oder eine 1,4-Piperazinylengruppe, wobei jeweils ein Stickstoffatom der vorstehend erwähnten Gruppen mit dem Rest A verknüpft ist, oder

A und B zusammen eine 2-Amino-ethoxy- oder 4-Chinuclidinylgruppe und

C eine $-CH_2CH_2-$Gruppe, eine 1,4-Phenylen-, 1,4-Cyclohexylen- oder 1,4-Bicyclo[2.2.2]octanylen-Gruppe

oder, wenn B eine Bindung darstellt, auch eine 1,4-Piperidinylengruppe, die über ein Stickstoffatom mit dem Rest A verknüpft ist, oder eine 1,2,3,4-Tetrahydro-2,6-naphthylengruppe, die in 2-Stellung mit dem Rest A verknüpft ist,

oder, falls B eine 1,4-Piperazinylengruppe darstellt, auch eine über die Carbonylgruppe mit der 1,4-Piperazinylengruppe des Restes B verbundene $-COCH_2-$Gruppe,

$R_b$ eine Gruppe der Formel

F - E - D -,

in der

D eine geradkettige Alkylengruppe mit 1 bis 4 Kohlenstoffatomen, eine 1,3-Phenylen- oder 1,4-Cyclohexylengruppe oder eine gegebenenfalls durch ein Fluoratom, durch eine Methyl-, Trifluormethyl- oder Cyanogruppe substituierte 1,4-Phenylengruppe, eine 2,6-Naphthylen- oder 1,2,3,4-Tetrahydro-2,6-naphthylengruppe oder, falls E eine 1,4-Piperidinylengruppe darstellt, eine über die Carbonylgruppe mit der 1,4-Piperidinylengruppe des Restes E verknüpfte $-COCH_2-$Gruppe,

E eine Bindung, eine -CH = CH-Gruppe, eine gegebenenfalls durch eine Alkylgruppe mit 1 bis 4 Kohlenstoffatomen, durch eine Phenylgruppe oder durch eine Alkylsulfonylaminogruppe mit 1 bis 4 Kohlenstoffatomen substituierte geradkettige Alkylengruppe mit 2 bis 4 Kohlenstoffatomen, eine $-O-CH_2-$Gruppe, wobei das Sauerstoffatom mit dem Rest D verknüpft ist, oder, falls D eine $-COCH_2-$

Gruppe darstellt, eine 1,4-Piperidinylengruppe, wobei das Stickstoffatom mit der Carbonylgruppe der $-COCH_2$-Gruppe des Restes D verknüpft ist, und
F eine Carbonylgruppe, die durch eine Hydroxygruppe, durch eine Alkoxygruppe mit 1 bis 5 Kohlenstoffatomen oder durch eine $R_7O$-Gruppe substituiert ist, wobei
$R_7$ eine Cyclopentyl-, Cyclohexyl-, Cyclohexylmethyl- oder 5-Indanylgruppe darstellt,
eine $R_8CO-O-CHR_9-O-CO-$, Phosphono- oder O-Ethyl-phosphono-Gruppe darstellen, wobei
$R_8$ eine tert.Butyl-, Ethoxy- oder Cyclohexyloxygruppe und
$R_9$ ein Wasserstoffatom oder eine Methylgruppe darstellen,
und der kürzeste Abstand zwischen dem Rest F und dem von dem Rest F am weitesten entfernten Stickstoffatom der Gruppe A-B-C- mindestens 11 Bindungen beträgt,
deren Tautomere, deren Stereoisomere und deren Salze.

6. Folgende cyclische Harnstoffderivate der allgemeinen Formel I gemäß Anspruch 1:
(a) 2-(4-Amidinophenyl)-4-[4-(2-carboxy-1-propyl)phenyl]-5-methyl-4H-1,2,4-triazol-3-on,
(b) 4-[4-(2-Carboxyethyl)phenyl]-5-methyl-2-[2-(4-piperidinyl)ethyl]-4H-1,2,4-triazol-3-on,
(c) 1-[4-(2-Carboxyethyl)phenyl]-3-[2-(4-piperidinyl)ethyl]-imidazolidin-2-on,
(d) 4-[4-(2-Carboxy-1-pentyl)phenyl]-5-methyl-2-[2-(4-piperidinyl)ethyl]-4H-1,2,4-triazol-3-on,
(e) 1-(4-Amidinophenyl)-3-[4-[2-(n-butylsulfonylamino)-2-carboxy-ethyl]phenyl]-imidazolidin-2-on,
(f) 2-(4-Amidinophenyl)-4-[trans-4-(2-carboxyethyl)cyclohexyl]-4H-1,2,4-triazol-3-on,
(g) 2-(4-Amidinophenyl)-4-[4-[2-(cyclohexyloxycarbonyl)ethyl]phenyl]-5-methyl-4H-1,2,4-triazol-3-on,
(h) 2-(4-Amidinophenyl)-4-[trans-4-(2-carboxyethyl)cyclohexyl]-5-methyl-4H-1,2,4-triazol-3-on,
(i) 1-(4-Amidinophenyl)-3-[trans-4-(2-carboxyethyl)cyclohexyl]-imidazolidin-2-on,
(j) 1-(4-Amidinophenyl)-3-[trans-4-(2-carboxyethyl)cyclohexyl]-imidazolidin-2,4-dion,
(k) 1-(4-Amidinophenyl)-3-[trans-4-[2-(methoxycarbonyl)ethyl]cyclohexyl]-imidazolidin-2-on,
(l) 1-(trans-4-Carboxycyclohexyl)-3-[4-(4-cyano-4-piperidinyl)phenyl]-imidazolidin-2-on,
(m) 1-[4-(4-Aminocarbonyl-4-piperidinyl)phenyl]-3-(trans-4-carboxycyclohexyl)-imidazolidin-2-on,
(n) 1-[4-[2-(Ethoxycarbonyl)ethyl]phenyl]-3-[2-(4-piperidinyl)ethyl]-imidazolidin-2-on,
(o) 1-(trans-4-Carboxycyclohexyl)-3-[4-(4-piperidinyl)phenyl]-imidazolidin-2-on,
(p) 1-[trans-4-(2-Carboxyethyl)cyclohexyl]-3-[2-(4-piperidinyl)ethyl)-imidazolidin-2-on,
(q) 1-[4-(2-Carboxyethyl)phenyl]-3-[2-(4-chinuclidinyl)ethyl]-imidazolidin-2-on,
(r) 1-[2-(4-Chinuclidinyl)ethyl]-3-[4-[2-(ethoxycarbonyl)ethyl]phenyl]-imidazolidin-2-on,
(s) 1-[trans-4-(5-Indanyloxycarbonyl)cyclohexyl]-3-[4-(4-piperidinyl)phenyl]-imidazolidin-2-on,
(t) 1-[trans-4-[[1-(Cyclohexyloxycarbonyloxy)ethyl]oxycarbonyl]cyclohexyl]-3-[4-(4-piperidinyl)-phenyl]-imidazolidin-2-on,
(u) 1-[trans-4-(Ethoxycarbonyl)cyclohexyl]-3-[4-(4-piperidinyl)phenyl]-imidazolidin-2-on,
(v) 1-[trans-4-[2-(Ethoxycarbonyl)ethyl]cyclohexyl]-3-[2-(4-piperidinyl)ethyl]-imidazolidin-2-on,
(w) 1-(2-Aminomethyl-1,2,3,4-tetrahydro-6-naphthyl)-3-(trans-4-carboxycyclohexyl)-imidazolidin-2-on,
(x) 1-[trans-4-[(Carboxymethyl)oxy]cyclohexyl]-3-[2-(4-piperidinyl)ethyl]-imidazolidin-2-on,
(y) 1-[4-(2-Carboxyethenyl)-2-fluor-phenyl]-3-[2-(4-piperidinyl)ethyl]-imidazolidin-2-on,
(z) 1-(trans-4-Carboxycyclohexyl)-3-[4-(4-piperidinyl)cyclohexyl]-imidazolidin-2-on,
(aa) 1-(trans-4-Carboxycyclohexyl)-3-[4-(4-chinuclidinyl)phenyl]-imidazolidin-2-on,
(bb) 1-(trans-4-Carboxycyclohexyl)-3-[4-(4-methyl-4-piperidinyl)phenyl]-imidazolidin-2-on,
(cc) 1-[4-(4-Chinuclidinyl)phenyl]-3-[trans-4-(ethoxycarbonyl)cyclohexyl]-imidazolidin-2-on,
(dd) 1-[trans-4-(Ethoxycarbonyl)cyclohexyl]-3-[4-(4-methyl-4-piperidinyl)phenyl]-imidazolidin-2-on,
(ee) 1-[4-(2-Carboxyethenyl)phenyl]-3-[2-(4-piperidinyl)ethyl]-imidazolidin-2-on,
(ff) 1-[trans-4-(Ethoxycarbonyl)cyclohexyl]-3-[4-(4-piperidinyl)phenyl]-3H-imidazol-2-on,
(gg) 1-[4-(Aminomethyl)-bicyclo[2.2.2]octan-1-yl]-3-[4-(2-carboxyethyl)phenyl]-imidazolidin-2-on,
(hh) 1-[4-(2-Carboxy-1-phenyl-ethyl)phenyl]-3-[2-(4-piperidinyl)ethyl]-imidazolidin-2-on,
(ii) 1-(2-Carboxy-1,2,3,4-tetrahydro-6-naphthyl)-3-[2-(4-piperidinyl)ethyl]-imidazolidin-2-on und
(jj) 1-(trans-4-Carboxycyclohexyl)-3-[4-[(2-aminoethyl)oxy]phenyl)-imidazolidin-2-on,
deren Stereoisomere und deren Salze.

7. Physiologisch verträgliche Salze der Verbindungen nach mindestens einem der Ansprüche 1 bis 6 mit anorganischen oder organischen Säuren oder Basen.

8. Arzneimittel, enthaltend eine Verbindung nach mindestens einem der Ansprüche 1 bis 6 oder ein physiologisch verträgliches Salz gemäß Anspruch 7 neben gegebenenfalls einem oder mehreren inerten Trägerstoffen und/oder Verdünnungsmitteln.

**9.** Verwendung einer Verbindung nach mindestens einem der Ansprüche 1 bis 7 zur Herstellung eines Arzneimittels, das zur Bekämpfung bzw. Verhütung von Krankheiten, bei denen kleinere oder größere Zell-Aggregate auftreten oder Zell-Matrixinteraktionen eine Rolle spielen, geeignet ist.

**10.** Verfahren zur Herstellung eines Arzneimittels gemäß Anspruch 8, dadurch gekennzeichnet, daß auf nichtchemischem Wege eine Verbindung nach mindestens einem der Ansprüche 1 bis 7 in einen oder mehrere inerte Trägerstoffe und/oder Verdünnungsmittel eingearbeitet wird.

**11.** Verfahren zur Herstellung der cyclischen Harnstoffderivate gemäß den Ansprüchen 1 bis 7, dadurch gekennzeichnet, daß

a) zur Herstellung von Verbindungen der allgemeinen Formel I, in der A wie eingangs erwähnt definiert ist und F eine Carboxygruppe darstellt oder F mit Ausnahme der Carboxygruppe wie eingangs erwähnt definiert ist und A eine Aminoalkylgruppe mit 1 bis 5 Kohlenstoffatomen, eine Amino-, Amidino- oder Guanidinogruppe, wobei in jeder der vorstehend erwähnten Gruppen jeweils an einem der Stickstoffatome ein oder zwei Wasserstoffatome jeweils durch eine Alkylgruppe ersetzt sein können, oder auch, falls A an ein Stickstoffatom der Reste B oder C gebunden ist, das nicht Teil einer Lactamgruppe ist, ein Wasserstoffatom darstellt, eine Verbindung der allgemeinen Formel

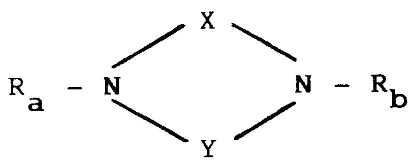

,(II)

in der

$R_a$, $R_b$, X und Y mit der Maßgabe wie in den Ansprüchen 1 bis 6 definiert sind, daß einer der Reste $R_a$ bis $R_d$ eine F'-E-D-Gruppe und der andere der Reste $R_a$ bis $R_d$ eine A-B-C-Gruppe oder einer der Reste $R_a$ bis $R_d$ eine F-E-D-Gruppe und der andere der Reste $R_a$ bis $R_d$ eine A'-B-C-Gruppe oder einer der Reste $R_a$ bis $R_d$ eine F'-E-D-Gruppe und der andere der Reste eine A'-B-C-Gruppe darstellen, in denen

A bis F wie in den Ansprüchen 1 bis 6 definiert sind,

A' eine mittels Hydrolyse, Behandeln mit Säure, Thermolyse oder Hydrogenolyse in eine Aminoalkyl-, Amino-, Amidino- oder Guanidinogruppe überführbare Gruppe, wobei in jeder der vorstehend erwähnten Gruppen jeweils an einem der Stickstoffatome ein oder zwei Wasserstoffatome jeweils durch eine Alkylgruppe ersetzt sein können, oder auch, falls A' an ein Stickstoffatom der Reste B oder C gebunden ist, das nicht Teil einer Lactamgruppe ist, eine mittels Hydrolyse, Behandeln mit Säure, Thermolyse oder Hydrogenolyse durch ein Wasserstoffatom ersetzbare Gruppe und

F' eine mittels Hydrolyse, Behandeln mit Säure, Thermolyse oder Hydrogenolyse in eine Carboxygruppe überführbare Gruppe darstellen,

in eine Verbindung der allgemeinen Formel I, in der A wie in den Ansprüchen 1 bis 6 erwähnt definiert ist und F eine Carboxygruppe darstellt oder F mit Ausnahme der Carboxygruppe wie in den Ansprüchen 1 bis 6 erwähnt definiert ist und A eine Aminoalkylgruppe mit 1 bis 5 Kohlenstoffatomen, eine Amino-, Amidino- oder Guanidinogruppe, wobei in jeder der vorstehend erwähnten Gruppen jeweils an einem der Stickstoffatome ein oder zwei Wasserstoffatome jeweils durch eine Alkylgruppe ersetzt sein können, oder auch, falls A an ein Stickstoffatom der Reste B oder C gebunden ist, das nicht Teil einer Lactamgruppe ist, ein Wasserstoffatom darstellt, umgewandelt wird oder

b) zur Herstellung von Verbindungen der allgemeinen Formel I, in der A eine $H_2N$-C(=NH)-Gruppe darstellt, in welcher ein Stickstoffatom durch eine oder zwei Alkylgruppen mit jeweils 1 bis 3 Kohlenstoffatomen substituiert sein kann, eine gegebenenfalls im Reaktionsgemisch gebildete Verbindung der allgemeinen Formel

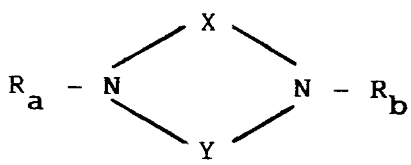

,(III)

in der
$R_a$, $R_b$, X und Y mit der Maßgabe wie in den Ansprüchen 1 bis 6 definiert sind, daß einer der Reste $R_a$ bis $R_d$ eine Gruppe der Formel

$Z_1$ - C(=NH) - B - C - darstellt,

in der
B und C wie in den Ansprüchen 1 bis 6 definiert sind und $Z_1$ eine Alkoxy-, Aralkoxy-, Alkylthio-, Aralkylthio- oder Aminogruppe darstellt, mit einem Amin der allgemeinen Formel

$R_{12}$ - NH - $R_{13}$ ,(IV)

in der
$R_{12}$ und $R_{13}$, die gleich oder verschieden sein können, Wasserstoffatome oder Alkylgruppen mit jeweils 1 bis 3 Kohlenstoffatomen bedeuten, oder mit deren Säureadditionssalzen umgesetzt wird oder
c) zur Herstellung von 4H-1,2,4-Triazol-3-onen der allgemeinen Formel I, eine Verbindung der allgemeinen Formel

($R_{14}CZ_2$) = N - $NR_{15}$ - CO - HN - $R_{16}$ ,(V)

in der
$R_{14}$ die für $R_c$ oder $R_d$ in den Ansprüchen 1 bis 6 erwähnten Bedeutungen besitzt,
einer der Reste $R_{15}$ oder $R_{16}$ die für $R_a$ in den Ansprüchen 1 bis 6 erwähnten Bedeutungen besitzt und
der andere der Reste $R_{15}$ oder $R_{16}$ die für $R_b$ in den Ansprüchen 1 bis 6 erwähnten Bedeutungen besitzt und
$Z_2$ eine nukleophile Austrittsgruppe darstellt, cyclisiert wird oder
d) zur Herstellung von Verbindungen der allgemeinen Formel I, in der X eine Carbonylgruppe und Y eine der in den Ansprüchen 1 bis 6 erwähnten Ethylen- oder Vinylengruppen darstellen, eine gegebenenfalls im Reaktionsgemisch gebildete Verbindung der allgemeinen Formel

$R_{17}$ - CO - $CHR_{18}$ - $NR_{15}$ - CO - $NHR_{16}$ ,(VI)

in der
einer der Reste $R_{15}$ oder $R_{16}$ die für $R_a$ in den Ansprüchen 1 bis 6 erwähnten Bedeutungen besitzt und
der andere der Reste $R_{15}$ oder $R_{16}$ die für $R_b$ in den Ansprüchen 1 bis 6 erwähnten Bedeutungen besitzt,
einer der Reste $R_{17}$ oder $R_{18}$ die für $R_c$ in den Ansprüchen 1 bis 6 erwähnten Bedeutungen besitzt und
der andere der Reste $R_{17}$ oder $R_{18}$ die für $R_d$ in den Ansprüchen 1 bis 6 erwähnten Bedeutungen besitzt, cyclisiert und gegebenenfalls eine so erhaltene Verbindung anschließend hydrolysiert wird oder
e) zur Herstellung von Verbindungen der allgemeinen Formel I, in der X eine Carbonylgruppe und Y eine der in den Ansprüchen 1 bis 6 erwähnten Ethylen- oder Vinylengruppen darstellen, eine Verbindung der allgemeinen Formel

$R_{15}$ - NH - $CHR_{18}$ - $R_{17}C(OR_{19})_2$, (VII)

mit einem Isocyanat der allgemeinen Formel

$O = C = N - R_{16}$ ,(VIII)

in denen
einer der Reste $R_{15}$ oder $R_{16}$ die für $R_a$ in den Ansprüchen 1 bis 6 erwähnten Bedeutungen besitzt und
der andere der Reste $R_{15}$ oder $R_{16}$ die für $R_b$ in den Ansprüchen 1 bis 6 erwähnten Bedeutungen besitzt,
einer der Reste $R_{17}$ oder $R_{18}$ die für $R_c$ in den Ansprüchen 1 bis 6 erwähnten Bedeutungen besitzt und
der andere der Reste $R_{17}$ oder $R_{18}$ die für $R_d$ in den Ansprüchen 1 bis 6 erwähnten Bedeutungen besitzt und $R_{19}$ jeweils eine Alkylgruppe mit 1 bis 4 Kohlenstoffatomen darstellt, umgesetzt und gegebenenfalls eine so erhaltene Verbindung anschließend hydriert wird oder
f) zur Herstellung von Verbindungen der allgemeinen Formel I, in der X eine Carbonylgruppe und Y eine der in den Ansprüchen 1 bis 6 erwähnten $-COCH_2-$, $-CH_2CO-$, -CONH- oder -NHCO-Gruppen darstellt, eine Verbindung der allgemeinen Formel

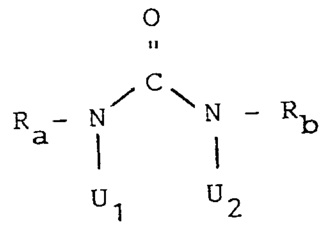

,(IX)

in der
$R_a$ und $R_b$ wie in den Ansprüchen 1 bis 6 definiert sind, einer der Reste $U_1$ oder $U_2$ ein Wasserstoffatom und
der andere der Reste $U_1$ oder $U_2$ entweder eine gegebenenfalls durch $R_c$ oder $R_d$ oder $R_c$ und $R_d$ substituierte $Z_3$-CO-$CH_2$-Gruppe darstellt, in welcher
$Z_3$ eine nukleophile Austrittsgruppe darstellt,
oder eine gegebenenfalls durch $R_c$ oder $R_d$ substituierte $Z_3$'-CONH-Gruppe darstellt, in welcher
$Z_3$' eine nukleophile Austrittsgruppe oder
$Z_3$' zusammen mit dem Wasserstoffatom der NH-Gruppe eine weitere Kohlenstoff-Stickstoff-Bindung darstellt,
cyclisiert wird oder
g) zur Herstellung von Verbindungen der allgemeinen Formel I, in der A eine durch eine Alkoxycarbonylgruppe, durch eine Benzyloxycarbonylgruppe oder durch eine $R_1$-CO-O-($R_2CR_3$)-O-CO-Gruppe substituierte Amino-, Aminoalkyl-, Amidino- oder Guanidinogruppe oder eine $R_1$-CO-O-($R_2CR_3$)-O-CO-Gruppe darstellt, eine Verbindung der allgemeinen Formel

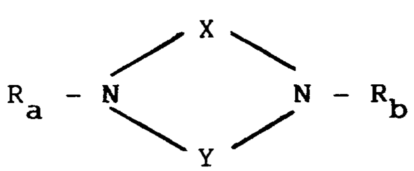

,(X)

in der
$R_a$, $R_b$, X und Y mit der Maßgabe wie in den Ansprüchen 1 bis 6 definiert sind, daß einer der Reste $R_a$ bis $R_d$ eine A'' - B - C -Gruppe darstellt, in der
B und C wie in den Ansprüchen 1 bis 6 definiert sind und
A'' eine $H_2N$-$C_{1-5}$ alkyl-, $H_2N$-C(=NH)- oder $H_2N$-C(=NH)-NH- oder $H_2N$-Gruppe oder ein Wasserstoffatom darstellt,
mit einer Verbindung der allgemeinen Formel

$Z_4 - R_{20}$ ,(XI)

in der
$R_{20}$ eine Alkoxycarbonylgruppe mit insgesamt 2 bis 5 Kohlenstoffatomen, eine $R_1$-CO-O-($R_2CR_3$)-O-CO- oder Benzyloxycarbonylgruppe und
$Z_4$ eine nukleophile Austrittsgruppe darstellen, umgesetzt wird oder
h) zur Herstellung von Verbindungen der allgemeinen Formel I, in der F eine durch eine Alkoxygruppe mit 1 bis 6 Kohlenstoffatomen, durch eine Phenylalkoxygruppe oder durch eine $R_7$O-Gruppe substituierte Carbonylgruppe darstellt, eine Verbindung der allgemeinen Formel

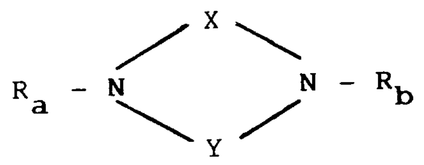
,(XII)

in der
$R_a$, $R_b$, X und Y mit der Maßgabe wie in den Ansprüchen 1 bis 6 definiert sind, daß einer der Reste $R_a$ bis $R_d$ eine F"- E - D - Gruppe darstellt, in der
E und D wie in den Ansprüchen 1 bis 6 definiert sind und
F" eine Carboxy- oder Alkoxycarbonylgruppe darstellt,
mit einem Alkohol der allgemeinen Formel

HO - $R_{21}$ ,(XIII)

in der
$R_{21}$ die für $R_7$ in den Ansprüchen 1 bis 6 erwähnten Bedeutungen aufweist oder auch eine Alkylgruppe mit 1 bis 6 Kohlenstoffatomen oder eine Phenylalkylgruppe darstellt, umgesetzt wird oder
i) zur Herstellung von Verbindungen der allgemeinen Formel I, in der A eine $H_2N$-$CH_2$-V-Gruppe, in der V eine Bindung oder eine geradkettige oder verzweigte Alkylengruppe mit 1 bis 4 Kohlenstoffatomen bedeutet, darstellt, eine Verbindung der allgemeinen Formel

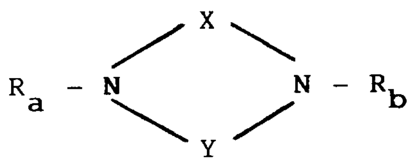
,(XIV)

in der
$R_a$, $R_b$, X und Y mit der Maßgabe wie in den Ansprüchen 1 bis 6 definiert sind, daß einer der Reste $R_a$ bis $R_d$ eine NC - V - B - C - Gruppe darstellt, in der
B und C wie in den Ansprüchen 1 bis 6 definiert sind und
V eine Bindung oder eine geradkettige oder verzweigte Alkylengruppe mit 1 bis 4 Kohlenstoffatomen darstellt,
reduziert wird oder
j) zur Herstellung von Verbindungen der allgemeinen Formel I, in der A eine Aminoalkylgruppe darstellt, in der die Aminogruppe nicht an ein quartäres Kohlenstoffatom gebunden ist, oder eine Aminogruppe darstellt, die an eine CH- oder $CH_2$-Gruppe des Restes B oder C gebunden ist, eine Verbindung der allgemeinen Formel

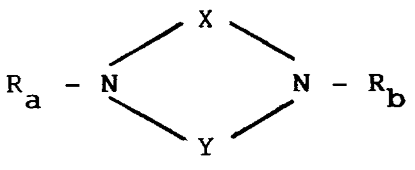
,(XV)

in der
$R_a$, $R_b$, X und Y mit der Maßgabe wie in den Ansprüchen 1 bis 6 definiert sind, daß einer der Reste $R_a$ bis $R_d$ eine A - B - C -Gruppe darstellt, in der

A, B und C mit der Maßgabe wie in den Ansprüchen 1 bis 6 definiert sind,

daß eine im Rest A, im Rest A und B zusammen oder im Rest A und C zusammen vorhandene $H_2N$-CH- oder $H_2N$-$CH_2$-Gruppe durch eine HO-N = C$<$ oder HO-N = CH-Gruppe ersetzt ist, reduziert wird oder

k) zur Herstellung von Verbindungen der allgemeinen Formel I, in der X eine durch eine Cyanogruppe substituierte Carbiminogruppe, eine Carbonyl-, Thiocarbonyl- oder Sulfonylgruppe darstellt, eine Verbindung der allgemeinen Formel

$R_a$ - NH - Y' - NH - $R_b$ ,(XVI)

in der
$R_a$ und $R_b$ wie in den Ansprüchen 1 bis 6 definiert sind und
Y' eine gegebenenfalls durch $R_c$ oder $R_d$ oder $R_c$ und $R_d$ substituierte geradkettige Alkylengruppe mit 2 oder 3 Kohlenstoffatomen, wobei eine Methylengruppe in einer Ethylengruppe zusätzlich durch eine Carbonylgruppe ersetzt sein kann, oder
eine gegebenenfalls durch $R_c$ oder $R_d$ substituierte -CO-NH-, -NH-CO-, -CH = N- oder -N = CH-Gruppe darstellt, mit einer Verbindung der allgemeinen Formel

$Z_5$ - X' - $Z_6$ ,(XVII)

in der
X' eine durch eine Cyanogruppe substituierte Carbiminogruppe, eine Carbonyl-, Thiocarbonyl- oder Sulfonylgruppe,
$Z_5$ und $Z_6$, die gleich oder verschieden sein können, nukleophile Austrittsgruppen darstellen, umgesetzt wird oder

l) zur Herstellung von Verbindungen der allgemeinen Formel I, in der X eine durch eine Cyanogruppe substituierte Carbiminogruppe, eine Carbonyl- oder Sulfonylgruppe und Y eine gege benenfalls durch $R_c$ oder $R_d$ oder $R_c$ und $R_d$ substituierte geradkettige Alkylengruppe mit 2 oder 3 Kohlenstoffatomen darstellt, eine Verbindung der allgemeinen Formel

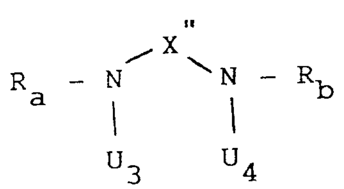

,(XVIII)

in der
$R_a$ und $R_b$ wie in den Ansprüchen 1 bis 6 definiert sind,
X'' eine durch eine Cyanogruppe substituierte Carbiminogruppe, eine Carbonyl- oder Sulfonylgruppe,
einer der Reste $U_3$ oder $U_4$ ein Wasserstoffatom und
der andere der Reste $U_3$ oder $U_4$ eine gegebenenfalls durch $R_c$ oder $R_d$ oder $R_c$ und $R_d$ substituierte geradkettige Alkylengruppe mit 2 oder 3 Kohlenstoffatomen, die zusätzlich endständig durch eine nukleophile Austrittsgruppe substituiert ist, cyclisiert wird oder

m) zur Herstellung von Verbindungen der allgemeinen Formel I, in der X eine Carbonyl- oder Sulfonylgruppe darstellt, eine Verbindung der allgemeinen Formel

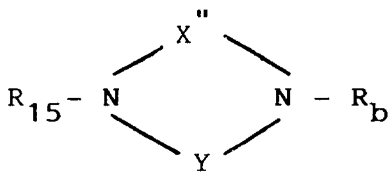

,(XIX)

mit einer Verbindung der allgemeinen Formel

$Z_7$ - $R_{16}$ ,(XX)

in denen
Y wie in den Ansprüchen 1 bis 6 definiert ist,
X'' eine durch eine Cyanogruppe substituierte Carbiminogruppe, eine Carbonyl- oder Sulfonylgruppe,
einer der Reste $R_{15}$ oder $R_{16}$ die für $R_a$ in den Ansprüchen 1 bis 6 erwähnten Bedeutungen besitzt und
der andere der Reste $R_{15}$ oder $R_{16}$ die für $R_b$ in den Ansprüchen 1 bis 6 erwähnten Bedeutungen besitzt und
$Z_7$ eine nukleophile Austrittsgruppe bedeuten, umgesetzt wird oder
n) zur Herstellung von Verbindungen der allgemeinen Formel I, in der A eine Alkylgruppe darstellt, eine Verbindung der allgemeinen Formel

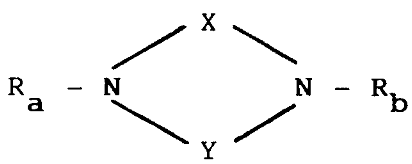

,(XXI)

in der
$R_a$, $R_b$, X und Y mit der Maßgabe wie in den Ansprüchen 1 bis 6 definiert sind, daß einer der Reste $R_a$ bis $R_d$ eine A''' - B - C -Gruppe darstellt, in der
B und C wie in den Ansprüchen 1 bis 6 definiert sind und
A''' ein Wasserstoffatom darstellt,
mit einer Verbindung der allgemeinen Formel

$Z_8$ - $R_{22}$ ,(XXII)

in der
$R_{22}$ eine Alkylgruppe mit 1 bis 3 Kohlenstoffatomen und $Z_8$ eine nukleophile Austrittsgruppe oder $Z_8$ zusammen mit einem benachbarten Wasserstoffatom des Restes $R_{22}$ ein Sauerstoffatom bedeuten, umgesetzt wird oder
o) zur Herstellung von Verbindungen der allgemeinen Formel I, in der mindestens einer der Reste B, C, D oder E eine gegebenenfalls durch eine Alkylgruppe substituierte Cyclohexylengruppe darstellt, eine Verbindung der allgemeinen Formel

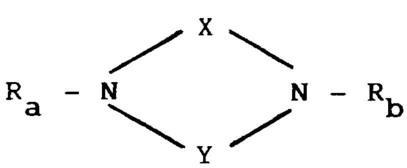

,(XXIII)

in der
$R_a$, $R_b$, X und Y mit der Maßgabe wie in den Ansprüchen 1 bis 6 definiert sind, daß mindestens einer der Reste B, C, D oder E eine gegebenenfalls durch eine Alkylgruppe substituierte Phenylengruppe

darstellt, hydriert wird oder
p) zur Herstellung von Verbindungen der allgemeinen Formel I, in der A eine Cyanogruppe, B eine Bindung und C eine gegebenenfalls durch eine Alkylgruppe substituierte 4- bis 7-gliedrige Cycloalkylengruppe oder B eine gegebenenfalls durch eine Alkylgruppe substituierte Cyclohexylengruppe darstellen, eine Verbindung der allgemeinen Formel

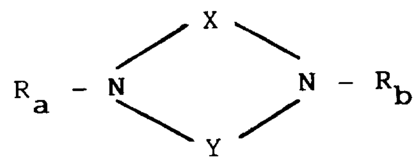
,(XXIV)

in der
$R_a$, $R_b$, X und Y mit der Maßgabe wie in den Ansprüchen 1 bis 6 definiert sind, daß einer der Reste $R_a$ bis $R_d$ eine A-B-C-Gruppe darstellt,
in der A zusammen mit einer -CH-Gruppe in einem der für B oder C in den Ansprüchen 1 bis 6 erwähnten 4- bis 7-gliedrigen Cycloalkylgruppen eine Carbonylgruppe darstellt, mit einer Verbindung der allgemeinen Formel

$Z_9$ - $CH_2$ - NC ,(XXV)

in der $Z_9$ eine nukleophile Austrittsgruppe darstellt, umgesetzt wird oder
q) zur Herstellung von Verbindungen der allgemeinen Formel I, in der F eine Carbonylgruppe darstellt, die durch eine Alkoxygruppe mit 1 bis 6 Kohlenstoffatomen, eine Phenylalkoxygruppe oder $R_8$-CO-O-$CHR_9$-O-Gruppe substituiert ist, darstellt, eine Verbindung der allgemeinen Formel

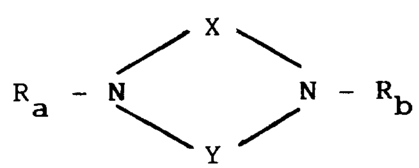
,(XXVI)

in der
$R_a$, $R_b$, X und Y mit der Maßgabe wie in den Ansprüchen 1 bis 6 definiert sind, daß einer der Reste $R_a$ bis $R_d$ eine F''' - E - D -Gruppe darstellt, in der
E und D wie in den Ansprüchen 1 bis 6 definiert sind und
F''' eine Carboxylgruppe darstellt,
mit einer Verbindung der allgemeinen Formel

$Z_{10}$ - $R_{23}$ ,(XXVII)

in der
$R_{23}$ eine Alkylgruppe mit 1 bis 6 Kohlenstoffatomen, eine Phenylalkyl- oder $R_8$-CO-O-$CHR_9$-Gruppe darstellt, wobei $R_8$ und
$R_9$ wie in den Ansprüchen 1 bis 6 definiert sind, und $Z_{10}$ eine nukleophile Austrittsgruppe bedeuten, umgesetzt wird oder
r) zur Herstellung von Verbindungen der allgemeinen Formel I, in der A zusammen mit B eine Cyanalkoxygruppe mit 1 bis 10 Kohlenstoffatomen im Alkoxyteil darstellt, die über das Sauerstoffatom mit einem Kohlenstoffatom der Gruppe C verknüpft ist, eine Verbindung der allgemeinen Formel

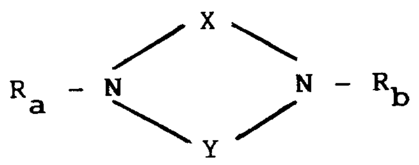

,(XXVIII)

in der
$R_a$, $R_b$, X und Y mit der Maßgabe wie in den Ansprüchen 1 bis 6 definiert sind, daß einer der Reste $R_a$ bis $R_d$ eine HO-C-Gruppe darstellt, wobei die Hydroxygruppe an ein Kohlenstoffatom der Gruppe C gebunden ist, mit einer Verbindung der allgemeinen Formel

$Z_{11}$ - $R_{24}$ ,(XXIX)

in der
$R_{24}$ eine Cyanoalkylgruppe mit 1 bis 10 Kohlenstoffatomen im Alkylteil und
$Z_{11}$ eine nukleophile Austrittsgruppe bedeuten, umgesetzt wird und
gewünschtenfalls eine so erhaltene Verbindung der allgemeinen Formel I, die eine Cyanogruppe enthält, in eine Verbindung der allgemeinen Formel I, die eine Aminocarbonyl- oder Carboxygruppe enthält, übergeführt wird und/oder
eine so erhaltene Verbindung der allgemeinen Formel I, die eine Kohlenstoff-Kohlenstoff-Doppelbindung enthält, in eine entsprechende gesättigte Verbindung übergeführt wird und/oder eine so erhaltene Verbindung der allgemeinen Formel I in ihre Stereoisomere aufgetrennt wird und/oder
erforderlichenfalls ein während den Umsetzungen zum Schutze von reaktiven Gruppen verwendeter Schutzrest abgespalten wird und/oder
eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.